# EUROPEAN PATENT APPLICATION

(11) **EP 4 450 497 A1**
(43) Date of publication of application: **23.10.2024**
(21) Application number: 23739998.5
(22) Date of filing: 10.01.2023
(51) Int. Cl.: C07D 413/10, C07D 413/12, C07D 417/10, C07D 417/12, A61K 31/553, A61P 11/00, A61P 3/00, A61P 9/00, A61P 37/00, A61P 35/00, A61P 31/00

(54) **PEPTIDYL NITRILE COMPOUND AND USE THEREOF**

(30) Priority: 11.01.2022 CN 202210028063; 26.10.2022 CN 202211316816
(71) Applicant: Shanghai Yidian Pharmaceutical Technology Development Co., Ltd, Shanghai 201108 (CN)
(72) Inventor: YE, Bin, Shanghai 201108 (CN); ZHENG, Mingfang, Shanghai 201108 (CN); YAN, Tao, Shanghai 201108 (CN)
(74) Representative: Cabinet Laurent & Charras
(86) International application number: PCT/CN2023/071503
(87) International publication number: WO 2023/134656

(57) **Abstract**

Provided are a peptidyl nitrile compound and a use thereof; specifically provided are a compound represented by formula (I) and/or a pharmaceutically acceptable salt thereof, a pharmaceutical composition comprising the compound and/or the pharmaceutically acceptable salt thereof, a method for preparing the compound, and a use of the compound in treating diseases caused by cathepsin C and a downstream serine protease thereof.

## Description

### TECHNICAL FIELD

The invention relates to the technical field of medicine, in particular to a novel peptide-based nitrile compound and its application thereof.

### BACKGROUND

Inflammatory diseases are currently an important area of drug research and development. Although there have been antibody drugs of interleukin class and small molecule drugs such as JAK inhibitors in clinical application, they have different degrees of defects, such as antibody drugs can only be injected, clinical compliance is low, JAK inhibitors are used in inflammatory diseases, because of the potential cardiovascular adverse reactions due to the target mechanism (Norman P, Expert Opinion on Investigational Drugs. 2014, 23 (8): 1067-77), so it is very necessary to explore new anti-inflammatory drugs.

Cathepsin C (CTSC), also known as dipeptidyl peptidase I (DPP1), is a 200KDa lysosomal cysteine protease belonging to the papain family. Cathepsin C acts as a key enzyme for the activation of neutrophil and mast cell granule serine peptidases in inflammatory cells, such as the four neutrophil proteases, elastase (NE), cathepsin G (CatG), proteinase 3 (PR3), and neutrophil serine protease (NSP4), as well as mast cell-associated chymase, tryptase, and serine proteases (Guay, D. et al, Curr. Top. Med. Chem. 2010, 10, 708-716; Korkmaz, B. et al, Pharmacol. Ther. 2018, 190, 202-236). Once activated by cathepsin C, these proteases are capable of degrading various extracellular matrix components, which leads to tissue damage and chronic inflammation. Thus, inhibitors of cathepsin C are useful as potential therapeutic drugs applicable to the treatment of neutrophil-dominated inflammatory diseases, including chronic obstructive pulmonary disease (COPD), emphysema, asthma, multiple sclerosis, idiopathic pneumonia, cystic fibrosis and the like. (Laine et al, Expert Opin. Ther. Patents 2010, 20, 497)

Cathepsin C inhibitors are also potential drugs for the treatment of other immune diseases, such as inflammatory bowel disease, rheumatoid arthritis, antineutrophil cytoplasmic antibody (ANCA)-associated necrotizing crescentic glomerulonephritis, ANCA-mediated vasculitis, and more serious systemic inflammatory diseases, such as sepsis, acute lung injury (acute respiratory distress syndrome), acute pancreatitis, which are caused by increased activity of some of these inflammatory proteases. In addition, the downstream serine protease elastase of cathepsin plays a very important role in the occurrence and metastasis of cancer, and cardiovascular and cerebrovascular diseases such as myocardial infarction. Theoretically, inhibition of cathepsin also has the same pharmacological action as inhibition of elastase. Theoretically, cathepsin inhibitors can also be used in the treatment of cancer and cardiovascular and cerebrovascular diseases. (Pharmacol Ther. 2018 oct;190:202-236;/nt J Mol Sci. 2021 Jan 13;22(2):722.) . It has been over 70 years since the discovery of cathepsin C, but the clinical drugs of cathepsin C inhibitors are still very limited(Korkmaz B. et al, J. Med. Chem., 2020, 63, 13258; Shen, XB et al, E. J. Med. Chem., 2021,225-113818). It was not until June 2020 that the US Food and Drug Administration granted Breakthrough Drug designation to Brensocatib, a cathepsin C inhibitor that had recently completed phase II clinical trials, for the treatment of non-cystic fibrosis bronchiectasis (NCFBE) in adults (Doyle K. et al, J. Med. Chem., 2016, 59, 9457). There remains a large unmet need for cathepsin C inhibitors. The existing compounds are difficult to inhibit the activities of cathepsin C and downstream serine proteases, so there is a need to provide novel peptidyl nitriles and applications thereof.

### SUMMARY

It is an object of the present invention to provide a compound of general formula (I) or a pharmaceutically acceptable salt thereof, 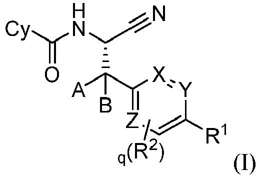 and/or a pharmaceutically acceptable salt thereof or a prodrug thereof, and/or solvates, hydrates, metabolites, N-oxides, racemic mixtures, enantiomers, diastereomers and tautomers thereof or mixtures thereof in any ratio including racemic mixtures, wherein:
Cy is 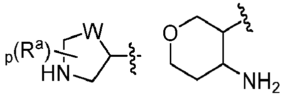
p is 0-6;
W is selected from CH₂-CH₂-O-, -O-, -S-, -SO₂-, -CH₂-, -OCH₂-, -CH₂O-, -CH₂S-, -SCH₂-, -CH₂SO₂-, - SO₂CH₂-, -CH₂-CH₂-, -(CH₂)₃-, -CH₂-CH₂-S-, -CH₂-CH₂-SO₂-, -CH₂-O-CH₂-, -CH₂-S-CH₂-, -CH₂-SO₂-CH₂-;
R^{a} are each independently selected from the group consisting of deuterium, halogen, hydroxy, cyano, mercapto, amino, C₁₋₆alkyl, C₃₋₆cycloalkyl, C₃₋₆heterocycloalkyl, C₃₋₆cycloalkyloxy, heterocycloalkoxy, - SC₁₋₆alkyl, C₁₋₆alkoxy, C₁₋₆alkylamino, (C₁₋₆alkyl)₂N-, C₁₋₆alkyl-C(O)HN-, -C(O)NHC₁₋₆alkyl, oxo, thio, and said alkyl, cycloalkyl, alkoxy, heterocycloalkyl and heterocycloalkoxy groups may all optionally be substituted with halogen and deuterium; Two R^{a} can be attached to the same carbon atom or to different carbon atoms; R^{a} or two R^{a} together form a C₁₋₄alkylene group or an ether chain containing 1 to 4 carbon atoms such as -CH₂-O-CH₂-CH₂-CH₂-, -CH₂-O-CH₂-, which alkylene or ether chain may form with the original ring cycloalkyl, cycloalkenyl, heterocycloalkyl, heterocycloalkenyl, bridged, fused and spiro rings with or without heteroatoms, which heteroatoms include N, S, O, which cycloalkyl, cycloalkenyl, heterocycloalkyl, heterocycloalkenyl, bridged, fused and spiro rings may be selectively substituted with deuterium, hydroxyl, halogen, alkyl and alkoxy groups, and wherein C, S may be selectively oxo to -C=O, -S=O, -S(O)2-; Or two R^{a} and their respective attached carbon atoms form 6-membered aryl, 5-membered heteroaryl, 6-membered heteroaryl, and form a co-ring with the original ring, said co-ring may be selectively substituted by hydroxyl, halogen, cyano, alkyl, alkoxy; And said alkyl, cycloalkyl, alkoxy, heterocycloalkyl and heterocycloalkoxy groups may all optionally be substituted with halogen and deuterium.
A and B are each independently selected from the group consisting of hydrogen, deuterium or fluorine, or
A and B form cyclopropane with the carbon atoms to which the both are attached;
X, Y and Z are each independently selected from CH, N, S, O or Se, or one of X, Y and Z is a bond in the ring, i.e., the atoms on either side of X, Y, or Z are directly linked, and the linked bond may be a single bond or a double bond;
R² is selected from the group consisting of hydrogen, deuterium, halogen, cyano, C₁₋₆ alkyl, C₁₋₆ alkoxy, C₃₋₆ cycloalkyl, or C₃₋₆ cycloalkoxy, and the said alkyl, alkoxy, cycloalkyl, and cycloalkoxy groups all may be optionally substituted with halogen and deuterium;
q is 0-3;

In the following four cases (four cases are in the relationship of "and/or")
1) Cy is not 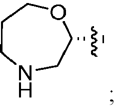
2) X, Y and Z are not all CH;
3) R² is not hydrogen and q is not 0;
4) A and B are not both hydrogen;

R¹ is selected from a cyclic group, specifically, aryl, heteroaryl, cycloalkyl, cycloalkenyl, heterocycloalkyl or heterocycloalkenyl; the said cyclic group may be monocyclic or bicyclic, and may optionally contain one or more heteroatoms of N, O, S and Se, and the C or S in the ring may be optionally substituted with - CO-, -CS-, -CO--SO-, or -SO2-; the said aryl, heteroaryl, cycloalkyl, cycloalkenyl, heterocycloalkyl, and heterocycloalkenyl groups may be optionally substituted with one or more R^{1a};
R^{1a} may be selected from the group consisting of hydrogen, deuterium, halogen, cyano, hydroxy, amino, mercapto, carboxyl, sulfone, sulfoxide, oxo, thio, nitro, alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkynyl, alkyloxy, alkoxy, haloalkoxy, cycloalkyloxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, - SOR³, -S (O) 2R³, -S (O) (NH) R³, -S (O) (NR⁴) R³, -S (O) 2NR³R⁴, -OS (O) 2R³, -NR³R⁴, -NR³ (CO) R⁴, -NR³ (SO₂) R⁴, -NR³R⁴ substituted alkyl, -CR³R⁴, -SR³, aryl, 5-6 membered heteroaryl; Wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, heteroaralkoxy may be selectively substituted with one or more R₃; The two R^{1a} can be attached to the same carbon or nitrogen atom, or to different carbon or nitrogen atoms; The two R^{1a} may selectively form a saturated or unsaturated cyclic group with a carbon or nitrogen atom in the original ring, said cyclic group including but not limited to cycloalkyl, cycloalkenyl, heterocycloalkyl, heterocycloalkenyl, bridged ring, spiro ring, fused ring, aromatic group, heteroaryl group, and the cyclic group may be further selectively substituted with one or more R³, and the C, S in the ring may also be selectively oxo or thio to -CO-, -CS-, -SO-, -SO2-;
R³ and R⁴ are each independently selected from the group consisting of hydrogen, deuterium, halogen, cyano, halogen, hydroxy, amino, alkyl, haloalkyl, alkoxy, haloalkoxy, cycloalkyl, cycloalkoxy, heterocycloalkyl, alkylcarbonyl, alkylsulfone, alkyl C(O)NH-, alkyl S(O)2NH-, carboxy, or alkylcarbonyl;
wherein amino, hydroxyl, carboxyl, alkyl, cycloalkyl, cycloalkoxy, heterocycloalkoxy, and heterocycloalkyl may be further substituted with alkyl, halogen, cyano, hydroxyl, hydroxyalkyl, and alkoxy;
two R³ may be connected to the same atom or to different atoms; or two R³, or R³ and R⁴, may optionally form a 3-10 membered cycloalkyl, heterocycloalkyl, spiro, bridged and fused rings, including but not limited to oxetane, azetidine, morpholine, piperidine, piperazine, aza-oxetane, furan and pyrrolidine, with each of the carbon or nitrogen atoms jointly attached, wherein C and S may be optionally substituted with CO-, -SO-, or -SO2-, and may be optionally substituted with one or more halogen, C1-3 alkyl, halo C₁₋₃ alkyl, C₁₋₃ alkoxy, or C₃₋₈ heterocyclyl;
When Cy is 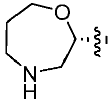 and X, Y and Z are CH, R² is hydrogen, and A/B is H:
R¹ is selected from the group consisting of pyrimidine, pyrazine, pyridazine, pyrazole, furan, imidazole, thiazole, oxazole, isoxazole, triazole, quinazoline, quinoline, cycloalkyl, cycloalkenyl, heterocycloalkyl, or
heterocycloalkenyl, wherein pyrimidine, pyrazine, pyridazine, triazinyl, pyrazole, furan, imidazole, thiazole, triazole, quinazoline, quinoline, cycloalkyl, cycloalkenyl, heterocycloalkyl and heterocycloalkenyl may be substituted with one or more R^{1a} (R^{1a} is defined as described above), or wherein S or 1-2 carbon atoms may be optionally substituted with oxo;
and/or R¹ is selected from: 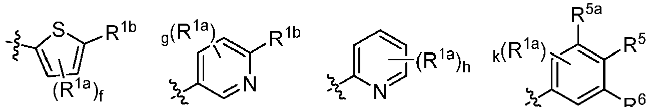
f is 0-2;
g is 0-3;
h is 0-5;
k is 0-2;
R^{1a} is as defined above, R^{1a} on the same ring may be simultaneously selected from the same substituent or from different substituents; two R^{1a} may, optionally with carbon or nitrogen atoms in the original ring, form saturated or unsaturated cyclic groups, which include but are not limited to cycloalkyl, cycloalkenyl, heterocycloalkyl, heterocycloalkenyl, bridged ring, spiro ring, fused ring, aryl and heteroaryl and may be further optionally substituted with one or more R³, and the C and S in the ring may also be optionally substituted with -CO-, -SO- and -SO2-;
R³ and R⁴ are as defined above unless otherwise specified;
When f and g are not 0 and R^{1a} is not hydrogen, R^{1b}=H, R^{1a}; R^{1b} and R^{1a}, or both R^{1a}, may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
When f and g are 0, or f and g are not 0 and R^{1a} is hydrogen, R^{1b} may be selected from the group consisting of hydrogen, deuterium, halogen, hydroxy, amino, mercapto, carboxyl, sulfone, sulfoxide, oxo, thio, nitro, alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkynyl, alkyloxy, haloalkoxy, cycloalkoxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, -SOR³, -S (O) 2R3, -S (O) (NH) R³, - S (O) (NR⁴) R³, -S (O) 2NR³R⁴, -OS (O) 2R³, -NR³R⁴, -NR³ (CO) R⁴, -NR³ (SO₂) R⁴, -NR³R⁴ substituted alkyl, -CR³R⁴, -SR³, aryl, 5-6 membered heteroaryl; Wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, heteroaralkoxy may be selectively substituted with one or more R₃; R^{1b} and R^{1a}, or both R^{1a}, may selectively form a saturated or unsaturated cyclic group with carbon or nitrogen atoms in the original ring, said cyclic group including but not limited to cycloalkyl, cycloalkenyl, heterocycloalkyl, heterocycloalkenyl, bridged ring, spiro ring, fused ring, aromatic group, heteroaryl group, and the cyclic group may be further selectively substituted with one or more R³, and the C, S in the ring may also be selectively oxylated to -CO-, -SO-, -SO2-;
When k is not 0 and R^{1a} is not hydrogen, R^{5a}, R⁵ and R⁶=R^{1a}, or two of R^{5a}, R⁵, R⁶ and 1-2 R^{1a} may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
When k is 0, or when k is not 0 and R^{1a} is hydrogen:
   If R^{5a} is deuterium, bromine, cyano, hydroxy, amino, mercapto, carboxyl, sulfone, sulfoxide, oxo, thio, nitro, cyano, C₂₋₈ alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocycloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, -SOR³, -S(O)2R³, -S(O)(NH)R³, - S(O)(NR⁴)R³, -S(O)2NR³R⁴, -OS(O)2R3, -NR³R⁴, -NR³(CO)R⁴, -NR³(SO₂)R⁴, -NR³R⁴ substituted alkyl, - CR³R⁴, -SR³, aryl, or 5-6 membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R³;
   R⁵ and R⁶=R^{1a}, or two of R⁵, R⁶, R^{5a} and 1-2 R^{1a} may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
   If R^{5a} is Chlorine, Fluorine, CH₃, thenR⁵, R⁶=R^{1a};
   If R^{5a} is H, R⁶ = R^{1a}, and R⁵ may be selected from the group consisting of deuterium, hydroxyl, amino, mercapto, carboxyl, sulfone, sulfoxide, oxo, thio, nitro, cyano, alkyl, 1-2 fluoro substituted alkyl, 1-3 bromo substituted alkyl, 1-3 chloro substituted alkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴ (wherein R³ and R⁴ are not simultaneously H), alkylcarbonyloxy, cycloalkylcarbonyloxy, heterocyclylcarbonyloxy, -SOR³, -S(O)2C₄₋₈ alkyl, -OS(O)2C₄₋₈ cycloalkyl, - OS(O)2C₄₋₈ heterocycloalkyl, -S(O)(NH)R³, -S(O)(NR⁴)R³, S(O)2NR³R⁴ (wherein R³ and R⁴ with the attached N atom may form heteroalkenyl, piperazinyl, 5 to 7-membered azaalkyl containing at least 1 O atom, morpholino, bridged morpholino, or R³ and R⁴ substituted alone do not constitute a ring), -S(O)2NH₂. -OS(O)2R³, -NR³R⁴, -NR³(CO)R⁴, -NR³(SO₂)R⁴, -NR³R⁴ substituted alkyl, -CR ³R⁴ , -SR³, aryl, 5 to 6-membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R³;
   or when R^{5a} is H, Cl, F, or CH₃, R¹ is selected from 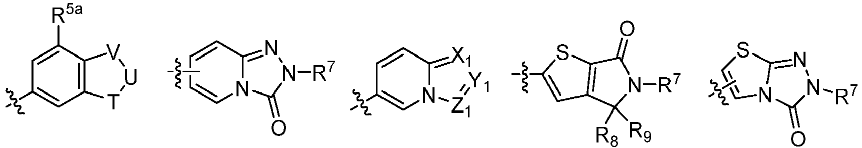 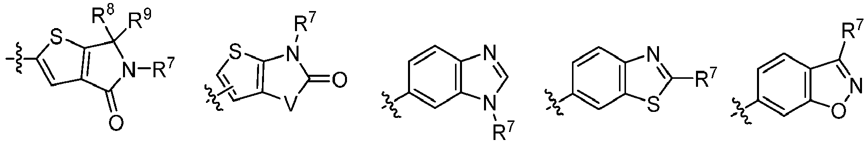
Wherein
   V is selected from the group consisting of -O-, -S-, -Se-, -CH₂-, -CF₂-, -CO-, -SO-, -SO2-, -N(R⁷)-, - C(R⁸R⁹)-, -O-CH(alkyl)-, -O-CH(cycloalkyl)-, -S-CH(cycloalkyl)-, -CH=C(alkyl)-, -N=C(alkyl)-, - CH=C(cycloalkyl)-, -N=C (cycloalkyl)-;
   U is selected from -O-, -S-, -Se-, -CO-, -SO-, -S(O)2, -NR⁷-, -CR⁸R⁹-;
   When V is -O-, -S- or -CF2- and U is -CO-, T is-O-, -S-, -Se-, -CO, -SO-, -S(O)2, -CR⁸R⁹-, -NH-, - N(CHF)-, -N(CF₂)-, -N(CH₂-CH(OH)-CH₃)-, -N(CH₂-CH(OCH₃)-CH₃)-, -N(CH₂-CH₂-OCH₃)-, -N(C₄₋₈ alkyl)-, -N(cycloalkyl)-, -N(cycloalkoxy)-, -N(oxetane)-, -N(tetrahydrofuran)-, or -N(tetrahydropyran)-, wherein said -N(C₄₋₈ alkyl)-, -N(cycloalkyl)-, -N(cycloalkoxy)-, -N(oxetane)-, -N(tetrahydrofuran)-, and - N(tetrahydropyran) may be optionally substituted with C₁₋₃ alkoxy, C₁₋₃ alkyl, C₃₋₆ cycloalkyl, NH(C₁₋₃ alkyl), N(C₁₋₃ alkyl)₂, or 1-3 halogen; otherwise T is -O-, -S-, -Se-, -CO-, -SO-, -SO2-, -C(R⁸R⁹)-, -N(R⁷)-;
   X1, Y1 and Z1 may be independently selected from CH and N;
   R⁷=R^{1a};
   R⁸, R⁹=R^{1a}, Or R⁸, R⁹ may selectively form a saturated or unsaturated cyclic group with the atoms to which both are originally attached, said cyclic group including but not limited to cycloalkyl, cycloalkenyl, heterocycloalkyl, heterocycloalkenyl, bridged ring, spiro ring, fused ring, aromatic group, heteroaryl group, and the cyclic group may be further selectively substituted with one or more R³, and C, S in the ring may also be selectively oxylated to -CO-, -SO-, -SO2-; And/or a pharmaceutically acceptable salt thereof or a prodrug thereof, and/or solvates, hydrates, metabolites, N-oxides, racemic mixtures, enantiomers, diastereomers and tautomers thereof or mixtures thereof in any ratio including racemic mixtures, wherein:
   Cy is 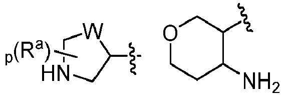
   p is 0-6;
   W is selected from CH₂-CH₂-O-, -O-, -S-, -SO₂-, -CH₂-, -OCH₂-, -CH₂O-, -CH₂S-, -SCH₂-, -CH₂SO₂-, - SO₂CH₂-, -CH₂-CH₂-, -(CH₂)₃-, -CH₂-CH₂-S-, -CH₂-CH₂-SO₂-, -CH₂-O-CH₂-, -CH₂-S-CH₂-, -CH₂-SO₂-CH₂-;
   R^{a} are each independently selected from the group consisting of deuterium, halogen, hydroxy, cyano, mercapto, amino, C₁₋₆alkyl, C₃₋₆cycloalkyl, C₃₋₆heterocycloalkyl, C₃₋₆cycloalkyloxy, heterocycloalkoxy, - SC₁₋₆alkyl, C₁₋₆alkoxy, C₁₋₆alkylamino, (C₁₋₆alkyl)₂N-, C₁₋₆alkyl-C(O)HN-, -C(O)NHC₁₋₆alkyl, oxo, thio, and said alkyl, cycloalkyl, alkoxy, heterocycloalkyl and heterocycloalkoxy groups may all optionally be substituted with halogen and deuterium; Two R^{a} can be attached to the same carbon atom or to different carbon atoms; R^{a} or two R^{a} together form a C₁₋₄alkylene group or an ether chain containing 1 to 4 carbon atoms such as -CH₂-O-CH₂-CH₂-CH₂-, -CH₂-O-CH₂-, which alkylene or ether chain may form with the original ring cycloalkyl, cycloalkenyl, heterocycloalkyl, heterocycloalkenyl, bridged, fused and spiro rings with or without heteroatoms, which heteroatoms include N, S, O, which cycloalkyl, cycloalkenyl, heterocycloalkyl, heterocycloalkenyl, bridged, fused and spiro rings may be selectively substituted with deuterium, hydroxyl, halogen, alkyl and alkoxy groups, and wherein C, S may be selectively oxo to -C=O, -S=O, -S(O)2-; Or two R^{a} and their respective attached carbon atoms form 6-membered aryl, 5-membered heteroaryl, 6-membered heteroaryl, and form a co-ring with the original ring, said co-ring may be selectively substituted by hydroxyl, halogen, cyano, alkyl, alkoxy; And said alkyl, cycloalkyl, alkoxy, heterocycloalkyl and heterocycloalkoxy groups may all optionally be substituted with halogen and deuterium.
   A and B are each independently selected from the group consisting of hydrogen, deuterium or fluorine, or A and B form cyclopropane with the carbon atoms to which the both are attached;
   X, Y and Z are each independently selected from CH, N, S, O or Se, or one of X, Y and Z is a bond in the ring, i.e., the atoms on either side of X, Y, or Z are directly linked, and the linked bond may be a single bond or a double bond;
   R² is selected from the group consisting of hydrogen, deuterium, halogen, cyano, C₁₋₆ alkyl, C₁₋₆ alkoxy, C₃₋₆ cycloalkyl, or C₃₋₆ cycloalkoxy, and the said alkyl, alkoxy, cycloalkyl, and cycloalkoxy groups all may be optionally substituted with halogen and deuterium;
   q is 0-3;
   In the following four cases (four cases are in the relationship of "and/or")
      1) Cy is not 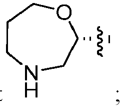
      2) X, Y and Z are not all CH;
      3) R² is not hydrogen and q is not 0;
      4) A and B are not both hydrogen;
   R¹ is selected from a cyclic group, specifically, aryl, heteroaryl, cycloalkyl, cycloalkenyl, heterocycloalkyl or heterocycloalkenyl; the said cyclic group may be monocyclic or bicyclic, and may optionally contain one or more heteroatoms of N, O, S and Se, and the C or S in the ring may be optionally substituted with - CO-, -CS-, -CO--SO-, or -SO2-; the said aryl, heteroaryl, cycloalkyl, cycloalkenyl, heterocycloalkyl, and heterocycloalkenyl groups may be optionally substituted with one or more R^{1a};
   R^{1a} may be selected from the group consisting of hydrogen, deuterium, halogen, cyano, hydroxy, amino, mercapto, carboxyl, sulfone, sulfoxide, oxo, thio, nitro, alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkynyl, alkyloxy, alkoxy, haloalkoxy, cycloalkyloxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, - SOR³, -S (O) 2R³, -S (O) (NH) R³, -S (O) (NR⁴) R³, -S (O) 2NR³R⁴, -OS (O) 2R³, -NR³R⁴, -NR³ (CO) R⁴, -NR³ (SO₂) R⁴, -NR³R⁴ substituted alkyl, -CR³R⁴, -SR³, aryl, 5-6 membered heteroaryl; Wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, heteroaralkoxy may be selectively substituted with one or more R₃; The two R^{1a} can be attached to the same carbon or nitrogen atom, or to different carbon or nitrogen atoms; The two R^{1a} may selectively form a saturated or unsaturated cyclic group with a carbon or nitrogen atom in the original ring, said cyclic group including but not limited to cycloalkyl, cycloalkenyl, heterocycloalkyl, heterocycloalkenyl, bridged ring, spiro ring, fused ring, aromatic group, heteroaryl group, and the cyclic group may be further selectively substituted with one or more R³, and the C, S in the ring may also be selectively oxo or thio to -CO-, -CS-, -SO-, -SO2-;
   R³ and R⁴ are each independently selected from the group consisting of hydrogen, deuterium, halogen, cyano, halogen, hydroxy, amino, alkyl, haloalkyl, alkoxy, haloalkoxy, cycloalkyl, cycloalkoxy, heterocycloalkyl, alkylcarbonyl, alkylsulfone, alkyl C(O)NH-, alkyl S(O)2NH-, carboxy, or alkylcarbonyl; wherein amino, hydroxyl, carboxyl, alkyl, cycloalkyl, cycloalkoxy, heterocycloalkoxy, and heterocycloalkyl may be further substituted with alkyl, halogen, cyano, hydroxyl, hydroxyalkyl, and alkoxy; two R³ may be connected to the same atom or to different atoms; or two R³, or R³ and R⁴, may optionally form a 3-10 membered cycloalkyl, heterocycloalkyl, spiro, bridged and fused rings, including but not limited to oxetane, azetidine, morpholine, piperidine, piperazine, aza-oxetane, furan and pyrrolidine, with each of the carbon or nitrogen atoms jointly attached, wherein C and S may be optionally substituted with CO-, -SO-, or -SO2-, and may be optionally substituted with one or more halogen, C1-3 alkyl, halo C₁₋₃ alkyl, C₁₋₃ alkoxy, or C₃₋₈ heterocyclyl;
   When Cy is 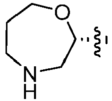 and X, Y and Z are CH, R² is hydrogen, and A/B is H:
      R¹ is selected from the group consisting of pyrimidine, pyrazine, pyridazine, pyrazole, furan, imidazole, thiazole, oxazole, isoxazole, triazole, quinazoline, quinoline, cycloalkyl, cycloalkenyl, heterocycloalkyl, or
      heterocycloalkenyl, wherein pyrimidine, pyrazine, pyridazine, triazinyl, pyrazole, furan, imidazole, thiazole, triazole, quinazoline, quinoline, cycloalkyl, cycloalkenyl, heterocycloalkyl and heterocycloalkenyl may be substituted with one or more R^{1a} (R^{1a} is defined as described above), or wherein S or 1-2 carbon atoms may be optionally substituted with oxo;
      and/or R¹ is selected from: 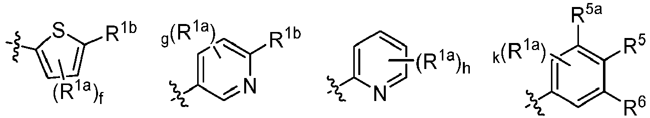
   f is 0-2;
   g is 0-3;
   h is 0-5;
   k is 0-2;
   R^{1a} is as defined above, R^{1a} on the same ring may be simultaneously selected from the same substituent or from different substituents; two R^{1a} may, optionally with carbon or nitrogen atoms in the original ring, form saturated or unsaturated cyclic groups, which include but are not limited to cycloalkyl, cycloalkenyl, heterocycloalkyl, heterocycloalkenyl, bridged ring, spiro ring, fused ring, aryl and heteroaryl and may be further optionally substituted with one or more R³, and the C and S in the ring may also be optionally substituted with -CO-, -SO- and -SO2-;
   R³ and R⁴ are as defined above unless otherwise specified;
   When f and g are not 0 and R^{1a} is not hydrogen, R^{1b}=H, R^{1a}; R^{1b} and R^{1a}, or both R^{1a}, may selectively form a saturated or unsaturated cyclic group with carbon or nitrogen atoms in the original ring, including but not limited to cycloalkyl, cycloalkenyl, heterocycloalkyl, heterocycloalkenyl, bridged ring, spiro ring, fused ring, aromatic group, heteroaryl group, and the cyclic group may be further selectively substituted with one or more R³, and the C, S in the ring may also be selectively oxylated to -CO-, -SO-, -SO2-;
   When f and g are 0, or f and g are not 0 and R^{1a} is hydrogen, R^{1b} may be selected from the group consisting of hydrogen, deuterium, halogen, hydroxy, amino, mercapto, carboxyl, sulfone, sulfoxide, oxo, thio, nitro, alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, -SOR³, -S(O)2R3, -S(O)(NH)R³, - S(O)(NR⁴)R³, -S(O)2NR³R⁴, -OS(O)2R³, -NR³R⁴, -NR³(CO)R⁴, -NR³(SO₂)R⁴, -NR³R⁴ substituted alkyl, - CR³R⁴, -SR³, aryl, or 5-6 membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R₃; R^{1b} and R^{1a}, or both R^{1a}, may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl, heteroaryl, and may be further optionally substituted with one or more R³, and the C and S in the ring may also be optionally substituted with -CO-, -SO-, and -SO2-;
When k is not 0 and R^{1a} is not hydrogen, R^{5a}, R⁵ and R⁶=R^{1a}, or two of R^{5a}, R⁵, R⁶ and 1-2 R^{1a} may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
When k is 0, or when k is not 0 and R^{1a} is hydrogen:
   If R^{5a} is deuterium, bromine, cyano, hydroxy, amino, mercapto, carboxyl, sulfone, sulfoxide, oxo, thio, nitro, cyano, C₂₋₈alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocyclenyl, alkynyl, alkoxy, haloalkoxy, cycloalkoxy, heterocycloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, -SOR³, -S(O)2R3, -S(O)(NH)R³, - S(O)(NR⁴)R³, -S(O)2NR³R⁴, -OS(O)2R³, -NR³R⁴, -NR³(CO)R⁴, -NR³(SO₂)R⁴, -NR³R⁴substituted alkyl, - CR³R⁴, -SR³, aryl, 5-6 membered heteroaryl; Wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, heteroaralkoxy may be selectively substituted with one or more R³;
   R⁵ and R⁶=R^{1a}, or two of R⁵, R⁶, R^{5a} and 1-2 R^{1a} may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
   If R^{5a} is chlorine, fluorine, or CH₃, R5 and R6 = R^{1a};
   If R^{5a} is H, R⁶ = R^{1a}, and R⁵ may be selected from the group consisting of deuterium, hydroxyl, amino, mercapto, carboxyl, sulfone, sulfoxide, oxo, thio, nitro, cyano, alkyl, 1-2 fluoro substituted alkyl, 1-3 bromo substituted alkyl, 1-3 chloro substituted alkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴ (wherein R³ and R⁴ are not simultaneously H), alkylcarbonyloxy, cycloalkylcarbonyloxy, heterocyclylcarbonyloxy, -SOR³, -S(O)2C₄₋₈ alkyl, -OS(O)2C₄₋₈ cycloalkyl, - OS(O)2C₄₋₈ heterocycloalkyl, -S(O)(NH)R³, -S(O)(NR⁴)R³, S(O)2NR³R⁴ (wherein R³ and R⁴ with the attached N atom may form heteroalkenyl, piperazinyl, 5 to 7-membered azaalkyl containing at least 1 O atom, morpholino, bridged morpholino, or R³ and R⁴ substituted alone do not constitute a ring), -S(O)2NH₂, -OS(O)2R³, -NR³R⁴, -NR³(CO)R⁴, -NR³(SO₂)R⁴, -NR³R⁴ substituted alkyl, -CR ³R⁴ , -SR³, aryl, 5 to 6-membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R³;
   or when R^{5a} is H, Cl, F, or CH₃, R¹ is selected from 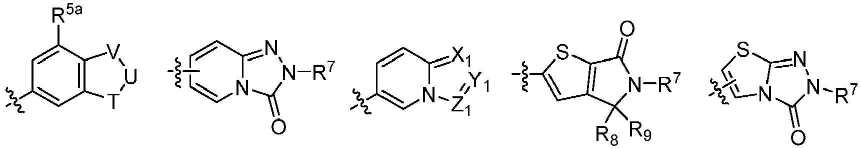 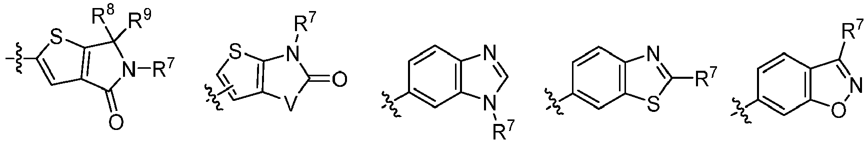
Wherein
   V is selected from the group consisting of -O-, -S-, -Se-, -CH₂-, -CF₂-, -CO-, -SO-, -SO2-, -N(R⁷)-, - C(R⁸R⁹)-, -O-CH(alkyl)-, -O-CH(cycloalkyl)-, -S-CH(cycloalkyl)-, -CH=C(alkyl)-, -N=C(alkyl)-, - CH=C(cycloalkyl)-, -N=C (cycloalkyl)-;
   U is selected from -O-, -S-, -Se-, -CO-, -SO-, -S(O)2, -NR⁷-, -CR⁸R⁹-;
   When V is -O-, -S-, -CF₂-, and U is -CO-, T is -O-, -S-, -Se-, -CO, -SO-, -S(O)2, -CR⁸R⁹-, -NH-, -N(CHF)-, -N(CF₂)-, -N(CH₂-CH(OH)-CH₃)-, -N(CH₂-CH (OCH₃)-CH₃)-, -N(CH₂-CH₂-OCH₃)-, -N(C₄₋₈alkyl)-, - N(cycloalkyl)-, -N(cycloalkoxy)-, -N(oxetane)-, -N(tetrahydrofuran)-, -N(tetrahydropyran)-, wherein said is -N(C₄₋₈alkyl)-, -N(cycloalkyl)-, -N(cycloalkoxy)-, -N(oxetane)-, -N(tetrahydrofuran)-, - N(tetrahydropyran) selectively substituted with C₁₋₃alkoxy, C₁₋₃alkyl, C₃₋₆cycloalkyl, NH(C₁₋₃alkyl), N(C₁₋₃alkyl)₂, or 1 to 3 halogen; otherwise T is -O-, -S-, -Se-, -CO-, -SO-, -SO2-, -C(R⁸R⁹)-, -N(R⁷)-;
   X1, Y1 and Z1 may be independently selected from CH and N;
   R⁷=R^{1a};
   R⁸, R⁹=R^{1a}, or R⁸, R⁹ may selectively form a saturated or unsaturated cyclic group, including but not limited to cycloalkyl, cycloalkenyl, heterocycloalkyl, heterocycloalkenyl, bridged ring, spiro ring, fused ring, aromatic group, heteroaryl group, with the atoms to which both are originally attached, and the cyclic group may be further selectively substituted with one or more R³, and C, S in the ring may also be selectively oxylated to -CO-, -SO-, -SO2-.

A preferred option of the present invention, a compound of general formula (I) or a pharmaceutically acceptable salt thereof, wherein Cy is selected from 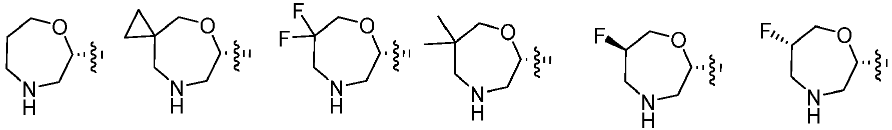 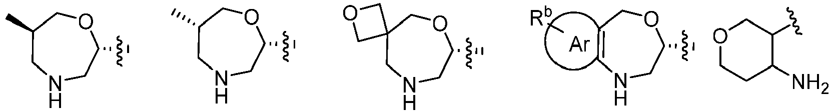
Ar is pyridine and benzene ring;
R^{b} is chlorine, fluorine, methyl, ethyl, propyl, isopropyl, cyclopropyl, trifluoromethyl, trifluoromethoxy A preferred option of the present invention, a compound of formula (I) or a pharmaceutically acceptable salt thereof, wherein A and B are both selected from H, or both are selected from F.

A preferred option of the present invention, a compound of formula (I) or a pharmaceutically acceptable salt thereof, wherein X, Y are each independently selected from CH, N, and Z is selected from CH.

A preferred option of the present invention, a compound of formula (I) or a pharmaceutically acceptable salt thereof, wherein R² is selected from hydrogen, fluorine, methyl, and q is selected from 1.

A preferred option of the present invention, a compound of formula (I) or a pharmaceutically acceptable salt thereof, wherein R¹ is selected from

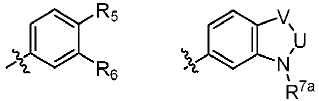

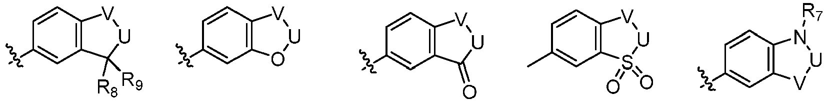

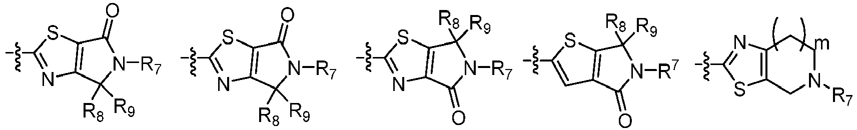

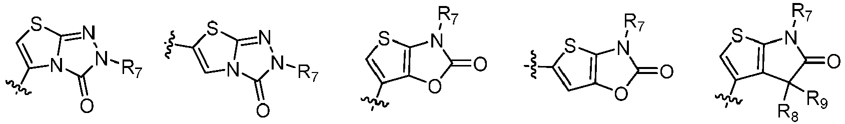

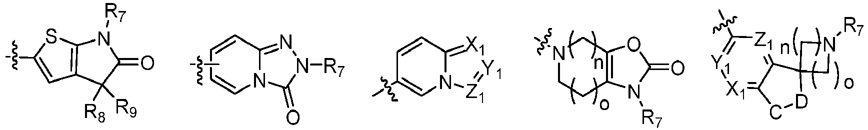

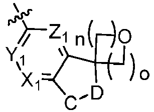

Wherein
X₁, Y₁ and Z₁ are independently selected from CH and N;
m, n and o are 0-3;
U may be selected from -C(=O)-, -S(O)2-, -O-, -NR⁷-, -CR⁸R⁹-;
V may be selected from -C(=O)-, -S(O)2-, -O-, -S-, -Se-, -NR⁷-, -CR⁸R⁹-;
R7 is selected from the group consisting of hydrogen, CH₃OCH₂CH₂-, oxetanyl, azetidine, tetrahydrofuranyl, tetrahydropyran, pyrrolidine, piperazine, morpholine, piperidine, -C₁₋₃ alkyl, and C₃₋₆ cycloalkyl, wherein said C₁₋₃ alkyl, C3-6 cycloalkyl, azetidine, tetrahydrofuranyl, tetrahydropyran, pyrrolidine, piperazine, morpholine or piperidine is optionally substituted with 1, 2 or 3 fluorines, and/or is optionally substituted with one substituent selected from the group consisting of: C₁₋₃ alkyl, hydroxy, -OC₁₋₃ alkyl,-N (C₁₋₃ alkyl)₂, or cyclopropyl; the same substituent or different substituent may be selected for R⁷ at different positions in the same structure;
R⁸ and R⁹ are hydrogen, fluorine, -C₁₋₆ alkyl, or -C₁₋₆ haloalkyl, or R⁸ and R⁹ together with the nitrogen atom or carbon atom to which they are attached form C₃₋₆ cycloalkyl, oxetane, azetidine, pyrrolidine, piperidine ring, piperazine ring, morpholine ring, tetrahydrofuranyl, or tetrahydropyranyl, and said cyclopropane, oxetane, azetidine, pyrrolidine, piperidine ring, piperazine ring, morpholine ring, tetrahydrofuranyl or tetrahydropyranyl may be optionally substituted with C₁₋₃ alkyl, cyclopropane, oxetane, azetidine, cyclopropyloxy;
C, D are independently selected from -NR⁷C(O)-, -C(O)NR⁷-, -CH₂-CH₂-, -C(O)-O-, -O-C(O)-, -CH₂-O-, -O-CH₂-, -CH₂-NR⁷-, -NR⁷-CH₂-, -CH₂-; Or one of C and D is a bond (single bond or double bond in the ring, the atoms at both ends are directly connected);
When Cy is 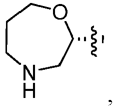 , and R² is H, and X, Y, Z are all selected from CH, and -O-, -S-, or -CF2- if V is present in the structure:
R5 is selected from the group consisting of -S(O)2C₁₋₃ alkyl, -CONH₂, -SO₂NHC₁₋₃ alkyl, or -SO₂NR³R⁴ (if R³ and R⁴, with the N atom to which they are attached, form a heterocyclic group, the said heterocyclic group is selected from the group consisting of piperazinyl or morpholinyl, which may be optionally substituted with C₁₋₃ alkyl or cyclopropyl);
R⁶ is selected from the group consisting of hydrogen, fluorine, chlorine, bromine, or methyl;
R^{7a} is selected from H, -CHF, -CF₂, -CH₂-CH (OH)-CH₃, -CH₂-CH(OCH₃)-CH₃, -C₄₋₈alkyl, -cycloalkyl, - cycloalkoxy, -CH₂-CH₂-OCH₃, -oxetane, -tetrahydrofuran, -tetrahydropyran, pyrrolidine, piperazine, morpholine, piperidine; Wherein said C₄₋₈alkyl, tetrahydrofuran, pyrrolidine, piperazine, morpholine, piperidine is optionally substituted with 1, 2 or 3 fluorine and
/or optionally substituted with a substituent selected from: hydroxyl, -OC₁₋₃alkyl, -N(C₁₋₃alkyl)₂, cyclopropyl;
Otherwise: R⁵ is selected from cyano, -SO₂C₁₋₃ alkyl, -CONH₂, or -SO₂NR³R⁴, wherein R³ and R⁴ are hydrogen and -C₁₋₆ alkyl, or R³ and R⁴ together with the nitrogen atom to which they are attached form azetidine, oxetane, pyrrolidine, piperidine ring, piperazine ring, or morpholine ring, which may be optionally substituted with C1-3 alkyl or cyclopropyl;
R⁶ is selected from the group consisting of hydrogen, fluorine, chlorine, bromine, or methyl;
R^{7a}=R⁷;

A preferred option of the present invention, a compound of formula (I) or a pharmaceutically acceptable salt thereof, wherein R¹ is selected from 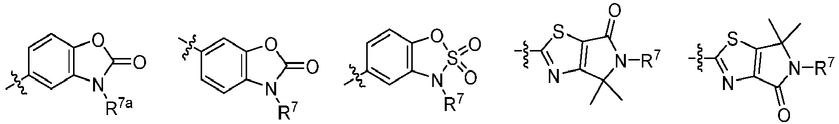 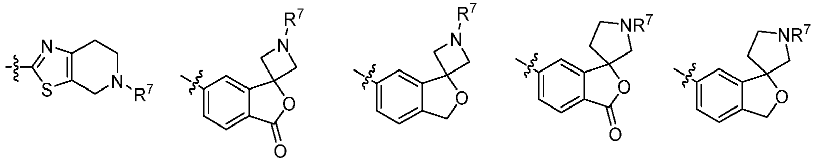 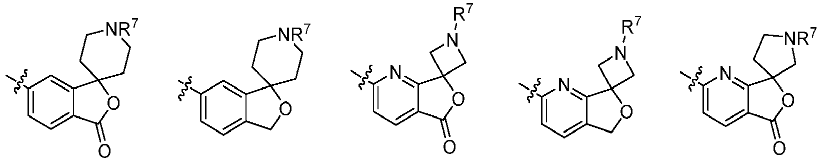 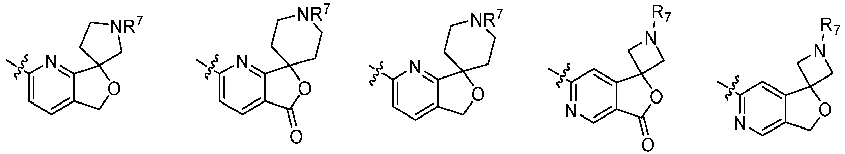 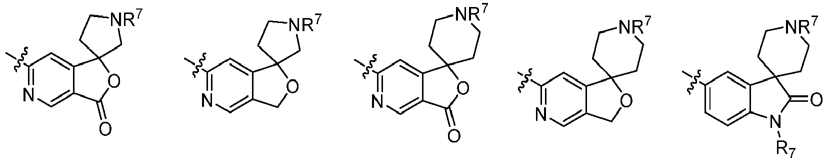 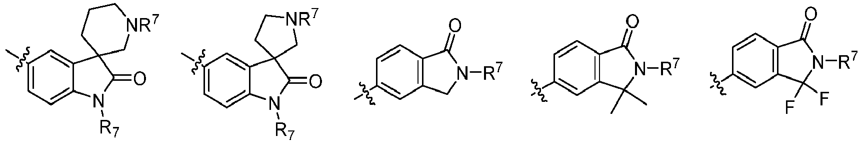 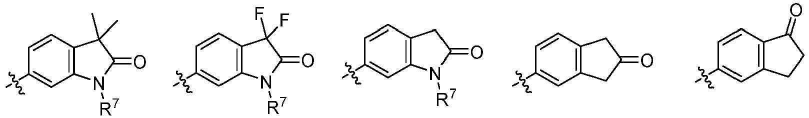 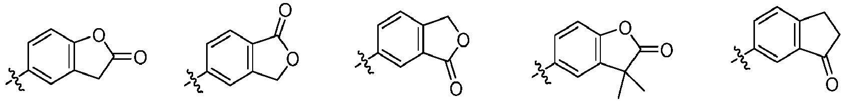 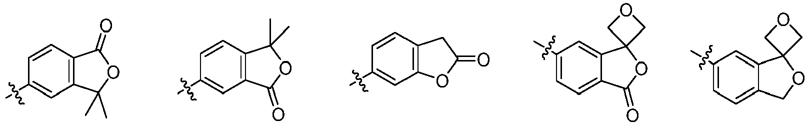
R⁷ is selected from the group consisting of hydrogen, -CF₂, -CHF, CH₃OCH₂CH₂-, oxetanyl, tetrahydrofuranyl, tetrahydropyran, -C₁₋₃ alkyl, or -C₃₋₆ cycloalkoxy; wherein said C₁₋₃ alkyl, oxetanyl, tetrahydrofuranyl, and tetrahydropyran may optionally be substituted with 1, 2 or 3 fluorines, and or optionally substituted with one substituent selected from the group consisting of: hydroxy, -OC₁₋₃ alkyl, - N(C₁₋₃ alkyl)₂, or cyclopropyl; the same substituent or different substituent may be selected for R⁷ at different positions in the same structure;
When Cy is 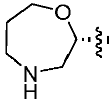 , and R² is H, and X, Y and Z are all selected from CH, and A and B are both H: R^{7a} is selected from H, -CHF, -CF₂, -CH₂-CH(OH)-CH₃, -CH₂-CH(OCH₃)-CH₃, -C₄₋₈alkyl, -C₃₋₆cycloalkyl, -C₃₋₆cycloalkoxy, -CH₂-CH₂-OCH₃, -oxetane, -tetrahydrofuran, -tetrahydropyran, said oxetane, tetrahydrofuran, tetrahydropyran being selectively substituted with C₁₋₃alkyl, cyclopropyl; otherwise R^{7a}=R⁷.

Typical compounds of the present invention include, but are not limited to:

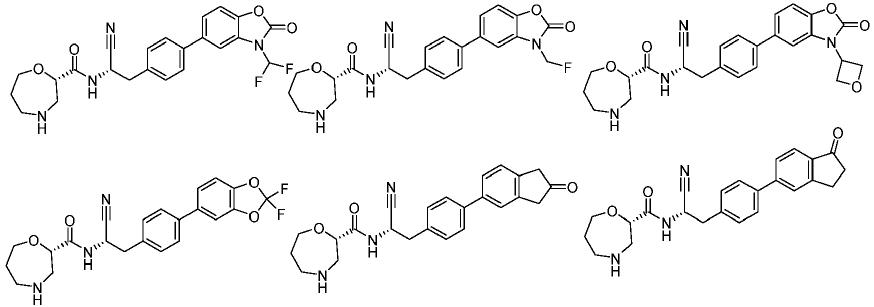

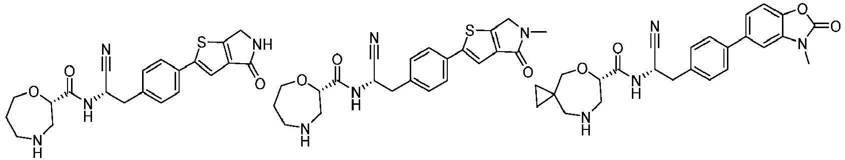

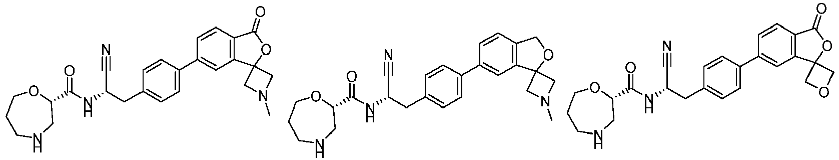

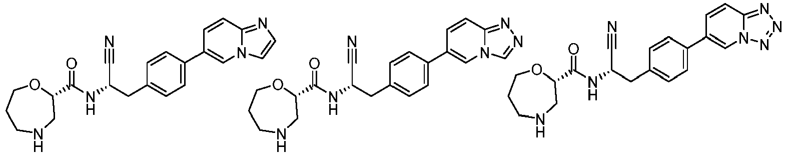

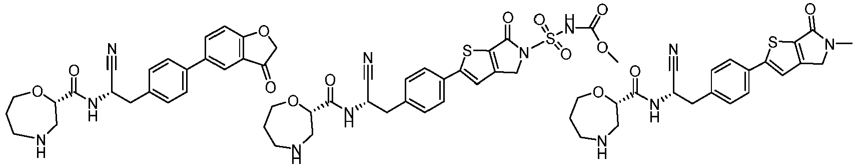

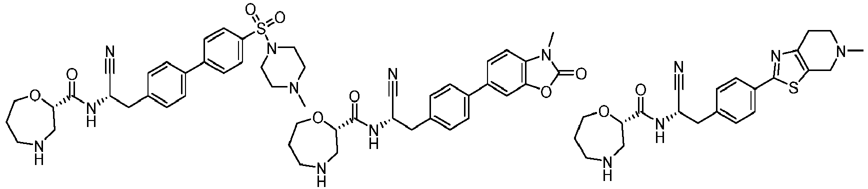

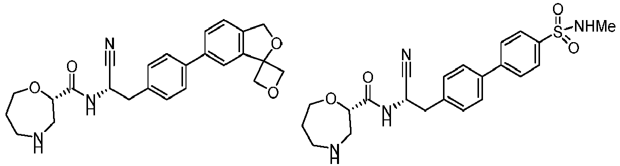

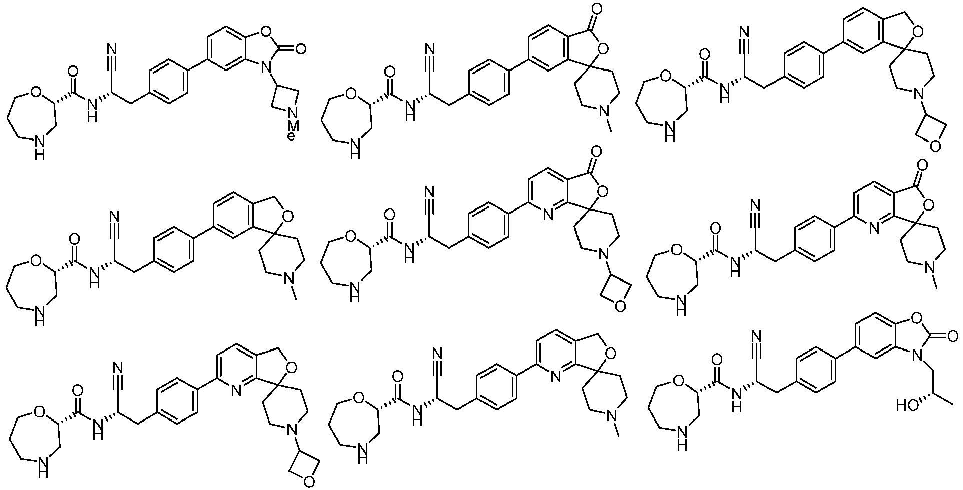

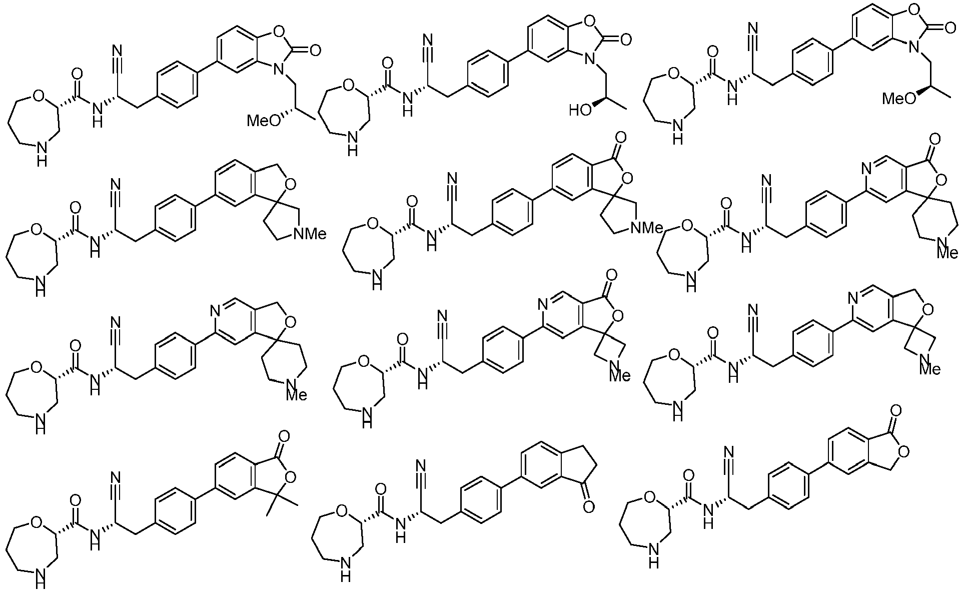

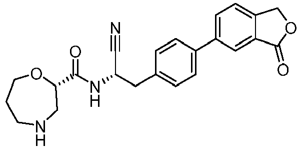

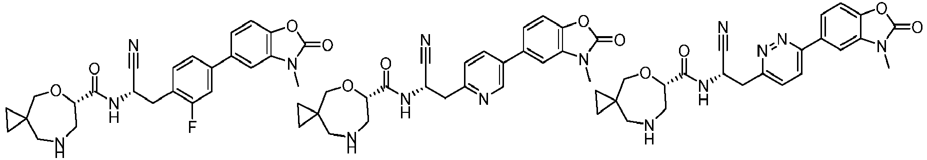

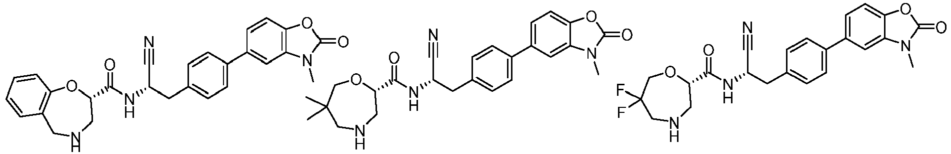

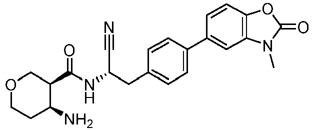

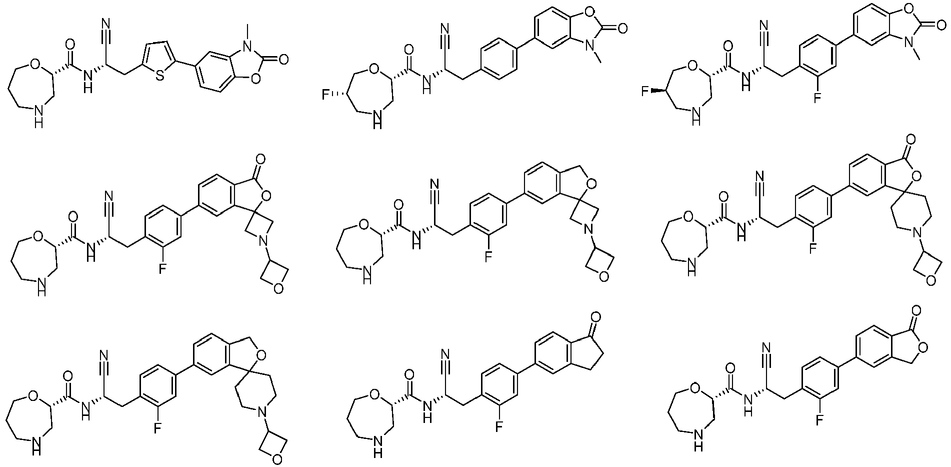

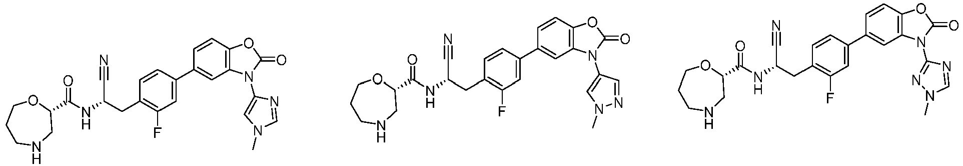

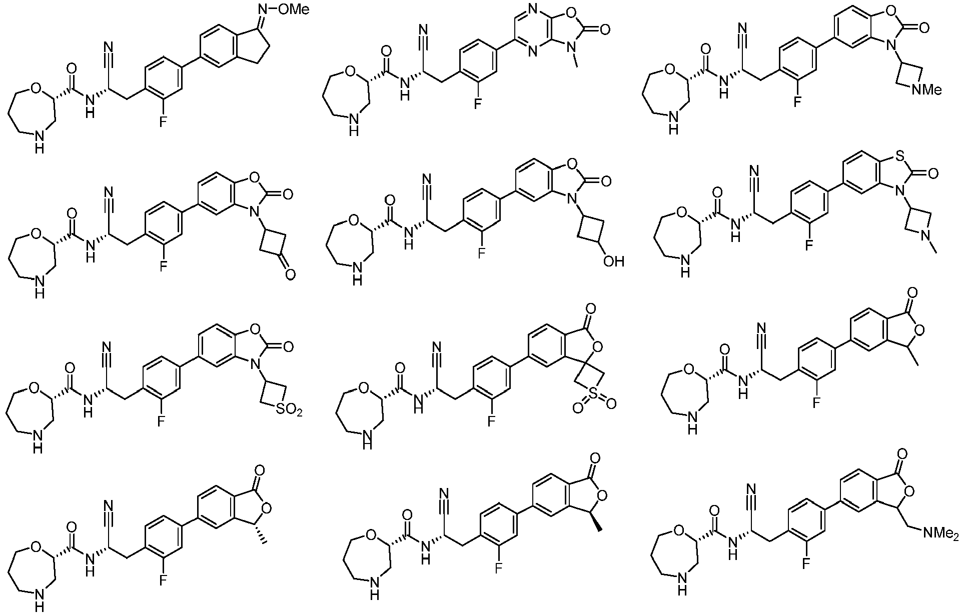

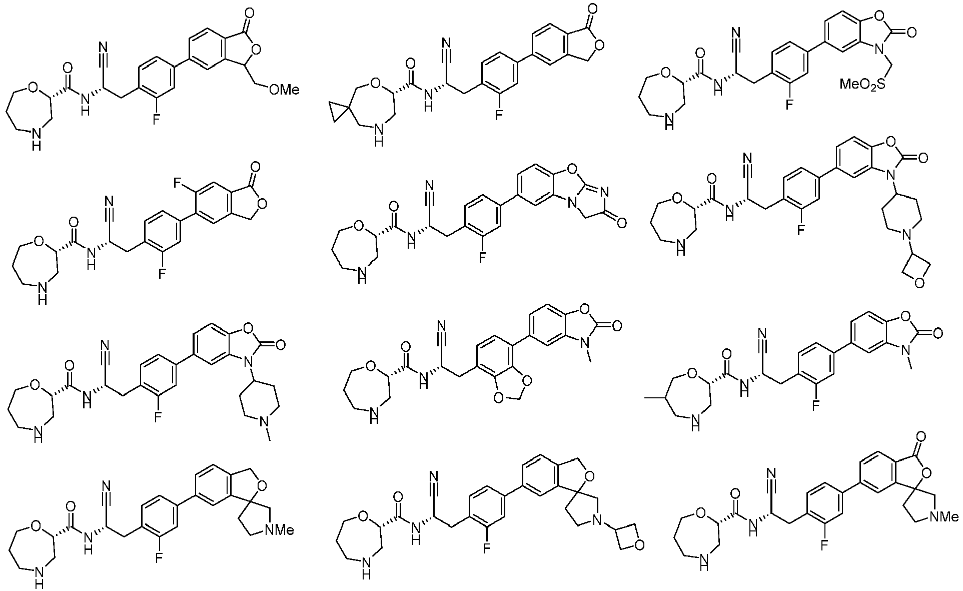

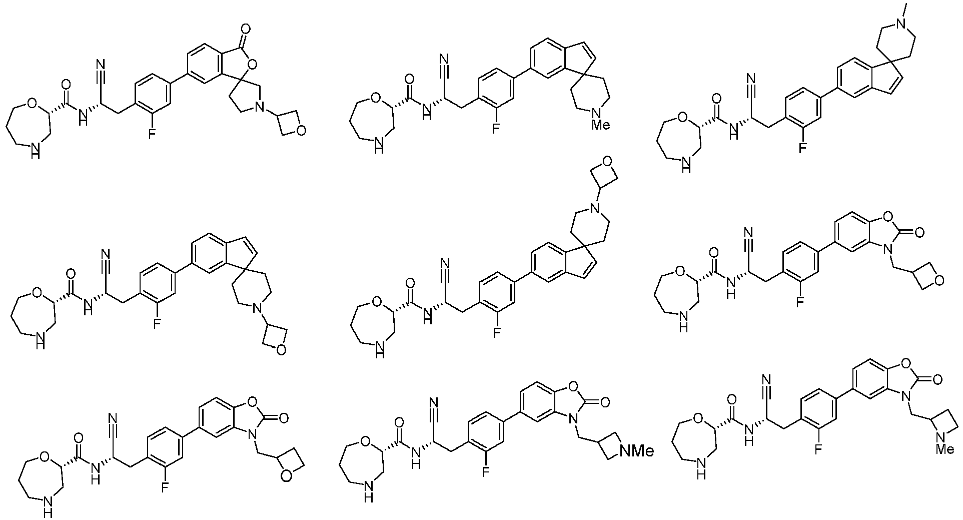

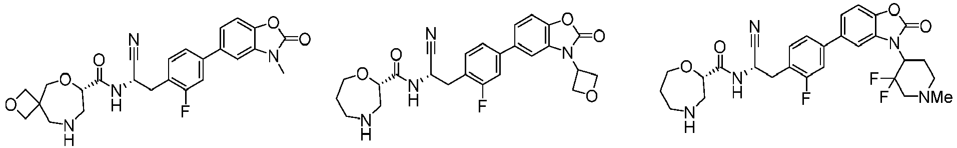

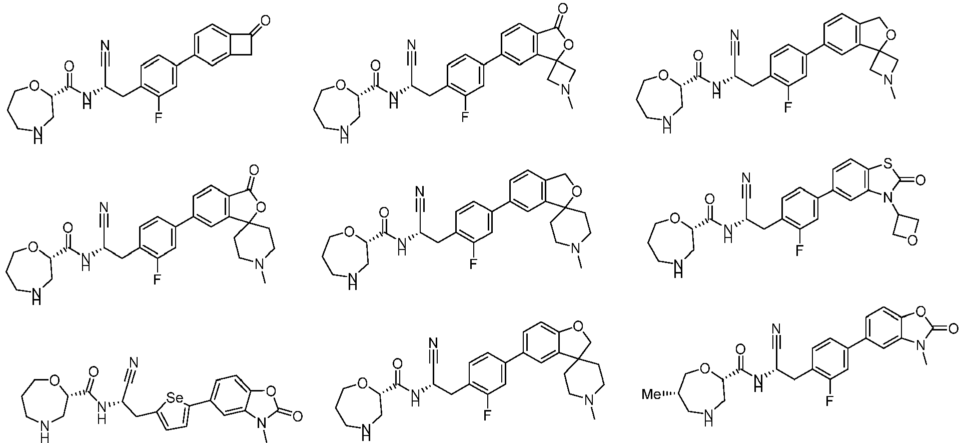

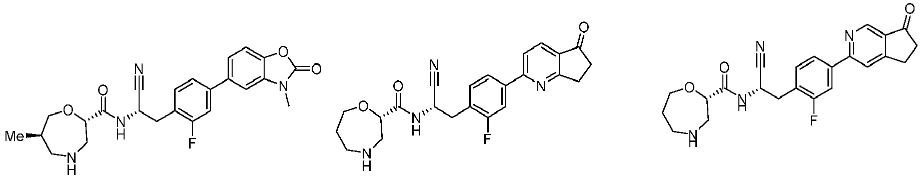

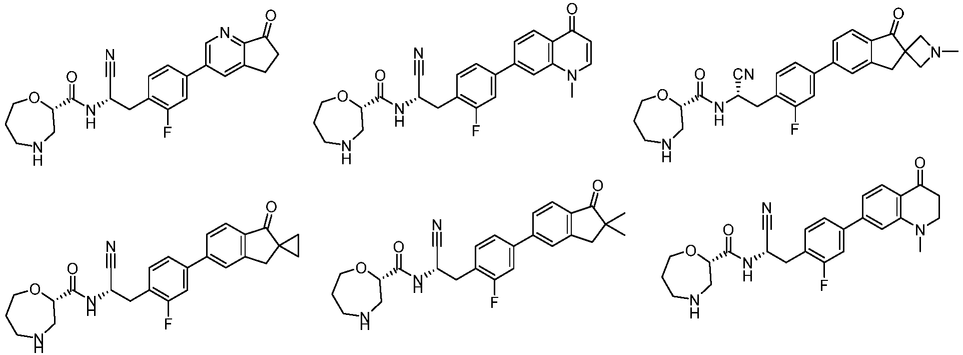

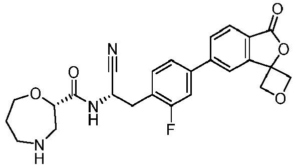

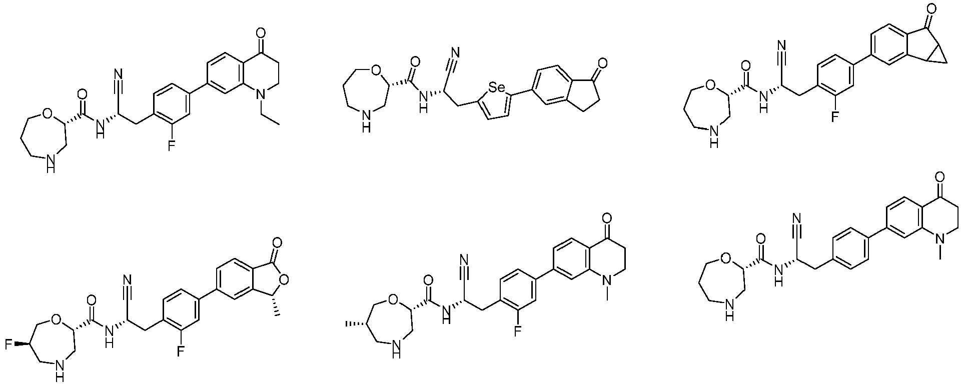

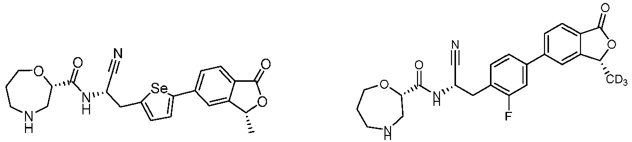

Or a pharmaceutically acceptable salt thereof.

The beneficial effect of the present invention relates to the use of a compound of formula (I) or a pharmaceutically acceptable salt thereof for the manufacture of a medicament for the treatment and prevention of diseases of cathepsin C and its downstream serine proteases NE, PR3, CaTG, NSP4.

Further relate to that use of a compound of formula (I) or a pharmaceutically acceptable salt thereof in the preparation of drugs for treating a patient suffering from respiratory disease, metabolic disease, cardio-cerebrovascular disease, autoimmune disease, cancer, infectious disease and other inflammatory related diseases including asthma, chronic obstructive pulmonary disease, pulmonary fibrosis, pulmonary hypertension, pulmonary hypertension, non-cystic fibrosis, cystic fibrosis, bronchiectasis, bronchitis, pneumonia, emphysema, acute lung injury (ALI), and acute respiratory distress syndrome (ARDS) , sepsis, allergic disorders, immune inflammatory bowel disease, rheumatoid arthritis, glomerulonephritis, eosinophilic disorders, neutrophil disorders, ANCA-associated inflammation, ANCA-associated necrotizing crescentic glomerulonephritis, acute brain trauma, acute myocarditis, acute kidney injury, a-1-antitrypsin deficiency (AATD) and associated inflammation, liver fibrosis, fatty liver and hepatic steatosis, obesity, insulin resistance, diabetes, pathogenic microbial infections, infectious gastrointestinal inflammatory diseases, lung cancer and/or radiation injury syndrome in a patient or at risk for said diseases.

The beneficial effect of the present invention relates to a pharmaceutical composition comprising a compound of formula (I), a pharmaceutically acceptable salt thereof, and at least one pharmaceutical carrier or excipient.

Further, the pharmaceutical composition contains one or more compounds of formula (I), and pharmaceutically active compounds selected from the group consisting of other compounds including, but not limited to: b mimetics, anticholinergic drugs, corticosteroids, PDE4 inhibitors, LTD4 antagonists, EGFR inhibitors, CRTH2 inhibitors, 5-LO inhibitors, histamine receptor antagonists, CCR9 antagonists and SYK inhibitors, NE inhibitors, MMP9 inhibitors, MMP 12 inhibitors and combinations of two or three active substances.

Further, the pharmaceutical composition also includes use in combination with a small molecule compound and/or a macromolecule antibody to treat cancer, inflammation, bone marrow-related diseases and autoimmune diseases. The small molecule compounds and/or macromolecule antibodies include, but are not limited to, glucocorticoids, adrenergic agonists, cholinergic receptor antagonists, theophylline drugs, antioxidants, elastase inhibitors, metalloproteinase inhibitors, PDE4 inhibitors, LTD4 antagonists, EGFR inhibitors, CRTH2 inhibitors, 5-LO inhibitors, histamine receptor antagonists, CCR9 antagonists and SYK inhibitors, chemokine receptor inhibitors, interleukin antibodies such as IL-6 antibodies, IL-23 antibodies, targeted anti-thymic stromal lymphopoietin (TSLP) antibodies such as tezepelumab, complement inhibitors.

The beneficial effect of the present invention relates to the use of a composition of a compound of formula (I) in a medicament for the treatment and prevention of diseases caused by cathepsin C and its downstream serine proteases NE, PR3, CaTG, NSP4, selected from respiratory diseases, metabolic diseases, cardiovascular and cerebrovascular diseases, autoimmune diseases, cancer, infectious diseases or inflammatory infectious diseases.

The beneficial effect of the present invention relates to solvates, racemic mixtures, enantiomers, diastereomers, tautomers or mixtures in any ratio including racemic mixtures of compounds of formula (I), wherein each of the substituents R¹, R², Cy, A, B, X, Y, Z and q:

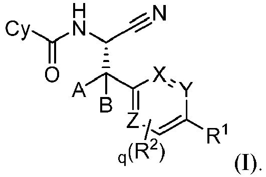

The compounds of the present invention may be asymmetric, such as having one or more stereocenters. Unless otherwise specified, all stereoisomers, are e.g., enantiomers and diastereomers. Containing asymmetrically substituted carbon atoms. The compounds of the present invention can be separated into optically pure or racemic forms. Optically pure forms may be prepared by resolution of the racemate or by the use of chiral synthons or chiral reagents.

The compounds of the present invention may also include tautomeric forms. The new form of tautomer is produced by the interchange of a single bond and an adjacent double bond together with the migration of a proton.

The compounds of the present invention may also include all isotopic forms of atoms present in the intermediate or final compound. Isotopes include those atoms that have the same atomic number but different mass numbers. For example, isotopes of hydrogen include deuterium and tritium.

The present invention also encompasses pharmaceutical salts of the compounds in formula (I). A pharmaceutical salt is a derivative of a compound in formula (I) in which the parent compound is modified by conversion of the base moiety present into its salt form, or a derivative of a compound in formula (I) in which the parent compound is modified by conversion of the acid moiety present into its salt form.

Specifically, examples of pharmaceutical salts include, but are not limited to: a salt of an inorganic or organic acid of a basic group (such as an amine), or a salt of an inorganic or organic base of an acidic group (such as a carboxylic acid). The pharmaceutical salts of the present invention can be synthesized from parent compounds in formula (I) by reacting the free base form of these compounds with 1-4 equivalents of the appropriate acid in a solvent system. Suitable salts are listed in Remington's Pharmaceuticals Sciences, 17th ed., Mack Publishing Company, Easton, Pa., 1985, p. 1418 and Journal of Pharmaceutical Science, 66, 2 (1977).

The compounds of the present invention, as well as their pharmaceutical salts, also include solvate forms or hydrate forms. In general, solvate forms or hydrate forms are equivalent to non-solvate forms or non-hydrate forms, both included within the scope of the present invention. Some of the compounds of the present invention may exist in a variety of crystalline or amorphous forms. Overall, all physical forms of the compounds are included within the scope of the present invention.

The present invention also encompasses prodrugs of the compounds in formula (I). A prodrug is a pharmacological substance (i.e., a drug) derived from the parent drug. Once administered, the prodrug is metabolized in the body to the parent drug. The prodrug may be prepared by substituting one or more functional groups present in the compound, wherein the substituent in the prodrug is removed *in vivo* in such a way that it is converted to the parent compound. The preparation and use of the prodrug is as documented in T. Higuchi and V. Stella, "Pro-drugs as Novel Delivery Systems" Vol. 0.14 of the A. C. S. Symposium Series and Bioreversible.

### DETAILED DESCRIPTION

The present invention is further described in connection with specific embodiments as follows:
The present invention is further described below by embodiments which are intended only to more specifically illustrate preferred embodiments of the present invention and are not intended to define technical solutions of the present invention. The solutions of the present invention are all technical solutions which can realize the purpose of the present invention. The temperatures and reagents used in the following embodiments may be substituted for the corresponding temperatures and reagents described above to achieve the purpose of the present invention.

The following terms, phrases and symbols used in the present invention have the meanings set forth below, unless otherwise specified in the context in which they are placed.

A short cross ("-") between two letters or symbols indicates the site of attachment of the substituents. For example, -O(C₁₋₄alkyl) refers to a C₁₋₄alkyl group attached to the rest of the molecule via an oxygen atom. However, when the site of attachment of a substituent is obvious to one skilled in the art, for example, a halogen substituent, the "-" may be omitted.

Unless otherwise explicitly stated, the use of terms such as "one" refers to one or more.

The term "alkyl" as used in the present invention refers to a linear or branched saturated hydrocarbon group containing 1-18 carbon atoms, e.g., 1-12 carbon atoms, 1-6 carbon atoms, or 1-4 carbon atoms. For example, "C₁₋₆alkyl" is in the range of "alkyl" and represents said alkyl group having 1-6 carbon atoms. Examples of alkyl groups include, but are not limited to, methyl ("Me"), ethyl ("Et"), n-propyl ("n-Pr"), isopropyl ("i-Pr"), n-butyl ("n-Bu"), isobutyl ("i-Bu"), sec-butyl ("sBu"), and tert-butyl ("t-Bu").

The term "alkenyl" as used in the present invention refers to a linear or branched hydrocarbon group containing one or more, e.g., 1, 2, or 3 carbon-carbon double bonds (C=C), containing 2-10 carbon atoms, e.g., 2-6 carbon atoms, or 2-4 carbon atoms. For example, "C2-6 alkenyl" is in the range of "alkenyl" and represents said alkenyl group having 2-6 carbon atoms. Examples of alkenyl groups include, but are not limited to, vinyl, 2-propenyl, and 2-butenyl.

The term "alkynyl" as used in the present invention refers to a linear or branched hydrocarbon group containing one or more, e.g., 1, 2 or 3 carbon-carbon triple bonds (C≡C), containing 2-10 carbon atoms, e.g., 2-6 carbon atoms, or 2-4 carbon atoms. For example, "C₂₋₆alkynyl" represents the said alkynyl group containing 1 carbon-carbon triple bond (C≡C) and having 2-6 carbon atoms. Examples of alkynyl groups include, but are not limited to, ethynyl, 2-propynyl, and 2-butynyl.

The term "halogenated" as used in the present invention refers to fluoro, chloro, bromo, and iodo, and "halogen" refers to fluorine, chlorine, bromine and iodine.

The term "haloalkyl" as used in the present invention refers to an alkyl group as defined in the present invention in which one or more hydrogen atoms, e.g. 1, 2, 3, 4 or 5 hydrogen atoms, are replaced by halogen atoms and which may be the same as or different from each other when more than one hydrogen atom is replaced by halogen atoms. In one embodiment, the term "haloalkyl" as used in the present invention refers to an alkyl group as defined in the present invention in which two or more hydrogen atoms, e.g., 2, 3, 4 or 5 hydrogen atoms, are replaced by halogen atoms, wherein the halogen atoms are the same as each other. In another embodiment, the term "haloalkyl" as used in the present invention refers to an alkyl group as defined in the present invention in which two or more hydrogen atoms, e.g. 2, 3, 4 or 5 hydrogen atoms, are replaced by halogen atoms, wherein the halogen atoms are different from each other. Examples of haloalkyl groups include, but are not limited to, -CF3, -CHF2, -CH2CF3, and the like.

The term "alkoxy" as used in the present invention refers to the group-O-alkyl, wherein alkyl is as defined above. Examples of alkoxy groups include, but are not limited to, methoxy, ethoxy, n-propoxy, isopropoxy, n-butoxy, isobutoxy, tert-butoxy, pentyloxy, and hexyloxy, including isomers thereof.

The term "cycloalkyl" as used in the present invention refers to a saturated or partially unsaturated cyclic hydrocarbon group containing 3-12 ring carbon atoms, e.g., 3-8 ring carbon atoms, or 3-6 ring carbon atoms, which may have one or more rings, e.g. 1 or 2 rings. For example, "C3-8 cycloalkyl" represents said cycloalkyl having 3-8 ring carbon atoms. Examples of cycloalkyl include, but are not limited to, cyclopropyl, cyclobutyl, cyclopentyl, cyclohexyl, cycloheptyl, cyclooctyl, adamantyl, and similar groups.

The term "heterocyclyl" or "heterocyclic" as used in the present invention refers to rings selected from the group consisting of 4 to 12 membered monocyclic, bicyclic and tricyclic, saturated and partially unsaturated, which contain at least one carbon atom in addition to at least one, e.g., 1-4, or 1-3, or 1 or 2 heteroatoms selected from the group consisting of O, S and N. The point of attachment of the heterocyclic group can be on the heteroatom or on the carbon. "Heterocyclyl" or "heterocyclic" also means a monocyclic ring comprising at least one heteroatom selected from O, S and N; Or is a fused ring, wherein at least one of the rings contains at least one heteroatom selected from O, S and N and the other rings are not heteroaryl or aryl, the point of attachment of which may be on the heterocycle or on the other rings.

The term "cycloalkoxy" as used in the present invention refers to the group-O-cycloalkyl, wherein cycloalkyl is as defined above. Examples of cycloalkoxy groups include, but are not limited to, cyclopropyloxy, cyclobutyloxy, including isomers thereof.

The term "heterocycloalkoxy" as used in the present invention refers to the group-O-heterocycloalkyl, wherein the heterocycloalkyl is as defined above. Examples of cycloalkoxy groups include, but are not limited to, azacyclopropyloxy, epoxypropyloxy, azetidinyloxy, oxetidinyloxy, including isomers thereof.

The term "cycloalkenyl" in the present invention refers to a non-aromatic cyclic hydroxyl group containing one or more, e.g. 1, 2 or 3 carbon-carbon double bonds, containing 3-12 ring carbon atoms, preferably 3-8 ring carbon atoms, more preferably 3-6 ring carbon atoms, which may have one or more rings, preferably 1 or 2 rings. For example, C₃₋₈ ring carbon atoms and a cycloalkenyl group, preferably "C₃₋₆cycloalkenyl group", i.e., the said cycloalkenyl group having 3-6 ring carbon atoms. Examples of cycloalkenyl groups include, but are not limited to, cyclopropenes and, cyclobutenyl, cyclopentenyl, cyclopentadienyl, cyclohexenyl, cycloheptenyl, and cyclooctenyl.

The "heterocyclic alkenyl group" in the present invention means that one or more carbon atoms in the "cycloalkenyl group" are replaced by N, O or S. For example, a heterocyclic alkenyl group of C₃₋₈ ring atoms, preferably "C₃₋₆heterocyclic alkenyl group", i.e., the said heterocyclic alkenyl group having 3-6 ring atoms. Examples of heterocyclenyl groups include, but are not limited to, azacyclobutylene and, oxacyclobutenyl, azacyclopentenyl, oxacyclopentenyl, oxacyclohexenyl.

The term "aryl" as used in the present invention, unless clearly defined, refers to a carbocyclic hydrocarbon group consisting of one or more rings fused to contain 6-14 ring carbon atoms, e.g. 6-12 ring carbon atoms, wherein at least one of the rings is an aromatic ring and the other rings are not heteroaryl as defined below, the point of attachment of which may be on the aromatic ring or on the other rings. Examples of aryl groups include, but are not limited to, phenyl, naphthyl, 1, 2, 3, 4-tetrahydronaphthyl, indenyl, indanyl, azulenyl, preferably phenyl and naphthyl. As used in the present invention, "aryl" or "aromatic" obeys the Hückel's rule in which the number of π electrons is equal to 4n + 2 and n is zero or any positive integer which is at most 6.

The term "heterocyclyl" or "heterocyclic" as used in the present invention refers to rings selected from the group consisting of 4 to 12 membered monocyclic, bicyclic and tricyclic, saturated and partially unsaturated, which contain at least one carbon atom in addition to at least one, e.g., 1-4, or 1-3, or 1 or 2 heteroatoms selected from the group consisting of O, S and N. The point of attachment of the heterocyclic group can be on the heteroatom or on the carbon. "Heterocyclyl" or "heterocyclic" also means a monocyclic ring comprising at least one heteroatom selected from O, S and N; Or is a fused ring, wherein at least one of the rings contains at least one heteroatom selected from O, S and N and the other rings are not heteroaryl or aryl, the point of attachment of which may be on the heterocycle or on the other rings.

The term "heteroaryl" as used in the present invention, unless clearly defined, refers to a monocyclic aromatic hydrocarbon group having 5, 6 or 7 ring atoms, e.g., having 6 ring atoms, containing in the ring one or more, e.g., 1, 2 or 3, e.g., 1 or 2, ring heteroatoms independently selected from N, O and S (e.g. N), with the remaining ring atoms being carbon atoms; and a bicyclic aromatic hydrocarbon group having 8-12 ring atoms, e.g., having 9 or 10 ring atoms, comprising in the ring one or more, e.g., 1, 2, 3 or 4, e.g., 1 or 2, ring heteroatoms independently selected from N, O and S (e.g. N), with the remaining ring atoms being carbon atoms, wherein at least one of the rings is an aromatic ring. For example, the bicyclic heteroaryl group comprises a 5-6 membered heteroaryl ring fused to a 5-6 membered cycloalkyl ring, heterocyclyl ring, or aryl ring, wherein the point of attachment may be on the heteroaryl ring or on the cycloalkyl ring/heterocyclyl ring/aryl ring. When the total number of S and O atoms in the heteroaryl group exceeds one, these S and O heteroatoms are not adjacent to each other. The heteroaryl groups also include those in which the N ring heteroatom is in the form of an N-oxide, for example, the N-oxypyrimidinyl group. In some embodiments, the ring heteroatom in the aforementioned heteroaryl groups is an N atom, and such heteroaryl groups are referred to as "nitrogen-containing heteroaryl groups". Nitrogen-containing heteroaryl groups also include those in which the N ring heteroatom is in the form of an N-oxide, such as pyridyl N-oxide.

Examples of heteroaryl groups include, but are not limited to: pyridyl, N-oxypyridyl; pyrazinyl; pyrimidinyl; pyrazolyl; imidazolyl; oxazolyl; isoxazolyl; thiazolyl; isothiazolyl; thiadiazole; tetrazolyl; triazolyl; thienyl; furyl; pyranyl; pyrrolyl; pyridazinyl; benzo[d]thiazolyl; benzodioxolyl, such as benzo[d][1,3]dioxolyl; benzoxazolyl, such as benzo[d]oxazolyl; imidazopyridyl, such as imidazo[1,2-a]pyridyl; triazolopyridyl, such as [1,2,4]triazolo[4,3-a]pyridyl and [1,2,4]triazolo[1,5-a]pyridyl; indazolyl; 2H-indazolyl; pyrrolopyrimidinyl, such as pyrrolo[3,4-d]pyrimidinyl, 7H-pyrrolo[2,3-d]pyrimidinyl; pyrazolopyrimidinyl, such as pyrazolo[1,5-a]pyrimidinyl; tetrazolopyridyl, such as tetrazolo[1,5-a]pyridyl; benzothienyl; benzofuranyl; benzimidazolinyl; indolyl; indolinyl; purinyl, such as 9H-purinyl and 7H-purinyl

Examples of nitrogen-containing heteroaryl groups include, but are not limited to: pyrrolyl; pyrazolyl; imidazolyl; pyridyl; pyrazinyl; pyrimidinyl, N-oxypyrimidinyl; pyridazinyl; pyrrolopyrimidinyl, such as pyrrolo[3,4-d]pyrimidinyl, 7H-pyrrolo[2,3-d]pyrimidinyl; Purine groups, such as 9H-purine groups and 7H-purinyl; quinolyl; indolyl; and indazolyl.

The term "hydroxyl" as used in the present invention refers to the -OH group.

The term "sulfhydryl" as used in the present invention refers to the -SH group.

The term "oxo" as used in the present invention refers to the =O group.

The term "carboxyl" as used in the present invention refers to the -C(O)-OH group.

The term "cyano" as used in the present invention refers to the -CN group.

The term "amino" as used in the present invention refers to the -NH2 group.

"Group" and "radical" used in the present invention are synonymous and are used to denote functional groups or molecular fragments that can be linked to other molecular fragments.

The numerical range of p, q, f, g, h, k, m, n, o described in the present invention refers to any integer encompassing the range, for example, 0-6 refers to any integer of 0, 1, 2, 3, 4, 5, 6.

If a structural formula of the present invention contains an asterisk "*", the compound represented by the structural formula is a chiral compound, i.e. the compound is in the R-configuration or the S-configuration. The configuration of a compound may be determined by a person skilled in the art using a variety of analytical techniques, such as single crystal X-ray crystallography and/or optical polarimetry, and according to conventional solutions.

The term "selectivity", "selective", "selectively", "option", "optional" or "optionally" as used in the present invention means that a subsequently described substitution pattern, event or situation may occur one or more times, or may not occur, and that description includes situations in which said substitution pattern occurs as well as situations in which said substitution pattern does not occur. For example, "optionally substituted alkyl" includes "unsubstituted alkyl" and "substituted alkyl" as defined in the present invention. It will be appreciated by those skilled in the art that, for any group containing one or more substituents, said group does not include any sterically impractical, chemically incorrect, synthetically infeasible and/or intrinsically labile substitution patterns.

The term "including but not limited to" as used in the present invention means that the selected range prefers subsequently exemplified groups, but groups not exemplified may also be used.

The term "substituted" or "substituted by..." as used in the present invention means that one or more hydrogen atoms on a given atom or group are replaced by one or more substituents selected from a given group of substituents, provided that the normal valence of that given atom is not exceeded. When the substituent is oxo (i.e. =O), two hydrogen atoms on a single atom are replaced by oxygen. Such combinations are permissible only if the combination of substituents and/or variables results in a chemically correct and stable compound. A chemically correct and stable compound means that the compound is sufficiently stable that it can be isolated from the reaction mixture and the chemical structure of the compound can be determined, and can subsequently be formulated into a preparation of at least practical utility.

Substituents are named in the core structure unless otherwise noted. For example, it should be appreciated that when a (cycloalkyl) alkyl group is listed as a possible substituent, it indicates that the point of attachment of the substituent to the core structure is in the alkyl moiety.

The term "substituted by one or more substituents" as used in the present invention means that one or more hydrogen atoms on a given atom or group are independently replaced by one or more substituents selected from a given group. In some embodiments, "substituted by one or more substituents" means that a given atom or group is substituted by 1, 2, 3 or 4 substituents independently selected from the given group.

It will be appreciated by those skilled in the art that some compounds of formula (I) may comprise one or more chiral centers and thus exist in two or more stereoisomers. Racemic mixtures of these isomers, mixtures enriched in individual isomers and one enantiomer, and mixtures partially enriched in diastereoisomers and specific diastereoisomer when there are two chiral centers are within the scope of the present invention. It will also be appreciated by those skilled in the art that the invention encompasses all individual stereoisomers (e.g. enantiomers), racemic mixtures or partially resolved mixtures of compounds of formula (I) and, where appropriate, individual tautomers thereof.

In other words, in some embodiments, the present invention provides compounds containing a variety of stereoisomeric purities, i.e., diastereomeric or enantiomeric purity represented by different "ee" or "de" values. In some embodiments, a compound of formula (I) (e.g. as described in the present invention) has an enantiomeric purity of at least 60% ee (e.g. 60%, 65%, 70%, 75%, 80%, 85%, 90%, 91%, 92%, 93%, 94%, 95%, 96%, 97%, 98%, 99%, 99.5%, 99.9% ee, or any value between these enumerated values). In some embodiments, a compound of formula (I) (e.g. as described in the present invention) has an enantiomeric purity of greater than 99.9% ee to 100% ee. In some embodiments, the compound of formula (I) (e.g. as described in the present invention) has a diastereomeric purity of at least 60% de (e.g. 60%, 65%, 70%, 75%, 80%, 85%, 90%, 91%, 92%, 93%, 94%, 95%, 96%, 97%, 98%, 99%, 99.5%, 99.9% de, or any value between these enumerated values). In some embodiments, a compound of formula (I) (e.g. as described in the present invention) has a diastereomeric purity of greater than 99.9% de.

The term "enantiomeric excess" or "ee" indicates the amount of one enantiomer relative to the other. For a mixture of R and S enantiomers, the percent enantiomeric excess is defined as |R-S|* 100, where R and S are the molar or weight fractions of the respective enantiomers in the mixture, and R+S=1. If the optical rotation of a chiral substance is known, the percent enantiomeric excess is defined as ([a]obs/[a]max)* 100, where [a]obs is the optical rotation of the enantiomeric mixture and [a]max is the optical rotation of the pure enantiomer.

The term "diastereomeric excess" or "de" denotes the amount of one diastereomer relative to the other and is defined by analogy in terms of enantiomeric excess. Therefore, for a mixture of diastereomers D1 and D2, the percent diastereomeric excess is defined as |D1-D2| * 100, where D1 and D2 are the molar or weight fractions of the respective diastereomers in the mixture, and D1+D2=1.

The determination of diastereomeric and/or enantiomeric excess can be performed using a variety of analytical techniques, including nuclear magnetic resonance spectroscopy, chiral column chromatography and/or optical polarimetry, and according to conventional solutions familiar to those skilled in the art.

The racemic mixture may be used in its own form or as a split into individual isomers. StereOCHemically pure compounds or mixtures enriched in one or more isomers can be obtained by resolution. Methods for separating isomers are well known and include physical methods such as chromatography using chiral adsorbents. Individual isomers in chiral form can be prepared from chiral precursors. Alternatively, individual isomers can be chemically separated from the mixture by forming diastereomeric salts with chiral acids (e.g. individual enantiomers of 10-camphorsulfonic acid, camphoric acid, alpha-bromo-camphoric acid, tartaric acid, diacetyltartaric acid, malic acid, pyrrolidone-5-carboxylic acid or the like), fractionally crystallizing said salts and then freeing one or both of the resolved bases, optionally repeating this process to thereby give one or two isomers substantially free of the other isomer, i.e. the desired stereoisomer having an optical purity by weight of e.g., at least 91%, 92%, 93%, 94%, 95%, 96%, 97%, 98%, 99% or 99.5%. Alternatively, as is well known to those skilled in the art, the racemate can be covalently attached to a chiral compound (auxiliary) to give the diastereomer, which can be separated by chromatography or fractional crystallization, after which the chiral auxiliary is chemically removed to give the pure enantiomer.

The "pharmaceutically acceptable salt" of the present invention means a salt of a free acid or base of a compound of formula (I) that is non-toxic, biologically tolerable or otherwise biologically suitable for administration to a treated individual.

"Pharmaceutically acceptable salts" include, but are not limited to: acid addition salts formed by compounds of formula (I) with mineral acids, such as hydrOCHloride, hydrobromide, carbonate, bicarbonate, phosphate, sulfate, sulfite, nitrate, and the like; and acid addition salts formed by compounds of formula (I) with organic acids, such as formate, acetate, malate, maleate, fumarate, tartrate, succinate, citrate, lactate, mesylate, p-toluenesulfonate, 2-hydroxyethanesulfonate, benzoate, salicylate, stearate and salts formed with alkanedicarboxylic acids of formula HOOC-(CH2)n-COOH, wherein n is 0-4, etc. The "pharmaceutically acceptable salts" also include base addition salts formed by compounds of formula (I) bearing acidic groups with pharmaceutically acceptable cations such as sodium, potassium, calcium, aluminum, lithium and ammonium. The molar ratios of the compound of formula (I) to the acid or cation in the resulting pharmaceutically acceptable salts include, but are not limited to, 1: 1, 1: 2, 1: 3 and 1: 4.

The "prodrug" of the present invention refers to a pharmacological substance (i.e. drug) derived from the parent drug, and it is metabolized into the parent drug *in vivo* once administered. The prodrug may be prepared by substituting one or more functional groups present in the compound, wherein the substituent in the prodrug is removed *in vivo* in such a way that it is converted to the parent compound. The preparation and use of the prodrugs is described in T .Higuchi and V Stella, "Pro-drugs asNovel Delivery Systems", Vol. 14 of the A.C.S. Symposium Series and Bioreversible. "Prodrugs" include, but are not limited to: esters of compounds of formula (I) such as phosphate, formate, carbamate; and amides such as formamide, acetamide.

Furthermore, if the compound described in the present invention is obtained in the form of an acid addition salt, its free base form can be obtained by basifying a solution of the acid addition salt. Conversely, if the product is in the form of a free base, an acid addition salt thereof, in particular a pharmaceutically acceptable acid addition salt, can be obtained by dissolving the free base in a suitable solvent and treating the solution with an acid, as is customary for the preparation of acid addition salts from basic compounds. One skilled in the art can determine, without excessive experimentation, various synthetic methods that can be used to prepare non-toxic pharmaceutically acceptable acid addition salts.

The term "solvate" means a solvent addition form comprising a stoichiometric or non-stoichiometric solvent. Some compounds have the propensity to snare a fixed molar ratio of solvent molecules in the solid state, thereby forming solvates. If the solvent is water, the solvate formed is a hydrate, and when the solvent is ethanol, the solvate formed is ethanolate. Hydrates are formed by one or more molecules of water with one molecule of said substance, wherein water retains its molecular state of H2O, such a combination being capable of forming one or more hydrates, such as hemihydrates, monohydrates and dihydrates, as well as variable hydrates.

The terms "group" and "group" used in the present invention are synonymous and are used to denote functional groups or molecular fragments that can be linked to other molecular fragments.

The term "active ingredient" is used to denote a chemical substance that is biologically active. In some embodiments, the "active ingredient" is a chemical substance having pharmaceutical use. In the United States, actual drug activity can be determined by appropriate preclinical testing, whether *in vitro* or *in vivo.* However, for drug activity that can be accepted by regulatory agencies (e.g., the FDA of the United States), there must be a higher standard for activity than that in preclinical trials. The success of such a higher standard for drug activity cannot generally be reasonably expected from preclinical trial results, but can be established through appropriate and valid randomized, double-blind, controlled clinical trials in humans.

The term "management" or "treatment" of a disease or disorder, where a beneficial therapeutic effect is achieved, refers to the administration of one or more pharmaceutical substances, particularly compounds of formula (I) as described in the present invention and/or pharmaceutically acceptable salts thereof, to an individual, e.g. a human, suffering from, or having symptoms of, or having a predisposition to, said disease or disorder, for the purpose of curing, healing, alleviating, mitigating, altering, treating, ameliorating, improving or influencing said disease or disorder, symptoms of said disease or disorder, or a predisposition to said disease or disorder. In some embodiments, the said disease or disorder is cancer.

When referring to chemical reactions, the terms "treatment", "contact", and "reaction" mean the addition or mixing of two or more reagents under appropriate conditions to produce the indicated and/or desired product. It should be appreciated that the reaction yielding the indicated and/or desired product may not necessarily arise directly from the combination of the two reagents initially added, i.e., there may be one or more intermediates formed in the mixture that ultimately lead to the formation of the indicated and/or desired product.

The term "effective amount" as used in the present invention refers to an amount or dose of a cathepsin C inhibitor generally sufficient to produce a beneficial therapeutic effect in a patient in need of treatment for a disease or disorder mediated by cathepsin C and downstream serine protease activity. The effective amount or dose of an active ingredient in the present invention can be determined by conventional methods, such as modeling, dose escalation studies or clinical trials, in combination with conventional influencing factors, such as mode or route of administration or application, pharmacokinetics of the drug ingredient, severity and course of the disease or disorder, prior or ongoing treatment of the individual, health status and response of the individual to the drug, and judgment of the attending physician. In the United States, determination of an effective dose is generally difficult to predict from preclinical trials. In fact, dosing is completely unpredictable, and new unpredictable dosing regimens will develop after the original use in randomized, double-blind, controlled clinical trials.

Typical dosage ranges are from about 0.0001 to about 200 mg of active ingredient per kg of individual body weight per day, e.g., from about 0.001 to 100 mg/kg/day, or about 0.01 to 35 mg/kg/day, or about 0.1 to 10 mg/kg, administered once daily or in divided dosage units (e.g., twice daily, three times daily, four times daily). For a person weighing 70 kg, a suitable dose range may be from about 0.05 to about 7 g/day, or from about 0.2 to about 5 g/day. Once the patient's disease or disorder has improved, the dose may be adjusted to maintain treatment. For example, the dose or number of administrations, or the dose and number of administrations, may be reduced to a level that maintains a desired therapeutic effect depending on the change in symptoms. Of course, treatment can be stopped if symptoms have been alleviated to an appropriate level. However, patients may require intermittent long-term treatment for recurrence of symptoms.

The term "inhibition" refers to a decrease in the baseline activity of a biological activity or process. The term "inhibition of cathepsin C activity" is the actual pharmaceutical activity used for the purposes of the present invention and refers to a decrease in cathepsin C activity resulting from a direct or indirect response to the presence of a compound of formula (I) described in the present invention and/or a pharmaceutically acceptable salt thereof relative to the cathepsin C activity in the absence of the compound of formula (I) and/or a pharmaceutically acceptable salt thereof. The decrease in activity may result from a direct interaction of the compounds of formula (I) and/or pharmaceutically acceptable salts thereof described in the present invention with cathepsin C, or from an interaction of the compounds of formula (I) and/or pharmaceutically acceptable salts thereof described in the present invention with one or more other factors which in turn affect cathepsin C activity. For example, the presence of a compound of formula (I) described in the present invention and/or a pharmaceutically acceptable salt thereof may reduce the activity of cathepsin C by directly binding to cathepsin C, may reduce the activity of cathepsin C by directly or indirectly affecting another factor, or may reduce the activity of cathepsin C by directly or indirectly reducing the amount of cathepsin C present in a cell or body.

The term "individuals" as used in the present invention refers to mammals and non-mammals. A mammal means any member of mammals, which includes, but is not limited to, humans; non-human primates such as chimpanzees and other ape and monkey species; farm animals such as cattle, horses, sheep, goats and pigs; domestic animals such as rabbits, dogs and cats; laboratory animals, including rodents such as rats, mice and guinea pigs; et al. Examples of non-mammals include, but are not limited to, birds, etc. The term "individual" does not define a particular age or sex. In some embodiments, the individual is a human.

In general, the term "about" is used in the present invention to adjust a given value to 20% above or below the value.

General method for synthesizing a compound of formula (I)

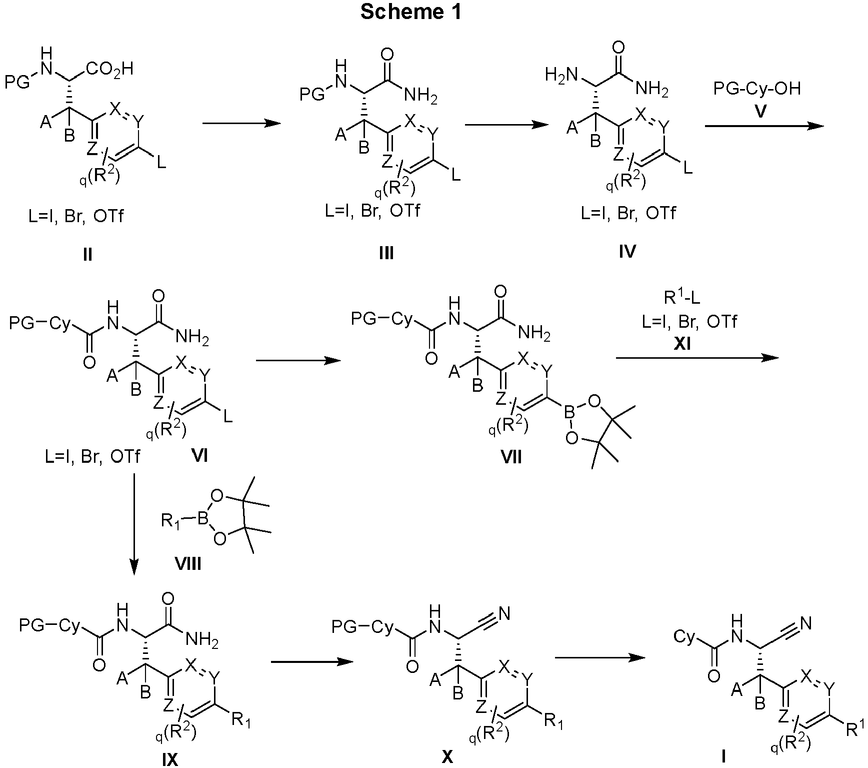

In Route 1, PG in the compound of formula II represents a protecting group, such as tert-butoxycarbonyl. As shown in Route 1, the compound of formula II is reacted with aqueous ammonia under basic conditions (e.g. With N, N-diisopropylethylamine as base), activated by 2-(7-azabenzotriazole)-N, N, N ', N'-tetramethyluronium hexafluorophosphate (HATU) or O-benzotriazole-tetramethyluronium hexafluorophosphate (HBTU) to give the compound of formula III. For the deprotection products of the compound of formula III, the compound of formula IV and the compound acid of formula V, the amide compound of formula VI is obtained by the conventional methods reported in literature, such as using N,N-diisopropylethylamine as base and HATU or HBTU as activating reagent.

The Suzuki coupling reaction of a compound of formula VI with a borate compound of formula VIII utilizes a palladium catalyst such as [1, 1 '-bis (diphenylphosphino) ferrocene] palladium dichloride in a suitable solvent such as dioxane, and using an appropriate base such as potassium acetate to give a compound of formula IX.

Another route is to convert a compound of formula VI into a boronate ester compound of formula VII. The compound of formula VI is reacted with divaleryldiboron in the presence of [1, 1 '-bis (diphenylphosphino) ferrocenyl] palladium dichloride catalyzed by potassium acetate as base in dimethyl sulfoxide to give the borate compound of formula VII. The Suzuki coupling reaction of a compound of formula XI with a borate compound of formula VII utilizes a palladium catalyst such as [1, 1 '-bis (diphenylphosphino) ferrocene] palladium dichloride in a suitable solvent such as dioxane, and using a suitable base such as potassium acetate to give a compound of formula IX.

The amide of the compound of formula IX can be dehydrated in methylene chloride utilizing methyl N-(triethylammonium sulfonyl) carbamate to give the compound of formula X. The deprotection reaction of the compound of formula X gives the final product the compound of formula I.

Compounds of formula (I) described in the present invention (e.g., any of the compounds of the present invention) and/or pharmaceutically acceptable salts thereof may be formulated into pharmaceutical compositions alone or in combination with one or more additional active ingredients. Pharmaceutical compositions include the following: (a) an effective amount of one compound of formula (I) described in the present invention and/or one pharmaceutically acceptable salt thereof; and (b) a pharmaceutically acceptable excipient (e.g., a pharmaceutically acceptable carrier).

A pharmaceutically acceptable carrier refers to a carrier that is compatible (in some embodiments, it stabilizes the active ingredient) with the active ingredient in the composition and is harmless to the individual being treated. For example, solubilizers including cyclodextrins, which are capable of forming specific, more soluble complexes with compounds of formula (I) described in the present invention and/or pharmaceutically acceptable salts thereof, can be used as pharmaceutical excipients to deliver active ingredients. Examples of other carriers include colloidal silicon dioxide, magnesium stearate, cellulose, sodium lauryl sulfate, and pigments including D&C Yellow #10. Suitable pharmaceutically acceptable carriers are disclosed in a reference to a standard in the art (Remington's Pharmaceutical Sciences, A. Osol).

Pharmaceutical compositions including a compound of formula (I) described in the present invention (e.g., any of the compounds herein) and/or a pharmaceutically acceptable salt thereof may be administered in various known ways, such as oral, topical, rectal, parenteral, inhalation, or implantation, and the like. The term "parenteral" used in the present invention includes subcutaneous, intradermal, intravenous, intramuscular, intra-articular, intra-arterial, intra-synovial, intra-sternal, intra-spinal, intra-affected site, and intracranial injections or infusions.

The pharmaceutical compositions described in the present invention can be prepared in the form of tablets, capsules, sachets, dragees, powders, granules, buccal tablets, powder injections, liquid preparations or suppositories. In some embodiments, pharmaceutical compositions including a compound of formula (I) and/or a pharmaceutically acceptable salt thereof may be prepared in a form for intravenous drip, topical administration, or oral administration.

The orally administered composition may be in any orally acceptable dosage form including, but not limited to: tablets, capsules, emulsions, and aqueous suspensions, dispersions, and solutions. Common tablet carriers include lactose and corn starch. Lubricants including magnesium stearate are also often added to the tablets. For oral administration in capsule form, useful diluents include lactose and dried corn starch. For oral administration in an aqueous suspension or emulsion form, an emulsifier or suspending agent may be used to suspend or dissolve the active ingredient in the oil phase. Certain sweeteners, flavoring agents or pigment may also be added if necessary.

In some embodiments, the amount of a compound of formula (I) and/or a pharmaceutically acceptable salt thereof in the tablet may be 1, 5, 10, 15, 20, 25, 50, 75, 80, 85, 90, 95, 100, 125, 150, 200, 250, 300, 400 and 500 mg. In some embodiments, the amount of a compound of formula (I) and/or a pharmaceutically acceptable salt thereof in the capsule may be 1, 5, 10, 15, 20, 25, 50, 75, 80, 85, 90, 95, 100, 125, 150, 200, 250, 300, 400 and 500 mg.

Sterile injectable compositions, including aqueous or oily suspensions, may be prepared according to techniques known in the art, by using a suitable dispersing or wetting agent (e.g., Tween 80) together with a suspending agent. The sterile injectable intermediate medium may also be a sterile injectable solution or suspension in a non-toxic parenterally acceptable diluent or solvent, including a solution in 1, 3-butanediol. The pharmaceutically acceptable carriers and solvents are mannitol, water, Ringer's solution and physiological saline which may be used in particular. In addition, sterile non-volatile oils, including synthetic monoglycerides or diglycerides, are commonly used as solvents or suspension media. Fatty acids, including oleic acid and glyceride derivatives thereof, as well as natural pharmaceutically acceptable oils, including olive oil or castor oil (especially polyoxyethylated forms thereof) are commonly used as injectable intermediate media. These oil solutions or suspensions may also contain long-chain alcoholic diluents or dispersants, or carboxymethyl cellulose or similar dispersants.

Inhalation compositions can be prepared according to techniques well known in the art of pharmaceutical preparations, or can be prepared as solutions in saline, by using benzyl alcohol or other suitable preservatives, using absorption enhancers that improve bioavailability, using fluorocarbons and/or other art-known solubilizers or dispersants.

The topical compositions may be prepared in the form of oils, creams, lotions, ointments, or the like. Suitable vehicles for use in the compositions include vegetable or mineral oils, white vaseline (white soft paraffin), branched chain fats or oils, animal fats, and alcohols of high molecular weight (i.e., alcohols having a number of carbon atoms greater than 12). In some embodiments, the pharmaceutically acceptable carrier is a carrier in which the active ingredient can be dissolved. The composition may further include emulsifiers, stabilizers, wetting agents, and antioxidants, as well as substances that impart color or fragrance thereto if necessary. In addition, a transdermal permeation enhancer may be added to the topical preparation. Examples of such enhancers can be found in US Patents No. 3,989,816 and 4,444,762.

The cream may be prepared from a mixture of mineral oil, self-emulsifying beeswax and water in which the active ingredient dissolved in a small amount of grease, such as almond oil, is mixed. An example of a cream includes, by weight, about 40 portions of water, about 20 portions of beeswax, about 40 portions of mineral oil, and about 1 portion of almond oil. The ointment may be prepared by mixing a solution of the active ingredient in a vegetable oil, such as almond oil, with warm soft paraffin and cooling the mixture. An example of an ointment includes, by weight, about 30% almond oil and about 70% white soft paraffin.

Suitable in vitro experiments can be used to evaluate the practical use of the compounds of formula (I) described in the present invention and/or their pharmaceutically acceptable salts in the inhibition of cathepsin C activity. Further practical use of the compounds of formula (I) described in the present invention and/or pharmaceutically acceptable salts thereof in the treatment of pulmonary diseases, inflammatory diseases, metabolic diseases, infectious diseases, cardio-cerebrovascular diseases or autoimmune diseases can be further tested by in vivo tests. For example, a compound of formula (I) and/or a pharmaceutically acceptable salt thereof according to the present invention can be administered to an animal (e.g., a mouse model) suffering from pulmonary diseases, inflammatory diseases, metabolic diseases, infectious diseases, cardiovascular and cerebrovascular diseases, cancer, autoimmune diseases, and then the therapeutic effect thereof can be evaluated. If the results of preclinical tests are successful, the dose range and administration route thereof to an animal, e.g., a human can also be predicted; Or it is administered to non-disease model animals (such as rats), and its inhibitory effect on serine protease downstream of cathepsin C is evaluated to predict its benefit to lung diseases such as bronchiectasis, for which there is no proven disease model.

The compounds of formula (I) and/or pharmaceutically acceptable salts thereof described in the present invention may be shown to have sufficient preclinical practical use to merit clinical trials and are expected to show beneficial therapeutic or prophylactic effects. For example, in individuals suffering from pulmonary diseases and inflammatory diseases.

The term "lung disorder" refers to a pathological state associated with lungs. Non-limiting examples of such diseases include bronchiectasis, idiopathic pulmonary fibrosis, pulmonary hypertension, asthma, chronic obstructive pulmonary disease, pneumonia, acute lung injury, acute respiratory distress syndrome.

The term "inflammatory disease" or "inflammatory disease" refers to a pathological state that leads to an inflammatory response, especially as a result of neutrophil chemotaxis. Non-limiting examples of such diseases include inflammatory skin diseases (including psoriasis and atopic dermatitis); systemic scleroderma and sclerosis; reactions related to inflammatory bowel disease (ibd) such as crohn's disease and ulcerative colitis; ischemia-reperfusion injury, including tissue reperfusion injury caused by surgery, myocardial ischemia such as myocardial infarction, cardiac arrest, reperfusion after cardiac surgery and abnormal contractile response of coronary vessels after percutaneous coronary angioplasty, stroke and abdominal aortic aneurysm surgery; cerebral edema secondary to stroke; cranial trauma; hemorrhagic shock; asphyxia; adult respiratory distress syndrome; acute lung injury; behcet's disease; dermatomyositis; polymyositis; autoimmune diseases such as rheumatoid arthritis (ra); pulmonary inflammation, including pleurisy, alveolitis, vasculitis, pneumonia, chronic bronchitis, bronchiectasis, diffuse panbronchiolitis, hypersensitivity pneumonitis, idiopathic pulmonary fibrosis (ipf) and cystic fibrosis; other inflammation anca-associated inflammation, necrotizing crescentic glomerulonephritis associated with antineutrophil cytoplasmic antibodies.

The compounds of formula (I) and/or pharmaceutically acceptable salts thereof can be used to achieve beneficial therapeutic or prophylactic effects, such as those beneficial therapeutic or prophylactic effects in individuals suffering from metabolic diseases, infectious diseases, cardiovascular and cerebrovascular diseases, infectious diseases, cancer, autoimmune diseases.

The term "autoimmune disease" refers to a disease or condition caused by the body's immune response to its own antigens, resulting in damage to its own tissues or organs. Examples of autoimmune diseases include, but are not limited to: chronic obstructive pulmonary disease (COPD), allergic rhinitis, lupus erythematosus, myasthenia gravis, multiple sclerosis (MS), rheumatoid arthritis (RA), psoriasis, inflammatory bowel disease (IBD), asthma, andidiopathic thrombocytopenicpurpura, myeloid proliferative disorder, myelofibrosis, postpolycythemia vera/essential thrombocytosis myelofibrosis (PV/ET).

The term "metabolic diseases" refers to diseases caused by metabolic disorders or diseases related to metabolism. The non-limiting examples include non-alcoholic fatty liver disease, non-alcoholic steatohepatitis, AATD, obesity, diabetes, etc.

The term "infectious disease" refers to a pathological state resulting from viral, bacterial, or parasitic infections. Non-limiting examples of such diseases include leishmaniasis, COVID-19, and sepsis.

The term "cardiovascular and cerebrovascular diseases" refers to diseases related to the cardiovascular system and brain organs. Non-limiting examples of such diseases are ischemia reperfusion injury, acute brain injury, heart failure, myocarditis, myocardial infarction and the like.

The term "cancer" as used in the present invention refers to a cellular disorder characterized by uncontrolled or dysregulated cell proliferation, reduced cell differentiation, inappropriate ability to invade surrounding tissue, and/or ability to establish new growth foci at other sites. The term "cancer" includes, but is not limited to, solid tumors and hematologic malignancies. The term "cancer" includes cancer of the skin, tissues, organs, bones, cartilage, blood, and blood vessels. The term "cancer" includes both primary and metastatic cancers. Non-limiting examples of solid tumors include lung cancer such as non-small cell lung cancer (NSCLC).

In some embodiments, the inflammatory and autoimmune diseases include rheumatoid arthritis, chronic obstructive pulmonary disease (COPD), allergic rhinitis, asthma, lupus erythematosus, systemic lupus erythematosus, psoriasis, and multiple sclerosis.

In addition, compounds of formula (I) described in the present invention (e.g., any of the compounds in the present invention) and/or pharmaceutically acceptable salts thereof may be used in combination with additional active ingredients for the treatment of respiratory diseases, inflammatory or autoimmune diseases and cancer. Additional active ingredients may be administered separately from the compounds of formula (I) described in the present invention and/or pharmaceutically acceptable salts thereof, or may be included in pharmaceutical compositions such as fixed-dose combination medicinal products according to the present disclosure. In a typical embodiment, additional active ingredients are those that are known or have been found to be effective in the treatment of diseases mediated by cathepsin C and its downstream serine protease activity, such as another cathepsin C modulator or a compound effective in antagonizing another target associated with that particular disease. The drug combination may be used to increase effectiveness (e.g., by including into the drug combination a compound that enhances the potency or efficacy of a compound of formula (I) described in the present invention and/or a pharmaceutically acceptable salt thereof), to reduce one or more side effects, or to reduce the required dose of a compound of formula (I) described in the present invention and/or a pharmaceutically acceptable salt thereof.

### Embodiment

The following embodiments are exemplary illustrations of the present invention and do not limit the present invention in any way. Unless otherwise stated, all fractions are weight fractions and the temperature is Celsius temperature. The pressure is atmospheric pressure or near-atmospheric pressure. All data are measured by Agilent 6120 and/or 1100. With the exception of synthetic intermediates, all reagents used in the present invention are obtained commercially. The names of all compounds except reagents are generated by ChemDrew 20.0.

The following abbreviations are used:
- ACN: Acetonitrile
- Boc: Tert-butoxycarbonyl
- (Boc)₂O: Di-tert-butyl dicarbonate
- BH₃: Borane
- DAST: Diethylaminosulfur trifluoride
- DCM: Dichloromethane
- DEA: Diethylamine
- DMF: N, N-Dimethylformamide
- DMA: Dimethylacetamide
- DIBAL-H: Diisobutylaluminum hydride
- DIEPA: N, N-Diisopropylethylamine
- EDCI: 1-(3-Dimethylaminopropyl)-3-ethylcarbodiimide hydrOCHloride
- EA: Ethyl acetate
- Et3N: Triethylamine
- HATU: 2-(7-azabenzotriazole)-N, N, N ', N'-tetramethylurea hexafluorophosphate
- HBTU: O-Benzotriazole-tetramethylurea hexafluorophosphate
- HOAc: Acetic acid
- HOBt: 1-Hydroxybenzotriazole
- ee: Enantiomeric excess
- mL: Milliliters
- g: Gram
- mg: Milligram
- ng: Nanogram
- mol: Mole
- mmol: Millimolar
- h: Hour
- MeOH: Methanol
- NaH: Sodium hydride
- NCS: N-Chlorosuccinimide
- NMP: N-methyl-2-pyrrolidone
- PE: Petroleum ether
- Pd(dppf)₂Cl₂: [1, 1 '-bis (diphenylphosphino) ferrocene] palladium dichloride
- Pd₂(dba)₃: Tri(dibenzylideneacetone) dipalladium
- Pd(PPh₃)₄: Tetrakis (triphenylphosphine) palladium
- PMB: PMBp-methoxyphenylacetonitrile
- PPh₃: Triphenylphosphin
- Pin₂B₂: Pinacol borate
- THF: Tetrahydrofuran
- TFA: Trifluoroacetic acid
- TsOH: 4-Toluene sulfonic acid
- Xphos: 2-dicyclohexylphosphine-2 ', 4', 6 '-triisopropylbiphenyl

Burgess reagent methyl N-(triethylammonium sulfonyl) carbamate 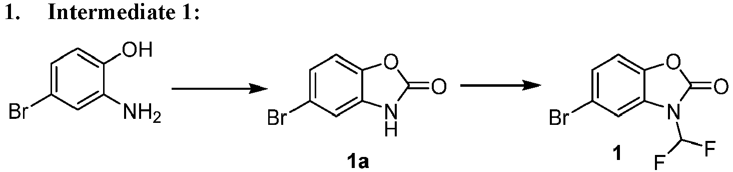
a) Under the protection of nitrogen at room temperature, CDI (20.7 g, 127.7 mmol) is added to the 2-Methf (300 ml) solution of compound 2-amino -4- bromophenol (20.0 g, 106.4 mmol), and then it is refluxed for 1 hour, is cooled to room temperature, and is used with 2M HCl (aq.) (300 mL), 8% NaHCO₃ (aq.) (300 mL) and saturated brine (150 mL) in turn, dried with Na₂SO₄, and concentrated to give a light brown solid product **1a** (21.9 g, 96%). MS (ESI): m/z =215.9 [M+H]⁺. ¹H NMR (400 MHz, DMSO-*d*₆): δ 11.85 (s, 1 H); 7.26-7.23 (m, 3 H).
b) To a solution of compound **1a** (1.0 g, 4.7 mmol) in DMF (15.0 mL) at room temperature under nitrogen, sodium chlorodifluoroacetate (3.6 g, 23.5 mmol), Ss (6.0 g, 23.5 mmol), sodium tert-butoxide (1.8 g, 18.8 mmol), 4 Å molecular sieve (200 mg, dry powder) are added sequentially. Then heat it to 70 °C and stir for 6 hours. Cool to room temperature, filter to remove molecular sieves, wash with EA (50 mL), further dilute with EA (100 mL), wash with saturated brine (50 mL × 5), dry with Na₂SO₄, and concentrate to obtain a crude product. The crude product is subject to preparative HPLC purification (TFA as buffer: A: 0.05% TFAaqueous solution; B: 0.05% TFA in acetonitrile; Column: Waters XBridge Peptide BEH C18, 19 × 250 mm, 10 µ m, 130 Å) to obtain a white solid product 1 (660 mg, 53%). MS (ESI): m/z =266.0 [M+H]⁺. ¹H NMR (400 MHz, DMSO-*d*₆): δ 7.73 (t, *J* = 57.4 Hz, 1 H), 7.58-7.57 (m, 1H), 7.50 (dd, *J* = 8.4, 2.0 Hz, 1 H), 7.45 (d, *J* = 8.4 Hz, 1H).

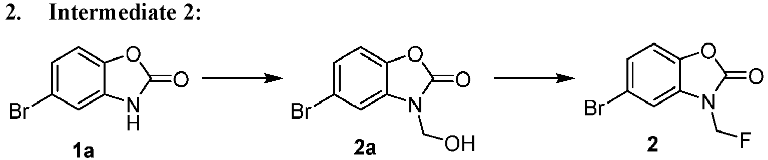

a) Add **1a** (1.0 g, 4.7 mmol) and formaldehyde (37% aqueous solution, 610 µL) to water (5 mL) at room temperature. The reaction solution is stirred overnight at 80 °C, then cooled to room temperature, filtered, and washed with water (3 × 10 mL). The resulting crude product is dried in vacuo to give a light brown solid **2a** (960 mg, 84%). MS (ESI): m/z =228.1 [M+H]⁺. ¹H NMR (400 MHz, DMSO-*d*₆): δ 7.60 (t, *J* = 1.2 Hz, 1 H); 7.33 (br, 3 H); 5.21 (s, 2H).
b) To a solution of **2a** (312 mg, 1.3 mmol) in dichloromethane (5 mL), DAST (265 µ L, 2.0 mmol) is added dropwise under nitrogen atmosphere at-50 °C. After addition, the reaction solution is slowly brought to room temperature and stirred overnight, then quenched with saturated NaHCO3 (10 mL), extracted with dichloromethane, washed with saturated brine (10 mL), dried with Na2SO4, and concentrated to give a light brown solid **2** (306 mg, 95%). MS (ESI): m/z =246.1 [M+H]⁺. ¹H NMR (400 MHz, DMSO-*d*₆): δ 7.86 (brs, 1 H); 7.41-7.39 (m, 2 H); 6.02 (d, *J* = 52 Hz, 2H).

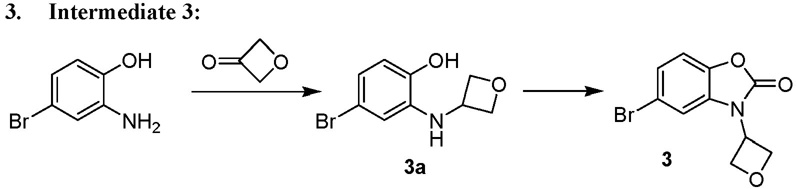

a) Compound 2-amino-4-bromophenol (1.0 g, 5.3 mmol) and compound 3-oxetidinone (764 mg, 10.6 mmol) are dissolved in dry THF (15.0 mL) at room temperature. After cooling in an ice bath, BH₃·THF (1M, 5.3 mL) is slowly added dropwise. After addition, the reaction solution is allowed to rise to room temperature, and stirring continues for 6 h. Then, compound 3-oxetidinone (764 mg, 10.6 mmol) and BH₃·THF (1M, 5.3 mL) are added and stirred overnight. The reaction solution is cooled in an ice bath, and excess BH₃ is quenched by dropwise addition of aqueous NaOH solution (1 M, 20 mL). The organic phase is separated, dried with Na₂SO₄, and concentrated to give a crude product. A light brown solid product **3a** (640 mg, 50%). is obtained by column chromatography (eluted with DCM/MeOH = 50/1). MS (ESI): m/z = 246.2 [M+H]⁺. ¹H NMR (400 MHz, DMSO-*d*₆): δ 9.64 (s, 1 H), 6.61-6.56 (m, 2 H), 6.32-6.28 (m, 1 H), 5.6 (brs, 1 H), 4.82-4.78 (m, 2 H), 4.53-4.45 (m, 3 H).
b) Under the protection of nitrogen at room temperature, CDI (535 mg, 3.3 mmol) is added to the solution of compound **3a** (603 mg, 2.5 mmol) in 2-MeTHF (10 mL). After refluxing for one hour, the reaction solution is brought to room temperature and subsequently treated with 2M HCl (aq.) (15 mL), 8% NaHCO₃ (aq.) (15 mL) and saturated saline (8 mL). The organic phase is separated, dried with Na₂SO₄, and concentrated to obtain a crude product. A light brown solid 3 (230 mg, 35%) is obtained by column chromatography (eluent PE/EA = 10: 1 ~ 2: 1, V/V). MS (ESI): m/z = 270.0 [M+H]⁺. ¹H NMR (400 MHz, DMSO-*d*₆): δ 7.68-7.66 (m, 1 H), 7.36-7.35 (m, 2 H), 5.42-5.37 (m, 1 H), 5.04-5.01 (m, 2 H), 4.93-4.88 (m, 2H).

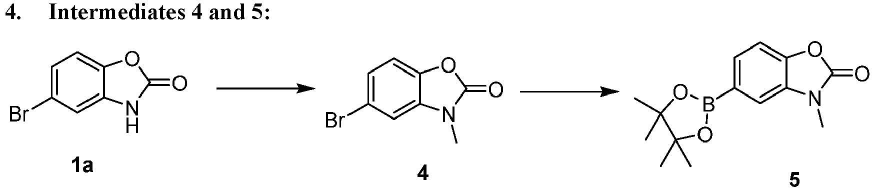

a) Add Cs₂CO₃ (1.6 g, 5.1 mmol) in DMF (15 mL) and MeI (658 µL, 10.2 mmol) to a DMF (15mL) solution of **1a** (1.1 g, 5.1 mmol) under nitrogen protection at 0 °C. Rise the temperature to room temperature and stir overnight. Cool to 0 °C and add water dropwise water (15 mL). Filter, wash with water and dry under vacuum at 55 °C to obtain a light brown solid **4** (826 mg, 71%). MS (ESI): m/z =230.3 [M+H]⁺. ¹H NMR (400 MHz, DMSO-*d*₆): δ 7.55-7.54 (m, 1 H), 7.29-7.28 (m, 2 H), 3.32 (s, 3 H).
b) Under the protection of argon gas, add 4 (228 mg, 1.0 mmol), Pin2B2 (305 mg, 1.2 mₘoₗ), Pd (OAc) 2 (7 mg, 0.03 mmol), _{X}Phos (29 mg, 0.06 mmol) and KOAc (294 mg, 3.0 mmol) to dioxane (5 mL), heat it to 75 °C, and keep stirring for 1 hour. Cool to room temperature, filter, and wash the solid with EA (3 × 5 mL). The filtrate is concentrated and purified by column chromatography (eluent PE/DCM = 0% ~ 100%, V/V) to obtain a gray solid product **5** (232 mg, 84.4%). MS (ESI): m/z = 276.4 [M+H]⁺. ¹H NMR (400 MHz, DMSO-*d*₆): δ 7.47 (dd, *J* = 8.0, 1.2 Hz, 1 H), 7.44 (s, 1 H), 7.33 (d, *J* = 8.0 Hz, 1 H), 3.35 (s, 3 H), 1.30 (s, 12 H).

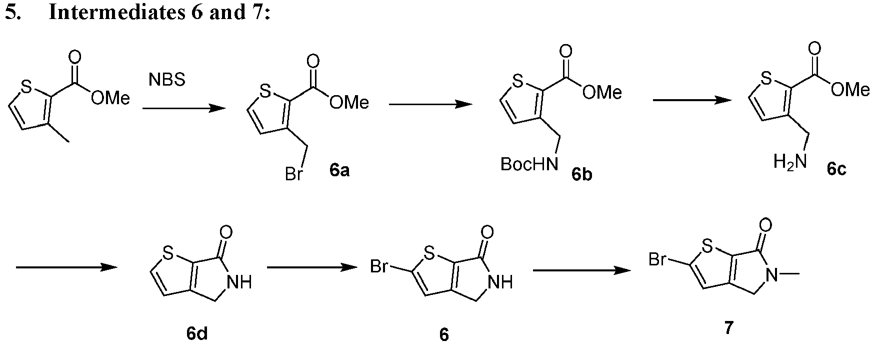

a) Add AIBN (524 mg, 3.2 mmol) to a suspension of methyl 3-methylthiophene-2-carboxylate (5 g, 32.05 mmol) and NBS (5.7 g, 32.02 mmol) in CC14 (80 mL) at 70 °C. Keep stirring for 3 hours, cool to 40 °C and add NBS (2.85 g, 16 mmol). Then increase the temperature to 70 °C and keep stirring for 1 hour. Cool to room temperature, filter to remove solids, spin-dry the filtrate, dissolve the resulting residue in EA (100 mL), wash successively with saturated NaHCO3 (30 mL) and water (30 mL), dry with Na₂SO₄, and concentrate to give the oily product **6a** (10 g). This crude product is used directly in the next reaction. MS (ESI): m/z = 155.4 [M-Br]⁺.
b) Add 7 N NH₃ in MeOH (100 mL) dropwise to a solution of **6a** (crude 10 g, approximately 32.05 mmol) in DMF (50 mL) at room temperature and keep stirring for 2 hours. Then add water H2O (200 mL) and EA (3 × 150 mL) to the reaction solution, and adjust the pH to pH 8 with solid NaHCO₃. Then, add dioxane (50 mL) and BoczO (5 g, 22.9 mmol) sequentially. Stir at room temperature for 3 hours. The dioxane was evaporated by concentration under reduced pressure, then it is extracted with EA (3 × 100 mL), dried with Na₂SO4, and concentrated to obtain the crude product. Purification by column chromatography (eluent PE: EA = 6: 1 ~ 5: 1 ~ 4: 1, product Rf = 0.7 in PE/EA 4/1) to obtain the oily product **6b** (4 g, with some BoczO). MS (ESI): m/z = 294.3 [M+Na]⁺.
c) Add TFA (20 mL) to a solution of **6b** (crude 4 g, about 14 mmol) in dichloromethane (60 mL) at room temperature and keep stirring for 1 hour. Solvent and TFA are concentrated and removed under reduced pressure. The residue obtained is dissolved in dichloromethane (100 mL) and spin dried again. The crude product is dissolved in MeOH (10 mL) and the pH is adjusted to pH10 by addition of 7 N NH3/MeOH. This liquid is purified by preparative HPLC (C18, CH₃CN, 10 mM NH₄HCO₃ in water) to give a pale white solid 6c (1.75 g, 4-step yield 31%). MS (ESI): m/z = 171.9 [M+H]⁺. ¹HNMR(400 MHz, CDCl₃): δ 7.94 (d, J = 4.8 Hz, 1H), 7.5 (brs, 2H), 7.32 (d, J = 4.8 Hz, 1H), 4.29 (s, 2H), 3.83 (s, 3H).
d) Mix compound 6c (1.55 g, 9.06 mmol) and K₂CO₃ (1.25 g, 9.06 mmol) in MeOH (120 mL) and EtOH (120 mL) and reflux overnight. Cool to room temperature and spin dry to obtain crude product. Purify to obtain a light brown solid product, 6d (680 mg, 53%) by column chromatography (eluent DCM:MeOH = 20:1 ~ 15:1, Rf = 0.2 in DCM/MeOH 20/1 solution). MS (ESI): m/z = 140.1 [M+H]⁺.
e) Drop slowly Br₂ (300 uL, 5.85 mmol) into a solution of **6d** (745 mg, 5.36 mmol) in AcOH (10 mL)/H₂O (10 mL) at 0 °C and continue stirring for 2 hours. Then, add 10% Na₂SO₃ (20 mL) followed by saturated NaHCO₃ and adjust the pH to pH 7. Extract with EA (3 × 50 ml), dry, concentrate light white solid 6 (1.09 g, 93%). MS (ESI): m/z = 218.2 [M+H]⁺. ¹HNMR (400 MHz, CD3OD): δ 7.29 (s, 1H), 4.39 (s, 2H).
f) Add NaH (60% in oil, 122 mg, 3.05 mmol) to a solution of 6 (600 mg, 2.75 mmol) in DMF (10 mL) at 0 °C. After stirring for half an hour, add dropwise MeI (205 uL, 3.29 mmol) and stir at room temperature for 2 hours. Quench the reacting liquid with water H₂O (100 mL), neutralize with saturated NH₄Cl solution to pH 7, extract with EA (50 mL* 3), dry with Na₂SO₄, and concentrate to obtain a crude product. Purify to obtain a light brown solid, 7 (400 mg, 62%) by column chromatography (EA:PE = 2:1 to EA, Rf = 0.5 in EA). MS (ESI): m/z = 232.1 [M+H]⁺.

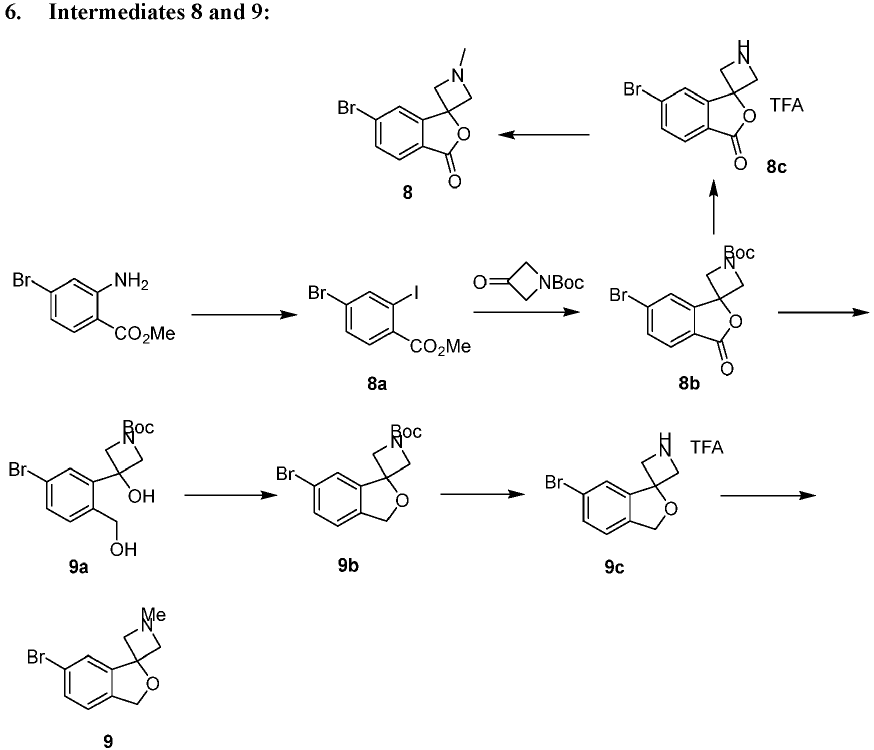

a) Add methyl 2-amino-4-bromobenzoate (20 g, 87 mmol) to a flask containing 10% H₂SO₄ (500 mL) at 0 °C, followed by 70 mL of NaNO₂ (12 g, 173.9 mmol) in water. After stirring for 40 minutes, add dropwise 70 mL of KI (57.73 g, 347.8 mmol) in water and continue stirring for 1 hour. Quench the reaction with saturated Na₂SO₃ (200 mL) and extract with EA (3 × 500 mL). Dry with Na₂SO₄ and concentrate to obtain crude product. Purify to obtain a oily product **8a** (25 g, 84%) by column chromatography (PE/EA = 10: 1, VN). MS (ESI): m/z = 342.8[M+H]⁺. ¹H NMR (400 MHz, CDCl₃): δ 8.19 (d, 1.6 Hz, 1 H), 7.71 (d, *J* = 8.4 Hz, 1 H), 7.57 - 7.54 (m, 1 H), 3.94 (s, 3 H).
b) Add slowly ⁱPrMgCl-LiCl (27.3 ml, 35.5 mmol, 1.3 mol/L in THF) dropwise at-70 °C to a solution of 8a (11 g, 32.3 mmol) and tert-butyl 3-oxoazetidine-1-carboxylate (6.08 g, 35.5 mmol) in THF (230 mL), and continue stirring for half an hour. Remove the dry ice bath, rise the temperature to room temperature, and continue stirring for 1 hour. Quench the reacting liquid with saturated aqueous NaHCO₃ (200 mL) and extract with EA (3 × 500 mL). Dry with Na₂SO₄ and concentrate to obtain crude product. Purify to obtain a yellow solid product **8b** (6 g, 53%) by column chromatography (eluent PE/EA = 10: 1, V/V). MS (ESI): m/z = 298.1 [M-55]⁺. ¹H NMR (400 MHz, CDCl₃): δ 7.93 - 7.91 (m, 1 H), 7.75 (d, *J* = 1.2 Hz, 2 H), 4.55 - 4.52 (m, 2 H),4.28 - 4.26 (m, 2 H), 1.51 (s, 9 H).
c) Add TFA (2 mL) to a solution of **8b** (0.8 g, 2.35 mmol) in dichloromethane (20 mL) at room temperature. After stirring for 7 hours, concentrate the brown oily product **8c** (1 g, 91%) under reduced pressure. MS (ESI): m/z = 256.0 [M+H]⁺.
d) Add 8c (1 g, 2.82 mmol) and paraformaldehyde (1.69 g, 56.3 mmol) to MeOH (40 mL) and AcOH (5 mL) at room temperature, after stirring for 1 hour, add NaBH(OAc)₃ (600 mg, 2.82 mmol), and continue stirring for half an hour. Repeat the step 9 times, followed by stirring overnight. Concentrate the reacting liquid under reduced pressure, add saturated NaHCO₃ (150 mL) to the residue, extract with EA (3 × 200 mL), dry with Na₂SO₄, and concentrate to obtain a crude product. Purify to obtain a white solid product, 8 (400 mg, 66.7%) by column chromatography (eluted with PE/EA = 3:1 to 1:1, V/V). MS (ESI): m/z = 270.0 [M+H]⁺. ¹H NMR (400 MHz, CDCl₃): δ 8.15 - 8.14 (m, 1 H), 7.70 - 7.69 (m, 2 H), 3.74 - 3.72 (m, 2 H), 3.67 - 3.65 (m, 2 H), 2.52 (s, 3 H).
e) Add LiBH₄ (1.49 g, 67.8 mmol) in THF (100 mL) to a solution of 8b (6 g, 16.9 mmol) in THF (100 mL) at room temperature. After stirring for 24h, quench the reacting liquid with water (200 mL), extract with EA (3 × 500 mL), dry with Na₂SO₄, and concentrate to obtain crude 9a. Preparative HPLC purification (use NH₄HCO₃ aqueous solution: A as buffer: 10 mM NH₄HCO₃ aqueous solution; B: acetonitrile; Column: Waters XBridge Peptide BEH C18, 19 × 250 mm, 10 µm, 130 Å) to obtain a white solid product **9a** (4.5 g, 75%). MS (ESI): m/z = 380.2 [M+Na]⁺.
f) Add p-toluenesulfonic anhydride (2.73 g, 8.4 mmol) batch wise to a solution of **9a** (2.5 g, 7.0 mmol) and TEA (3.53 g, 35 mmol) in THF (100 mL) at 0 °C. After stirring for 30 min, rise the temperature to room temperature and continue stirring for 48 h. Concentrate under reduced pressure, add water (200 mL), extract with EA (3 × 200 mL), dry with Na₂SO₄, and concentrate to obtain a crude product. Purify to obtain a white solid product **9b** (1.9 g, 80%) by column chromatography (eluted with PE/EA = 5:1, V/V). MS (ESI): m/z = 284.2 [M-55]⁺. ¹H NMR (400 MHz, CDCl₃): δ 7.62 (d, *J* = 1.6 Hz, 1 H), 7.48 - 7.46 (m, 1 H), 7.10 (d, *J* = 8.0 Hz, 1 H), 5.07 (s, 2 H), 4.32 (d, *J* = 10.0 Hz, 2 H), 4.13 (d, *J* = 10.0 Hz, 2 H), 1.50 (s, 9 H).
g) Add TFA (3 mL) dropwise to a solution of **9b** (1 g, 2.95 mmol) in dichloromethane (20 mL) at room temperature. After stirring for 3 h, concentrate under reduced pressure to obtain brown oil **9c** (1.05 g, 99%) MS (ESI): m/z = 242.1 [M+H]⁺.
h) Add **9c** (1.05 g, 2.97 mmol) and paraformaldehyde (1.78 g) to a flask containing MeOH (60 mL) and AcOH (10 mL) at room temperature, after stirring for 1 h, add NaBH(OAc)₃ (629 mg, 2.97 mmol), and continue stirring for 30 minutes. Repeat the step 9 times and stir the reacting liquid overnight. Concentrate under reduced pressure, add saturated NaHCO₃ (200 mL), extract with EA (3 × 200 mL), dry with Na₂SO₄, and concentrate to obtain a crude product. Purify to obtain a colorless oil product 9 (650 mg, 86%) by column chromatography (eluent PE/EA = 2:1 ~ 1:1, V/V). MS (ESI): m/z = 256.0 [M+H]⁺. ¹H NMR (400 MHz, CDCl₃): δ 7.88 (d, *J* = 1.6 Hz, 1 H), 7.45 - 7.42 (m, 1 H), 7.06 (d, *J* = 8.0 Hz, 1 H), 5.02 (s, 2 H), 3.69 - 3.66 (m, 2 H), 3.43 - 3.40 (m, 2 H), 2.46 (s, 3 H).

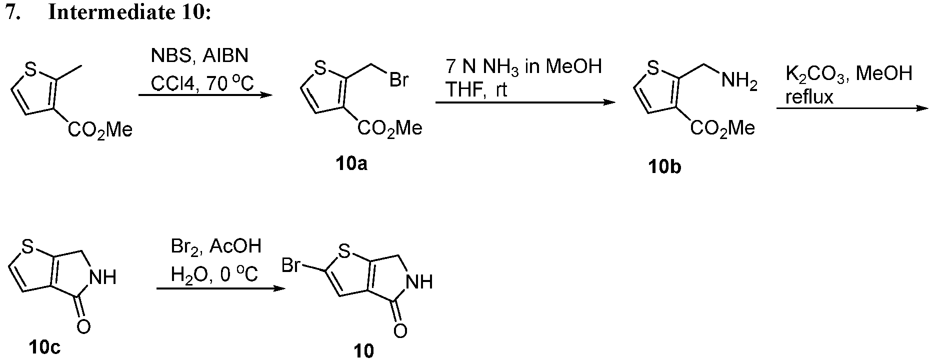

a) Add AIBN (840 mg, 5.1 mmol) to a suspension of methyl 2-methylthiophene-3-carboxylate (8 g, 51.3 mmol)/NBS (9.1 g, 51.1 mmol) in carbon tetrachloride (120 mL) at 70 °C. Stir the reaction mixture at 70 °C for 4 h. Cool the reaction mixture to room temperature and filter. Wash the filtrate with saturated NaHCO₃ (50 mL)/water (50 mL). Separate the organic layer, dry with Na₂SO₄, filter and evaporate to obtain a light brown oily **10a** crude product (13g). Use the crude product directly in the next step. MS (ESI): m/z = 155.4 [M-Br]+.
b) Add a solution of 7N NH₃ (30 mL, 210 mmol) in MeOH to a solution of 10a (crude **13g**, approx. 51.3 mmol) in THF (100 mL). Stir the reaction mixture at room temperature for 1 h. Evaporate the reaction mixture and wash the residual light brown solid with EA/MeOH 10/1 (50 mL) and filter to obtain a white solid **10b** (3.5 g, yield 39%). MS (ESI): m/z = 172.0 [M+H]+. ¹HNMR (400 MHz, CD3OD): δ 7.59-7.55 (m, 2 H), 4.61 (s, 2 H), 3.94 (s, 3 H)
c) Reflux a solution of a 10b (3.3 g, 19.3 mmol)/K₂CO₃ (2.66 g, 19.3 mmol) mixture in MeOH (120 mL) for 24 h, after which cool the reaction mixture to room temperature and evaporate. Separate the residue by silica gel chromatography (DCM:MeOH=20:1, Rf=0.5 in DCM/MeOH 20/1) and purify to obtain a **10c** white solid (890 mg, 33%). MS (ESI): m/z = 140.4 [M+H]+. ¹HNMR (400 MHz, MeOD): δ 7.56 (d, *J* = 5.2 Hz, 1 H), 7.21 (d, *J* = 5.2 Hz, 1 H), 4.53 (s, 2 H).
d) Add Br₂ (320 uL, 6.24 mmol) to a solution of 10c (0.86 g, 6.19 mmol) in AcOH (10 mL)/H₂O (10 mL) at 0 °C. Then stir the reaction mixture at 0 °C for 1 h. Add Br₂ (40 uL, 0.78 mmol) to the reaction mixture and stir for an additional 1 h at 0 °C. Add 10% Na₂SO₃ (20 mL) to the reaction mixture until it is colorless, then add saturated NaHCO₃ to pH7 and extract with EA (3 × 50 mL). Combine the EA layers, dry with Na₂SO₄, filter, and evaporate. Wash the residue with EA (10 mL)/PE (5 mL) to obtain **10** white solid (1.22 g, yield 90%). MS (ESI): m/z = 218.2 [M+H]+. ¹HNMR (400 MHz, CD3OD): δ 7.27 (s, 1 H), 4.49 (s, 2 H).

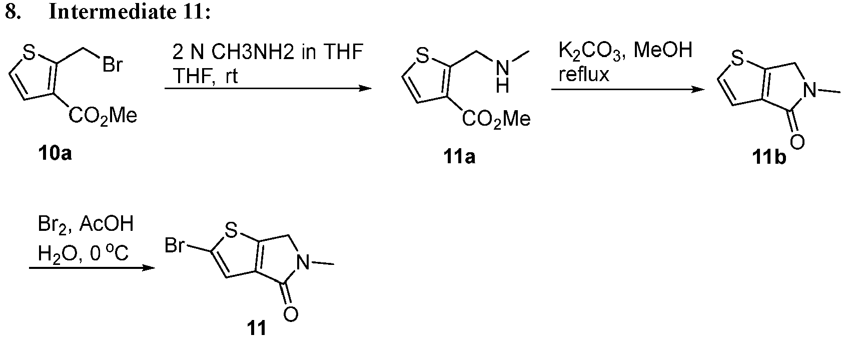

a) Add CH₃NH₂ (2N, THF solution, 20 mL, 40 mmol) to a solution of **10a** (crude 3g, approximately 11.54 mmol) in THF (20 mL). Stir the reaction mixture at room temperature for 2 h. Filter the reaction mixture and evaporate the filtrate. Purify the residue by silica gel chromatography (EA: PE = 1:1 ~ 2:1 ~ 100%EA, Rf=0.1 in EA) to obtain a brown oily **11a** (1.4 g, yield 66% in 2 steps). MS (ESI): m/z = 186.4 [M+H]⁺.
b) Reflux a mixture of **11a** (1.4 g, 7.57 mmol)/K₂CO₃ (1.05 g, 7.6 mmol) in MeOH (40 mL) overnight. Cool the reaction mixture to room temperature and evaporate to dryness. Add water (10 mL) to the residue and extract with EA (3 × 20 mL). Combine all EA layers, dry with Na₂SO₄, filter, and evaporate. Use silica gel chromatography (EA:PE = 1:1~2:1~100%EA, Rf--0.5 in EA/PE 2/1) to obtain **11b** as a white solid (500mg, 43%). MS (ESI): m/z = 154.3 [M+H]⁺.¹HNMR (400 MHz, MeOD): δ 7.55 (d, *J* = 4.8 Hz, 1 H), 7.19 (d, *J* = 4.8 Hz, 1 H), 4.56 (s, 2 H), 3.17 (s, 3 H).
c) Add Br₂ (168 uL, 3.27 mmol) to a solution of 11b (500 mg, 3.27 mmol) in AcOH (5 mL)/H₂O (5mL) at 0 °C. Stir the reaction mixture at 0 °C for 1h. Add Br₂ (35 uL, 0.68 mmol) to the reaction mixture and stir for an additional 2h at 0 °C. Add 10% Na₂SO₃ (10 mL) to the reaction mixture until it is colorless, then add the saturated solution. Add NaHCO; to pH7 and extract with EA (3 × 30 mL). Combine the EA layers, dry with Na₂SO₄, filter, and evaporate. Purify the residue by silica gel chromatography (EA:PE = 1:1∼2:1, Rf=0.4 in EA/PE 2/1) to obtain **11** as a light solid (400 mg, 52%).

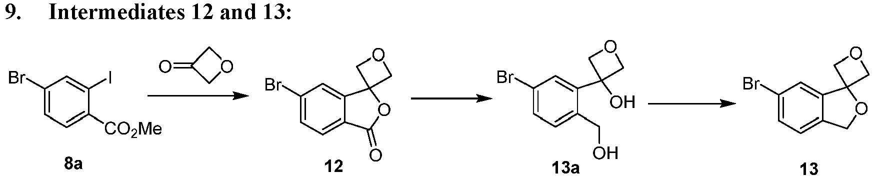

a) Add dropwise iPrMgCl-LiCl (2.5 mL, 3.2 mmol, 1.3 mol/L in THF) to a solution of compound **8a** (1 g, 2.9 mmol) and 3-oxetanone (232 mg, 3.2 mmol) in THF (25 mL) at -70 °C. After stirring for half an hour, rise the temperature to room temperature, add saturated NaHCO₃ (40 mL), extract with EA (3 × 100 mL), dry with Na₂SO₄, and concentrate to obtain a crude product. Preparative HPLC purification (TFA as buffer: A: 0.05% TFA aqueous solution; B: Acetonitrile; Column: Waters XBridge Peptide BEH C18, 19 × 250 mm, 10 µm, 130 Å)) to obtain a white solid product **12** (300 mg, 40%). MS (ESI): m/z = 257.0 [M+H]⁺. ¹H NMR (400 MHz, CDCl₃): δ 8.12 (s, 1 H), 7.77 - 7.73 (m, 2 H), 5.24 (d, *J* = 8.0 Hz, 2 H), 4.91 (d, *J* = 7.6 Hz, 1 H).
b) Add LiBH₄ (621 mg, 28.2 mmol) to a solution of compound **12** in THF (70 mL) at room temperature. After stirring for 24 h, add water (200 mL), extract with EA (3 × 300 mL), dry with Na₂SO₄, and concentrate to obtain a crude product. Purify to obtain a white solid product **13a** (1.6 g, 89%) by column chromatography (eluent PE/EA = 2:1, V/V). MS (ESI): m/z = 281.0 [M+Na]⁺.
c) Add p-toluenesulfonic anhydride (1.51 g, 4.6 mmol) batch wise to a solution of **13a** (1 g, 3.9 mmol) and TEA (19.5 g, 19.3 mmol) in THF (50 mL) at 0 °C. After stirring for half an hour, rise the temperature to room temperature and continue stirring for 48 hours. Concentrate under reduced pressure, add water (100 mL), extract with EA (3 × 300 mL), dry with Na₂SO₄, and concentrate to obtain a crude product. Purify to obtain a white solid product **13** (440 mg, 47%) by column chromatography (eluent PE/EA = 10: 1, V/V). MS (ESI): m/z = 243.1 [M+H]⁺. ¹H NMR (400 MHz, DMSO-*d*₆): δ 7.88 (d, *J* = 1.6 Hz, 1 H), 7.56 - 7.54 (m, 1 H), 7.28 (d, *J* = 8.0 Hz, 1 H), 5.00 (s, 2 H), 4.83 - 4.77 (m, 4 H).

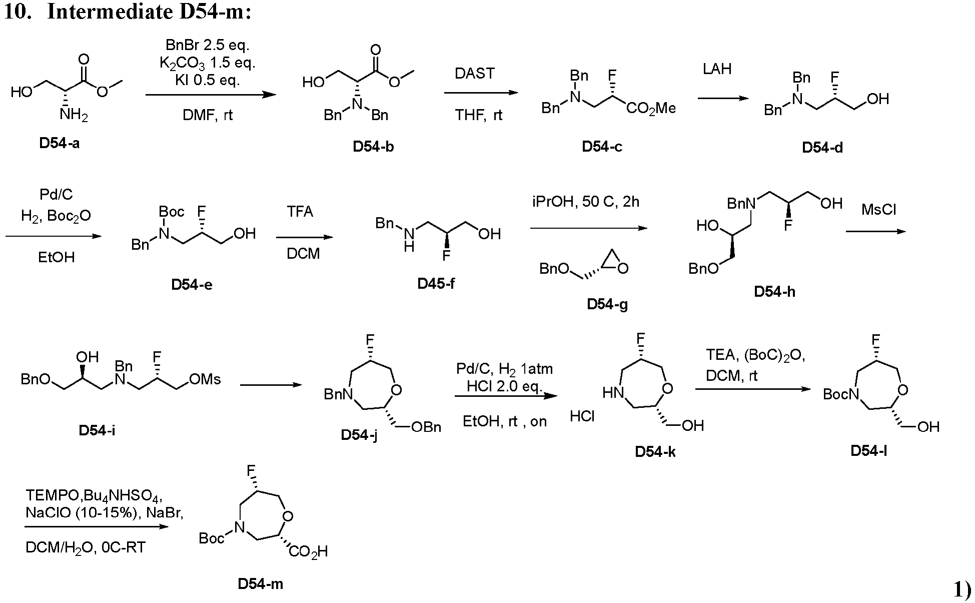

### Preparation of dibenzyl-D-serine methylester (D54-b):

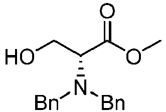

Add anhydrous K₂CO₃ (31.0 g, 224.1 mmol), D-serine methyl ester hydrOCHloride (**D54-a**, 23.25 g, 149.4 mmol), KI (12.4 g, 74.7 mol), and benzyl bromide (44.4 mL, 373.6 mol) to DMF (250 mL) sequentially at room temperature. Stir overnight at room temperature and then pour the reacting liquid into water (1 L). Extract with ethyl acetate (500 mL × 3), wash sequentially with water (300 mL) and saturated saline (300 mL), dry with anhydrous Na₂SO₄, concentrate, and purify PE/EA (9/1 to 8/2) by column chromatography to obtain a colorless oily liquid product (**D54-b**, 42.3 g, 88%). MS (ESI): m/z = 300.2 [M+H]⁺.

### 2) Preparation of (S)-3-(dibenzylamino)-2-fluoropropanoic acid methyl ester (D54-c):

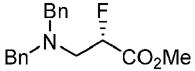

Add **54-b** (41.3 g, 138 mmol) to a solution of diethylamine sulphur trifluoride (18.23 mL, 138 mmol) in THF (500 mL) over 3 hours at room temperature. After stirring at room temperature for 1 hour, add ethyl acetate (500 mL) and neutralize to pH 7 with saturated NaHCO₃ solution. Dry with anhydrous Na₂SO₄, concentrate, and purify PE/EA (9/1 to 8/2) by column chromatography to obtain a colorless oily liquid product (**D54-c**, 38.1 g, 92%).

### 3) Preparation of (S)-3-(dibenzylamino)-2-fluoropropanol (D54-d):

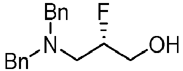

Add dropwise LAH (60 mL, 2.5 M in THF, 150 mmol) to a solution of **54-c** (37.8 g, 125 mmol) in THF (500 mL) at 0 °C. After 1 hour of reaction, cautiously add 5.7 mL of water. After 30 minutes of reaction, add 5.7 mL of 15% NaOH. After stirring for 10 minutes, add 17 mL of water. Add anhydrous Na₂SO₄, filter off the solid, and concentrate to obtain a colorless oily liquid product (**D54-d** , 34.2 g, 99% ). MS (ESI): m/z = 274.1 [M+H]⁺.

### 4) Preparation of (S)-benzyl (2-fluoro-3-hydroxypropyl) tert-butyl carbamate (D54-e):

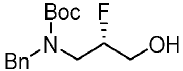

Place a mixture of **54-d** (33.9 g, 124 mmol) and 10% Pd/C (2.4 g) in ethanol (500 mL) in hydrogen at 1 atmosphere at room temperature. Allow to react overnight, filter off the solid and concentrate to obtain a colorless oily liquid product. Purify by column chromatography to obtain (**D54-e**, 33.6 g, 95%). MS (ESI): m/z = 306.2 [M+Na]⁺.

### 5) Preparation of (S)-3-(benzylamino)-2-fluoro-1-propanol (D54-f):

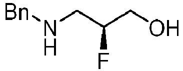

Add (20 mL) to **D54-e** (10 g, 35.3 mmol) in DCM (120 mL) at room temperature. After reaction for 3 hours, concentrate, dissolve with DCM, add 30 g K₂CO₃. After stirring for 20 minutes, filter off the solid, concentrate, and purify by reverse-phase column chromatography to obtain (C18, using 10 nm NH₄HCO₃ buffer) (**D54-f**, 6.5 g, 91%). MS (ESI): m/z = 184.1 [M+H]⁺.

### 6) Preparation of (S)-3-(benzyl ((S)-3-(benzyloxy)-2-hydroxypropyl) amino)-2-fluoro-1-propanol (D54-h):

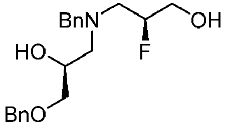

Mix **54-f** (6.3 g, 34.4 mmol) and (S)-2-((benzyloxy)methyl)oxirane (D54-g, 6.77 g, 41.3 mmol) in iPrOH (500 mL) at room temperature. Stir overnight at 55 °C. Cool to room temperature, concentrate, and purify by column chromatography to give a colorless oily product (**D54-h**, 4.5 g, 38%). MS (ESI): m/z = 348.2 [M+H]⁺.

### 7) Preparation of (S)-3-(benzyl ((S)-3-(benzyloxy)-2-hydroxypropyl) amino)-2-fluoropropyl methanesulfonate (D54-i):

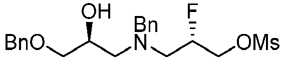

Add dropwise a solution of MsCl (346 mg, 3.02 mmol) in DCM (3 mL) to a solution of **54-h** (1.0 g, 2.88 mmol) and DIPEA (447 mg, 3.46 mmol) in DCM (12 mL) at -10 °C. Allow to react at -2 °C for 1 hour, add water (5 mL) and extract with DCM (10 mL × 3). Dry with anhydrous Na₂SO₄ and concentrate to obtain a colorless oily liquid product (**D54-I**, 1.2 g). MS (ESI): m/z = 348.2 [M+H]⁺.

### 8) Preparation of (2S, 6S)-4-benzyl-2-((benzyloxy) methyl)-6-fluoro-1,4-oxazepane (D54-j):

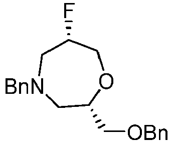

Add NaH batch wise (60% in oil, 276 mg, 6.91 mmol) to a solution of **D54-h** (1.2 g, 2.88 mmol) in THF (15 mL) at 0 °C. Rise the temperature to room temperature and stir overnight. Add saturated NaHCO₃ (5 mL) solution and concentrate to remove THF. Extract the residue with ethyl acetate (50 mL × 3), wash sequentially with water (50 mL) and saturated saline (50 mL), dry with anhydrous Na₂SO₄, concentrate, and purify by column chromatography to obtain a colorless oily liquid product (**54-j**, 350 mg, 37%). MS (ESI): m/z = 330.2 [M+H]⁺.

### 9) Preparation of ((2S, 6S)-6-fluoro-1,4-oxazepan-2-yl) methanol (D54-k):

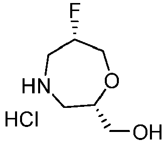

Place a compound of **D54-i** (680 mg, 2.06 mmol), 10% Pd/C (100 mg) and 4 M HCl in dioxane (1.03 mL, 4.12 mmol) in ethanol (20 mL) in a hydrogenation apparatus at room temperature. After 4 hours of hydrogenation, filter off the solid, and concentrate the filtrate to obtain a colorless oily liquid product (**54-k,** 0.4 g). MS (ESI): m/z =150.1 [M+H]⁺.

### 10) Preparation of tert-butyl (2S, 6S)-6-fluoro-2-(hydroxymethyl)-1,4-oxazepan-4-carboxylate (D54-l):

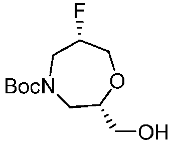

Add (Boc)₂O (919 µL, 4.0 mmol) and TEA (1.4 mL, 10.0 mmol) sequentially to a solution of **D54-k** (382 mg, 2.0 mmol) in DCM (20 mL) at room temperature. After stirring for 4 hours, add water (50 mL), extract with DCM (3 × 20 mL), wash with saturated saline (20 mL), dry with anhydrous Na₂SO₄, and concentrate to obtain a colorless oily liquid product (**D54-l**, 0.50 g, 100%). MS (ESI): m/z = 194.1 [M-tBu+H]⁺.

### 11) Step 11: Step 11: Preparation of (2S, 6S)-4-(tert-butoxycarbonyl)-6-fluoro-1, 4-oxazepan-2-formic acid

### (D54-m):

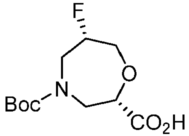

Add water (3 mL), TEMPO (5.9 mg, 0.038 mmol) and Bu₄NHSO₄ (45 mg, 0.133 mmol) sequentially to a solution of **D54-l** (475 mg, 1.9 mmol) in DCM (13 mL) at room temperature. Adjust pH of 6.8 mL of NaClO solution (10-15%) to pH of 8-9 with saturated NaHCO₃. Add NaBr (60 mg, 5.8 mmol) aqueous solution (1.2 mL) to the prepared buffer solution. Take out 10 mL of buffer solution and add dropwise to the reaction system at 0 °C. After stirring at room temperature for 3 hours, adjust the pH value to pH 2-3 with KHSO₄ (2 N) solution. Extract with DCM (3 × 20 mL), dry with anhydrous Na₂SO₄, concentrate, and purify by reverse-phase column chromatography to obtain a white solid product (**D54-m**, 385 mg, total yield of 68% in 3 steps). MS (ESI): m/z =208.3 [M-tBu+H]⁺.

### ■ Intermediate (2S, 6R)-4-(tert-butoxycarbonyl)-6-fluoro-1, 4-oxazepan-2-carboxylic acid is synthesized in a similar manner:

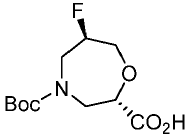

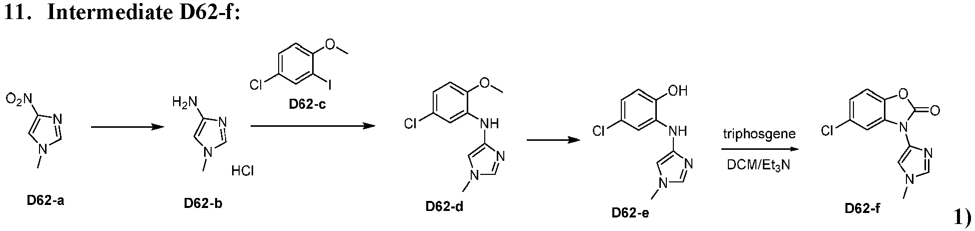

### Preparation of 1-methyl-1H-imidazol-4-amine hydrOCHloride (D62-b):

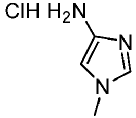

Add Pd(OH)₂/C (65 mg) to a solution of **D62-a** (530 mg, 4.17 mmol) in EtOH (20 mL) at room temperature. Place the reacting liquid under hydrogen overnight, filter off the solid, add HCl (4 M in dioxane, 2 mL) to the filtrate, and concentrate to obtain a yellow solid product (**D62-b**, 575 mg.). MS (ESI): m/z = 98.2 [M+H]⁺.

### 2) Preparation of N-(5-chloro-2-methoxyphenyl)-1-methyl-1H-imidazol-4-amine (D62-d)

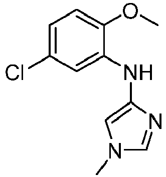

Add 4-chloro-2-iodo-1-methoxybenzene (**D62-c**, 1.07 g, 3.99 mmol), **D62-b** (280 mg, 2.1 mmol), Pd₂(dba)₃ (261 mg, 0.285 mmol), XPhos (272 mg, 0.571 mmol) and NaOtBu (549 mg, 5.7 mmol) to Dioxane (15 mL) under argon at room temperature. Stir the reacting liquid at 110 °C for 4 hours, cool to room temperature, concentrate, and directly purify by column chromatography to obtain a brown solid product (**D62-d**, 235 mg, 47%). MS (ESI): m/z = 238.2 [M+H]⁺.

### 3) Preparation of 4-chloro-2-((1-methyl-1H-imidazol-4-yl) amino) phenol (D62-e):

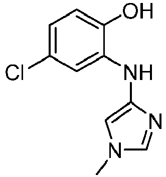

To a solution of **D62-d** (130 mg, 0.55 mmol) in DCM (4 mL) at 0 °C, add BBr₃ (2 mL, 17% Wt in DCM) and allow to react overnight at room temperature. Add saturated NaHCO₃ aq. (20 mL), extract with DCM (3x10 mL), dry with anhydrous Na₂SO₄, concentrate, and purify by reverse-phase column chromatography to obtain (**D62-e**, 165 mg). MS (ESI): m/z = 224.1 [M+H]⁺.

### 4) Preparation of 5-chloro-3-(1-methyl-1H-imidazol-4-yl) benzo[d]oxazol-2 (3H)-one (D62-f):

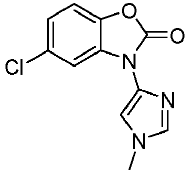

To a solution of **D62-e** (165 mg crude, 0.55 mmol) in DCM (3 mL), add Et₃N (206 µL, 1.48 mmol) and triphosgene (109 mg, 0.37 mmol) sequentially at 0 °C. Stir overnight at room temperature and extract with saturated NH₄Cl aq. (20 mL) and DCM (20 mL × 3). Dry with anhydrous Na₂SO₄ and concentrate, purify by reverse-phase column chromatography (C18, TFA buffer) to obtain a brown solid product (**D62-f**, 21 mg, total yield of 15% in 2 steps). MS (ESI): m/z = 250.3 [M+H]⁺.

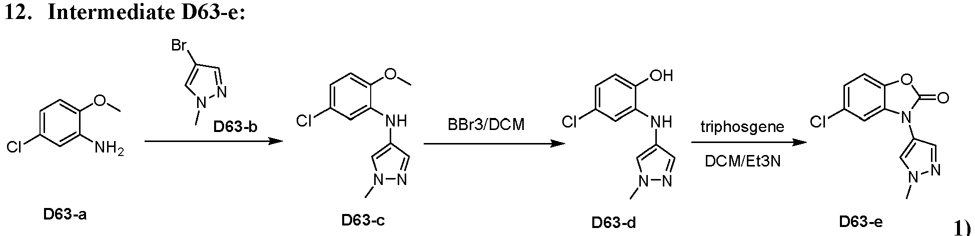

### Preparation of N-(5-chloro-2-methoxyphenyl)-1-methyl-1H-pyrazol-4-amine (D63-c):

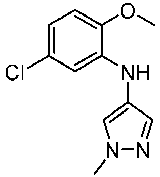

Stir a mixture of **D63-a** (473 mg, 3.0 mmol), **D63-b** (483 mg, 3.0 mmol), XPhos Pd G3 (51.0 mg, 0.06 mmol) and NaOtBu (576 mg, 6.0 mmol) in Dioxane (10 mL) under argon at 110 °C for 7 hours. Cool to room temperature, concentrate, and purify by column chromatography to obtain a brown solid product (**D63-c**, 546 mg, 77%). MS (ESI): m/z = 238.1 [M+H]⁺.

### 2) Preparation of 4-chloro-2-((1-methyl-1H-pyrazol-4-yl) amino) phenol (D63-d):

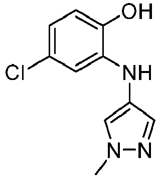

Add BBr₃ (5 mL, 17% Wt in DCM) dropwise to a solution of **D63-c** (546 mg, 2.3 mmol) in DCM (20 mL) at 0 °C. Stir overnight at room temperature and add saturated NaHCO₃ (20 mL). Extract with DCM (20 mL × 3). Dry with anhydrous Na₂SO₄ and concentrate to obtain a product (**D63-d**, 450 mg crude). MS (ESI): m/z = 224.0 [M+H]⁺.

### 3) Preparation of 5-chloro-3-(1-methyl-1H-pyrazol-4-yl) benzo[d]oxazol-2 (3H)-one (D63-e)

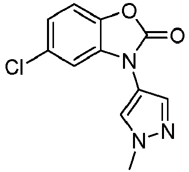

To a solution of **D63-d** (450 mg crude, 2.0 mmol) in DCM (5 mL), add Et₃N (840 uL, 6 mmol) and triphosgene (297 mg, 1.0 mmol) sequentially at 0 °C. Stir overnight at room temperature and add saturated NH₄Cl (20 mL) in water. Extract with DCM (20 mL × 3). Dry with anhydrous Na₂SO₄, concentrate, and purify by column chromatography to obtain a yellow solid (**D63-e**, 194 mg,). MS (ESI): m/z = 250.1 [M+H]⁺.

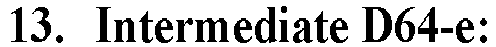

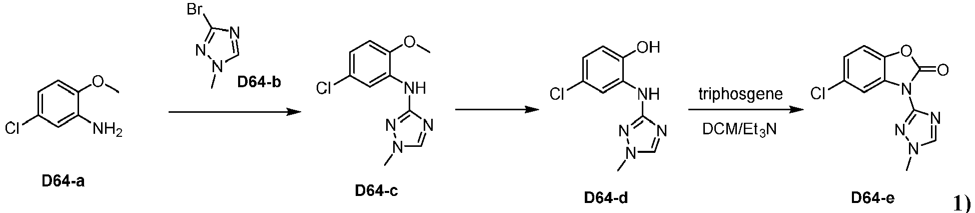

### Preparation of N-(5-chloro-2-methoxyphenyl)-1-methyl-1H-1,2,4-triazol-3-amine (D64-c):

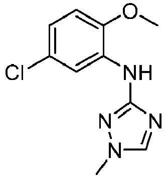

Stir the mixture of **D64-a** (486 mg, 3.1 mmol, 1 eq.), **D64-b** (500 mg, 3.1 mmol, 1 eq.), Pd₂(dba)₃ (283 mg, 0.3 mmol, 0.1 eq.), XPhos (294 mg, 0.6 mmol, 0.2 eq.) and NaOtBu (593 mg, 6.2 mmol, 2 eq.) in Dioxane (20 mL) for 4h under argon at 110 °C. Cool to room temperature, concentrate, and purify by column chromatography to obtain a brown solid product (**D64-c**, 340 mg, 46% yield). MS (ESI): m/z = 239.2 [M+H]⁺.

### 2) Preparation of 4-chloro-2-((1-methyl-1H-1,2,4-triazol-3-yl) amino) phenol (D64-d):

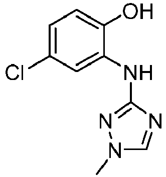

To a solution of **D64-c** (340 mg, 1.42 mmol, 1 eq.) in DCM (5 mL), add dropwise BBr₃ (2.5 mL, 17% Wt in DCM) at 0 °C. Stir overnight at room temperature and add saturated NaHCO₃ (20 mL). Extract with DCM (20 mL × 3). Dry with anhydrous Na₂SO₄ and concentrate to obtain a product (**D64-d**, 340 mg crude). MS (ESI): m/z = 225.1 [M+H]⁺.

### 3) Preparation of 5-chloro-3-(1-methyl-1H-1,2,4-triazol-3-yl) benzo[d]oxazol-2 (3H)-one (D64-e):

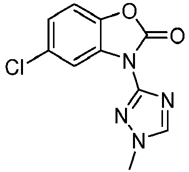

To a solution of **D64-d** (340 mg crude, 1.4 mmol, 1.0 eq.) in DCM (20 mL), add Et₃N (418 µL, 3.0 mmol, 2.0 eq.) and triphosgene (222 mg, 0.75 mmol, 0.5 eq.) sequentially at 0 °C. Stir overnight at room temperature and add saturated NH₄Cl (20 mL). Extract with DCM (20 mL × 3). Dry with anhydrous Na₂SO₄, concentrate, and purify by column chromatography to obtain a yellow solid **product** (**D64-e**, 234 mg, 42%).

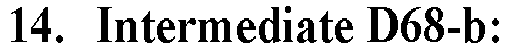

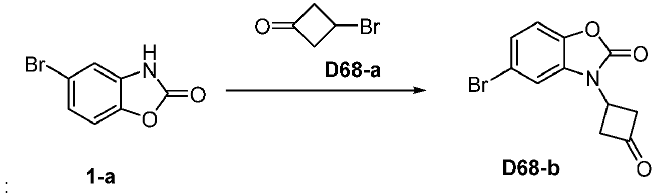

### Preparation of 5-bromo-3-(3-oxocyclobutyl) benzodoxazol-2 (3H)-one (D68-b):

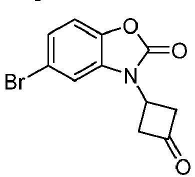

Add NaH (376 mg, 9.4 mmol, 60% in oil) to a solution of **1-a** (1.0 g, 4.7 mmol) in DMF (15 mL) at room temperature. After stirring for 30 minutes, add **D68-a** (1.4 g, 9.4 mmol). After stirring for 3 hours, cool to 0 °C and add water (20 mL, 1/1, v/v). Extract with DCM (20 mL × 3). Dry with anhydrous Na₂SO₄, concentrate, and purify by reverse-phase column chromatography to obtain a yellow solid product (**D68-b**, 550 mg, 42%). MS (ESI): m/z = 284.0 [M+H]⁺.

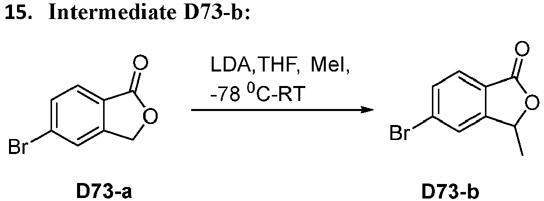

### Preparation of 5-bromo-3-methylisobenzofuran-1 (3H)-one (D73-b):

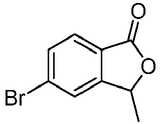

Add dropwise LDA (8.8 mL, 17.6 mmol, 2 mol/L) to a solution of **D73-a** (1.5 g, 7 mmol) in THF (40 mL) over 15 minutes at -78 °C. After stirring for 15 minutes, add MeI (10 g, 70.4 mmol). Raise the reacting liquid to 0 °C and continue stirring for 6 hours. Add water (100 mL), extract with ethyl acetate (250 mL × 3), dry with Na₂SO₄, concentrate, and purify by column chromatography to obtain a white solid product (**D73-b**, 528 mg). MS (ESI): m/z = 227.1 [M+H]⁺. ¹HNMR (400 MHz, CDCl₃): δ 7.77 (s, 1 H), 7.70-7.67 (m, 1H), 7.63 (d, *J* = 0.8 Hz, 1 H), 5.58-5.53 (m, 1 H), 1.65 (d, *J* = 6.4 Hz, 3 H).

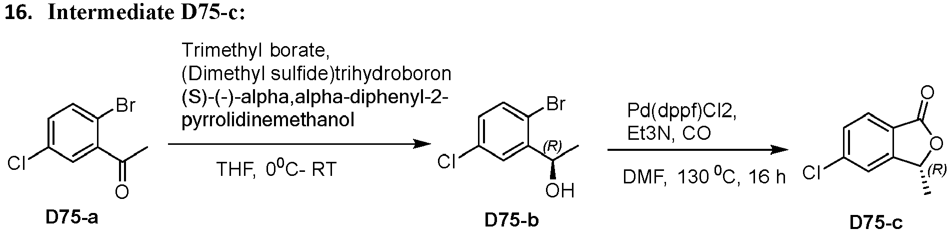

### 1) Preparation of (R)-1-(2-bromo-5-chlorophenyl) ethanol (D75-b):

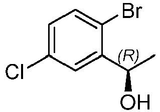

Add Trimethylborate (161 mg, 1.5 mmol) to a solution of (S)-(-)-alpha, alpha-diphenyl-2-pyrrolidinemethanol (297 mg, 1.3 mmol) in tetrahydrofuran (35 mL) at room temperature. After stirring for 1.25 hours, slowly add dropwise borane-methyl sulfide complex (7.1 mL of a 2 M solution in tetrahydrofuran, 14.2 mmol). Cool the reacting liquid to 0 °C and add dropwise a solution of **D122-a** (3 g, 12.8 mmol) in tetrahydrofuran (15 mL) over 1 hour. Rise the temperature to room temperature and stir overnight. Concentrate to remove most of the THF and pour into 1N HCl solution. Extract with ethyl acetate (200 mL × 3), wash with saturated saline (200 mL), dry with Na₂SO₄, concentrate and purify by column chromatography to obtain a white solid product (**D122-b**, 2.9 g, 97%). MS (ESI): m/z = 217.1 [M-17]⁺.

### 2) Preparation of (R)-5-chloro-3-methylisobenzofuran-1 (3H)-one (D75-c):

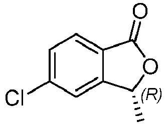

To a solution of **D122-b** (200 mg, 0.85 mmol) in DMF (10 mL) at room temperature, add sequentially Pd(dppf)Cl₂ (62 mg, 0.09 mmol), and TEA (171 mg, 1.7 mmol). Fill the reaction system with nitrogen for 3 times, and then fill carbon monoxide. Stir for 16 hours at 130 °C. Cool to room temperature, concentrate, and purify by column chromatography to obtain a white solid product (**D122-c**, 80 mg, 52%). MS (ESI): m/z = 183.1 [M+H]⁺.

### ■ Intermediate Intermediate (S)-5-Chloro-3-methylisobenzofuran-1 (3H)-one is synthesized in a similar manner:

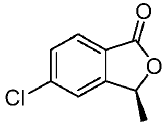

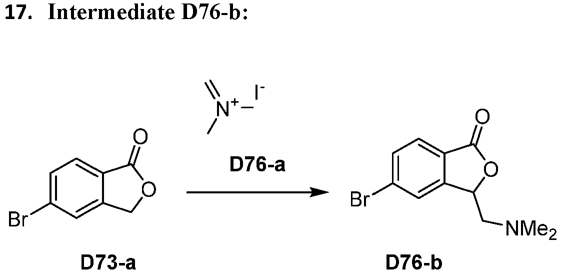

### Preparation of 5-bromo-3-((dimethylamino) methyl) isobenzofuran-1 (3H)-one (D76-b):

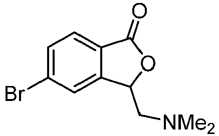

Add dropwise LiHMDS (4.8 mL, 4.8 mmol, 1 M in THF/hexane) to a solution of **D73-a** (1.035 g, 4.86 mmol) in THF (20 mL) at -78 °C. After stirring for 1 hour, add **D76-b** (900 mg, 4.86 mmol) once. Continue stirring at -78 °C for 2 hours. Add saturated NH₄Cl aq. (100 mL) and extract with DCM/MeOH (9:1) (3 * 20 mL). Dry with Na₂SO₄, concentrate, and purify by reverse-phase column chromatography to obtain a white solid product (**D76-b**, 0.6 g, 47%). MS (ESI): m/z = 272.1 [M+H]⁺.

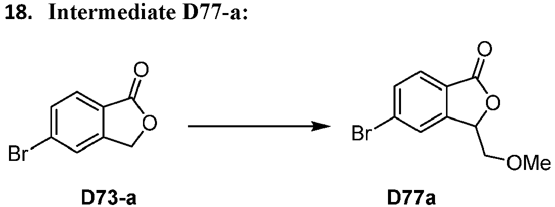

### Preparation of 5-bromo-3-(methoxymethyl) isobenzofuran-1 (3H)-one (D77-a):

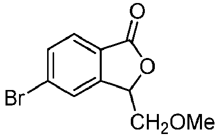

Add dropwise LiHMDS (5.6 mL, 5.63 mmol, 1 M in THF/hexane) to a solution of **D73-a** (1.0 g, 4.69 mmol) in THF (20 mL) at -78 °C. After stirring for 15 minutes, add dropwise bromo(methoxy)methane (417.88 µL, 5.15 mmol). Continue stirring for 1 hour at -78 °C, add saturated NH₄Cl aq. (100 mL) and extract with DCM/MeOH (9:1) (3 × 20 mL). Dry with Na₂SO₄, concentrate, and purify by reverse-phase column chromatography to obtain a white solid product (**D77-a**, 0.6 g, 50%). MS (ESI): m/z = 257.0 [M+H]⁺.

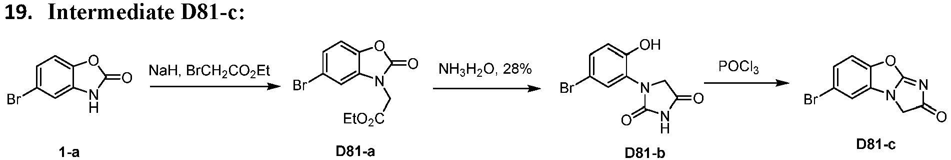

### 1) Preparation of ethyl 2-(5-bromo-2-oxobenzo[d]oxazol-3 (2H)-yl) acetate (D81-a):

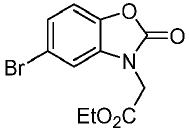

Add NaH (1.03 g, 25.83 mmol) to a solution of **1-a** (5 g, 23.48 mmol) in DMF (40 mL) at 0 °C. After stirring for 30 minutes, add dropwise BrCH2CO2Et (3.9 g, 23.48 mmol). Stir at room temperature for 3 hours, add water, extract with ethyl acetate, dry with Na₂SO₄, concentrate, and purify by reverse-phase column chromatography to obtain a yellow solid product (**D81-a**, 4.8 g, 68.47%).

### 2) Preparation of 1-(5-bromo-2-hydroxyphenyl) imidazoline-2,4-dione (D81-b):

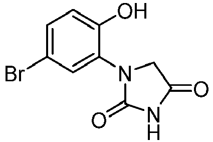

Add NH4OH (300 mL) to a solution of **D81-a** (4.8 g, 16.05 mmol) in EtOH (30 mL) at room temperature. After stirring at room temperature for 24 hours, remove ethanol under reduced pressure, add ice water, and filter to obtain a yellow solid product (**D81-b**, 2.4 g, 55.42%).

### 3) Preparation of 6-bromobenzo[d]imidazol[2,1-b] oxazol-2 (3H)-one (D81-c):

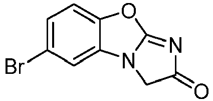

To a solution of **D81-b** (2.4 g, 8.89 mmol) in toluenee (25 mL) at 0 °C, add POCl₃ (25 mL). Stir at 110 °C for 1 hour, cool to room temperature, add ice water, extract with ethyl acetate, dry with Na₂SO₄, concentrate, and purify by reverse-phase column chromatography to obtain a yellow solid product (**D81-c**, 1.4 g, 5.56 mmol, 62.5%). ¹H NMR (400 MHz, DMSO-d*₆*) δ 7.75 (s, 1H), 7.44 - 7.31 (m, 2H), 5.13 (s, 2H).

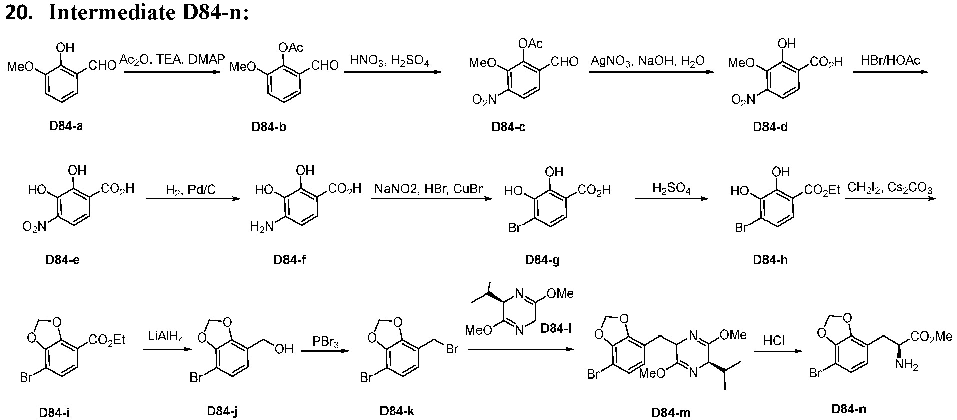

### 1) Preparation of 2-hydroxy-3-methoxybenzaldehyde (D84-b):

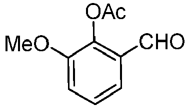

To a solution of **D84-b** (10 g, 65.77 mmol) in DCM (100 mL) at room temperature, add sequentially Ac2O (10.07 g, 98.65 mmol), TEA (6.92 g, 68.40 mmol) and DMAP (0.8 g, 6.58 mmol). After stirring for 30 minutes, add water and extract with dichloromethane, wash sequentially with dilute hydrOCHloric acid HCl (4.0 M) and saturated saline, dry with Na₂SO₄, and concentrate to obtain a white solid product (**D84-b**, 12 g, 94%). 1H NMR (400 MHz, DMSO-*d*₆) δ 10.10 (s, 1H), 7.51 - 7.42 (m, 3H), 3.84 (s, 3H), 2.34 (s, 3H).

### 2) Preparation of 6-formyl-2-methoxy-3-nitrophenyl acetate (D84-c):

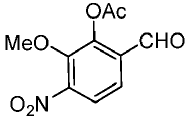

Add dropwise TFAA:TFA (3: 1, 37.5 mL) to a mixture of **D84-b** (10 g, 51.50 mmol), KNO3 (5.31 g, 52.53 mmol) in DCM (40 mL) at -40 °C. Slowly raise the reacting liquid to room temperature and stir overnight. Add ice water and extract with dichloromethane. Dry with Na₂SO₄, concentrate, and purify by column chromatography to obtain a red solid product (**D84-c**, 10.3 g, 83.6%).

### 3) Preparation of 2-hydroxy-3-methoxy-4-nitrobenzoic acid (D84-d):

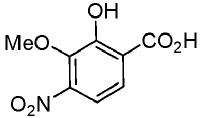

To a solution of **D84-c** (10 g, 41.81 mmol) in MeOH (150 mL) at room temperature, add sequentially NaOH (6.8 g, 171.42 mmol) and water (800 mL). After stirring for 5 minutes, add AgNO3 (8.5 g, 50.17 mmol) and stir at 85 °C overnight. Filter off the solid, and adjust the pH of the filtrate to 2. Extract with ethyl acetate, wash with saturated saline, dry with Na₂SO₄, and concentrate to obtain a yellow solid product (**D84-d**, 7g, 78.5%) (RT=0.483 min, m/z=211.9).**LCMS**: 211.9 [M-H]⁺;

### 4) Preparation of 2, 3-dihydroxy-4-nitrobenzoic acid (D84-e):

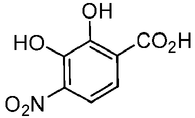

To a solution of **D84-d** (13.45 g, 63.10 mmol) in HOAc (158 mL) at room temperature, add 47% aq.HBr (79 mL). Reflux the reaction for 16 hours and cool to room temperature. Extract with ethyl acetate, wash with water, dry with Na₂SO₄, and concentrate to obtain a yellow solid product (**D84-e**, 9 g, 71.6%).

### 5) Preparation of 4-amino-2, 3-dihydroxybenzoic acid (D84-f):

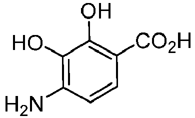

Dissolve **D84-d** (7.2 g, 36.16 mmol) in MeOH (120 mL) at room temperature, add 10% Pd/C (0.6 g) and place in 40 Psi hydrogen gas. When the hydrogen pressure does not continue to decrease, filter off the solid and concentrate the filtrate to obtain a yellow solid product (**D84-f**, 3.6 g, 58.9%). **LCMS**: 170.2 [M+H]⁺.

### 6) Preparation of 4-bromo-2, 3-dihydroxybenzoic acid (D84-g):

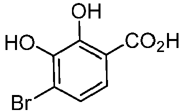

Add 48% aq. HBr (6 mL) followed by a solution of NaNO₂ (1.2 g, 17.29 mmol) in water (8 mL) to a suspension of **D84-f** (3.9 g, 23.06 mmol) in H₂O (8 mL) at 0 °C. After stirring at 0 °C for 2 hours, add a solution of CuBr (2.5 g, 17.29 mmol) in HBr (6 mL). After stirring at 0 °C for 1 h, stir at room temperature overnight. Extract with ethyl acetate, wash with water, dry with Na₂SO₄, and concentrate to obtain a black solid product (**D84-g**, 2.5 g, 46.5%).**LCMS:** 231.0 [M-H]⁺;

### 7) Preparation of ethyl 4-bromo-2, 3-dihydroxybenzoate (D84-h):

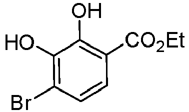

To a solution of (**D84-g**, 1.2 g, 5.15 mmol) in EtOH (38 mL) at room temperature, add concentrated H₂SO₄ (2 mL) and reflux with stirring overnight. Cool to room temperature and remove EtOH under reduced pressure. Add ethyl acetate and wash with saturated NaHCO₃ followed by saline. Dry with Na₂SO₄, concentrate, and purify by column chromatography to obtain a white solid product (**D84-h**, 0.537 g, 39.9%). **LCMS:** 261.0 [M+H]⁺;

### 8) Preparation of ethyl 7-bromobenzo[d][1,3]dioxolo-4-carboxylate (D84-i):

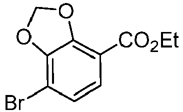

To a solution of **D84-h** (1.0 g, 3.83 mmol) in DMF (8 mL) at room temperature, add Cs₂CO₃ (2.7 g, 8.43 mmol), after stirring for 1 hour, add CH₂I₂ (1.7 g, 6.21 mmol) and allow to react at 75 °C for 12 hours. Cool to room temperature and add ethyl acetate and water. Dry with Na₂SO₄, concentrate, and purify by column chromatography to obtain a white solid product (**D84-i**, 598 mg, 57.2%). **LCMS:** 275.0 [M+H]⁺;

### 9) Preparation of ethyl (7-bromobenzo[d][1,3]-dioxolo-4-yl methanol (D84-i):

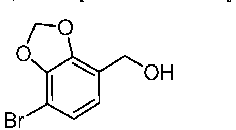

Add dropwise LiAlH₄ (35.89 mL, 35.89 mmoL) to a solution of **D84-i** (4.9 g, 17.94 mmol) in THF (80 mL) at -78 °C. Raise the reacting liquid to room temperature and continue stirring for 2 hours. Add water, extract with ethyl acetate, dry with Na₂SO₄, and concentrate to obtain the product (**D84-j**, 3.8 g, 92%). **GCMS:** 230.0 [M]⁺;

### 10) Preparation of 4-bromo-7-(bromomethyl) benzo[d][1,3]dioxo (D84-k):

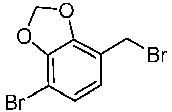

To a solution of **D84-j** (3800 mg, 16.45 mmol) in THF (40 mL), add a solution of PBr₃ (4897.14 mg, 18.09 mmoL) in THF (20 mL) at 0 °C. After stirring at 0 °C for 2 hours, add saturated NH₄Cl, extract with ethyl acetate (300 mL × 4), dry with Na₂SO₄, concentrate, and purify by column chromatography to obtain a white solid product (**D84-k**, 2.6 g, 54%). **GCMS:** 294 [M]⁺;

### 11) Preparation of (5R)-2-((7-bromobenzo[d][1,3]dioxolo-4-yl) methyl)-5-isopropyl-3, 6-dimethoxy-2, 5-dihydropyrazine (D84-m):

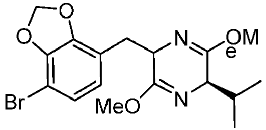

Add dropwise n-butyllithium (2.5 Min hexanes, 6.29 mL, 15.72 mmol) to a solution of **D84-l** (2895.79 mg, 15.72 mmol) in tetrahydrofuran (dry, 60 mL) at -78 °C. After stirring for 30 minutes, add dropwise a solution of **D84-j** (4200 mg, 14.29 mmol) in THF (dry, 20 mL), continue stirring for 1 hour, add saturated NH₄Cl solution, extract with ethyl acetate, dry with Na₂SO₄, concentrate, and purify by column chromatography to obtain a white solid product (**D84-m**, 1800 mg, 32%).**LCMS**: [M+H]⁺ :397.1

### 12) Preparation of (S)-2-amino-3-(7-bromobenzo[d][1,3]dioxolo-4-yl) methyl propionate (D84-n):

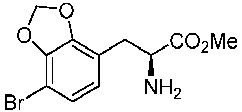

To a solution of **D84-m** (1800 mg, 4.53 mmol) in acetonitrile (60 mL) at room temperature, add aq. 0.2 M HCl (47.57 mL, 9.51 mmol). After stirring for 12 hours, adjust pH to 8 with saturated NaHCO₃ solution, extract with ethyl acetate, dry with Na₂SO₄, concentrate, and purify by column chromatography to obtain a white solid product (**D84-n** 1300.0 mg, 95%).1H NMR (400 MHz, CDCl3) δ 6.87 (d, *J* = 8.4 Hz, 1H), 6.54 (d, *J* = 8.4 Hz, 1H), 6.01 - 5.94 (m, 2H), 3.74 (dd, *J* = 7.5, 5.6 Hz, 1H), 3.68 (s, 3H), 2.99 (dd, *J* = 13.8, 5.3 Hz, 1H), 2.77 (dd, *J* = 13.8, 8.0 Hz, 1H).

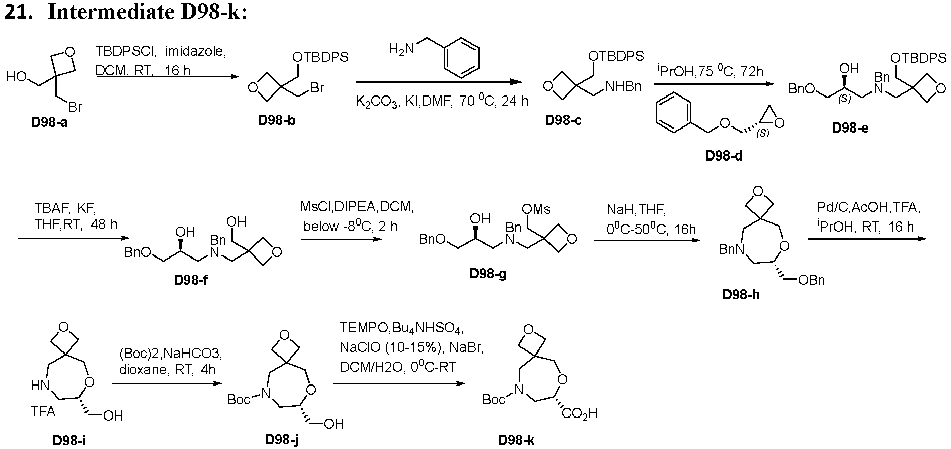

### 1) Preparation of (3-(bromomethyl) oxetan-3-yl) methoxy) (tert-butyl) diphenylsilane (D98-b):

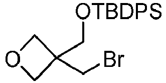

To a solution of **D98-b** (25 g, 138.1 mmol) in DCM (600 mL) at room temperature, add sequentially TBDPSCl (45.6 g, 165.7 mmol) and imidazole (37.6 g, 552.5 mmol). Stir at room temperature for 16 hours and concentrate to remove dichloromethane. Add EtOAc (1000 mL), wash sequentially with water (250 mLx3) and saline (250 mL), dry with Na₂SO₄, concentrate, and purify by column chromatography to obtain a colorless oily product (**D98-b**, 60.1 g, 96%). MS (ESI): m/z = 441.1 [M+Na]⁺.

### 2) Preparation of N-benzyl-1-(3-(((tert-butyldiphenylsilyl) oxy) methyl) oxetan-3-yl) carboxamide (D98-c)

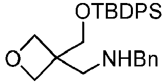

Stir a mixture of **D98-b** (70 g, 143.2 mmol), BnNH₂ (38.3 g, 358 mmol), K₂CO₃ (39.8 g, 286.4 mmol) and KI (23.8 g, 143.2 mmol) in DMF (600 mL) at 70 °C for 24 hours under nitrogen. Cool to room temperature, add EtOAc (1500 mL), wash with saturated saline water (350 mLx4), dry with Na₂SO₄, concentrate, and purify by column chromatography to obtain a colorless oily product (**D98-c**, 41.5 g, 65%). MS (ESI): m/z = 446.3 [M+H]⁺.

### 3) Preparation of (S)-1-(benzyl ((3-(((tert-butyldiphenylsilyl) oxy) methyl) oxetan-3-yl) methyl) amino)-3-(benzyloxy) propan-2-ol (D98-e):

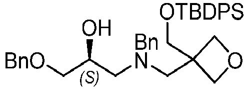

Stir a mixture of **D98-c** (40 g, 89.9 mmol) and **D98-D** (29.5 g, 179.8 mmol) in ⁱPrOH (200 mL) at 75 °C for 72 hours under nitrogen. Cool to room temperature, concentrate, and purify by column chromatography to obtain a colorless oily product (**D98-e**, 58.4 g, 95%). MS (ESI): m/z = 610.2 [M+H]⁺.

### 4) (S)-1-(benzyl ((3-(hydroxymethyl) oxetan-3-yl) methyl) amino)-3-(benzyloxy) propan-2-ol Preparation of (D98-f):

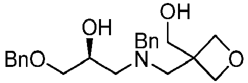

Stir the compound of **D98-e** (58.4 g, 95.9 mmol), KF (55.6 g, 959 mmol) and TBAF (4 mL, 1 mol/l) in THF (1000 mL) at room temperature for 48 hours under nitrogen. Concentrate and add water (300 mL). Extract with ethyl acetate (500 mLx3), dry with Na₂SO₄, concentrate, and purify by column chromatography to obtain a colorless solid product (**D98-f**, 18 g, 50.6%). MS (ESI): m/z = 372.3 [M+H]⁺.

### 5) Preparation of (S)-Methyl (3-((benzyl (3-(benzyloxy)-2-hydroxypropyl) amino) methyl) oxetan-3-yl) methanesulfonate (D98-g):

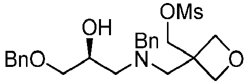

To a solution of **D98-f** (15.3 g, 41.2 mmol) in DCM (250 mL), add DIPEA (6.92 g, 53.6 mmol) and MsCl (4.96 g, 43.3 mmol) in DCM (50 mL) dropwise at -8 °C over 10 minutes. Stir continuously at -8 °C for 2 hours, add ice water (150 mL), extract with DCM (200 mLx3), dry with Na₂SO₄, filter, and concentrate to obtain a colorless oily crude (**D98-g**, 18.5 g, 99%). MS (ESI): m/z = 450.1 [M+H]⁺.

### 6) Preparation of (S)-9-Benzyl-7-((benzyloxy) methyl)-2, 6-dioxa-9-azaspiro [3.6] decane (D98-h):

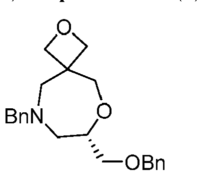

To a suspension of NaH (8.24 g, 206 mmol, purity: 60%) in THF (200 mL), add a solution of **D98-g** (18.5 g, 41.2 mmol) in THF (100 mL) dropwise at -0 °C. Then, stir at 50 °C for 16 hours. Cool to 0 °C, add saturated NaHCO₃ (500 ml) solution, extract with ethyl acetate (500 mLx3), dry with Na₂SO₄, concentrate, and purify by column chromatography to obtain a colorless oily product (**D98-h**, 12.5 g, 86%). MS (ESI): m/z = 354.2 [M+H]⁺.

### 7) Preparation of (S)-(2, 6-dioxa-9-azaspiro [3.6] decan-7-yl) methanol TFA salt (D98-i):

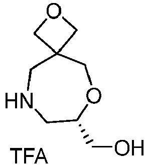

Place a mixture of **D98-h** (5 g, 14.2 mmol), Pd/C (2 g, 10% on C), TFA (1.61 g, 14.2 mmol) and AcOH (10 mL) in ⁱPrOH (200 mL) in a hydrogenation unit at room temperature. After 16 hours of hydrogen absorption under stirring at room temperature, filter the solids, and concentrate the filtrate to obtain a brown oily crude product (**D98-i**, 4.5 g, 95%). MS (ESI): m/z = 174.2 [M+H]⁺.

### 8) Preparation of (S)-tert-butyl 7-(hydroxymethyl)-2, 6-dioxa-9-azaspiro [3.6] decane-9-carboxylate (D98-j):

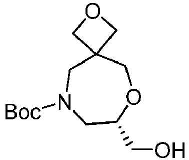

Stir a mixture of **D98-i** (Crude 4.5 g, 14.2 mmol), (Boc)₂O (6.18 g, 28.3 mmol) and saturated NaHCO₃ (150 ml) in dioxane (150 mL) at room temperature for 4 hours. Extract with ethyl acetate (3 × 200 mL), dry with Na₂SO₄, concentrate, and purify by column chromatography to obtain a brown oily product (**D98-j**, 3.5 g, 90%). MS (ESI): m/z = 274.2 [M+H]⁺.

### 9) Preparation of (S)-9-(tert-butoxycarbonyl)-2, 6-dioxa-9-azaspiro [3.6] decane-7-formic acid (D98-k):

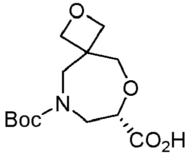

To DCM (20 mL) and water (9 mL), add **D98-j** (1 g, 3.7 mmol), TEMPO (11 mg, 0.07 mmol), and Bu₄NHSO₄ (87 mg, 0.3 mmol) sequentially at room temperature. Meanwhile, adjust 7.4 mL of NaClO solution 10-15% to pH 8-9 with saturated NaHCO₃ (liquid + solids) (approx 15 mL), and add NaBr (64 mg, 0.6 mmol) aqueous solution (1 mL) to the buffer solution. Add freshly prepared buffer solution to the reaction solution dropwise at 0 °C. Stir at room temperature for 3 hours, add KHSO₄ (2N) solution to adjust pH to 2-3. Extract with DCM (100 mLx3), dry with Na₂SO₄, and concentrate to obtain a white solid product (**D98-k**, 850 mg, 81%). MS (ESI): m/z =310.2 [M+Na]⁺.

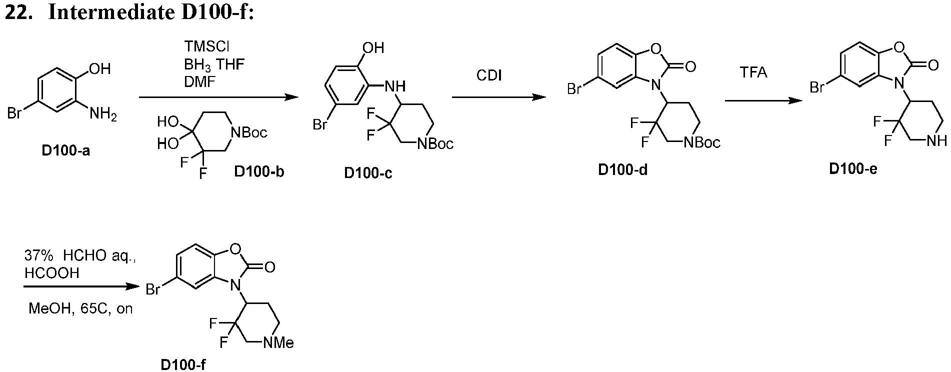

### 1) Preparation of tert-butyl 4-((5-bromo-2-hydroxyphenyl) amino)-3, 3-difluoropiperidine-1-carboxylate (D100-c);

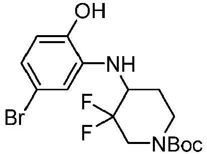

To **D100-b** (1.0 g, 4.0 mmol, 4 eq.) and **D100-a** (376 mg, 2.0 mmol, 1 eq.) in DMF (10 mL) add TMSCl (1.0 mL, 8.0 mmol, 4.0 eq.) at 0 °C. Stir at 0 °C for 2 hours, add BH₃·THF (1 M, 10 mL, 5 eq.) dropwise. Stir at room temperature for 3 hours, add saturated Na₂CO₃ (200 mL) and water (10 mL). Extract with ethyl acetate (100 mL × 2), wash with water, dry with Na₂SO₄, concentrate, and purify by column chromatography to obtain an orange oily product **(D100-c,** 730 mg, 90%). MS (ESI): m/z = 353.0 [M-tBu+H]⁺.

### 2) Preparation of tert-butyl 4-(5-bromo-2-oxobenzo[d]oxazol-3(2H)-yl)-3, 3-difluoropiperidine-1-carboxylate (D100-d)

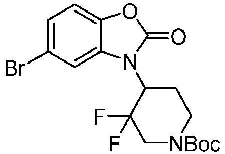

Stir a mixture of **D100-c** (690 mg, 1.69 mmol), CDI (330 mg, 2.03 mmol) in dioxane (20 mL) at 110 °C for 6 hours under argon. Cool to room temperature, concentrate, and purify by column chromatography to obtain a yellow solid product **(D100-d,** 586 mg, 76% yield). MS (ESI): m/z = 455.0 [M+Na]⁺.

### 3) Preparation of 5-bromo-3-(3, 3-difluoropiperidin-4-yl) benzo[d]oxazol-2 (3H)-one (D100-e):

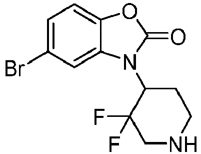

Stir a mixture of **D 100-d** (566 mg, 1.3 mmol) in TFA/DCM (1:6, 7 mL) at room temperature for 2 hours under argon, and concentrate to obtain the product to use directly in the next reaction. MS (ESI): m/z = 334.9 [M+H]⁺.

### 4) Preparation of 5-bromo-3-(3, 3-difluoro-1-methylpiperidin-4-yl) benzo[d]oxazol-2(3H)-one (D100-f):

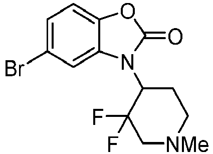

To a mixture of **D100-e** (490 mg crude, TFA salt, 1.1 mmol) in MeOH/HCHO aq. (10 mL, 1:1) at room temperature, add 250 µL of HCOOH reaction solution, stir at 65 °C for 24 hours. Cool to room temperature, concentrate, and lyophilize to obtain a yellow solid product**(D100-f,** 390 mg, 99% yield).MS (ESI): m/z = 347.1 [M+H]⁺.

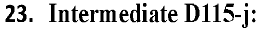

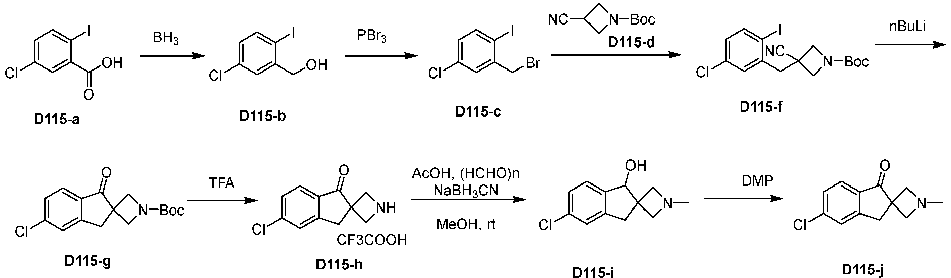

### 1) Preparation of (5-Chloro-2-iodophenyl) methanol (D115-b):

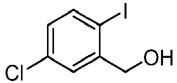

To **D115-a** (2.0 g, 7.1 mmol, 1.0 eq.) in THF (20 mL), add BH₃·THF (1M in THF, 17.8 mL, 2.5 eq.) dropwise at 0 °C, then stir at room temperature overnight. Add MeOH (25 mL) to the reaction solution and concentrate to obtain a white solid product **(D115-b,** 1.9 g, 99%). MS (ESI): m/z = 251.0 [M-OH]⁺.

### 2) Preparation of 2-(Bromomethyl)-4-chloro-1-iodobenzene (D115-c):

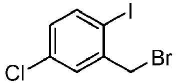

To **D115-b** (1.34 g, 5.0 mmol, 1.0 eq.) in DCM (50 mL), add PBr₃ (522 µL, 5.5 mmol, 1.1 eq.) dropwise at 0 °C. Stir at 0 °C for 1 hour and at room temperature for 2 hours, add saturated NaHCO₃ aq. (100 mL). Extract with dichloromethane (20 mLx2), dry with Na₃SO₄. concentrate, and purify by column chromatography to obtain a white solid **(D115-c,** 0.92 g, 56%).

### 3) Preparation of tert-butyl 5'-chloro-1'-oxo-1',3'-dihydrospiro [azetidine-3,2'-indene]-1-carboxylate (D115-f):

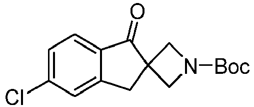

To a solution of **D115-d** (420 mg, 2.3 mmol) in THF (40 ml), add LiHMDS (1 M in hexane, 2.3 mL) dropwise at -78 °C. Stir continuously at -78 °C for 30 minutes, add **D115-c** (713 mg, 2.15 mmol) at a time. Allow the reaction to slowly rise to room temperature and continue to stir for 3 hours. Cool to -78 °C again and add nBuLi (2.5 M in hexane, 1.9 mL) dropwise. Allow the reaction to slowly rise to room temperature and continue to stir overnight. Extract with water (H₃O) (100 mL), ethyl acetate (EtOAc) (30 mL × 3), dry with Na₂SO₄, concentrate, and purify by reverse-phase column chromatography to obtain a yellowish oily product **(D115-f,** 570 mg, 86%). MS (ESI): m/z = 252.2, 330.1 [M-tBu+H]⁺, [M+Na]⁺.

### 4) Preparation of 5'-Chlorospiro [azetidin-3,2'-indene]-1'(3'H)-one (D115-g):

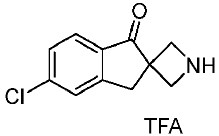

Dissolve **D115-f** (570 mg, 1.85 mmol) in TFA/DCM (1: 10, 5.5 mL) at room temperature, stir for 2 hours, and concentrate to obtain the product **(D115-g,** 0.6 g, TFA salt). MS (ESI): m/z = 208.1 [M+H]⁺.

### 5) Preparation of 5'-chloro-1-methyl-1',3'-dihydrospiro [azetidin-3,2'-indene]-1'-ol (D115-h):

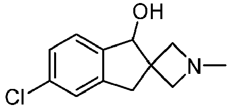

To **D115-g** (0.6 g crude, 1.85 mmol, 1.0 eq.) in MeOH/AcOH (5:1, 24 mL), paraformaldehyde (555 mg, 18.5 mmol, 10.0 eq.) at room temperature . Stir for 5 hours, add NaBH₃CN (465 mg, 7.4 mmol, 4.0 eq.).. Stir overnight at room temperature, filter solids, and concentrate. Add saturated NaHCOs aq. (50 mL) and a mixture of dichloromethane/methanol (9/1, 20 mLx6) to the residue for extraction. Dry with Na₂SO₄ and concentrate to obtain a yellowish oily product **(D115-h,** 330 mg). MS (ESI): m/z = 224.2 [M+H]⁺.

### 6) Preparation of 5'-chloro-1-methylspiro [azetidin-3,2'-indene]-1'(3'H)-one (D115-i):

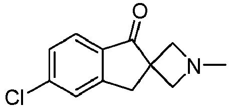

To **D115-h** (308 mg, 1.38 mmol, 1.0 eq.) in DCM (20 mL), add Dess-Martin periodinane (642 mg, 1.51 mmol, 1.1 eq.) at room temperature. Stir for 1 hour, add saturated NaHCOs aq. (30 mL) and DCM (20 mLx3) for extraction. Dry with Na₂SO₄, concentrate, and purify by reverse-phase column chromatography to obtain a white solid product **(D115-i,** 280 mg). MS (ESI): m/z = 222.1 [M+H]⁺.

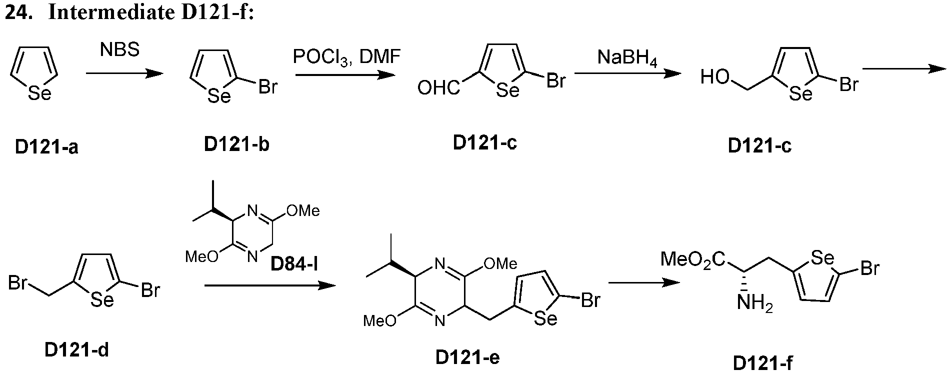

### 1) Preparation of 2-bromoselenophene (D121-b):

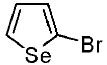

To a solution of **D121-a** (5000 mg, 38.15 mmol) in DCM (60 mL) and CH₃COOH (60 mL) at 0 °C, add NBS (6791 mg, 38.15 mmol). Stir at 0 °C for 2 hours, add water, extract with dichloromethane, dry with Na₂SO₄, concentrate, and purify by column chromatography to obtain a white oily product **(D121-b,** 5100 mg, 63.7%).

### 2) Preparation of 5-bromoselenophene-2-carbaldehyde (D121-c): Page: IDSU22-32-060

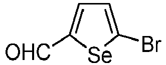

To a solution of **D121-b** (6100 mg, 29.06 mmol) in THF (60 mL), add LDA (43.6 mL, 43.58 mmol) dropwise at -78 °C. Stir for 30 minutes, add DMF (2 mL) dropwise. Stir continuously at -78 °C for 4 hours, add water, extract with dichloromethane, dry with Na₂SO₄, concentrated, and purify by column chromatography to obtain a brown oily product (**D121-c**, 2100 mg, 30.4%). **GCMS:** 237 [M]

### 3) Preparation of (5-Bromoselenophen-2-yl) methanol (D121-c):

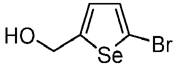

To a solution of **D121-b** (2600 mg, 10.93 mmol) in MeOH (50 mL), add NaBH4 (827 mg, 21.85 mmol) at 0 °C. Stir at 0 °C for 2 hours, add water, extract with dichloromethane, dry with Na₂SO₄, and concentrate to obtain a brown oil product YD02-I46-4 **(D121-c,** 2100 mg).

### 4) Preparation of 2-Bromo-5-(bromomethyl) selenophene (D121-d):

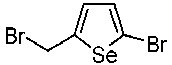

To a solution of **D121-c** (2100 mg, 8.75 mmol) in diethyl ether (20 mL), add PBr₃ (3079 mg, 11.37 mmol) dropwise at 0 °C. Stir at 0 °C for 2 hours, add water, extract with dichloromethane, dry with Na₂SO₄, and concentrate to obtain a brown oily product **(D121-d,** 2500 mg).

### 5) Preparation of (SR)-2-((5-bromoselenophen-2-yl) methyl)-5-isopropyl-3, 6-dimethoxy-2, 5-dihydropyrazine (D121-e):

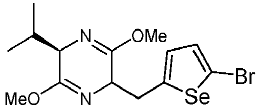

To a solution of **D84-l** (1825 mg, 9.91 mmol) in THF (50 mL), add n-BuLi (4.95 mL, 12.38 mmol) dropwise at -78 °C. Stir continuously for 1 hour, add **D121-f** (2500 mg, 8.25 mmol) in THF (10 mL) dropwise. After 2 hours of reaction at -78 °C, add saturated aqueous NH₄Cl solution, extract with ethyl acetate, dry with Na₂SO₄, concentrate, and purify by column chromatography to obtain a white solid product **(D121-e,** 2000 mg, 59.7%). LCMS: 407.0 [M+H]⁺

### 6) Preparation of methyl 2-amino-3-(5-bromoselenophen-2-yl) propionate (D121-f):

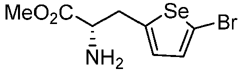

To a solution of **D121-e** (2000 mg, 4.92 mmol) in CH₃CN (20 mL) at room temperature, add HCl (52 mL, 0.2 mol/L). After stirring for half an hour, concentrate and purify by HPLC to obtain a white solid product **(D121-f,** 1002 mg, 65.4%). LCMS: 312.0 [M+H]⁺

### Embodiment 1: Synthesis of compound D01

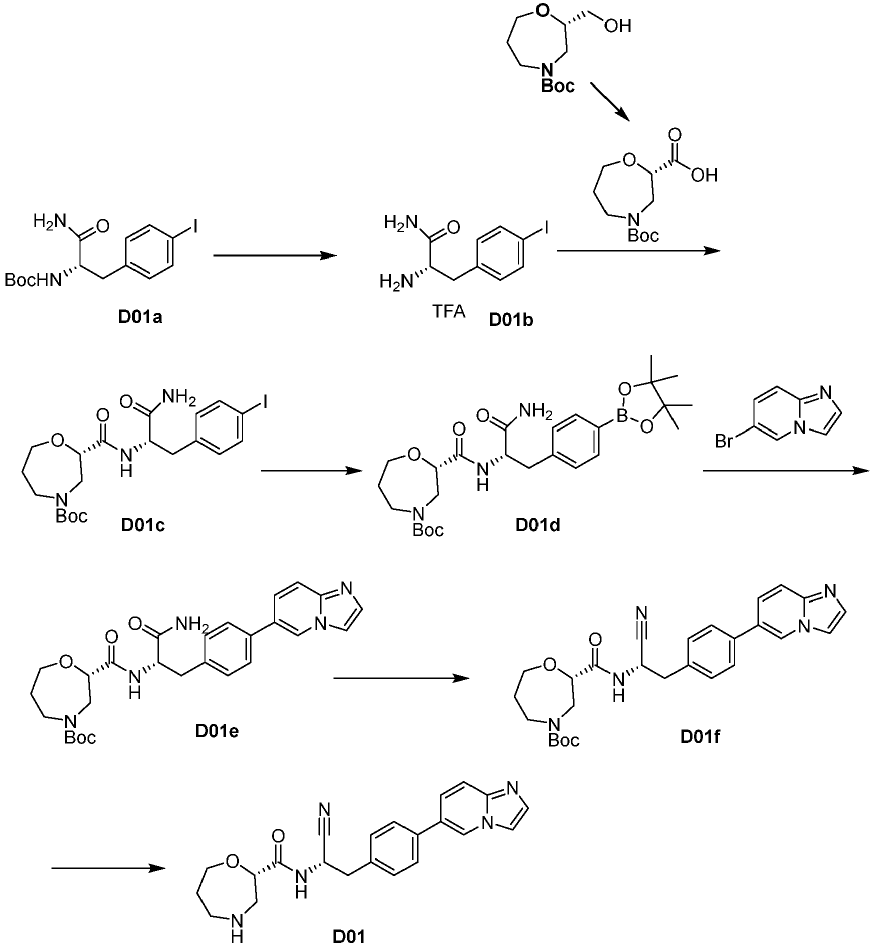

a) Dissolve the compound **D01a** (1 g, 2.6 mmol) in dichloromethane (30 mL) at room temperature, then add trifluoroacetic acid (6 mL) dropwise. Stir for 2 h and spin dry to obtain a brown solid product **D01b** (1 g, 96.5%). MS (ESI): m/z = 291.0 [M+H]⁺.
b) (*S*)-4-(tert-butoxycarbonyl)-1, 4-oxoazacycloheptane-2-carboxylic acid: Dissolve the compound (*S*)-4-(tert-butoxycarbonyl)-1, 4-oxoazacycloheptane-2-methanol (7.9 g, 34.2 mmol) in dichloromethane (134 mL) and water (28 mL) with cooling in an ice bath. (*S*)-4-(tert-butoxycarbonyl)-1, 4-oxoazacycloheptane-2-methanol is synthesized according to the route reported in patent (WO2015110826). Then sequentially add TEMPO (107 mg, 0.68 mmol) and Bu₄NHSO₄ (812 mg, 2.4 mmol). At the same time, prepare buffer solution. To 68 mL of NaClO solution (10-15%) add saturated NaHCOs solution (approximately 100 mL) to adjust acidity and alkalinity to pH 8-9. Then, dissolve NaBr (599 mg, 5.8 mmol) in water (11.6 mL), and add the above buffer solution. Slowly drip the prepared buffer solution into the reaction solution. Note that the reaction is exothermic, keep the temperature in the bottle below 10 °C for 1 h. Stir overnight at room temperature, adjust the acidity and alkalinity to pH 2-3 with KHSO₄ (2N), extract the reaction solution with dichloromethane (3 × 500 mL), dry, and concentrate to obtain a crude product. Purify by column chromatography (elute with PE/EA = 2:1 to 1:1, V/V) to obtain a white solid product (*S*)-4-(tert-butoxycarbonyl)-1, 4-oxoazacycloheptane-2-carboxylic acid (30.9 g, 65%). MS (ESI): m/z =268.1 [M+Na]⁺. ¹H NMR (400 MHz, CDCl3): δ 9.14 (m, 1 H); 4.34-4.00 (m, 3 H); 3.76-3.34 (m, 3 H); 3.28-3.16 (m, 1 H); 1.93-1.92 (m, 2 H); 1.46 (s, 9 H).
c) Dissolve the compound **D01b** (10.16 g, 25 mmol) and (S)-4-(tert-butoxycarbonyl)-1, 4-oxoazacycloheptane-2-carboxylic acid (5.56 g, 22.7 mmol) in DCM (300 mL) at room temperature, followed by DIPEA (11.71 g, 90.8 mmol), HOBt (3.06 g, 22.7 mmol) and HBTU (9.46 g, 25 mmol) and stir overnight. Add water (200 mL) to the reaction solution, extract with dichloromethane (3 × 400 mL), dry, and concentrate to obtain a crude product. Purify by column chromatography (elute with PE/EA = 1: 1 to 1:4, V/V) to obtain a white solid product **D01c** (10.6 g, 91%). MS (ESI): m/z =540.1 [M+Na]⁺. ¹H NMR (400 MHz, CDCl₃): δ 7.61(s, 2 H); 7.20-7.11 (m, 1 H); 6.98 (d, *J* = 8.4 Hz, 2 H); 6.29 (s, 0.5 H); 5.96 (s, 0.5 H); 5.45 (d, *J* = 8.0 Hz, 1 H); 4.63-4.55 (m, 1 H); 4.18-3.97 (m, 3 H); 3.81-3.77 (m, 0.5 H); 3.50-2.93 (m, 5.5 H); 2.00-1.86 (m, 2 H); 1.45 (s, 9 H).
d) Dissolve compounds D01c (9.7 g, 18.8 mmol) and Pin₂B₂ (9.53 g, 37.5 mmol) in DMSO (250 mL) at room temperature under argon, then add Pd(dppf)Cl₂ (960 mg, 1.3 mmol) and KOAc (5.52 g, 56.3 mmol). Heat the reaction solution to 85 °C and stir continuously for 8 h. Cool to room temperature, dilute with EA (1000 mL), wash with saturated saline water (5 × 200 mL), dry with Na₂SO₄, and concentrate to obtain the crude product. Purify to obtain the white solid product by column chromatography (eluent PE/EA = 1:1-1:6, V/V) **D01d** (8.5 g, 87.6%). MS (ESI): m/z =540.3 [M+Na]⁺. ¹H NMR (400 MHz, CDCl₃): δ 7.76 (d, *J* = 7.2 Hz, 2 H); 7.25 (d, *J* = 8.0 Hz, 2 H); 7.18 (d, *J* = 7.2 Hz, 1 H); 6.15 (s, 0.5 H); 5.88 (s, 0.5 H); 5.48 (d, *J* = 18 Hz, 1 H); 4.67-4.61 (m, 1 H); 4.20-3.99 (m, 3 H); 3.85-3.80 (m, 0.5 H); 3.54-3.11 (m, 5 H); 2.96-2.90 (m, 0.5 H); 2.02-1.96 (m, 2 H); 1.46 (s, 9 H), 1.34 (s, 12 H).
e) Dissolve compound **D01d** (200 mg, 0.39 mmol) and 6-bromimidazolo[1,2-*a*]pyridine (115 mg, 0.58 mmol) in dioxane (8 mL) and water (0.8 mL) in argon gas at room temperature, then transfer into PdCl₂dppf (30 mg, 0.04 mmol) and KOAc (115 mg, 1.17 mmol), raise temperature to 85 °C, and continue stirring for 4 h. Cool the reacting liquid to room temperature, add EA (50 mL), extract, wash with saturated salt water (10 mL), dry with Na₂SO₄, concentrate to obtain the crude product. Purify to obtain the brown oily product by column chromatography (eluent PE-EA:MeOH = 10: 1, Rf = 0.3 in EA/MeOH 10/1) **DOle** (120 mg, 60%). MS (ESI): m/z = 508.3 [M+H]⁺. ¹HNMR (400 MHz, CDCl₃): δ 8.35-8.31 (m, 1H), 7.76-7.70 (m, 3H), 7.66-7.41 (m, 3H), 7.38-7.36 (m, 2H), 7.26-7.19 (m, 1H), 6.24 (brs, 0.5H), 5.93 (brs, 0.5H), 5.45 (brs, 1H), 4.71-4.64 (m, 1H), 4.22-4.02 (m, 3H), 3.80-3.76 (m, 0.5H), 3.52-3.02 (m, 5.5H), 1.92-1.90 (m, 2H), 1.47 (s, 9H).
f) To a solution of **D01e** (120 mg, 0.237 mmol) in dichloromethane (10 mL) at room temperature, add Burgess reagent (338 mg, 1.42 mmol). After stirring at room temperature for 4 h, dilute the reacting liquid with dichloromethane (20 mL), wash with water (10 mL), dry with Na₂SO₄, and concentrate to obtain the crude product. Purify the crude product by -HPLC (using TFA buffer: A: 0.05% TFA aqueous solution; B: 0.05% TFA in acetonitrile; Column: Waters XBridge Peptide BEH C18, 19 × 250 mm, 10 µm, 130 Å) and freeze-dry. Neutralize the resulting product with saturated NaHCOs to pH 7, extract the resulting solution with EA (3 × 50 mL), dry with Na₂SO₄, and concentrate to obtain the brown solid product **D01f** (15 mg, yield 12%). MS (ESI): m/z = 490.3 [M+H]⁺.
g) At room temperature, add HCOOH (3 mL) to compound **D01f** (10 mg, 0.02 mmol), then heat to 50 °C and continue stirring for 10 min. Concentrate the reacting liquid to obtain the crude product, which is purified by -HPLC (using HCOOH buffer: A: 0.1% HCOOH aqueous solution; B: acetonitrile; Column: Waters XBridge Peptide BEH C18, 19 × 250 mm, 10 µm, 130 Å) to obtained the white solid product **D01** (1.5 mg, yield 17%)¹H NMR (400 MHz, DMSO-d₆): δ 8.91 (s, 1H), 8.71 (d, J = 8.4 Hz, 1H), 8.20 (s, 1H), 7.95 (s, 1H), 7.68-7.55 (m, 5H), 7.41 (d, J = 8 Hz, 2H), 5.07-5.01 (m, 1H), 4.09-4.06 (m, 1H), 3.88-3.83 (m, 1H), 3.79-3.70 (m, 1H), 3.26-3.18 (m, 2H), 3.12-3.08 (m, 1H), 2.88-2.82 (m, 1H), 2.72-2.66 (m, 1H), 2.63-2.57 (m, 1H), 1.80-1.73 (m, 2H).

| Compound No. | Structural formula | MS (M+H) ⁺ | ¹HNMR |
|---|---|---|---|
| D01 | 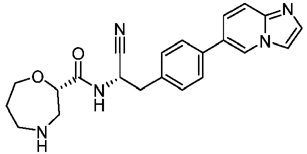 | 390.4 | ¹H NMR (400 MHz, DMSO-d₆): δ 8.91 (s, 1H), 8.71 (d, J = 8.4 Hz, 1H), 8.20 (s, 1H), 7.95 (s, 1H), 7.68-7.55 (m, 5H), 7.41 (d, J = 8 Hz, 2H), 5.07-5.01 (m, 1H), 4.09-4.06 (m, 1H), 3.88-3.83 (m, 1H), 3.79-3.70 (m, 1H), 3.26-3.18 (m, 2H), 3.12-3.08 (m, 1H), 2.88-2.82 (m, 1H), 2.72-2.66 (m, 1H), 2.63-2.57 (m, 1H), 1.80-1.73 (m, 2H). |

The following compounds are prepared by replacing 6-bromoimidazo[1,2-*a*]pyridine with different bromides according to the method of Embodiment 1:

| **Comp ound** | **Bromide structure** | **Product structure** | **MS (M+H)⁺** | **¹H NMR** |
|---|---|---|---|---|
| D02 | 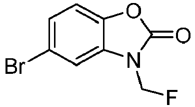 | 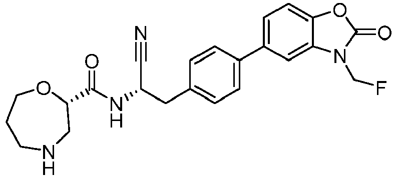 | 439.2 | ¹H NMR (400 MHz, DMSO-*d*₆): δ 8.80-8.72 (m, 1 H); 8.12 (s, 1 H); 7.87 (d, *J* = 1.6 Hz, 1 H); 7.67 (d, *J* = 8.8 Hz, 2 H); 7.54-7.47 (m, 2 H); 7.40 (d, *J* = 8.8 Hz, 2 H); 6.16 (s, 1 H); 6.03 (s, 1 H); 5.06-5.00 (m, 1 H); 4.13-4.10 (m, 1 H); 3.89-3.83 (m, 1 H); 3.76-3.70 (m, 1 H); 3.61-3.55 (m, 1 H); 3.25-2.62 (m, 6 H); 2.02-1.75 (m, 2 H). F¹⁹ NMR (400 MHz, DMSO-*d*₆):-174.42. |
| D03 | 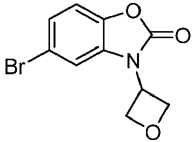 | 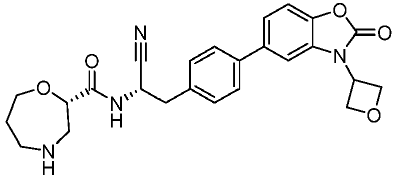 | 463.0 | ¹H NMR (400 MHz, DMSO-*d*₆): δ 8.62 (d, *J* = 8.4 Hz, 1H), 7.72 (s, 1 H ), 7.64-7.62 (m, 2 H), 7.47-7.39 (m, 4 H), 5.52-5.45 (m, 1 H), 5.14-5.10 (m, 2 H), 5.06-5.00 (m, 1 H), 4.96-4.92 (m, 2 H), 4.00-3.97 (m, 1 H), 3.86-3.80 (m, 1 H), 3.74-3.68 (m, 1 H), 3.23-3.15 (m, 2 H), 3.02 (dd, *J* = 14.0, 3.4 Hz, 1 H), 2.78-2.72 (m, 1 H), 2.62-2.51 (m, 2 H), 1.72-1.70 (m, 2 H). |
| D04 | 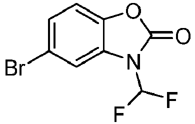 | 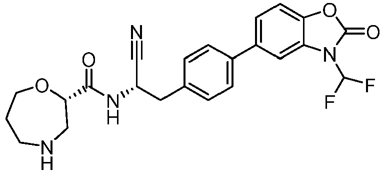 | 457.2 | ¹H NMR (400 MHz, DMSO-*d*₆): δ 8.65 (d, *J* = 8.4 Hz, 1H), 7.78 (t, *J* = 57.2 Hz, 1 H), 7.59 (d, *J* = 9.0 Hz, 2 H), 7.57-7.54 (m, 3 H), 7.39 (d, *J* = 8.4 Hz, 2 H), 5.03 (q, *J* = 8.0 Hz, 1 H), 4.02 (dd, *J* = 8.0, 4.0 Hz, 1H), 3.86-3.81 (m, 1 H), 3.73-3.69 (m, 1 H), 3.25-3.15 (m, 2 H), 3.04 (dd, *J* = 14.0, 4.0 Hz, 1 H), 2.81-2.75 (m, 1 H), 2.65-2.50 (m, 2 H), 1.76-1.69 (m, 2 H). F¹⁹ NMR (400 MHz, DMSO-*d*₆):-102.129, -102.281. |
| D05 | 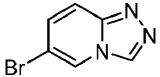 | 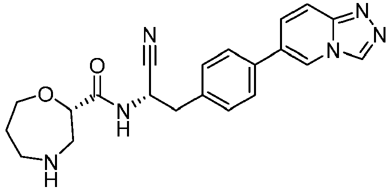 | 391.0 | ¹H NMR (400 MHz, DMSO-d₆): δ 9.24 (s, 1H), 8.90 (s, 1H), 8.63 (d, J = 8.4 Hz, 1H), 7.86 (d, J = 9.6 Hz, 1H), 7.73 (dd, J1 = 1.6 Hz, J2 = 10 Hz, 1H), 7.68 (d, J = 8 Hz, 2H), 7.43 (d, J = 8 Hz, 2H), 5.08-5.01 (m, 1H), 4.00-3.97 (m, 1H), 3.87-3.81 (m, 1H), 3.75-3.69 (m, 1H), 3.27-3.15 (m, 2H), 3.02 (dd, J1 = 3.6 Hz, J2 = 14 Hz, 1H), 2.78-2.72 (m, 1H), 2.63-2.53 (m, 2H), 1.78-1.67 (m, 2H) |
| D06 | 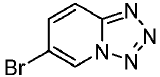 | 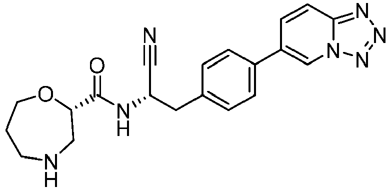 | 392.3 | ¹H NMR (400 MHz, DMSO-*d*₆): δ 9.69 (s, 1 H), 8.71 (d, *J* = 6.8 Hz, 1 H), 8.32-8.29 (m, 1 H), 8.26-8.23 (m, 1 H), 8.19 (s, 1 H), 7.85 (d, *J* = 8.4 Hz, 2 H), 7.47 (d, *J* = 8.4 Hz, 2 H), 5.09-5.03 (m, 1 H), 4.07-4.04 (m, 1 H), 3.89-3.83 (m, 1 H), 3.76-3.70 (m, 1 H), 3.29-3.19 (m, 2 H), 3.08 (dd, *J* = 14.0, 4.0 Hz, 1 H), 2.85-2.80 (m, 1 H), 2.71-2.66 (m, 1 H), 2.60-2.55 (m, 1 H), 1.79-1.73 (m, 2 H). |
| D07 | 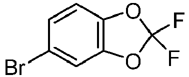 | 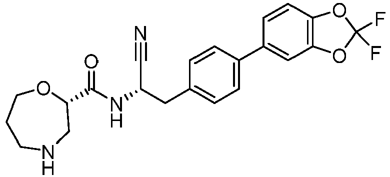 | 430.0 | ¹H NMR (400 MHz, DMSO-d₆): δ 8.62 (d, J = 8.4 Hz, 1H), 7.73 (d, J = 1.2 Hz, 1H), 7.62 (d, J = 8 Hz, 2H), 7.51-7.46 (m, 2H), 7.38 (d, J = 8 Hz, 2H), 5.05-4.99 (m, 1H), 4.01-3.98 (m, 1H), 3.87-3.81 (m, 1H), 3.74-3.68 (m, 1H), 3.25-3.14 (m, 2H), 3.02 (dd, J1 = 3.6 Hz, J2 = 14 Hz, 1H), 2.79-2.73 (m, 1H), 2.63-2.53 (m, 2H), 1.76-1.68 (m, 2H) ¹⁹F NMR (400 MHz, DMSO-d₆): δ -49.04 |
| D08 | 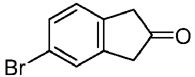 | 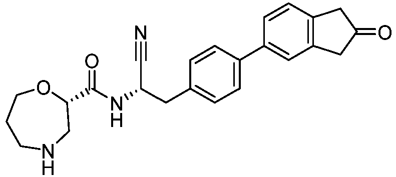 | 404.2 | ¹H NMR (400 MHz, DMSO-*d*₆): δ 8.65 (d, *J* = 8.8 Hz, 1 H), 7.25-7.75 (m, 7 H), 5.08-5.01 (m, 1 H), 4.00-4.35 (m, 1 H), 3.40-3.90 (m, 6H), 2.60-3.20 (m, 6H), 1.70-2.05 (m, 2H). |
| D09 | 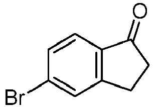 | 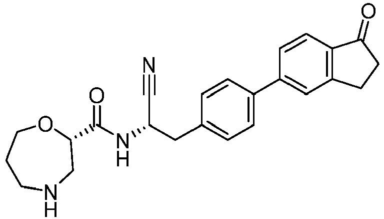 | 404.2 | ¹H NMR (400 MHz, DMSO-*d*₆): δ 8.65 (d, *J* = 8.8 Hz, 1H), 7.85 (s, 1 H ), 7.72-7.70 (m, 4 H), 7.42 (d, *J* = 8.0 Hz, 2 H), 5.08-5.01 (m, 1 H), 4.00 (dd, *J* = 8.0, 3.6 Hz, 1 H), 3.85-3.81 (m, 1 H), 3.75-3.69 (m, 1 H), 3.27-3.14 (m, 5 H), 3.03 (dd, *J* = 14.0, 3.2 Hz, 1 H), 2.78-2.72 (m, 1 H), 2.68-2.51 (m, 4 H), 1.75-1.70 (m, 2H). |
| D10 | 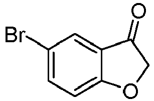 | 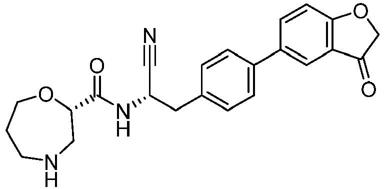 | 406.4 | ¹H NMR (400 MHz, DMSO-*d*₆): δ 8.64 (d, *J* = 8.8 Hz, 1 H), 8.06-8.02 (m, 1 H), 7.87-7.85 (m, 1 H), 7.67-7.61 (m, 2 H), 7.39-7.36 (m, 3 H), 5.05-4.99 (m, 1 H), 4.87 (s, 2 H), 4.01 (dd, *J* = 8.0, 4.0 Hz, 1 H), 3.94-3.81 (m, 2 H), 3.75-3.68 (m, 1 H), 3.22-3.18 (m, 2 H), 3.04 (dd, *J* = 14.4, 3.6 Hz, 1 H), 2.81-2.75 (m, 1 H), 2.67-2.54 (m, 2 H), 1.78-1.69 (m, 2H). |
| D12 | 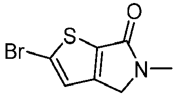 | 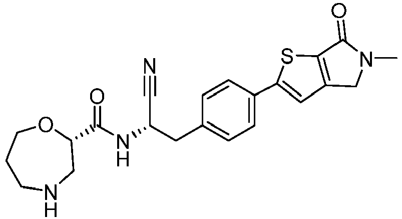 | 425.1 | ¹H NMR (400 MHz, MEOD): δ 7.69 (d, *J* = 8.0 Hz, 2 H), 7.48 (s, 1H), 7.39 (d, *J* = 8.0 Hz, 2 H), 5.16-5.05 (m, 1 H), 4.46 (s, 2 H), 4.15-3.96 (m, 2 H), 3.80-3.76 (m, 1 H), 3.31-3.16 (m, 6 H), 2.92-2.77 (m, 2 H), 2.65 (dd, *J* = 14.4, 8.4 Hz, 1 H), 1.95-1.80 (m, 2H). |
| D13 | 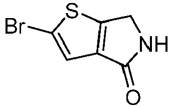 | 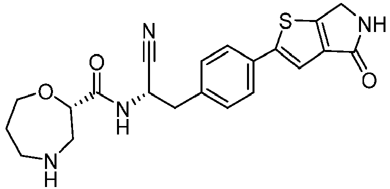 | 411.1 | ¹H NMR (400 MHz, DMSO-*d*₆): 6 8.61 (d, *J* = 8.4 Hz, 1H), 8.36 (s, 1 H), 7.67-7.63 (m, 2 H ), 7.55 (s, 1 H), 7.35-7.31 (m, 2 H), 5.05-4.98 (m, 1 H), 4.47 (s, 2 H), 4.00-3.97 (m, 1 H), 3.86-3.80 (m, 1 H), 3.74-3.68 (m, 1 H), 3.20-3.13 (m, 2 H), 3.01 (dd, *J* = 14.0, 3.6 Hz, 1 H), 2.79-2.72 (m, 1 H), 2.67-2.51 (m, 2 H), 1.78-1.65 (m, 2H). |
| D14 | 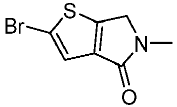 | 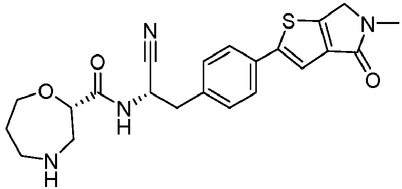 | 425.0 | ¹H NMR (400 MHz, DMSO-*d*₆): 6 8.61 (d, *J* = 8.8 Hz, 1 H), 7.68-7.63 (m, 2 H), 7.58 (s, 1H), 7.35-7.31 (m, 2 H), 5.05-4.97 (m, 1 H), 4.55 (s, 2 H), 3.99-3.95 (m, 1 H), 3.87-3.79 (m, 1 H), 3.74-3.68 (m, 1 H), 3.19-3.15 (m, 2 H), 3.02 (s, 3 H), 3.00-2.97 (m, 1 H), 2.78-2.72 (m, 1 H), 2.68-2.50 (m, 2 H), 1.78-1.65 (m, 2H). |
| D16 | 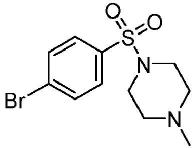 | 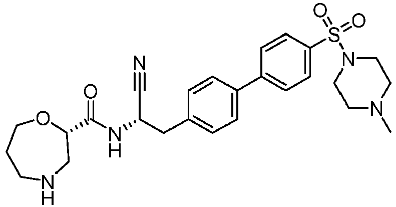 | 512.3 | ¹H NMR (400 MHz, DMSO-*d*₆): δ 8.64-8.61 (m, 1 H), 7.95-7.92 (m, 2 H ), 7.80-7.78 (m, 2 H), 7.74-7.71 (m, 2H), 7.43 (d, *J* = 8.4 Hz, 2 H), 5.08-4.95 (m, 1 H), 4.00-3.68 (m, 3 H), 3.27-3.18 (m, 3 H), 3.01 (dd, *J* = 14.4, 3.6 Hz, 1 H), 2.95-2.87 (m, 4 H), 2.77 -2.51 (m, 3 H), 2.37-2.35 (m, 4 H), 2.13 (s, 3 H), 1.76-1.70 (m, 2H). |
| D17 | 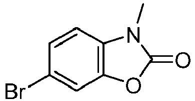 | 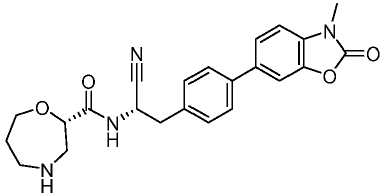 | 421.2 | ¹H NMR (400 MHz, DMSO-*d*₆): δ 8.64 (d, *J* = 8.4 Hz, 1 H), 7.65-7.62 (m, 3 H ), 7.55-7.52 (m, 1 H), 7.37-7.30 (m, 3 H), 5.05-4.98 (m, 1 H), 4.03-3.99 (m, 1 H), 3.87-3.81 (m, 1 H), 3.75-3.69 (m, 1 H), 3.36 (s, 3 H), 3.24-3.13 (m, 3 H), 3.04 (dd, *J* = 14.0, 3.6 Hz, 1 H), 2.81-2.75 (m, 1 H), 2.65-2.52 (m, 2 H), 1.76-1.68 (m, 2 H). |
| D18 | 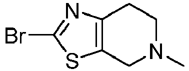 | 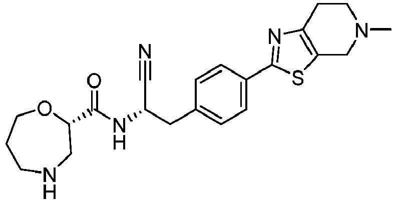 | 426.2 | ¹H NMR (400 MHz, DMSO-*d*₆): δ 9.2 (brs, 1H), 9.09-8.81 (m, 1 H), 7.87 (d, *J* = 8.0 Hz, 2 H), 7.42 (d, *J* = 8.0 Hz, 2 H), 5.09-5.01 (m, 1 H), 4.71-4.67 (m, 1 H), 4.48-4.38 (m, 2 H), 4.10-3.88 (m, 2 H), 3.80-3.65 (m, 4 H), 3.46-3.35 (m, 2 H), 3.30-3.09 (m, 5 H), 2.94 (s, 3 H), 1.81-1.63 (m, 1H). |
| D19 | 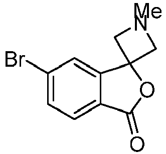 | 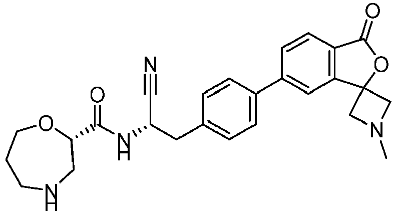 | 461.2 | ¹H NMR (400 MHz, DMSO-*d*₆): δ 8.65 (d, *J* = 8.4 Hz, 1H), 8.15 (s, 1 H), 7.92-7.86 (m, 2 H), 7.79 - 7.77 (m, 2 H), 7.46 (d, *J* = 8.0 Hz, 2 H), 5.09 - 4.98 (m, 1 H), 4.13 - 3.46 (m, 8 H), 3.27 - 3.21 (m, 2 H), 3.05-3.01 (m, 1 H), 2.81 - 2.52 (m, 3 H), 2.43 (s, 3 H), 1.79 - 1.66 (m, 2 H). |
| D20 | 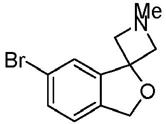 | 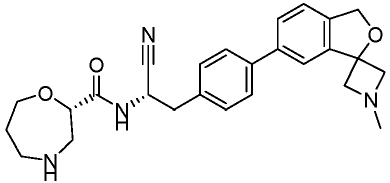 | 447.4 | ¹H NMR (400 MHz, DMSO-*d*₆): δ 8.79 (d, *J* = 8.8 Hz, 0.43 H), 8.62 (d, *J* = 8.2 Hz, 0.5 H), 7.85 (s, 0.4 H), 7.82 (s, 0.6 H), 7.64-7.57 (m, 3 H), 7.40 - 7.33 (m, 3 H), 5.08 - 5.00 (m, 3 H), 4.13-3.40 (m, 8 H), 3.33 - 3.13 (m, 3 H), 3.05 - 2.99 (m, 1 H), 2.78 - 2.74 (m, 0.5 H), 2.67 - 2.50 (m, 1.5 H), 2.42 (s, 1.3 H), 2.36 (s, 1.7 H), 1.76- 1.70 (m, 2 H). |
| D21 | 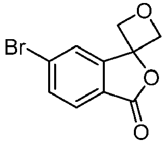 | 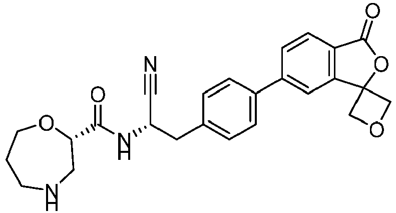 | 448.2 | ¹H NMR (400 MHz, DMSO-*d*₆): δ 8.65 (d, *J* = 8.4 Hz, 1H), 8.34 (s, 1 H), 7.95-7.83 (m, 4 H), 7.47 (d, *J* = 8.4 Hz, 2 H), 5.12-4.99 (m, 5 H), 4.01 - 3.98 (m, 1 H), 3.87 - 3.81 (m, 1 H), 3.75-3.69 (m, 1 H), 3.27 - 3.21 (m, 2 H), 3.05 - 3.00 (m, 1 H), 2.79-2.73 (m, 1 H), 2.63 -2.55 (m, 1 H), 2.53 - 2.52 (m, 1 H), 1.80-1.64 (m, 2 H). |
| D22 | 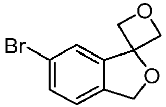 | 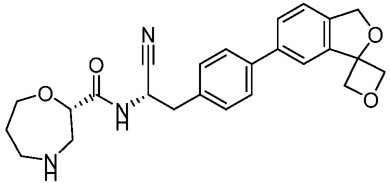 | 434.1 | ¹H NMR (400 MHz, DMSO-*d*₆): δ 8.63 (d, *J* = 8.8 Hz, 1H), 7.92 (s, 1 H), 7.70 (d, *J* = 8.4 Hz, 2 H), 7.66 - 7.64 (m, 1 H), 7.41 - 7.37 (m, 3 H), 5.08 (s, 2 H), 5.05 - 4.98 (m, 1 H), 4.87 (s, 4 H), 4.01 - 3.98 (m, 1 H), 3.87-3.78 (m, 1 H), 3.75 - 3.69 (m, 1 H), 3.26 - 3.16 (m, 2 H), 3.05-3.01 (m, 1 H), 2.78 - 2.70 (m, 1 H), 2.63 - 2.52 (m, 2 H), 1.75 - 1.67 (m, 2 H). |
| D23 | 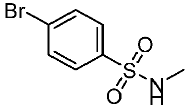 | 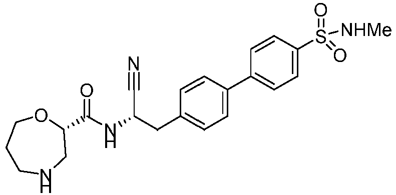 | 443.0 | ¹H NMR (400 MHz, DMSO-*d*₆): δ 8.71-8.63 (m, 1 H), 7.91-7.82 (m, 4 H), 7.71-7.69 (m, 2 H), 7.51-7.41 (m, 3 H), 5.08-5.01 (m, 1 H), 4.07-3.97 (m, 1 H), 3.87-3.81 (m, 1 H), 3.75-3.68 (m, 1 H), 3.26-3.00 (m, 4 H), 2.68-2.65 (m, 1 H), 2.61-2.51 (m, 2 H), 2.43 (d, *J =* 5.2 Hz, 3 H), 1.80-1.67 (m, 2 H). |

Further, the following compounds are prepared by replacing 6-bromoimidazo[1,2-*a*]pyridine with different bromides according to the method of Embodiment 1:

| **Compoun d** | **Bromide structure** | **Product structure** | **MS (M+H)⁺** |
|---|---|---|---|
| D24 | 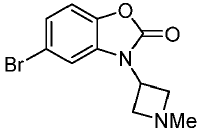 | 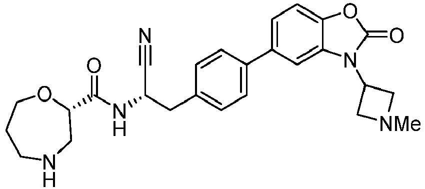 | 476.3 |
| D25 | 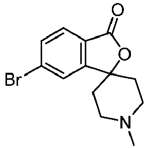 | 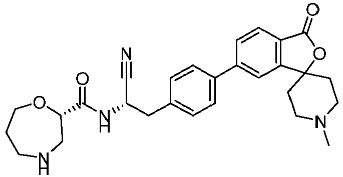 | 489.2 |
| D26 | 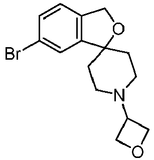 | 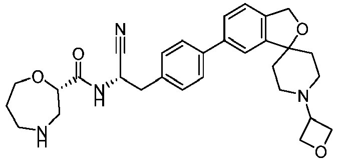 | 517.3 |
| D27 | 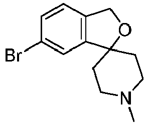 | 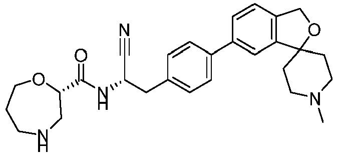 | 475.3 |
| D28 | 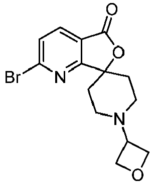 | 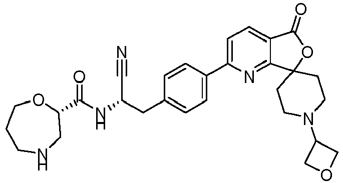 | 532.3 |
| D29 | 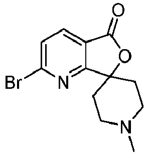 | 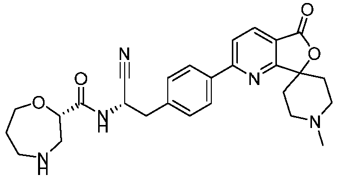 | 490.2 |
| D30 | 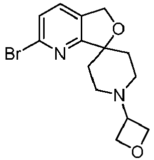 | 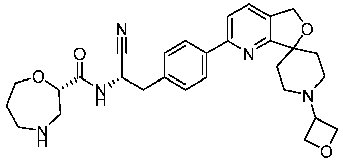 | 518.3 |
| D31 | 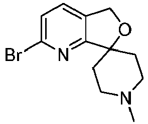 | 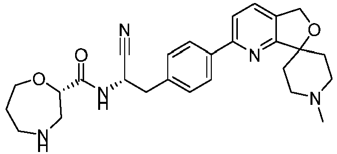 | 476.3 |
| D32 | 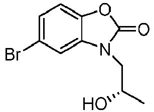 | 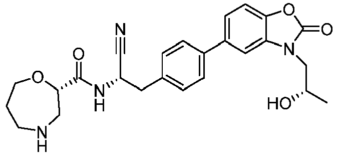 | 465.2 |
| D33 | 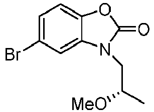 | 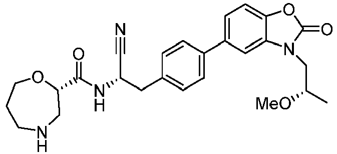 | 479.2 |
| D34 | 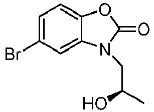 | 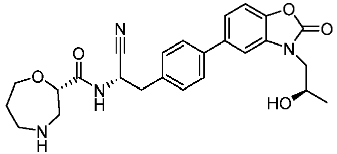 | 465.2 |
| D35 | 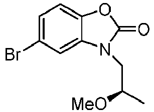 | 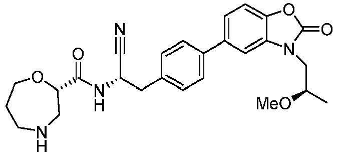 | 479.2 |
| D36 | 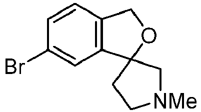 | 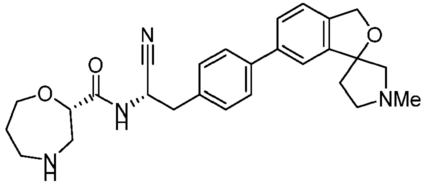 | 461.3 |
| D37 | 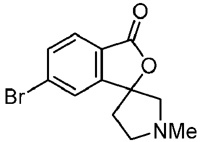 | 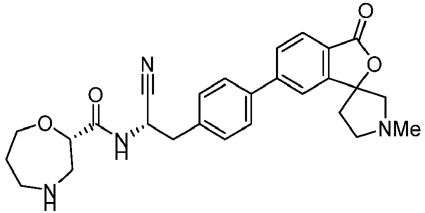 | 475.2 |
| D38 | 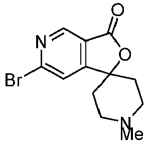 | 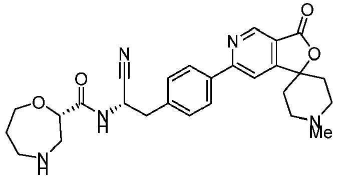 | 490.2 |
| D39 | 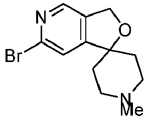 | 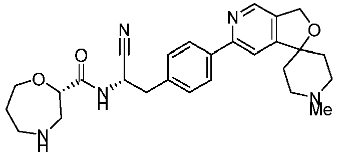 | 476.3 |
| D40 | 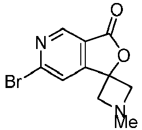 | 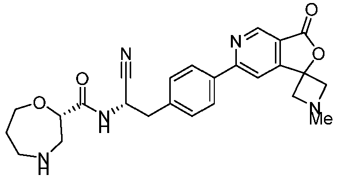 | 462.2 |
| D41 | 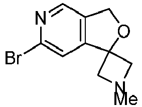 | 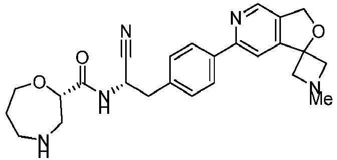 | 448.2 |
| D42 | 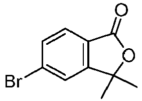 | 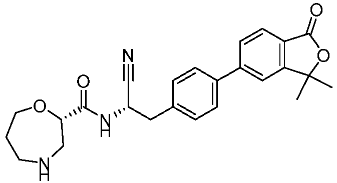 | 434.2 |
| D43 | 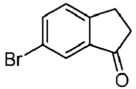 | 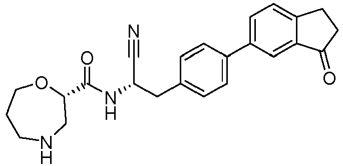 | 404.2 |
| D44 | 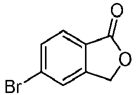 | 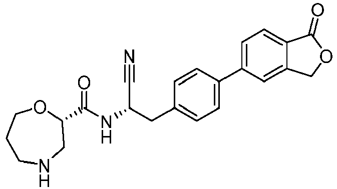 | 406.2 |
| D45 | 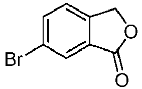 | 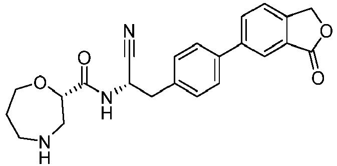 | 406.2 |

Further, the following compounds are similarly prepared from different analogs of fluoro-substituted L-phenylalanine (L-2-Fluoro-Phenylalanine) according to the method of Embodiment 1.

| **Compo und** | **Product structure** | **MS (M+H) ⁺** | **¹H NMR** |
|---|---|---|---|
| D55 | 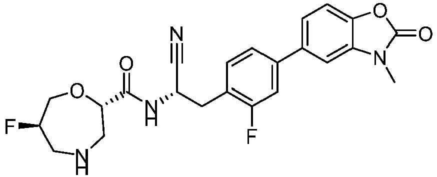 | 457.5 | ¹H NMR (400 MHz, DMSO-*d*₆): δ 8.81 (d, *J* = 8.0 Hz, 1 H), 7.65 (s, 1 H), 7.60-7.54 (m, 2 H), 7.48-7.39 (m, 3 H), 5.07-4.99 (m, 1 H), 4.85-4.72 (m, 1 H), 4.13-4.02 (m, 2 H), 3.82-3.73 (m, 1 H), 3.40 (s, 3 H), 3.31-3.26 (m, 1 H), 3.24-3.05 (m, 3 H), 2.86-2.73 (m, 1 H), 2.47-2.37 (m, 2 H). |
| D56 | 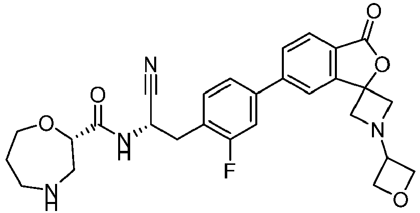 | 521.6 | ¹H NMR (400 MHz, DMSO-*d*₆): δ8.12 (s, 1 H), 7.77 (d, *J* = 8.0 Hz, 1 H), 7.68 (s, 1 H), 7.56 (m, 2 H), 7.51 (m, 1 H), 4.61 (m, 1 H), 4.23 (m, 2 H), 4.18 (m, 2 H), 4.01 (m, 1 H), 3.95 (m, 2 H), 3.85 (m, 2 H), 2.89-2.71 (m, 1 H), 2.48-2.35 (m, 2H). |
| D57 | 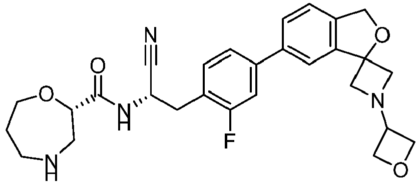 | 507.5 | ¹H NMR (400 MHz, DMSO-*d*₆): δ7.95 (s, 1 H), 7.72 (d, *J* = 8.0 Hz, 1 H), 7.65 (s, 1 H), 7.58 (m, 2 H), 7.54 (m, 1 H), 4.61 (m, 1 H), 4.23 (m, 2 H), 4.18 (m, 2 H), 4.01 (m, 1 H), 3.95 (m, 2 H), 3.85 (m, 2 H), 3.68 (dd, 1 H), 3.61(dd, 1H), 2.87-2.72 (m, 1 H), 2.43-2.31 (m, 2 H). |
| D58 | 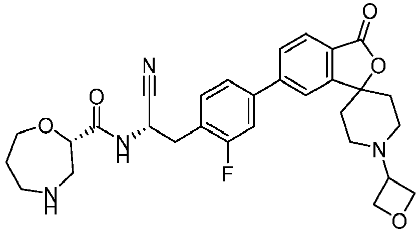 | 549.6 | ¹H NMR (400 MHz, DMSO-*d*₆): δ 8.64 (dd, 1H), 8.41 (s, 1H), 7.92 (dd, 1H), 7.73 (dd, 1H), 7.71 (dd, 1H), 7.69 (dd, 1 H), 7.52 (t, 1 H), 5.07 (m, 1 H), 4.58 (t, 1 H), 4.47 (t, 1 H), 4.02 (m, 1 H), 3.75 (m, 1 H), 3.52 (m, 1 H), 3.18 (m, 1 H), 2.74 (m, 3 H), 2.59 (m, 2 H), 2.41 (m, 2 H), 2.18 (m, 2H), 1.66 (m, 4 H). |
| D59 | 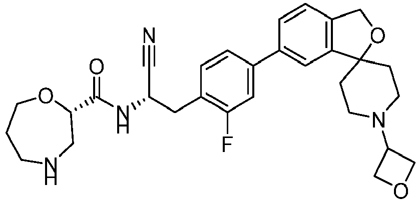 | 535.6 | ¹H NMR (400 MHz, DMSO-*d*₆): δ 8.74 (d, 1H), 7.68 (s, 1H), 7.62 (d, 1H), 7.7-7.54 (m, 2H), 7.43 (t, 1H), 7.32 (t, 1 H), 5.2-5.0 (m, 3 H), 4.57 (t, 2 H), 4.45 (t, 2 H), 4.1 (br., 1 H), 3.85 (m, 1 H), 3.75 (m, 1 H), 3.47 (m, 1 H), 3.37-3.12 (m, 2H), 2.81 (m, 1 H), 2.63 (m, 4 H), 2.32-2.1 (m, 4 H), 1.61-1.42 (m, 4 H). |
| D60 | 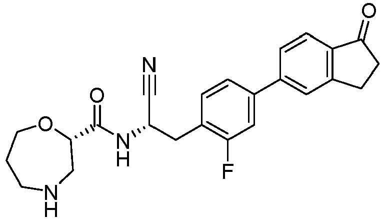 | 422.5 | ¹H NMR (400 MHz, DMSO-*d*₆): δ 8.75 (d, 1H), 7.91 (s, 1H), 7.77-7.58 (m, 4H), 7.48 (t, 1H), 5.07 (m, 1 H), 3.98-3.56 (m, 4H), 3.36-3.12 (m, 6H), 2.79-2.58 (m, 4H), 1.77-1.69 (m, 2H). |
| D61 | 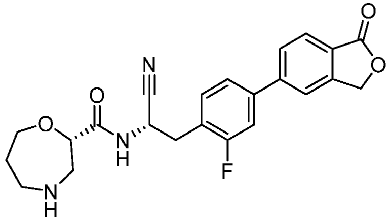 | 424.4 | ¹H NMR (400 MHz, DMSO-*d*₆): δ 8.76 (d, 1H), 8.04 (s, 1H), 7.92 (s, 2H), 7.71-7.47 (m, 4H), 5.09 (m, 1 H), 3.86 (m, 1H), 3.74 (m, 1H), 3.41-3.22 (m, 5H), 3.12 (dd, 1H), 2.83-2.54 (m, 3H), 1.72 (m, 2H). |
| D62 | 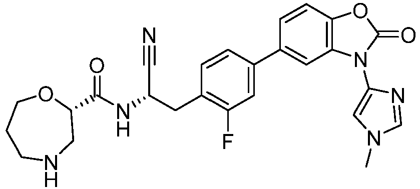 | 505.5 | ¹H NMR (400 MHz, DMSO-*d₆*): δ 8.97 (d, *J* = 8.4 Hz, 1H), 8.03 (s, 1H), 7.76 (s, 1H), 7.56 - 7.49 (m, 3H), 7.46 (d, *J* = 2.2 Hz, 2H), 5.05 (dd, *J* = 16.3, 8.1 Hz, 1H), 4.29 (dd, *J* = 9.1, 3.0 Hz, 1H), 3.99-3.82 (m, 1H), 3.82 - 3.67 (m, 4H), 3.29 (d, *J* = 7.2 Hz, 2H), 3.19 (dd, *J* = 13.6, 8.4 Hz, 2H), 3.13 - 3.03 (m, 1H), 2.91 (dd, *J* = 12.9, 5.9 Hz, 1H), 2.81 (dd, *J* = 13.7, 9.5 Hz, 1H), 1.95 - 1.84 (m, 2H). |
| D63 | 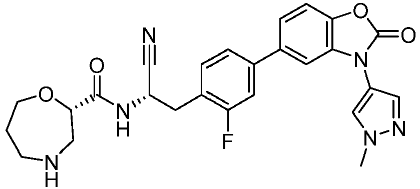 | 505.5 | ¹H NMR (400 MHz, DMSO-*d₆*): δ 8.73 (d, *J* = 8.6 Hz, 1H), 8.30 (s, 1H), 7.90 (d, *J* = 0.5 Hz, 1H), 7.58 (dd, *J* = 11.5, 1.5 Hz, 1H), 7.55 - 7.49 (m, 3H), 7.43 (d, *J* = 8.0 Hz, 1H), 7.40 (d, *J* = 6.4 Hz, 1H), 5.03 (dd, *J* = 16.2, 8.3 Hz, 1H), 4.01 (dd, *J* = 8.1, 3.6 Hz, 1H), 3.93 (s, 3H), 3.89 - 3.81 (m, 1H), 3.71 (ddd, *J* = 12.0, 7.5, 4.1 Hz, 1H), 3.28 (dd, *J* = 13.6, 7.5 Hz, 2H), 3.17 (dd, *J* = 13.6, 8.6 Hz, 1H), 3.05 (dd, *J* = 14.2, 3.6 Hz, 1H), 2.82 - 2.73 (m, 1H), 2.67 - 2.59 (m, 1H), 2.56 (dd, *J* = 14.2, 8.2 Hz, 1H), 1.83 - 1.63 (m, 2H). |
| D64 | 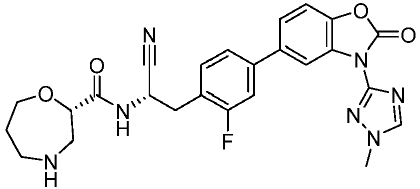 | 506.5 | ¹H NMR (400 MHz, DMSO-*d₆*): δ 8.81 (d, *J* = 8.5 Hz, 1H), 8.71 (s, 1H), 7.83 (d, *J* = 1.4 Hz, 1H), 7.60 - 7.52 (m, 2H), 7.52 - 7.44 (m, 3H), 5.05 (q, *J* = 8.2 Hz, 1H), 4.11 (dd, *J* = 8.5, 3.5 Hz, 1H), 3.98 (s, 3H), 3.91 - 3.83 (m, 1H), 3.75 (ddd, *J* = 12.0, 9.4, 5.9 Hz, 1H), 3.31 (dd, *J* = 13.6, 7.7 Hz, 1H), 3.17 (ddd, *J* = 14.4, 11.0, 5.9 Hz, 2H), 2.95 - 2.85 (m, 1H), 2.78 - 2.70 (m, 1H), 2.66 (dd, *J* = 14.1, 8.5 Hz, 1H), 1.85 - 1.73 (m, 2H). |
| D65 | 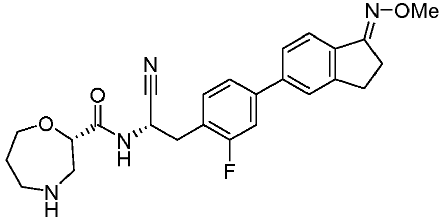 | 451.5 | N/A |
| D66 | 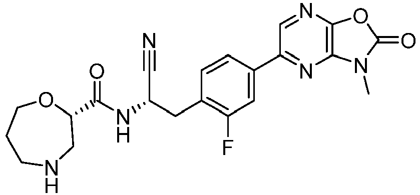 | 441.4 | ¹H NMR (400 MHz, DMSO-*d*₆): δ 8.27 (d, *J* = 8.0 Hz, 1 H), 7.98 (m, 1 H), 782 (m, 1 H), 4.52 (m, 1 H), 4.13-4.02 (m, 2 H), 3.81 (m, 1 H), 3.72 (m, 1 H), 3.24-3.11 (m, 3 H), 2.88-2.76 (m, 1 H), 2.47- 2.37 (m, 2H). |
| D67 | 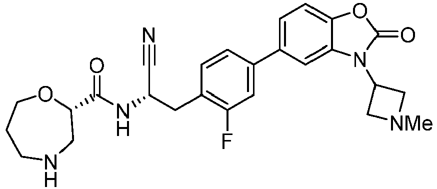 | 494.5 | ¹H NMR (400 MHz, DMSO-*d*₆): δ 8.72 (d, *J* = 8.0 Hz, 1 H), 7.78 (s, 1H), 7.57-7.38 (m, 5H), 5.05 (t, 1H), 4.93 (t, 1H), 4.02 (m, 1 H), 3.87-3.58 (m, 6 H), 3.52-3.11 (m, 3 H), 2.87 (m, 1H), 2.74-2.43 (m, 2 H), 1.67 (m, 2 H). |
| D68 | 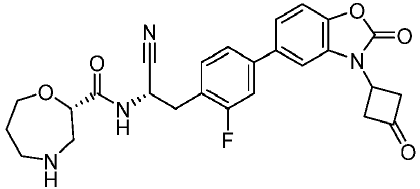 | 493.5 | ¹H NMR (400 MHz, DMSO-*d*₆): δ 8.73 (d, *J* = 8.0 Hz, 1 H), 7.65-7.34 (m, 6H), 5.11 (m, 2H), 4.04-3.52 (m, 6H), 3.42-3.11 (m, 3 H), 2.83-2.51 (m, 2 H), 1.75 (m, 2H). |
| D69 | 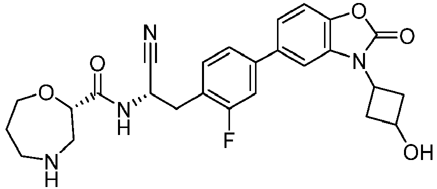 | 495.5 | N/A |
| D70 | 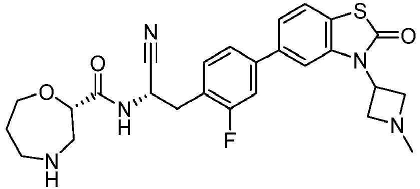 | 510.6 | ¹H NMR (400 MHz, DMSO-*d*₆): δ 8.17 (d, *J* = 8.0 Hz, 1 H), 7.91-7.66 (m, 5H), 5.02 (m, 2H), 4.13-3.82 (m, 5H), 3.31-3.17 (m, 4 H), 2.62 (s, 3 H). |
| D71 | 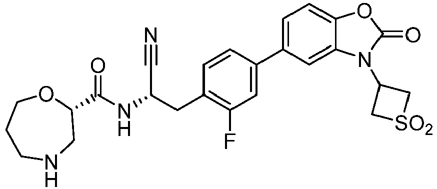 | 529.5 | ¹H NMR (400 MHz, DMSO-*d*₆): δ 8.27 (d, *J* = 8.0 Hz, 1 H), 7.96 (s, 1H), 7.63-7.51 (m, 5H), 5.48 (m, 1H), 5.11-4.54 (m, 4H), 4.02-3.58 (m, 3H), 3.37-2.99 (m, 2 H), 2.01 (m, 2H). |
| D72 | 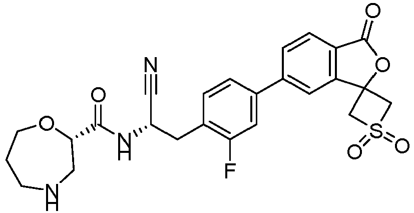 | 514.5 | ¹H NMR (400 MHz, DMSO-*d*₆): δ 8.77 (d, *J* = 8.0 Hz, 1 H), 8.44 (s, 1H), 8.02 (dd, 1H), 7.84 (d, 1H), 7.73 (dd, 1H), 7.62 (dd, 1H), 7.56 (t, 1H), 5.31 (m, 2H), 5.07 (m, 1H), 4.73 (m, 2H), 4.14-3.66 (m, 4 H), 3.41-3.05 (m, 3 H), 2.77 (m, 1H), 2.66-2.51 (m, 3H), 1.76 (m, 2H). |
| D73 | 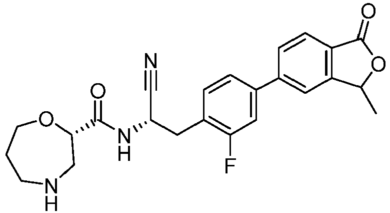 | 438.45 | 1H NMR (400 MHz, DMSO-*d*₆): δ 8.73 (d, J = 8.0 Hz, 1 H), 8.06 (s, 1 H), 7.94-7.88 (m, 2 H), 7.67-7.62 (m, 2 H), 7.51 (t, J = 7.8 Hz, 1 H), 5.76-5.71 (m, 1 H), 5.11-5.05 (m, 1 H), 4.02-3.99 (m, 1 H), 3.89-3.83 (m, 1 H), 3.75-3.69 (m, 1H), 3.29-3.19 (m, 2 H), 3.07-3.02 (m, 1 H), 2.80-2.74 (m, 1 H), 5.67-2.51 (m, 2 H), 1.79-1.66 (m, 2H), 1.62 (d, J = 6.4 Hz, 3 H). |
| D74 | 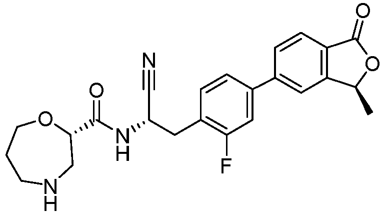 | 438.5 | ¹H NMR (400 MHz, DMSO-*d*₆): δ 8.72 (d, J = 8.8 Hz, 1H), 8.05 (s, 1H), 7.93-7.87 (m, 2H), 7.70-7.62 (m, 2H), 7.53-7.49 (m, 1H), 5.76-5.71 (m, 1H), 5.08 (q, J = 8 Hz, 1H), 4.01-3.99 (m, 1H), 3.88-3.84 (m, 1H), 3.75-3.69 (m, 1H), 3.25-3.20 (m, 2H), 3.06-3.02 (m, 1H), 2.78-2.73 (m, 1H), 2.62-2.54 (m, 2H), 1.74-1.71 (m, 2H), 1.62 (d, J = 6.4 Hz, 3H). |
| D75 | 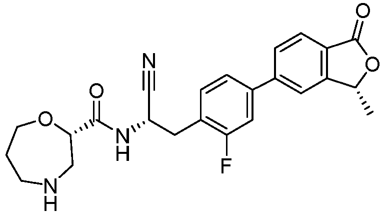 | 438.5 | ¹H NMR (400 MHz, DMSO-*d*₆): δ 8.73 (d, *J* = 8.8 Hz, 1H), 8.06 (s, 1H), 7.94-7.87 (m, 2H), 7.70-7.63 (m, 2H), 7.53-7.49 (m, 1H), 5.76-5.71 (m, 1H), 5.08 (q, *J* = 8.8 Hz, 1H), 4.01-3.98 (m, 1H), 3.89-3.83 (m, 1H), 3.75-3.69 (m, 1H), 3.35-3.20 (m, 2H), 3.06-3.02 (m, 1H), 2.80-2.73 (m, 1H), 2.70-2.56 (m, |
| | | | 2H), 1.77-1.68 (m, 2H), 1.62 (d, J = 6.4 Hz, 3H). |
| D76 | 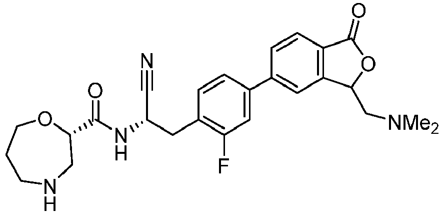 | 481.4 | ¹H NMR (400 MHz, DMSO-*d*₆) δ 8.76 (d, *J* = 8.4 Hz, 1H), 8.06 (s, 1H), 7.98 - 7.86 (m, 2H), 7.69 (d, *J* = 11.4 Hz, 1H), 7.64 (d, *J* = 8.0 Hz, 1H), 7.52 (t, *J* = 7.9 Hz, 1H), 5.76 (dd, *J* = 7.4, 3.3 Hz, 1H), 5.08 (q, *J* = 8.1 Hz, 1H), 4.03 (dd, *J* = 8.0, 3.5 Hz, 1H), 3.93 - 3.81 (m, 1H), 3.79 - 3.68 (m, 1H), 3.30 - 3.19 (m, 2H), 3.05 (td, *J* = 14.1, 3.3 Hz, 2H), 2.85 - 2.76 (m, 1H), 2.67 - 2.54 (m, 3H), 2.29 (s, 6H), 1.85 - 1.63 (m, 2H). |
| D77 | 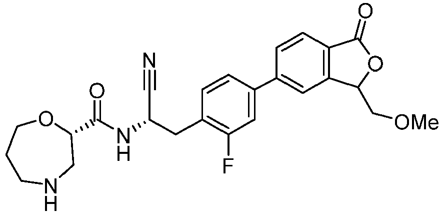 | 468.5 | ¹H NMR (400 MHz, DMSO-*d₆*): δ 8.73 (d, *J* = 7.9 Hz, 1H), 8.08 (d, *J* = 1.9 Hz, 1H), 7.99 - 7.86 (m, 2H), 7.74 - 7.58 (m, 2H), 7.52 (t, *J* = 7.9 Hz, 1H), 5.81 (dd, *J* = 4.9, 2.8 Hz, 1H), 5.08 (q, *J* = 8.2 Hz, 1H), 4.06 - 3.96 (m, 2H), 3.91-3.82 (m, 1H), 3.82 - 3.68 (m, 2H), 3.32 - 3.29 (m, 1H), 3.27 (d, *J* = 0.5 Hz, 3H), 3.23 (dd, *J* = 13.7, 8.8 Hz, 1H), 3.04 (dd, *J* = 14.2, 3.6 Hz, 1H), 2.82 - 2.72 (m, 1H), 2.66 - 2.59 (m, 1H), 2.56 (dd, *J* = 14.2, 8.0 Hz, 1H), 1.83 - 1.63 (m, 2H). |
| D78 | 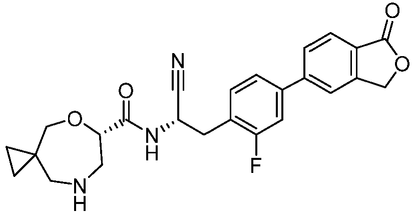 | 450.5 | ¹H NMR (400 MHz, DMSO-*d₆*): δ 8.64 (d, 1H), 8.02 (s 1H), 7.82 (s, 2H), 7.68 - 7.47 (m, 3H), 5.08 (m 1H), 4.17 (m, 1H), 3.61-3.18 (m, 5H), 2.65- - 2.41 (m, 3H), 3.32-3.29 (m, 1H), 3.27 (d, *J* = 0.5 Hz, 3H), 3.23 (dd, *J* = 13.7, 8.8 Hz, 1H), 0.51-0.33 (m, 4H). |
| D79 | 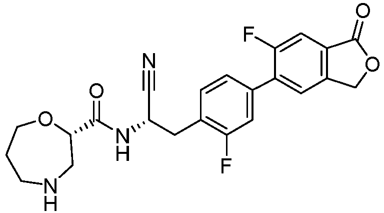 | 442.4 | ¹H NMR (400 MHz, DMSO-*d₆*): δ 7.71 (d, 1H), 7.56 (d, 1H), 7.46 (t, 1H), 7.37 - 7.25 (m, 3H), 5.22 (t, 1H), 4.11 (m, 1H), 3.82 (m, 1H), 3.24 (m, 2H), 2.92 (m, 2H), 1.83 (m, 2H). |
| D80 | 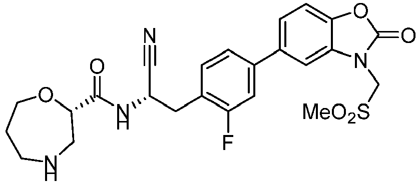 | 517.5 | ¹H NMR (400 MHz, DMSO-*d₆*): δ 8.75 (d, 1H), 7.86 (s, 1H), 7.57-7.42 (m, 3H), 5.20 (s, 2H), 5.04 (m, 1H), 4.02 (m, 1H), 3.74 (m, 1H), 3.65 (m, 1H), 3.58 - 3.11 (m, 6H), 2.77 (m, 1H), 2.62-2.51 (m, 2H), 1.68 (m, 2H). |
| D81 | 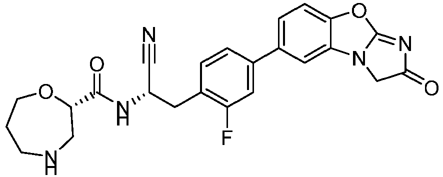 | 464.5 | ¹H NMR (400 MHz, DMSO-*d₆*): δ 7.91 (s, 1H), 7.63 -7.45 (m, 5H), 5.20 (s, 2H), 5.11 (m, 1H), 4.04 (m, 1H), 3.86 (m, 1H), 3.75 (m, 1H), 3.51 - 3.11 (m, 3H), 2.79-2.57 (m, 3H), 1.77 (m, 2H). |
| D82 | 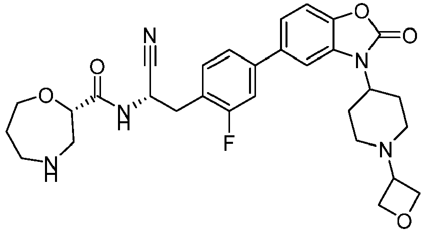 | 564.3 | ¹H NMR (400 MHz, DMSO-*d₆*): δ 7.71-7.28 (m, 6H), 5.01 (m, 1H), 4.58-4.47 (m, 4H), 4.21-3.67 (m, 5H), 3.42 (m, 1H), 3.27 - 3.11 (m, 4H), 2.83-2.51 (m, 5H), 2.38 (m, 1H), 1.97(m, 1H), 1.81-1.65 (m, 4H). |
| D83 | 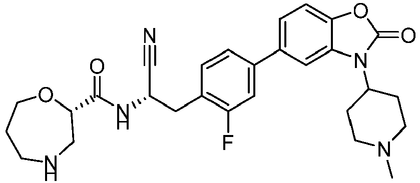 | 522.6 | ¹H NMR (400 MHz, DMSO-*d₆*): δ 7.71-7.38 (m, 6H), 5.05 (m, 1H), 4.27-3.65 (m, 4H), 3.41-2.77 (m, 6H), 2.65 - 2.47 (m, 4H), 2.37 (m, 2H), 2.12 (m, 2H), 1.69(m, 4H). |
| D85 | 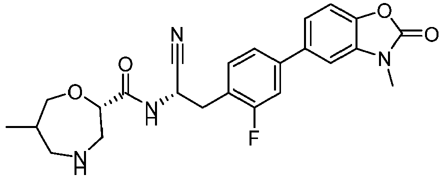 | 453.5 | ¹H NMR (400 MHz, DMSO-*d₆*): δ 8.72 (d, *J* = 8.4 Hz, 1H), 7.64 (d, *J* = 1.4 Hz, 1H), 7.61- 7.52 (m, 2H), 7.45 (dd, *J* = 8.8, 7.2 Hz, 2H), 7.39 (d, *J* = 8.4 Hz, 1H), 5.05 (q, *J* = 8.2 Hz, 1H), 4.02 (dd, *J* = 6.7, 4.6 Hz, 1H), 3.82 (dd, *J* = 12.4, 3.9 Hz, 1H), 3.39 (s, 3H), 3.35 - 3.32 (m, 2H), 3.19 (dd, *J* = 13.3, 8.6 Hz, 1H), 3.03 (dd, *J* = 14.2, 4.4 Hz, 1H), 2.78 (dd, *J* = 13.3, 4.4 Hz, 1H), 2.70 (dd, *J* = 14.2, 7.0 Hz, 1H), 2.38 (dd, *J* = 13.2, 7.4 Hz, 1H), 2.02 - 1.79 (m, 1H), 0.75 (d, *J* = 6.9 Hz, 3H). |
| D86 | 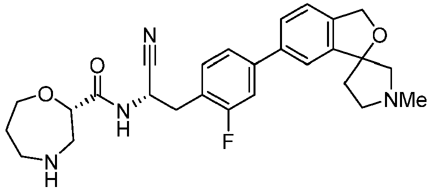 | 479.6 | ¹H NMR (400 MHz, DMSO-*d₆*): δ 7.63-7.32 (m, 6H), 5.07 (m, 1H), 4.87 (s, 2H), 4.11-3.72 (m, 4H), 3.37 - 3.05 (m, 4H), 2.87-2.58 (m, 7H), 2.38 (s, 3H), 2.1 (m, 2H), 1.73 (m, 2H). |
| D87 | 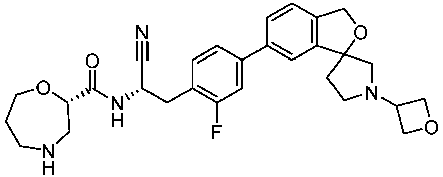 | 521.6 | ¹H NMR (400 MHz, DMSO-*d₆*): δ 7.68-7.32 (m, 6H), 5.11 (m, 1H), 4.98 (s, 2H), 4.57 (m, 1H), 4.52 (m, 1H), 4.11-3.63 (m, 5H), 3.31-3.12 (m, 3H), 2.91-2.62 (m, 7H), 2.25-2.11 (m, 2H), 1.72 (m, 2H). |
| D88 | 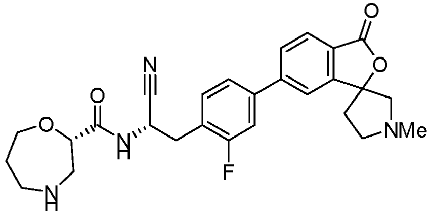 | 493.6 | ¹H NMR (400 MHz, DMSO-*d₆*): δ 8.07 (s, 1H), 7.93 (d, 1H), 7.86 (d, 1H), 7.75 (d, 1H), 7.67 (d, 1H), 7.52 (t, 1H), 5.09 (m, 1H), 4.11 (d, 1H), 3.89-3.64 (m, 2H), 3.22 (m, 1H), 3.11-2.5 (m, 8H), 2.38 (s, 3H), 2.27 (m, 1H), 1.73 (m, 2H). |
| D89 | 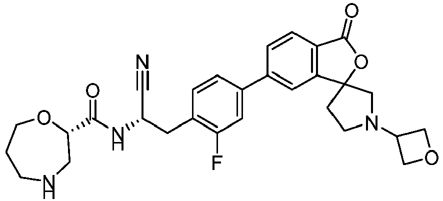 | 535.6 | ¹H NMR (400 MHz, DMSO-*d₆*): δ 8.11 (s, 1H), 7.94 (d, 1H), 7.86 (d, 1H), 7.72 (d, 1H), 7.65 (d, 1H), 7.52 (t, 1H), 5.08 (m, 1H), 4.63-4.52 (m, 4H), 4.11 (dd, 1H), 3.92-3.66 (m, 3H), 3.31-2.98 (m, 5H), 2.83 (m, 2H), 2.71-2.55 (m, 5H), 2.27 (m, 1H), 1.76 (m, 2H). |
| D90 | 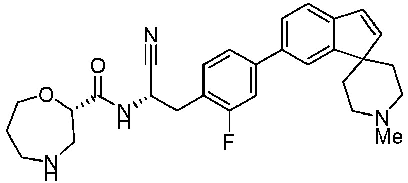 | 489.6 | ¹H NMR (400 MHz, DMSO-*d₆*): δ 7.76 (s, 1H), 7.59-7.4 (m, 5H), 7.02 (d, 1H), 6.82 (d, 1H), 5.05 (m, 1H), 4.12-3.59 (m, 4H), 3.32-3.04 (m, 4H), 2.91-2.52 (m, 4H), 2.83 (m, 2H), 2.31 (s, 3H), 1.76 (m, 2H), 1.22 (m, 3H). |
| D91 | 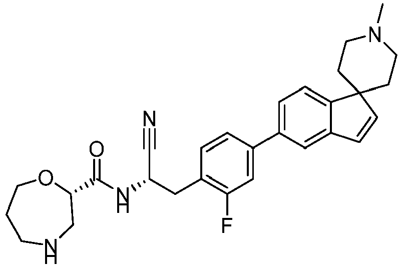 | 489.6 | ¹H NMR (400 MHz, DMSO-*d₆*): δ 7.45 (s, 1H), 7.38-7.15 (m, 5H), 6.84 (d, 1H), 6.74 (d, 1H), 5.12 (m, 1H), 4.14-3.91 (m, 4H), 3.69 (m, 1H), 3.26-3.11 (m, 3H), 2.94-2.75 (m, 5H), 2.36 (s, 3H), 1.75 (m, 2H), 1.33 (m, 3H). |
| D92 | 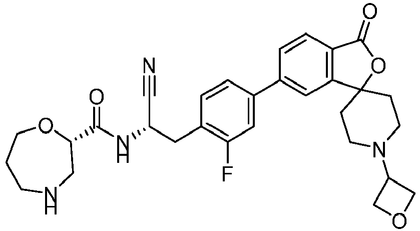 | 549.6 | ¹H NMR (400 MHz, DMSO-*d₆*): δ 7.84 (s, 1H), 7.62-7.39 (m, 5H), 7.04 (d, 1H), 6.82 (d, 1H), 5.06 (m, 1H), 4.59 (t, 2H), 4.49 (t, 2H), 4.02 (m, 1H), 3.86 (m, 1H), 3.73 (m, 1H), 3.56 (m, 1H), 3.2-3.04 (m, 3H), 2.8-2.57 (m, 6H), 2.31-2.16 (m, 4H), 1.74 (m, 2H), 1.32 (m, 2H). |
| D93 | 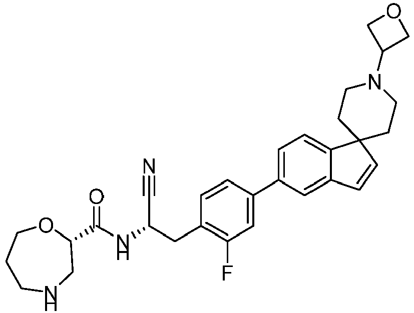 | 531.6 | ¹H NMR (400 MHz, DMSO-*d₆*): δ 744 (s, 1H), 7.4-7.25 (m, 5H), 6.82 (d, 1H), 6.73 (d, 1H), 5.12 (m, 1H), 4.65 (m, 4H), 3.99-3.57 (m, 3H), 3.17-2.79 (m, 8H), 2.17 (m, 4H), 1.82 (m, 2H). |
| D94 | 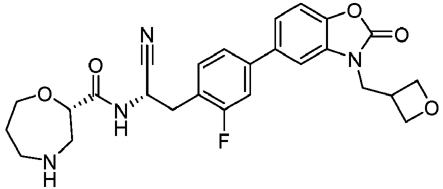 | 495.5 | N/A |
| D95 | 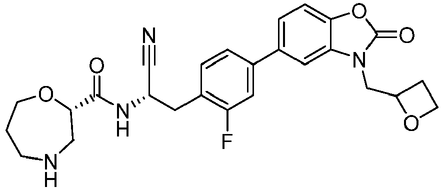 | 495.5 | ¹H NMR (400 MHz, DMSO-*d₆*): δ 7.52-7.31 (m, 7H), 5.18 (m, 2H), 4.61 (m, 1H), 4.43 (m, 1H), 4.21-4.02 (m, 4H), 3.78 (m, 1H), 3.37-3.21 (m, 3H), 2.98-2.76 (m, 4H), 2.62 (m, 1H), 1.84 (m, 2H). |
| D96 | 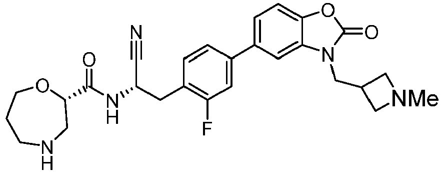 | 508.6 | ¹H NMR (400 MHz, DMSO-*d₆*): δ 7.7 (1H, s), 7.61-7.4 (m, 5H), 5.05 (m, 2H), 4.07 (d, 1H), 4.02 (m, 1H), 3.85 (m, 1H), 3.73 (m, 1H), 3.43-3.16 (m, 5H), 2.96 (m, 1H), 2.83-2.51 (m, 4H), 2.42 (s, 3H), 1.84 (m, 2H). |
| D97 | 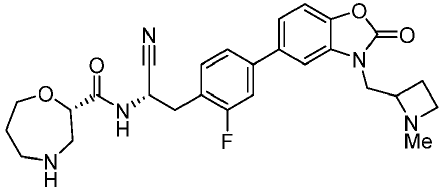 | 508.6 | ¹H NMR (400 MHz, DMSO-*d₆*): δ 7.68 (1H, s), 7.58-7.4 (m, 5H), 5.03 (m, 2H), 4.01-3.7 (m, 5H), 3.34-3.04 (m, 5H), 2.79-2.57 (m, 4H), 2.15 (s, 3H), 1.97-1.72 (m, 4H). |
| D98 | 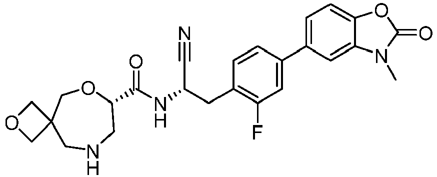 | 481.5 | ¹H NMR (400 MHz, DMSO-*d₆*): δ 7.65 (1H, s), 7.6-7.39 (m, 5H), 5.05 (m, 1H), 4.41 (d, 1H), 4.25 (m, 2H), 4.17 (d, 1H), 4.03 (d, 1H), 3.94 (m, 1H), 3.86 (d, 1H), 3.4-2.53 (m, 7H), 2.22 (s, 3H). |
| D99 | 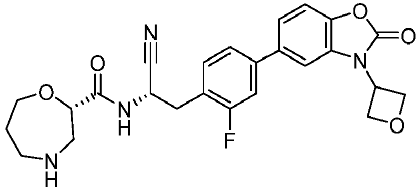 | 481.5 | ¹H NMR (400 MHz, DMSO-*d₆*): δ 7.76 (1H, s), 7.58-7.45 (m, 5H), 5.51 (m, 1H), 5.14 (m, 2H), 5.07 (m, 1H), 5.05 (m, 1H), 4.94 (m, 1H), 4.01 (dd, 2H), 3.88-3.69 (m, 2H), 3.23-3.03 (m, 3H), 2.81-2.49 (m, 3H), 1.75 (m, 2H). |
| D100 | 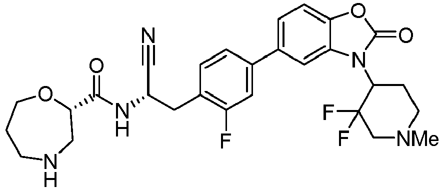 | 558.6 | ¹H NMR (400 MHz, DMSO-*d₆*): δ 8.73 (d, *J* = 8.4 Hz, 1H), 7.61 (s, 1H), 7.58 -7.40 (m, 5H), 5.13-4.94 (m, 1H), 4.82 - 4.63 (m, 1H), 4.02 (dd, *J* = 7.9, 3.4 Hz, 1H), 3.91 - 3.81 (m, 1H), 3.79 - 3.67 (m, 1H), 3.20 (dd, *J* = 13.4, 8.4 Hz, 3H), 3.10 - 3.02 (m, 1H), 3.02 - 2.85 (m, 2H), 2.84 - 2.74 (m, 1H), 2.69 - 2.53 (m, 3H), 2.39 - 2.22 (m, 4H), 2.01 (br, 1H), 1.82 - 1.64 (m, 2H). |
| D101 | 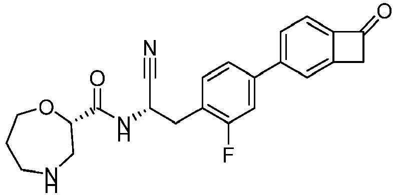 | 409.5 | ¹H NMR (400 MHz, DMSO-*d₆*): δ 7.98 (s, 1H), 7.81 (d, 1H), 7.66-7.45 (m, 4H), 5.09 (m, 1H), 4.03 (s, 2H), 4.01 (m, 1H), 3.87 (m, 1H), 3.74 (m, 1H), 3.22 - 3.03 (m, 3H), 2.78 (m, 1H), 265 - 2.55 (m, 2H), 1.75 (m, 2H). |
| D102 | 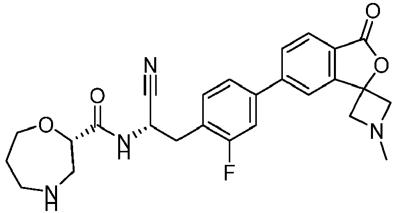 | 479.6 | ¹H NMR (400 MHz, DMSO-*d₆*): δ 8.21 (s, 1H), 7.92 (d, 1H), 7.88 (d, 1H), 7.68 (d, 1H), 7.55 (d, 1H), 7.53 (t, 1H), 5.09 (t, 1H), 4.04 (m, 1H), 3.87 (m, 1H), 3.77-3.64 (m, 4H), 3.26-3.21 (m, 1H), 3.07 (dd, 1H), 2.85-2.57 (m, 4H), 2.29 (s, 3H), 1.76 (m, 2H). |
| D103 | 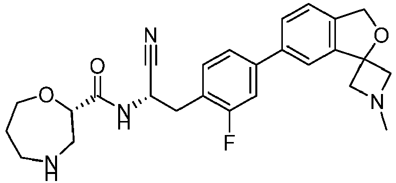 | 465.5 | ¹H NMR (400 MHz, DMSO-*d₆*): δ 7.86 (s, 1H), 7.65 (d, 1H), 7.63-7.36 (m, 5H), 5.05 (m, 1H), 4.11 (m, 1H), 3.89-3.57 (m, 7H), 3.34-3.16 (m, 3H), 2.78 (m, 1H), 2.61-2.52 (m, 4H), 2.38 (s, 3H), 1.75 (m, 2H). |
| D104 | 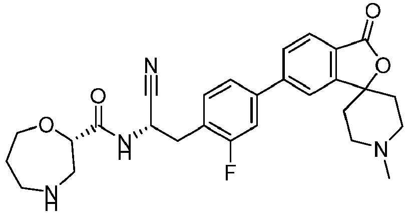 | 507.6 | ¹H NMR (400 MHz, DMSO-*d₆*): δ 8.12 (s, 1H), 7.91 (d, 1H), 7.74 (d, 1H), 7.53 (d, 1H), 7.5 (t, 1H), 5.07 (t, 1H), 4.06 (m, 1H), 3.87 (m, 1H), 3.77 (m, 1H), 3.31-3.07 (m, 3H), 2.84 (d, 2H), 2.67-2.52 (m, 2H), 2.4-2.31 (m, 4H), 2.28 (s, 3H), 1.76 (m, 2H), 1.64 (m, 2H). |
| D105 | 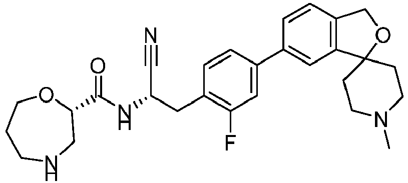 | 493.6 | ¹H NMR (400 MHz, DMSO-*d₆*): δ 7.62-7.56 (m, 4H), 7.45 (t, 1H), 7.35 (d, 1H), 5.07 (m, 1H), 5.03 (s, 2H), 4.03 (m, 1H), 3.87 (m, 1H), 3.75 (m, 1H), 3.31-3.06 (m, 4H), 2.72-2.58 (m, 5H), 2.33-2.24 (m, 2H), 2.22 (s, 3H), 1.75 (m, 2H), 1.63 (m, 2H). |
| D106 | 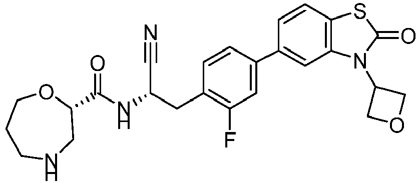 | 497.6 | ¹H NMR (400 MHz, DMSO-*d₆*): δ 8.72 (d, 1H), 7.8-7.78 (m, 2H), 7.64-7.46 (m, 3H), 5.75 (m, 1H), 5.19-4.94 (m, 5H), 4.02 (m, 1H), 3.87 (m, 1H), 3.75 (m, 1H), 3.3-3.03 (m, 3H), 2.77 (m, 1H), 2.65-2.51 (m, 2H), 1.74 (m, 2H). |
| D108 | 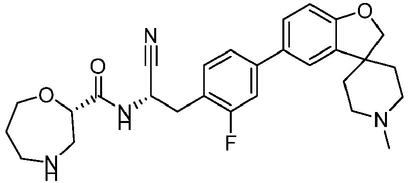 | 493.6 | N/A |
| D109 | 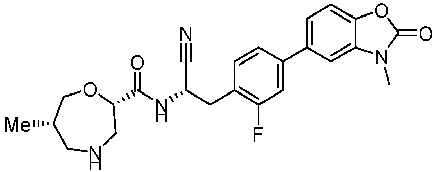 | 453.5 | ¹H NMR (400 MHz, DMSO-*d₆*): δ 8.79 (d, 1H), 7.65 (d, 1H), 7.6-7.38 (m, 5H), 5.05 (m, 1H), 4.01 (m, 1H), 3.74 (m, 1H), 3.53-3.09 (m, 7H), 2.97 (dd, 1H), 2.51 (s, 3H), 2.43 (dd, 1H), 1.87 (m, 2H). |
| D110 | 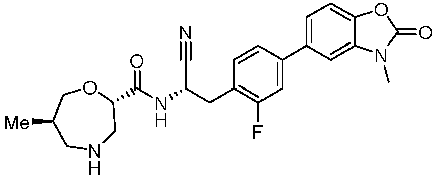 | 453.5 | ¹H NMR (400 MHz, DMSO-*d₆*): δ 8.69 (d, 1H), 7.67-7.35 (m, 6H), 5.05 (m, 1H), 3.98 (m, 1H), 3.81 (m, 1H), 3.39-2.98 (m, 4H), 2.78-2.66 (m, 2H), 2.35 (m, 1H), 1.89 (m, 2H). |
| D111 | 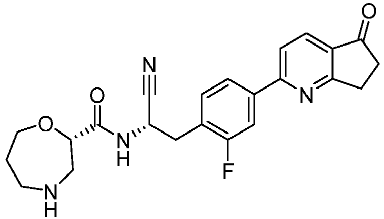 | 423.5 | ¹H NMR (400 MHz, DMSO-*d₆*): δ 8.11 (d, 1H), 7.9 (d, 1H), 7.75 (d, 1H), 7.49 (t, 1H), 5.22 (m, 1H), 4.12-4.02 (m, 2H), 3.79 (m, 1H), 3.35-3.27 (m, 5H), 2.94-2.84 (m, 5H), 1.89 (m, 2H). |
| D112 | 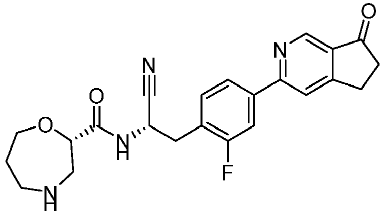 | 423.5 | ¹H NMR (400 MHz, DMSO-*d₆*): δ 7.91-7.85 (m, 3H), 7.48 (m, 1H), 7.28 (m, 1H), 5.22 (m, 1H), 4.12-4.0 (m, 2H), 3.78 (m, 1H), 3.33-3.21 (m, 5H), 2.99-2.78 (m, 5H), 1.89 (m, 2H). |
| D113 | 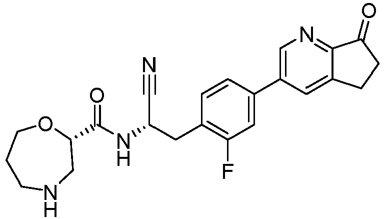 | 423.5 | ¹H NMR (400 MHz, DMSO-*d₆*): δ 8.98 (d, 1H), 8.03 (d, 1H), 7.53-7.28 (m, 4H), 5.22 (m, 1H), 4.13-4.02 (m, 2H), 3.81 (m, 1H), 3.35-3.23 (m, 5H), 3.11-2.84 (m, 5H), 1.91 (m, 2H). |
| D114 | 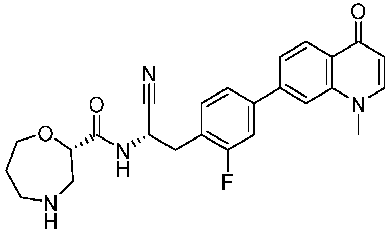 | 449.5 | ¹H NMR (400 MHz, DMSO-*d₆*): δ 8.73 (d, 1H), 8.21 (d, 1H), 7.99 (d, 1H), 7.86 (d, 1H), 7.79-7.69 (m, 3H), 7.52 (t, 1H), 6.05 (d, 1H), 5.09 (m, 1H), 4.01 (dd, 1H), 3.94 (s, 3H), 3.9-3.71 (m, 2H), 3.23-3.02 (m, 2H), 2.81-2.49 (m, 3H), 1.77 (m, 2H). |
| D115 | 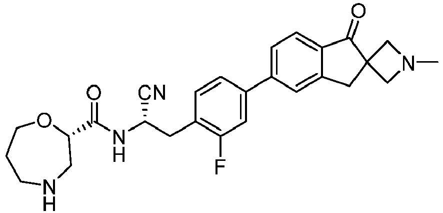 | 477.6 | ¹H NMR (400 MHz, DMSO-*d₆*): δ 8.74 (d, 1H), 8.21 (d, 1H), 7.92 (d, 1H), 7.78-7.47 (m, 5H), 5.09 (m, 1H), 4.02 (m, 1H), 3.85 (m, 1H), 3.74 (m, 1H), 3.54 (s, 2H), 3.36-3.23 (m, 7H), 3.05 (m, 1H), 2.77 (m, 1H), 2.65-2.53 (m, 2H), 2.27 (s, 3H), 1.73 (m, 2H). |
| D116 | 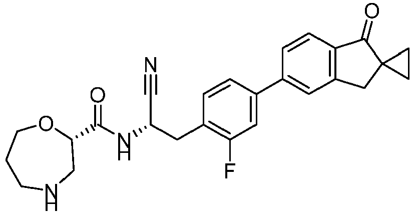 | 448.5 | ¹H NMR (400 MHz, DMSO-*d₆*): δ 8.72 (d, 1H), 7.96 (s, 1H), 7.82-7.47 (m, 5H), 5.09 (m, 1H), 4.0 (dd, 1H), 3.89-3.69 (m, 2H), 3.22 (s, 2H), 3.24-3.02 (m, 2H), 2.78 (m, 1H), 2,64-2.51 (m, 4H), 1.75 (m, 2H), 1.27 (d, 4H). |
| D117 | 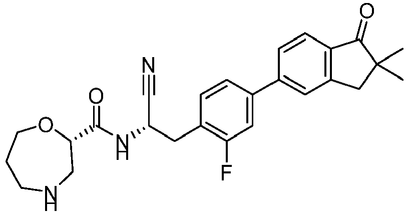 | 450.5 | ¹H NMR (400 MHz, DMSO-*d₆*): δ 8.73 (d, 1H), 7.89 (s, 1H), 7.77-7.47 (m, 5H), 5.09 (m, 1H), 4.02 (m, 1H), 3.87 (m, 1H), 3.74 (m, 1H), 3.34-3.25 (m, 3H), 3.07 (m, 3H), 2.79 (m, 1H), 2,69-2.51 (m, 2H), 1.77 (m, 2H), 1.17 (s, 6H). |
| D118 | 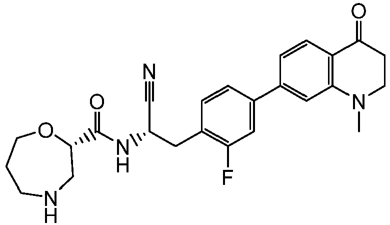 | 451.5 | ¹H NMR (400 MHz, DMSO-*d₆*): δ 8.72 (d, 1H), 7.75 (d, 1H), 7.64-7.43 (m, 5H), 7.06-7.01 (m, 2H), 5.08 (m, 1H), 4.0 (m, 1H), 3.88 (m, 1H), 3.76 (m, 1H), 3.50 (m, 2H), 3.34-3.21 (m, 3H), 3.05 (s, 3H), 2.81-2.52 (m, 4H), 1.74 (m, 2H). |
| D119 | 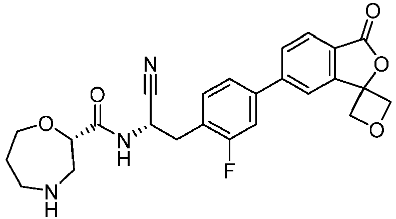 | 466.5 | ¹H NMR (400 MHz, DMSO-*d₆*): δ 8.73 (d, 1H), 8.42 (s, 1H), 8.01-7.52 (m, 5H), 5.14-4.98 (m, 5H), 4.01-3.65 (m, 3H), 3.27-3.02 (m, 2H), 2.81-2.51 (m, 3H), 1.75 (m, 2H). |
| D 120 | 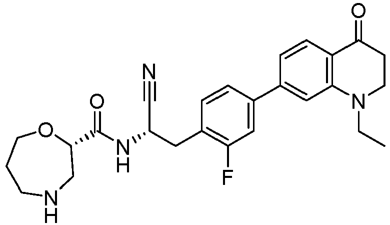 | 465.3 | 1H NMR (400 MHz, DMSO-*d*₆) δ 8.72 (d, *J =* 8.4 Hz, 1H), 7.74 (d, *J* = 8.4 Hz, 1H), 7.60 - 7.53 (m, 2H), 7.48 - 7.43 (m, 1H), 7.05 (s, 1H), 6.96 - 6.94 (m, 1H), 5.08 - 5.02 (m, 1H), 4.01 - 3.98 (m, 1H), 3.88 - 3.82 (m, 1H), 3.75 - 3.69 (m, 1H), 3.62 - 3.57 (m, 2H), 3.53-3.46 (m, 3H), 3.31 - 3.28 (m, 1H), 3.25 - 3.17 (m, 1H), 3.03 (dd, *J* = 14.4, 4 Hz, 1H), 2.79 - 2.73 (m, 1H), 2.63 - 2.60 (m, 2H), 2.58-2.54 (m, 1H), 1.76 - 1.70 (m, 2H), 1.11 (t, *J* = 7.2 Hz, 3H). |
| D122 | 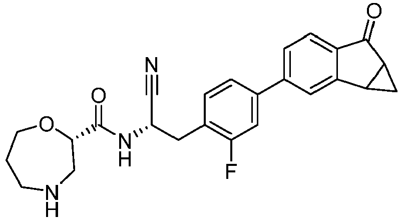 | 434.1 | ¹H NMR (400 MHz, DMSO-*d*₆) δ 10.20 (br, 1H), 8.77 (d, *J* = 8.6 Hz, 1H), 8.19 (d, *J* = 8.7 Hz, 2H), 7.79 (dd, *J* = 8.7, 1.9 Hz, 1H), 7.71-7.63 (m, 2H), 7.49 (t, *J* = 8.1 Hz, 1H), 7.42 (d, *J* = 8.1 Hz, 1H), 7.38 - 7.30 (m, 1H), 6.88 (dd, *J* = 7.4, 0.8 Hz, 1H), 5.07 (dd, *J* = 16.1, 8.4 Hz, 1H), 4.04 (dd, *J* = 8.1, 3.6 Hz, 1H), 3.92 - 3.82 (m, 1H), 3.80-3.70 (m, 1H), 3.30 (d, *J* = 7.5 Hz, 1H), 3.22 (dd, *J* = 13.6, 8.7 Hz, 1H), 3.09 (dd, *J* = 14.2, 3.6 Hz, 1H), 2.86 - 2.77 (m, 1H), 2.70-2.55 (m, 2H), 1.83 - 1.67 (m, 2H). |
| D123 | 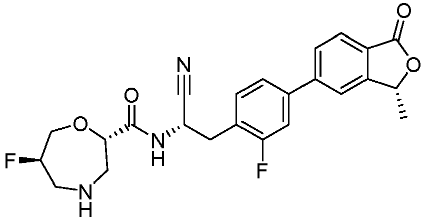 | 455.9 | ¹H NMR (400 MHz, DMSO-*d*₆) δ 8.82 (d, *J* = 8.4 Hz, 1H), 8.06 (s, 1H), 7.94 - 7.88 (m, 2H), 7.71-7.63 (m, 2H), 7.53 - 7.49 (m, 1H), 5.76 - 5.71 (m, 1H), 5.06 (t, *J* = 8 Hz, 1H), 4.86 - 4.70 (m, 1H), 4.13 - 3.97 (m, 2H), 3.82 - 3.72 (m, 1H), 3.31 - 3.28 (m, 1H), 3.23-3.05 (m, 3H), 2.86 - 2.73 (m, 1H), 2.43 - 2.36 (m, 1H), 1.62 (d, *J* =6.8 Hz, 3H). |
| D124 | 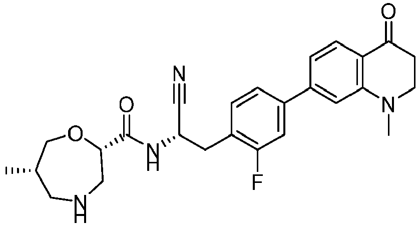 | 465.3 | 1H NMR (400 MHz, DMSO-*d*₆) δ 8.69 (d, *J* = 8.4 Hz, 1H), 7.75 (d, *J* = 8.0 Hz, 1H), 7.62 - 7.54 (m, 2H), 7.47 - 7.43 (m, 1H), 7.04 - 7.01 (m, 2H), 5.09 - 5.01 (m, 1H), 4.01 (dd, *J* = 9.2, 3.6 Hz, 1H), 3.72 (dd, *J* = 12.4, 3.6 Hz, 1H), 3.51 - 3.47 (m, 2H), 3.44 - 3.38 (m, 1H), 3.23 - 3.17 (m, 2H), 3.12 - 3.05 (m, 4H), 2.90 - 2.85 (m, 1H), 2.65 (t, *J* = 6.8 Hz, 2H), 2.40 (dd, *J* = 14.0, 9.6 Hz, 1H), 2.15 (dd, *J* = 13.2, 9.6 Hz, 1H), 1.90 - 1.79 (m, 1H), 0.78 (d, *J* = 7.2 Hz, 3H). |
| D125 | 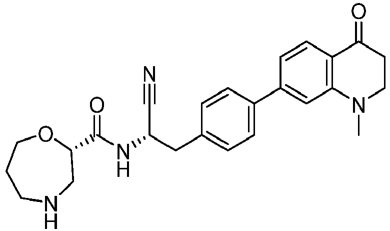 | 433.3 | 1H NMR (400 MHz, DMSO-*d*₆) δ 8.63 (d, *J* = 8.4 Hz, 1H), 7.75 (d, *J* = 8.4 Hz, 1H), 7.67 (d, *J* = 8.4 Hz, 2H), 7.39 (d, *J* = 8.4 Hz, 2H), 7.00 - 6.97 (m, 2H), 5.05 - 5.00 (m, 1H), 4.00 (dd, *J* = 7.6, 3.2 Hz, 1H), 3.85 - 3.80 (m, 1H), 3.74 - 3.71 (m, 1H), 3.48 (t, *J* = 6.7 Hz, 2H), 3.26 - 3.17 (m, 2H), 3.04 - 3.00 (m, 4H), 2.78 - 2.73 (m, 1H), 2.66 - 2.52 (m, 4H), 1.75 - 1.70 (m, 2H). |
| D127 | 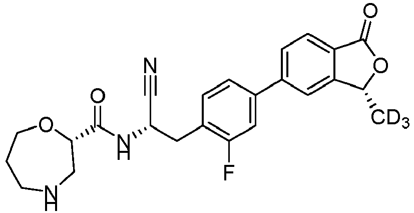 | 441.1 | 1H NMR (400 MHz, DMSO-*d*₆) δ 8.73 (d, *J* = 8.4 Hz, 1H), 8.06 (s, 1H), 7.94 - 7.87 (m, 2H), 7.71-7.63 (m, 2H), 7.53 - 7.49 (m, 1H), 5.72 (s, 1H), 5.10 - 5.04 (m, 1H), 4.00 (dd, *J* = 8.0, 4.0 Hz, 1H), 3.87 - 3.82 (m, 1H), 3.75 - 3.69 (m, 1H), 3.33 - 3.19 (m, 2H), 3.03 (dd, *J* = 14.0, 3.2 Hz, 1H), 2.80 - 2.73 (m, 1H), 2.67 - 2.51 (m, 2H), 1.79 - 1.67 (m, 2H). |

### Embodiment 2: Synthesis of compound D11

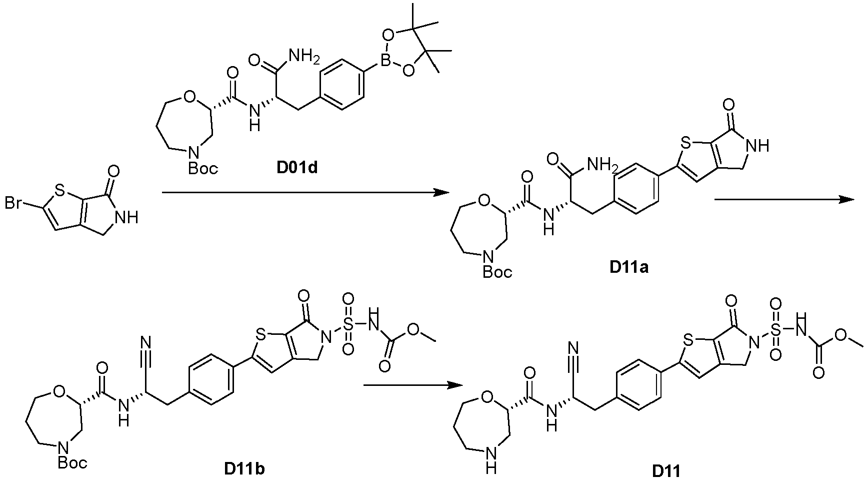

a) Add **D01d** (200 mg, 0.387 mmol), 2-bromo-4,5-dihydro-6H-thieno[2,3-c]pyrrol-6-one (170 mg, 0.78 mmol), PdCl₂dppf (60 mg, 0.08 mmol) and KOAc (114 mg, 1.16 mmol) to dioxane (8 mL)/and H₂O (0.8 mL) under argon at room temperature. Stir overnight at 90 °C. Cool the reacting liquid to room temperature, add EA (50 mL) and saturated saline water (10 mL) Extract with EA (3 × 300 mL), dry with Na₂SO₄, and concentrate to obtain the crude product. Purify to obtain a relatively pure product by column chromatography (eluent EA, EA:MeOH = 20: 1-10: 1, Rf = 0.5 in EA/MeOH 10/1), further purify to obtain the white solid product by preparative HPLC (C18, CH₃CN, 10 mM NH₄HCO₃ aqueous solution) **D11a** (120 mg, yield 58%). MS (ESI): m/z = 551.2 [M+Na]⁺.
   ¹HNMR (400 MHz, CDCl₃): δ 7.56-7.52 (m, 2H), 7.32-7.20 (m, 3H), 6.33-6.28 (m, 1.5H), 5.97 (brs, 0.5H), 5.46-5.35 (m, 1H), 4.69-4.63 (m, 1H), 4.40 (s, 2H), 4.22-4.01 (m, 3H), 3.81-3.76 (m, 0.5H), 3.53-2.99 (m, 5.5H), 2.03-1.82 (m, 2H), 1.47 (s, 9H).
b) At room temperature, add Burgess reagent (325 mg, 1.36 mmol) to a solution of **D11a** (120 mg, 0.227 mmol) in dichloromethane (15 mL) and THF (5 mL), stir for 4 h, dilute with dichloromethane DCM (20 mL), wash with water (10 mL), dry with Na₂SO₄, and concentrate to obtain the crude product. Purify to obtain the white solid product **D11b** (130 mg, 88% yield) by column chromatography (eluent EA to EA:MeOH = 20:1, Rf = 0.1 in EA). MS (ESI): m/z = 548.2 [M-Boc]⁺. ¹HNMR (400 MHz, DMSO-d₆): δ 8.78 (d, J = 8 Hz, 1H), 7.70 (d, J = 8.4 Hz, 2H), 7.62 (s, 1H), 7.38 (d, J = 8 Hz, 2H), 5.05-4.99 (m, 1H), 4.86 (s, 2H), 4.12-4.08 (m, 1H), 3.95-3.76 (m, 2H), 3.57-3.47 (m, 2H), 3.37 (s, 3H), 3.26-2.95 (m, 4H), 1.80-1.69 (m, 2H), 1.39-1.34 (m, 9H).
c) At room temperature, to a solution of **D11b** (50 mg, 0.077 mmol) in MeOH (0.5 mL) and CH₃CN (3 mL), add dropwise 4 N HCl (0.2 mL) in methanol (0.5 mL). After stirring for 2 h, add 4 N HCl (0.2 mL) and continue stirring for 2 h. Then, add Et₂O (40 mL) to the reacting liquid, and precipitate some white sediment. Centrifuge for 2 min, remove the liquid, and purify the residue using preparative HPLC (using NH₄HCO₃ aqueous solution as buffer: A: 10 mM NH₄HCO₃ aqueous solution; B: acetonitrile;) Column: Waters XBridge Peptide BEH C18, 19 × 250 mm, 10 µm, 130 Å) to obtain the white solid product **D11**, white solid (20 mg, yield 47%)MS (ESI): m/z = 548.1 [M+H]⁺.¹H NMR (400 MHz, DMSO-d₆): δ 8.96 (d, J = 8.4 Hz, 1H), 8.2 (brs, 1H), 7.67 (d, J = 8.4 Hz, 2H), 7.56 (s, 1H), 7.36 (d, J = 8.4 Hz, 2H), 5.09-5.03 (m, 1H), 4.82 (s, 2H), 4.37-4.34 (m, 1H), 3.92-3.86 (m, 1H), 3.79-3.73 (m, 1H), 3.40-3.38 (m, 1H), 3.31 (s, 3H), 3.26-3.19 (m, 3H), 3.09-3.03 (m, 1H), 2.82-2.76 (m, 1H), 1.96-1.93 (m, 2H)

### Embodiment 3: Synthesis of compound D15

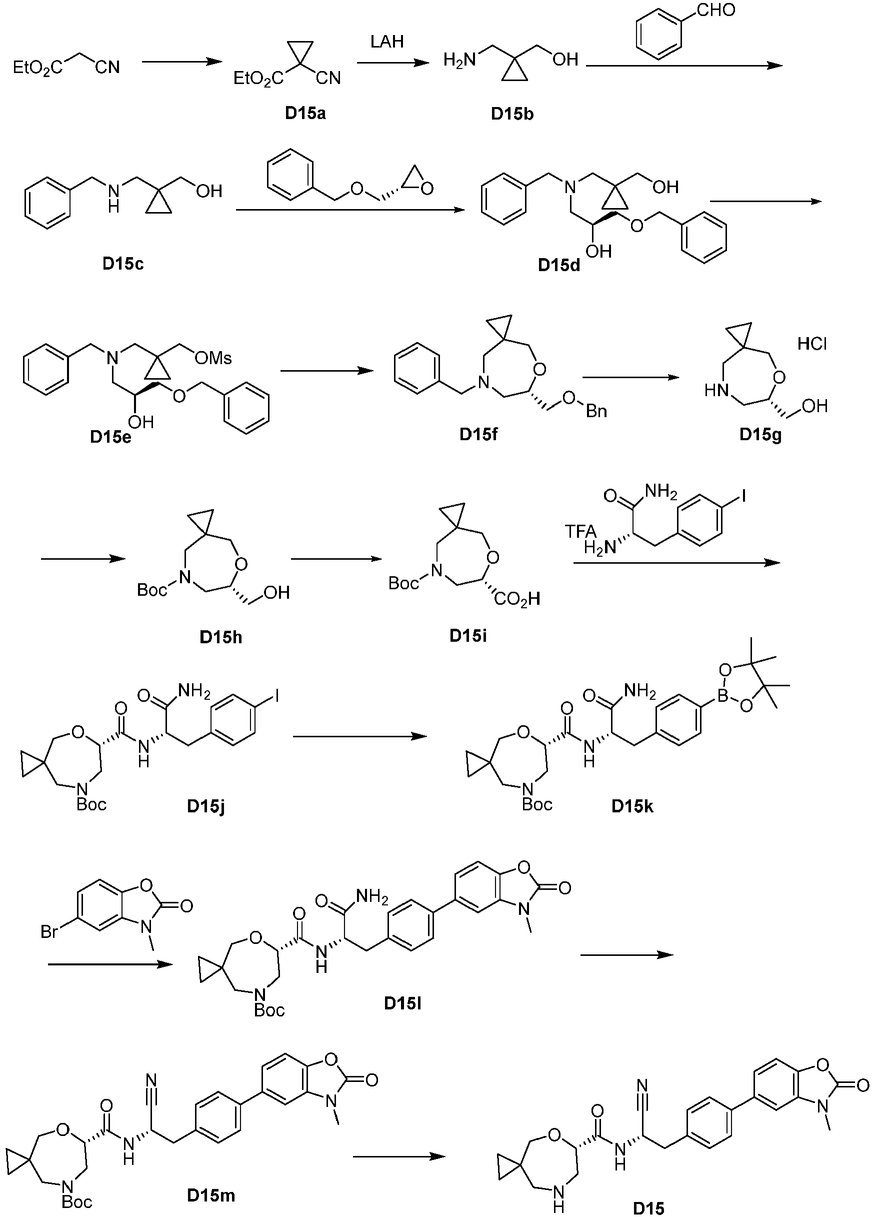

a) At room temperature, sequentially add the compounds ethyl cyanoacetate (20 g, 177 mmol), 1,2-dibromoethane (66.5 g, 354 mmol) and K₂CO₃ (73.3 g, 531 mmol) into acetone (300 mL). Heat and reflux overnight, then cool to room temperature and filter off solids. Concentrate the filtrate to obtain the yellow oily product D15a (22 g, 89%) MS (ESI): m/z = 140.4 [M+H]⁺.
b) At 0 °C, dissolve **D15a** (22 g, 158.3 mmol) in dried THF (150 mL) and slowly add dropwise to THF (250 mL) containing LiAlH4 (24.06 g, 633.1 mmol). Stir the reacting liquid overnight, then cool to 0 °C, dilute with Et₂O (250 mL), carefully quench with water (22.06 mL), 15% *aq.* NaOH (22.06 mL), and chase of water (22.06 mL), filter off the solid, and rinse with MeOH (400 mL). Dry the filtrate with Na₂SO₄, filter off the solids, add HCl (100 mL, 4 mol/L dioxane solution), and concentrate to obtain the brown oily crude product**D15b** (22 g, 80%). ¹H NMR (400 MHz, DMSO-*d*₆): δ 8.09 (s, 3 H); 3.32 (s, 2 H); 2.79-2.75 (m, 2 H); 0.58-0.55 (m, 2 H); 0.45-0.42 (m, 2 H).
c) At room temperature, add **D15b** (5 g, purity: 80%, 29.1 mmol), benzaldehyde (4.63 g, 43.6 mmol) and TEA (2.94 g, 29.1 mmol) in MeOH (100 mL), stir for 2 h, and add NaBH₄ (2.21 g, 58.2 mmol) in batches. After stirring for 2 h, filter off the solid, concentrate the filtrate, and purify to obtain the brown oily product by column chromatography (eluent MeOH/DCM = 1:15, V/V)**D15c** (4.8 g, 87%). MS (ESI): m/z = 192.4 [M+H]⁺. ¹H NMR (400 MHz, CDCl₃): δ 7.37-7.26 (m, 5 H); 3.82 (s, 2 H); 3.58 (s, 2 H); 2.76 (s, 2 H); 0.52-0.50 (m, 2 H); 0..41-0.39 (m, 2 H).
d) At room temperature, add compound **D15c** (9.3 g, 48.7 mmol) and (S)-benzyloxymethyloxirane (8.38 g, 51.1 mmol) to ⁱPrOH (100 mL) and stir at 50 °C for 16 h. Cool to room temperature, concentrate, and purify to obtain the brown oily product by column chromatography (eluent PE/EA = 1: 1 to 1:2, V/V)D15d (14 g, 81%). MS (ESI): m/z =356.2 [M+H]⁺. ¹H NMR (400 MHz, CDCl₃): δ 7.38-7.26 (m, 10 H); 4.52 (s, 2 H); 4.09-4.03 (m, 1 H); 3.86 (d, *J* = 13.2 Hz, 1 H); 3.59-3.36 (m, 5 H); 2.87 (d, *J* = 13.2 Hz, 1 H); 2.77-2.71 (m, 1 H); 2.52-2.48 (m, 1 H); 2.35 (d, *J* = 13.2 Hz, 1 H); 0.60-0.56 (m, 1 H); 0.47-0.40 (m, 2 H); 0.35-0.31 (m, 1 H).
e) In an ice bath, add DIPEA (5.6 g, 43.4 mmol) to **D15d** (11 g, 31 mmol) in dichloromethane (100 mL), and then slowly add MsCl (3.9 g, 34.1 mmol) dropwise within 1 h. After stirring for 1 h, add ice water (100 mL) and extract with dichloromethane (3 × 200 mL). Dry with Na₂SO₄ and concentrate to obtain the brown oily crude product **D15e.** MS (ESI): m/z =434.2 [M+H]⁺.
f) At 0 °C, to a suspension of NaH (3.88 g, 97 mmol, purity: 60% in oil) in THF (300 mL) slowly add dropwise a solution of D15e (14 g, 32.3 mmol) in THF (100 mL). Stir at 50 °C for 16 h, cool to 0 °C, add sat. NaHCOs (200 mL) and EA (3 × 400 mL) to extract. Dry with Na₂SO₄ and concentrate to obtain crude product. Purify to obtain the brown oily product by column chromatography (eluent PE/EA = 6:1 to 5:1, V/V) **D15f** (8 g, 73%). MS (ESI): m/z = 338.2 [M+H]⁺. ¹H NMR (400 MHz, CDCl₃): δ 7.36-7.23 (m, 10 H); 4.57-4.49 (m, 2 H); 4.15-4.08 (m, 1 H); 3.75-3.62 (m, 3 H); 3.56-3.49 (m, 2 H); 3.40-3.36 (m, 1 H); 2.97 (d, *J* = 13.6 Hz, 1 H); 2.70-2.60 (m, 2 H); 2.34 (d, *J* = 12.4 Hz, 1 H); 0.58-0.44 (m, 3 H); 0.35-0.30 (m, 1 H).
g) At room temperature, dissolve **D15f** (4 g, 11.9 mmol) in a hydrogenation unit containing methanol (100 mL), then add Pd/C (800 mg, 10% on C) and AcOH (4 mL). After being replaced by hydrogenation four times, the air in the hydrogenation unit is hydrogenated and shaken for 7 h. Then add HCl (1 mL, 12 mol/L, 12 mmol) and continue hydrogenation for 16 h. Filter off Pd/C and concentrate to obtain the brown oily product **D15g** (2.4 g, 90%). MS (ESI): m/z =158.2 [M+H]⁺.
h) At room temperature, dissolve **D15g** (4.8 g, 24.8 mmol) in dioxane (150 mL) and water (150 mL), then add (Boc)₂O (10.82 g, 49.6 mmol) and NaHCO₃ (10.42 g, 124 mmol). After stirring for 16 h, extract with EA (3 × 500 mL). Dry with Na₂SO₄ and concentrate to obtain crude product. Purify to obtain the colorless oily product by column chromatography (PE/EA = 2:1 to 1:1, V/V)**D15h** (5.7 g, 90%). MS (ESI): m/z =280.0 [M+Na]⁺. ¹H NMR (400 MHz, CDCl₃): δ 3.85-2.92 (m, 9 H); 1.58-1.40 (m, 9 H); 0.66-0.45 (m, 4H).
i) At room temperature, dissolve **D15h** (2 g, 7.78 mmol) in dichloromethane (35 mL), and sequentially add water (7, TEMPO (24 mg, 0.16 mmol), Bu₄NHSO₄ (185 mg, 0.54 mmol). Meanwhile, adjust 15.5 mL of NaClO solution (10-15%) to pH 8-9 using sat. NaHCO₃ (liquid + solids) (approximately 30 mL). Add 1 mL of NaBr (136 mg, 1.32 mmol) aqueous solution to the buffer solution. Cool the obtained solution to 0 °C and slowly add it dropwise to the reacting liquid. After reaction at 0 °C for 1 h, stir overnight. Adjust the pH of the reacting liquid KHSO₄(2N) to 2-3. Extract with dichloromethane, dry with Na₂SO₄, and concentrate to obtain the crude product. Purify to obtain the white solid product **D15i** (1.08 g, 40%) by column chromatography (eluent PE/EA = 2:1 to 1:1, V/V). MS (ESI): m/z =294.2 [M+Na]⁺.
j) At room temperature, add **D15i** (1 g, 3.7 mmol), DIPEA (1.9 g, 14.8 mmol), HOBt (498 mg, 3.7 mmol), and HBTU (1.68 g, 4.4 mmol) to (*S*) -2-amino-3-(4-iodophenyl) propionamide trifluoroacetate (1.79 g, 4.4 mmol) in dichloromethane (40 mL). After stirring for 5 h, add water (50 mL) and extract with dichloromethane (3 × 150 mL). Dry with Na₂SO₄ and concentrate to obtain crude product. Purify by column chromatography (eluent PE/EA = 1: 1 to 1:5, V/V) followed by flash column chromatography to obtain the crude product, (C18, CH₃CN, 10 mM NH₄HCO₃ aqueous solution), a white solid product**D15j** (1 g, 50%). MS (ESI): m/z =566.0 [M+Na]⁺. ¹H NMR (400 MHz, CDCl₃): δ 7.66-7.64 (m, 2 H); 7.19 (d, *J* = 8.4 Hz, 0.5 H); 7.10 (d, *J* = 8.4 Hz, 0.5 H); 7.00 (d, *J* = 8.4 Hz, 2 H); 6.23 (s, 0.5 H); 5.89 (s, 0.5 H); 5.37 (s, 1 H); 4.66-4.59 (m, 1 H); 4.30-4.06 (m, 2 H); 3.88 (d, *J* = 13.6 Hz, 0.5 H); 3.76-3.71 (m, 1 H); 3.51 (d, *J* = 14 Hz, 0.5 H); 3.38-3.34 (m, 0.5 H); 3.30 (d, *J* = 12.8 Hz, 1 H); 3.14-3.00 (m, 3 H); 2.84 (d, *J=* 14 Hz, 0.5 H); 1.49 (s, 5 H); 1.47 (s, 4 H); 0.66-0.47 (m, 4 H).
k) At room temperature, add Pin₂B₂ (936 mg, 3.68 mmol), Pd(dppf)Cl₂ (269 mg, 0.37 mmol), and KOAc (541 mg, 5.52 mmol) to a DMSO (30 mL) solution of **D15j** (1 g, 1.84 mmol). Remove air using reduced pressure in the reaction flask before injecting argon gas. After repeating 3 times, stir at 85 °C and react for 5 h. Cool to room temperature, dilute with EA (500 mL), and wash with saturated saline (5 × 100 mL). Dry with Na₂SO₄ and concentrate to obtain crude product. Purify to obtain the brown solid by column chromatography (eluent PE/EA = 1:2 to 1:6, V/V)**D15k** (900 mg, 90%). MS (ESI): m/z =566.3 [M+Na]⁺. ¹H NMR (400 MHz, CDCl₃): δ 7.73-7.75 (m, 2 H); 7.26(d, *J* = 7.6 Hz, 2 H); 7.22-7.15 (m, 1H); 6.15 (s, 0.5 H); 5.84 (s, 0.5 H); 5.44 (s, 1 H); 4.71-4.61 (m, 1 H); 4.29-4.06 (m, 2 H); 3.86 (d, *J* = 13.6 Hz, 0.5 H); 3.71-3.65 (m, 1 H); 3.49 (d, *J* = 14 Hz, 0.5 H); 3.33-3.27 (m, 1.5 H); 3.15-3.04 (m, 3 H); 2.81 (d, *J* = 13.6 Hz, 0.5 H); 1.48 (s, 5 H); 1.46 (s, 4 H); 1.35 (s, 12 H); 0.65-0.46 (m, 4 H).
l) At room temperature, dissolve **D15k** (200 mg, 0.37 mmol) in dioxane (10 mL), then sequentially add 5-bromo-3-methylbenzo[*d*]oxazole-2(3*H*)-ketone (126 mg, 0.55 mmol), Pd(dppf)Cl₂ (54 mg, 0.07 mmol), KOAc (108 mg, 1.1 mmol), and water (1 mL), and remove air using reduced pressure in the reaction flask before injecting argon gas. Stir the reaction mixture at 85 °C for 6 h. After cooling to room temperature, concentrate, and purify to obtain a relatively pure product by column chromatography (eluent PE/EA = 1: 1 to 1:6 to MeOH/DCM = 1: 10, V/V). Further column purification (C18, CH₃CN, 10 mM NH₄HCO₃ aqueous solution) to obtain the brown solid product **D15l** (150 mg, 72%). MS (ESI): m/z = 587.3 [M+Na]⁺.
m) At room temperature, dissolve **D15l** (140 mg, 0.25 mmol) in dichloromethane (10mL), and then add Burgess reagent (354 mg, 1.49 mmol). After stirring at room temperature for 3 h, concentrate and purify by preparative HPLC (Using NH₄HCO₃ buffer: A: 10 mM NH₄HCO₃ aqueous solution; B: acetonitrile; Column: Waters XBridge Peptide BEH C18, 19 × 250 mm, 10 µm, 130 Å) to obtain the brown solid product **D15m** (80 mg, 59%). MS (ESI): m/z = 569.3 [M+Na]⁺.
n) At room temperature, drop HCl-dioxane (1.172 mmol, 4 mol/L, 0.293 mL) into a MeCN (4.8 mL) solution of **D15m** (80 mg, 0.147 mmol). After stirring at room temperature for 1.5 h, add cold Et₂O (45 mL), centrifuge for separation and remove the liquid phase. Wash the residue twice with Et₂O (15 mL). Purify by preparative HPLC (A: 0.8% NH₄HCO₃ aqueous solution B: CH₃CN; Column: Xbridge BEH peptide C18, 19 mm × 250 mm) to obtain the white solid product D15 (35 mg, 53%). HPLC purity: 99.46%.

| Compound No. | Structural formula | MS (M+H) ⁺ | ¹H NMR |
|---|---|---|---|
| D15 | 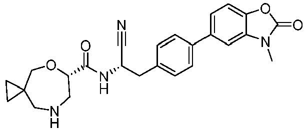 | 447.3 | ¹H NMR (400 MHz, DMSO-*d*₆): δ 8.79-8.61 (m, 1 H); 7.64 (d, *J* = 8.0 Hz, 2 H); 7.58-7.56 (m, 1 H); 7.42-7.37 (m, 4 H); 5.06-4.93 (m, 1 H); 4.15-4.06 (m, 1 H); 3.63-3.43 (m, 2 H); 3.39 (s, 3 H); 3.25-3.15 (m, 2 H); 3.08-3.04 (m, 1 H); 2.73-2.53 (m, 2 H); 2.47-2.42 (m, 1 H); 0.55-0.33 (m, 4 H). |

The following compounds are prepared from analogues of different L-phenylalanine using a similar method:

| **Compound No.** | **Structure of analogues of L-phenylalanine** | **Product structure** | MS (M+H)⁺ |
|---|---|---|---|
| D46 | 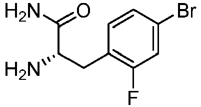 | 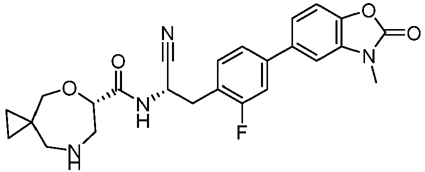 | 465.2 |
| D47 | 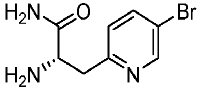 | 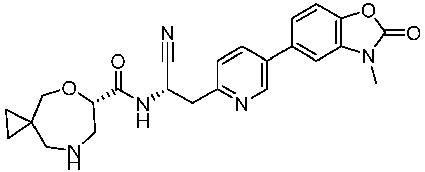 | 448.2 |
| D48 | 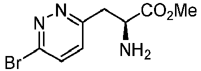 | 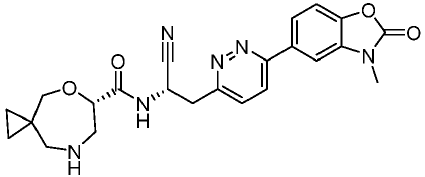 | 449.2 |
| D53 | 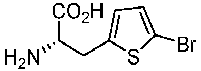 | 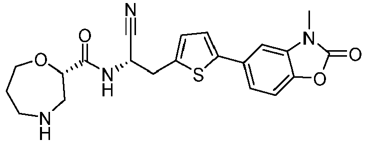 | 426.49 |
| D84 | 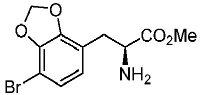 | 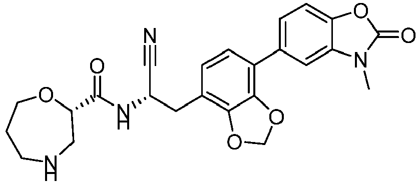 | 426.49 |
| D107 | 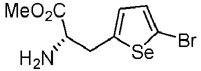 | 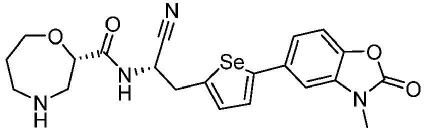 | 426.49 |
| D121 | 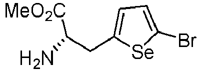 | 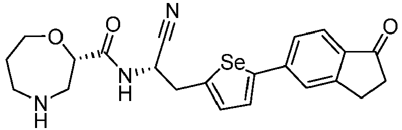 | 458.09 |
| D126 | 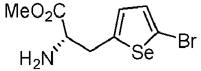 | 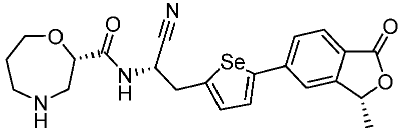 | 474.10 |

The following compounds are prepared from different 6- and 7-membered cyclic amino acids using a similar method:

| **Compound No.** | **Structure of 6-membered and 7-membered cyclic amino acids** | **Product structure** | MS (M+H)⁺ |
|---|---|---|---|
| D49 | 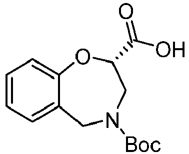 | 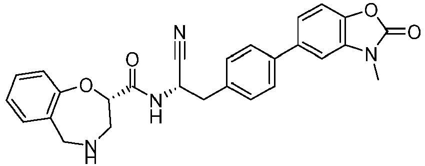 | 469.2 |
| D50 | 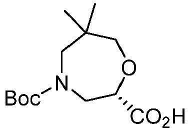 | 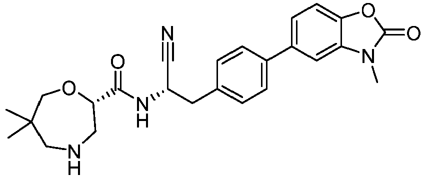 | 449.2 |
| D51 | 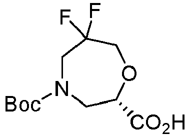 | 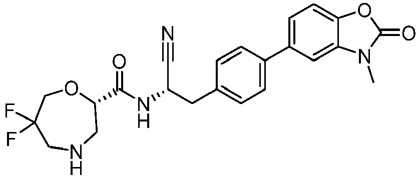 | 457.1 |
| D52 | 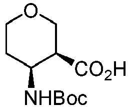 | 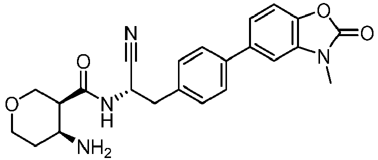 | 421.2 |
| D54 | 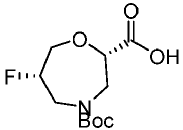 | 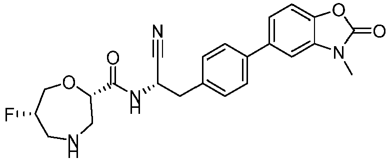 | 438.46 |

### Embodiment 4: DPP1 activity inhibition test on U937 cells

Measure intracellular enzyme activity in 384-well plates with cell culture medium: 1640, 10% FBS, 1*PS. Add 30 µL cell culture medium suspension containing U937 cells to a 384-well plate, so that each well contains 2 × 10⁴ cells; And add 30 nL of AZD7986, vehicle control (100% DMSO), or continuous dilution of the tested compound to the wells through Echo. After incubation at 37 °C for 1 h, add h-Gly-phe-AFC (10 µL) to each well and start reaction. Incubate further at 37 °C for 1 h, then read fluorescence absorption at EXλ 400 nm and EMλ 505 nm. The IC50 values are calculated using Graphpad 8.0 for the above test results, and the results are shown in the table below:

| **Compou nd** | **Average Inhibition% @50 nM** | **Average Inhibition % @5 nM** | **IC50 (nM)** |
|---|---|---|---|
| AZD798 6 | ++ | + | 6 |
| D01 | - | - | / |
| D02 | ++ | + | 5.7 |
| D03 | + | - | / |
| D04 | ++ | + | 5.2 |
| D05 | - | - | / |
| D06 | - | - | / |
| D07 | + | - | / |
| D08 | + | - | / |
| D09 | ++ | + | 10 |
| D10 | + | - | / |
| D11 | - | - | / |
| D12 | - | - | / |
| D13 | - | - | / |
| D14 | + | - | / |
| D15 | ++ | + | 9 |
| D16 | + | - | / |
| D17 | + | - | / |
| D18 | + | - | / |
| D19 | ++ | + | 41 |
| D20 | ++ | + | 35 |
| D21 | ++ | + | 29 |
| D22 | ++ | + | / |
| D23 | - | - | / |
| D24 | ++ | + | / |
| D25 | ++ | + | / |
| D26 | ++ | + | 16 |
| D27 | + | - | / |
| D28 | ++ | + | / |
| D29 | ++ | + | / |
| D30 | + | - | / |
| D31 | + | - | / |
| D32 | + | - | / |
| D33 | + | - | / |
| D34 | + | - | / |
| D35 | + | - | / |
| D36 | + | - | / |
| D37 | + | - | / |
| D38 | + | - | / |
| D39 | + | - | / |
| D40 | + | - | / |
| D41 | + | - | / |
| D42 | ++ | + | 15 |
| D43 | + | - | / |
| D44 | ++ | + | 14 |
| D45 | + | - | / |
| D46 | ++ | + | 3 |
| D47 | ++ | - | / |
| D48 | ++ | + | 12 |
| D49 | + | - | / |
| D50 | / | / | >1000 |
| D51 | / | / | >1000 |
| D52 | + | - | / |
| D53 | / | / | 16 |
| D54 | + | + | / |
| D55 | / | / | 6.4 |
| D56 | + | - | / |
| D57 | ++ | + | 10 |
| D58 | - | - | / |
| D59 | ++ | + | 11 |
| D60 | ++ | + | 4.6 |
| D61 | ++ | + | 5.3 |
| D62 | + | - | / |
| D63 | ++ | + | 6.1 |
| D64 | - | - | / |
| D65 | + | / | / |
| D66 | - | - | / |
| D67 | ++ | + | 32 |
| D68 | - | - | / |
| D69 | - | - | / |
| D70 | ++ | + | 13.2 |
| D71 | - | - | / |
| D72 | - | - | / |
| D73 | ++ | + | 3.8 |
| D74 | ++ | + | 8 |
| D75 | ++ | + | 3.8 |
| D76 | ++ | - | / |
| D77 | ++ | - | / |
| D78 | ++ | + | 2.9 |
| D79 | - | - | / |
| D80 | - | - | / |
| D81 | + | - | / |
| D82 | - | - | / |
| D83 | - | - | / |
| D84 | ++ | + | 24.1 |
| D85 | ++ | + | 3 |
| D86 | ++ | - | / |
| D87 | ++ | - | 12.8 |
| D88 | ++ | - | / |
| D89 | - | - | / |
| D90 | ++ | - | / |
| D91 | ++ | + | 18 |
| D92 | ++ | + | 5.9 |
| D93 | ++ | + | 15.8 |
| D94 | - | - | / |
| D95 | ++ | - | 17 |
| D96 | / | / | 367 |
| D97 | / | / | 108.6 |
| D98 | - | - | / |
| D99 | + | - | / |
| D100 | + | - | / |
| D101 | ++ | + | 7.0 |
| D 102 | ++ | - | 13.1 |
| D103 | ++ | + | 12.2 |
| D104 | - | - | / |
| D105 | - | - | / |
| D106 | / | / | 4.3 |
| D107 | / | / | 6.9 |
| D108 | ++ | - | / |
| D109 | ++ | + | 5.3 |
| D110 | / | / | 4.3 |
| D111 | ++ | - | 32.9 |
| D112 | + | - | / |
| D113 | - | - | / |
| D114 | / | / | 867 |
| D115 | / | / | 29.4 |
| D116 | + | - | / |
| D117 | ++ | + | 6 |
| D118 | / | / | 2.1 |
| D119 | / | / | 6.1 |
| D120 | / | / | 3.42 |
| D121 | / | / | 4.7 |
| D122 | / | / | 17 |
| D123 | / | / | 15 |
| D127 | / | / | 4.6 |

| | | | |
|---|---|---|---|
| "++": 75% ≤ inhibition% < 100%; "+": 30% ≤ inhibition% < 75%; "-": inhibition% < 30%; "/": Not tested. | | | |

### Embodiment 5: Test for inhibition of DPP1 enzyme activity

Experimental materials:
Recombinant human cathepsin, rhCathepsin C/DPP1, purchased from R&D systems
Recombinant human cathepsin, rhCathepsin L, purchased from R&D systems
AZD7986, purchased from MCE
Gly-Arg-AMC (hydrOCHloride), purchased from Cayman chemical
DMSO, purchased from Sigma-Aldrich

Experimental methods:
1) Preparation and handling of compounds: Accurately weigh the compound and dissolve it in 10 mL of DMSO to prepare a 10-mL of the bulk, which is further diluted as required.
2) Screening test: Dilute recombinant human cathepsin rhCathepsin C/DPP1 and recombinant human cathepsin rhCathepsin L with an activating buffer solution, and incubate at 37 °C for 60 min. Add 4 µL of test compound to the white well of the 384-well plate. Add 4 µL of the enzyme mixture after incubation to the white wells. Block the 384-well plate and allow to stand at room temperature for 30 min, and add 8 µL of 2-fold diluted Gly-Arg-AMC (hydrochloride). Block the 384-well plate and allow to stand at room temperature for 2 h. Prepare 4x stop solution and add 4 µL stop solution to white wells. Read fluorescence data on the fluorescence meter Victor Nivo35.
3) Data analysis:
All IC50 values are converted to percent inhibition using Prism Graphpad 8.0. The results are shown in the table below:

| **Compound** | **IC50 (nM)** |
|---|---|
| AZD7986 | 4.2 |
| D02 | 4.1 |
| D04 | 3.9 |
| D09 | 5.4 |
| D15 | 1.4 |
| D19 | 3.5 |
| D20 | 3.6 |
| D21 | 8 |
| D26 | 3.1 |
| D44 | 5.2 |
| D46 | 0.45 |
| D57 | 2.2 |
| D59 | 0.79 |
| D60 | 3.7 |
| D61 | 2.5 |
| D63 | 1.7 |
| D67 | 0.18 |
| D70 | 0.58 |
| D73 | 2.5 |
| D74 | 2.9 |
| D75 | 2.3 |
| D78 | 0.72 |
| D85 | 3.2 |
| D67 | 0.18 |
| D92 | 0.32 |
| D106 | 1.36 |
| D118 | 1.6 |
| D120 | 2.3 |
| D123 | 1.4 |
| D124 | 11.2 |
| D125 | 6.7 |
| D126 | 10.8 |
| D127 | 4.2 |

Embodiments of the present patent are provided only in an illustrative and not limiting manner. One skilled in the art will readily recognize that various non-critical parameters may be altered or modified to produce substantially similar results.

Although the technical solutions of the present invention have been described and enumerated in greater detail, it will be understood that modifications or equivalent alternatives to the above embodiments are obvious to those skilled in the art, and that such modifications or improvements, without departing from the spirit of the present invention, are within the scope of the present invention.

## Claims

1. A compound of formula (I) or a pharmaceutically acceptable salt thereof, **characterized in that**: 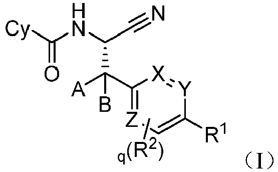 and/or a pharmaceutically acceptable prodrug thereof, and/or solvates, hydrates, metabolites, oxides of nitrogen, racemic mixtures, enantiomers, diastereomers and tautomers thereof or mixtures thereof in any ratio including racemic mixtures, wherein:
Cy is 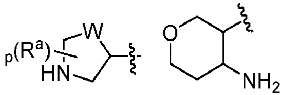
p is 0-6;
W is selected from CH₂-CH₂-O-, -O-, -S-, -SO₂-, -CH₂-, -OCH₂-, -CH₂O-, -CH₂S-, -SCH₂-, -CH₂SO₂-,-SO₂CH₂-, -CH₂-CH₂-, -(CH₂)₃-, -CH₂-CH₂-S-, -CH₂-CH₂-SO₂-, -CH₂-O-CH₂-, -CH₂-S-CH₂-, -CH₂-SO₂-CH₂-;
R^{a} is each independently selected from the group consisting of deuterium, halogen, hydroxy, cyano, mercapto, amino, C₁₋₆ alkyl, C₃₋₆ cycloalkyl, C₃₋₆ heterocycloalkyl, C₃₋₆ cycloalkyloxy, heterocycloalkoxy,-SC₁₋₆ alkyl, C₁₋₆ alkoxy, C₁₋₆ alkylamino, (C₁₋₆ alkyl)₂N-, C₁₋₆ alkyl-C(O)HN-, -C(O)NHC₁₋₆ alkyl, oxo, or
thio, and the said alkyl, cycloalkyl, alkoxy, heterocycloalkyl and heterocycloalkoxy groups may all be optionally substituted with halogen and deuterium; two R^{a} may be attached to the same carbon atom or to different carbon atoms; R^{a} or two R^{a} together forms a C₁₋₄ alkylene group or an ether chain containing 1 to 4 carbon atoms such as -CH₂-O-CH₂-CH₂-CH₂- or -CH₂-O-CH₂-, and the said alkylene group or ether chain may form with the original ring cycloalkyl, cycloalkenyl, heterocycloalkyl, heterocycloalkenyl, bridged,
fused or spiro rings with or without heteroatoms, the said heteroatoms include N, S and O, and the said cycloalkyl, cycloalkenyl, heterocycloalkyl, heterocycloalkenyl, bridged, fused and spiro rings may be optionally substituted with deuterium, hydroxyl, halogen, alkyl and alkoxy groups, and wherein C and S may be optionally substituted with -C=O, -S=O, and -S(O)2-; or two R^{a} and their respective attached carbon atoms form 6-membered aryl, 5-membered heteroaryl, or 6-membered heteroaryl, and form a co-ring with the original ring, the said co-ring may be optionally substituted with hydroxyl, halogen, cyano, alkyl, or
alkoxy; and the said alkyl, cycloalkyl, alkoxy, heterocycloalkyl and heterocycloalkoxy groups may all be optionally substituted with halogen and deuterium;
A and B are each independently selected from the group consisting of hydrogen, deuterium or fluorine, or A and B form cyclopropane with the carbon atoms to which the both are attached;
X, Y and Z are each independently selected from CH, N, S, O or Se, or one of X, Y and Z is a bond in the ring, i.e., the atoms on either side of X, Y, or Z are directly linked, and the linked bond may be a single bond or a double bond;
R² is selected from the group consisting of hydrogen, deuterium, halogen, cyano, C₁₋₆ alkyl, C₁₋₆ alkoxy, C₃₋₆ cycloalkyl, or C₃₋₆ cycloalkoxy, and the said alkyl, alkoxy, cycloalkyl, and cycloalkoxy groups all may be optionally substituted with halogen and deuterium;
q is 0-3;
in the following four cases (four cases are in the relationship of "and/or")
1) Cy is not 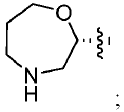
2) X, Y and Z are not all CH;
3) R² is not hydrogen and q is not 0;
4) A and B are not both hydrogen;
R¹ is selected from a cyclic group, specifically, aryl, heteroaryl, cycloalkyl, cycloalkenyl, heterocycloalkyl or heterocycloalkenyl; the said cyclic group may be monocyclic or bicyclic, and may optionally contain one or more heteroatoms of N, O, S and Se, and the C or S in the ring may be optionally substituted with - CO-, -CS-, -SO-, or -SO2-; the said aryl, heteroaryl, cycloalkyl, cycloalkenyl, heterocycloalkyl, and heterocycloalkenyl groups may be optionally substituted with one or more R^{1a};
R^{1a} may be selected from the group consisting of hydrogen, deuterium, halogen, cyano, hydroxy, amino, mercapto, carboxy, sulfone, sulfoxide, oxo, thio, nitro, alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocycloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy,-SOR³, -S(O)2R³, -S(O)(NH)R³, -S(O)(NR⁴)R³, -S(O)2NR³R⁴, -OS(O)2R³, -NR³R⁴, -NR³(CO)R⁴,-NR³(SO₂)R⁴, -NR³R⁴substituted alkyl, -CR³R⁴, -SR³, aryl, 5-6 membered heteroaryl; the said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R₃; two R^{1a} can be attached to the same carbon or nitrogen atom, or to different carbon or nitrogen atoms; two R^{1a} may optionally form with a carbon or nitrogen atom in the original ring a saturated or unsaturated cyclic group, which includes but is not limited to cycloalkyl, cycloalkenyl, heterocycloalkyl, heterocycloalkenyl, bridged ring, spiro ring, fused ring, aryl, and heteroaryl and may be further optionally substituted with one or more R³, and the C and S in the ring may also be optionally substituted with -CO-, -CS-, -SO-, -SO2-;
R³ and R⁴ are each independently selected from the group consisting of hydrogen, deuterium, halogen, cyano, halogen, hydroxy, amino, alkyl, haloalkyl, alkoxy, haloalkoxy, cycloalkyl, cycloalkoxy, heterocycloalkyl, alkylcarbonyl, alkylsulfone, alkyl C(O)NH-, alkyl S(O)2NH-, carboxy, or alkylcarbonyl; wherein amino, hydroxyl, carboxyl, alkyl, cycloalkyl, cycloalkoxy, heterocycloalkoxy, and heterocycloalkyl may be further substituted with alkyl, halogen, cyano, hydroxyl, hydroxyalkyl, and alkoxy; two R³ may be connected to the same atom or to different atoms; or two R³, or R³ and R⁴, may optionally form a 3-10 membered cycloalkyl, heterocycloalkyl, spiro, bridged and fused rings, including but not limited to oxetane, azetidine, morpholine, piperidine, piperazine, aza-oxetane, furan and pyrrolidine, with each of the carbon or nitrogen atoms jointly attached, wherein C and S may be optionally substituted with CO-, -SO-, or -SO2-, and may be optionally substituted with one or more halogen, C1-3 alkyl, halo C₁₋₃ alkyl, C₁₋₃ alkoxy, or C₃₋₈ heterocyclyl;
when Cy is 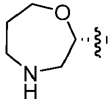 and X, Y and Z are all CH, R² is hydrogen, and A/B is H:
R¹ is selected from the group consisting of pyrimidine, pyrazine, pyridazine, pyrazole, furan, imidazole, thiazole, oxazole, isoxazole, triazole, quinazoline, quinoline, cycloalkyl, cycloalkenyl, heterocycloalkyl, or heterocycloalkenyl, wherein pyrimidine, pyrazine, pyridazine, triazinyl, pyrazole, furan, imidazole, thiazole, triazole, quinazoline, quinoline, cycloalkyl, cycloalkenyl, heterocycloalkyl and heterocycloalkenyl may be substituted with one or more R^{1a} (R^{1a} is defined as described above), or wherein S or 1-2 carbon atoms may be optionally substituted with oxo;
and/or R¹ is selected from: 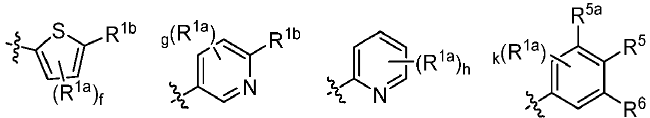
f is 0-2;
g is 0-3;
h is 0-5;
k is 0-2;
R^{1a} is as defined above, R^{1a} on the same ring may be simultaneously selected from the same substituent or from different substituents; two R^{1a} may, optionally with carbon or nitrogen atoms in the original ring, form saturated or unsaturated cyclic groups, which include but are not limited to cycloalkyl, cycloalkenyl, heterocycloalkyl, heterocycloalkenyl, bridged ring, spiro ring, fused ring, aryl and heteroaryl and may be further optionally substituted with one or more R³, and the C and S in the ring may also be optionally substituted with -CO-, -SO- and -SO2-;
R³ and R⁴ are as defined above unless otherwise specified;
when f and g are not 0 and R^{1a} is not hydrogen, R^{1b}=H, R^{1a}; R^{1b} and R^{1a}, or both R^{1a}, may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
when f and g are 0, or f and g are not 0 and R^{1a} is hydrogen, R^{1b} may be selected from the group consisting of hydrogen, deuterium, halogen, hydroxy, amino, mercapto, carboxyl, sulfone, sulfoxide, oxo, thio, nitro, alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, -SOR³, -S(O)2R³, -S(O)(NH)R³,-S(O)(NR⁴)R³, -S(O)2NR³R⁴, -OS(O)2R³, -NR³R⁴, -NR³(CO)R⁴, -NR³(SO₂)R⁴, -NR³R⁴ substituted alkyl,-CR³R⁴, -SR³, aryl, or 5-6 membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R₃; R^{1b} and R^{1a}, or both R^{1a}, may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl, heteroaryl, and may be further optionally substituted with one or more R³, and the C and S in the ring may also be optionally substituted with -CO-, -SO-, and -SO2-;
when k is not 0 and R^{1a} is not hydrogen, R^{5a}, R⁵ and R⁶=R^{1a}, or two of R^{5a}, R⁵, R⁶ and 1-2 R^{1a} may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
when k is 0, or when k is not 0 and R^{1a} is hydrogen:
if R^{5a} is deuterium, bromine, cyano, hydroxy, amino, mercapto, carboxyl, sulfone, sulfoxide, oxo, thio, nitro, cyano, C₂₋₈ alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocycloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, -SOR³, -S(O)2R³, -S(O)(NH)R³,-S(O)(NR⁴)R³, -S(O)2NR³R⁴, -OS(O)2R³, -NR³R⁴, -NR³(CO)R⁴, -NR³(SO₂)R⁴, -NR³R⁴ substituted alkyl,-CR³R⁴, -SR³, aryl, or 5-6 membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R³; R⁵ and R⁶=R^{1a}, or two of R⁵, R⁶, R^{5a} and 1-2 R^{1a} may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
if R^{5a} is chlorine, fluorine, or CH₃, R5 and R6 = R^{1a};
if R^{5a} is H, R⁶ = R^{1a}, and R⁵ may be selected from the group consisting of deuterium, hydroxyl, amino, mercapto, carboxyl, sulfone, sulfoxide, oxo, thio, nitro, cyano, alkyl, 1-2 fluoro substituted alkyl, 1-3 bromo substituted alkyl, 1-3 chloro substituted alkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴ (wherein R³ and R⁴ are not simultaneously H), alkylcarbonyloxy, cycloalkylcarbonyloxy, heterocyclylcarbonyloxy, -SOR³, -S(O)2C₄₋₈ alkyl, -OS(O)2C₄₋₈ cycloalkyl,-OS(O)2C₄₋₈ heterocycloalkyl, -S(O)(NH)R³, -S(O)(NR⁴)R³, S(O)2NR³R⁴ (wherein R³ and R⁴ with the attached N atom may form heteroalkenyl, piperazinyl, 5 to 7-membered azaalkyl containing at least 1 O atom, morpholino, bridged morpholino, or R³ and R⁴ substituted alone do not constitute a ring), -S(O)2NH₂, -OS(O)2R³, -NR³R⁴, -NR³(CO)R⁴, -NR³(SO₂)R⁴, -NR³R⁴ substituted alkyl, -CR ³R⁴ , -SR³, aryl, 5 to 6-membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R³;
or when R^{5a} is H, Cl, F, or CH₃, R¹ is selected from 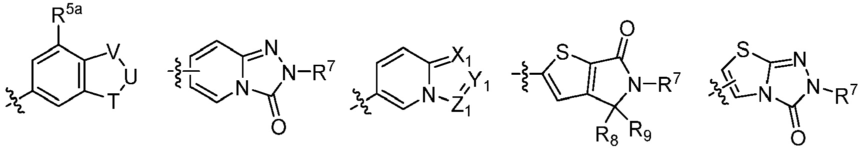 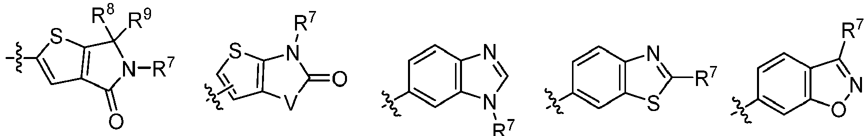
wherein
V is selected from the group consisting of -O-, -S-, -Se-, -CH₂-, -CF₂-, -CO-, -SO-, -SO2-, -N(R⁷)-,-C(R⁸R⁹)-, -O-CH(alkyl)-, -O-CH(cycloalkyl)-, -S-CH(cycloalkyl)-, -CH=C(alkyl)-, -N=C(alkyl)-,-CH=C(cycloalkyl)-, -N=C (cycloalkyl)-;
U is selected from -O-, -S-, -Se-, -CO-, -SO-, -S(O)2, -NR⁷-, -CR⁸R⁹-;
when V is -O-, -S- or -CF2- and U is -CO-, T is-O-, -S-, -Se-, -CO, -SO-, -S(O)2, -CR⁸R⁹-, -NH-, -N(CHF)-, -N(CF₂)-, -N(CH₂-CH(OH)-CH₃)-, -N(CH₂-CH(OCH₃)-CH₃)-, -N(CH₂-CH₂-OCH₃)-, -N(C₄₋₈ alkyl)-,-N(cycloalkyl)-, -N(cycloalkoxy)-, -N(oxetane)-, -N(tetrahydrofuran)-, or -N(tetrahydropyran)-, wherein said -N(C₄₋₈ alkyl)-, -N(cycloalkyl)-, -N(cycloalkoxy)-, -N(oxetane)-, -N(tetrahydrofuran)-, and-N(tetrahydropyran) may be optionally substituted with C₁₋₃ alkoxy, C₁₋₃ alkyl, C₃₋₆ cycloalkyl, NH(C₁₋₃ alkyl),
N(C₁₋₃ alkyl)₂, or 1-3 halogen; otherwise T is -O-, -S-, -Se-, -CO-, -SO-, -SO2-, -C(R⁸R⁹)-, -N(R⁷)-;
X1, Y1 and Z1 may be independently selected from CH and N;
R⁷=R^{1a};
R⁸ and R⁹=R^{1a}, or R⁸ and R⁹ may, optionally with the atoms to which the both are originally attached, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-; and/or a pharmaceutically acceptable salt thereof or a prodrug thereof, and/or solvates, hydrates, metabolites, N-oxides, racemic mixtures, enantiomers, diastereomers and tautomers thereof or mixtures thereof in any ratio including racemic mixtures, wherein:
Cy is 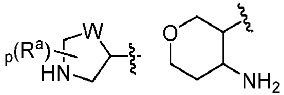
p is 0-6;
W is selected from CH₂-CH₂-O-, -O-, -S-, -SO₂-, -CH₂-, -OCH₂-, -CH₂O-, -CH₂S-, -SCH₂-, -CH₂SO₂-,-SO₂CH₂-, -CH₂-CH₂-, -(CH₂)₃-, -CH₂-CH₂-S-, -CH₂-CH₂-SO₂-, -CH₂-O-CH₂-, -CH₂-S-CH₂-, -CH₂-SO₂-CH₂-;
R^{a} is each independently selected from the group consisting of deuterium, halogen, hydroxy, cyano, mercapto, amino, C₁₋₆ alkyl, C₃₋₆ cycloalkyl, C₃₋₆ heterocycloalkyl, C₃₋₆ cycloalkyloxy, heterocycloalkoxy,-SC₁₋₆ alkyl, C₁₋₆ alkoxy, C₁₋₆ alkylamino, (C₁₋₆ alkyl)₂N-, C₁₋₆ alkyl-C(O)HN-, -C(O)NHC₁₋₆ alkyl, oxo, or
thio, and the said alkyl, cycloalkyl, alkoxy, heterocycloalkyl and heterocycloalkoxy groups may all be optionally substituted with halogen and deuterium; two R^{a} may be attached to the same carbon atom or to different carbon atoms; R^{a} or two R^{a} together forms a C₁₋₄ alkylene group or an ether chain containing 1 to 4 carbon atoms such as -CH₂-O-CH₂-CH₂-CH₂- or -CH₂-O-CH₂-, and the said alkylene group or ether chain may form with the original ring cycloalkyl, cycloalkenyl, heterocycloalkyl, heterocycloalkenyl, bridged,
fused or spiro rings with or without heteroatoms, the said heteroatoms include N, S and O, and the said cycloalkyl, cycloalkenyl, heterocycloalkyl, heterocycloalkenyl, bridged, fused and spiro rings may be optionally substituted with deuterium, hydroxyl, halogen, alkyl and alkoxy groups, and wherein C and S may be optionally substituted with -C=O, -S=O, and -S(O)2-; or two R^{a} and their respective attached carbon atoms form 6-membered aryl, 5-membered heteroaryl, or 6-membered heteroaryl, and form a co-ring with the original ring, the said co-ring may be optionally substituted with hydroxyl, halogen, cyano, alkyl, or
alkoxy; and the said alkyl, cycloalkyl, alkoxy, heterocycloalkyl and heterocycloalkoxy groups may all be optionally substituted with halogen and deuterium;
A and B are each independently selected from the group consisting of hydrogen, deuterium or fluorine, or
A and B form cyclopropane with the carbon atoms to which the both are attached;
X, Y and Z are each independently selected from CH, N, S, O or Se, or one of X, Y and Z is a bond in the ring, i.e., the atoms on either side of X, Y, or Z are directly linked, and the linked bond may be a single bond or a double bond;
R² is selected from the group consisting of hydrogen, deuterium, halogen, cyano, C₁₋₆ alkyl, C₁₋₆ alkoxy,
C₃₋₆ cycloalkyl, or C₃₋₆ cycloalkoxy, and the said alkyl, alkoxy, cycloalkyl, and cycloalkoxy groups all may be optionally substituted with halogen and deuterium;
q is 0-3;
in the following four cases (four cases are in the relationship of "and/or")
1) Cy is not 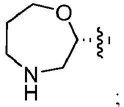
2) X, Y and Z are not all CH;
3) R² is not hydrogen and q is not 0;
4) A and B are not both hydrogen;
R¹ is selected from a cyclic group, specifically, aryl, heteroaryl, cycloalkyl, cycloalkenyl, heterocycloalkyl or heterocycloalkenyl; the said cyclic group may be monocyclic or bicyclic, and may optionally contain one or more heteroatoms of N, O, S and Se, and the C or S in the ring may be optionally substituted with - CO-, -CS-, -CO--SO-, or -SO2-; the said aryl, heteroaryl, cycloalkyl, cycloalkenyl, heterocycloalkyl, and heterocycloalkenyl groups may be optionally substituted with one or more R^{1a};
R^{1a} may be selected from the group consisting of hydrogen, deuterium, halogen, cyano, hydroxy, amino, mercapto, carboxyl, sulfone, sulfoxide, oxo, thio, nitro, alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkyloxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, - SOR³, -S(O)2R³, -S(O)(NH)R³, -S(O)(NR⁴)R³, -S(O)2NR³R⁴, -OS(O)2R³, -NR³R⁴, -NR³(CO)R⁴, - NR³(SO₂)R⁴, -NR³R⁴substituted alkyl, -CR³R⁴, -SR³, aryl, 5-6 membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R₃; two R^{1a} may be attached to the same carbon or nitrogen atom, or to different carbon or nitrogen atoms; two R^{1a} may, optionally with a carbon or nitrogen atom in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl, and heteroaryl, and may be further optionally substituted with one or more R³, and the C and S in the ring may also be optionally substituted with -CO-, -CS-, -SO-, or -SO2-;
R³ and R⁴ are each independently selected from the group consisting of hydrogen, deuterium, halogen, cyano, halogen, hydroxy, amino, alkyl, haloalkyl, alkoxy, haloalkoxy, cycloalkyl, cycloalkoxy, heterocycloalkyl, alkylcarbonyl, alkylsulfone, alkyl C(O)NH-, alkyl S(O)2NH-, carboxy, or alkylcarbonyl; wherein amino, hydroxyl, carboxyl, alkyl, cycloalkyl, cycloalkoxy, heterocycloalkoxy, and heterocycloalkyl may be further substituted with alkyl, halogen, cyano, hydroxyl, hydroxyalkyl, and alkoxy; two R³ may be connected to the same atom or to different atoms; or two R³, or R³ and R⁴, may optionally form a 3-10 membered cycloalkyl, heterocycloalkyl, spiro, bridged and fused rings, including but not limited to oxetane, azetidine, morpholine, piperidine, piperazine, aza-oxetane, furan and pyrrolidine, with each of the carbon or nitrogen atoms jointly attached, wherein C and S may be optionally substituted with CO-, -SO-, or -SO2-, and may be optionally substituted with one or more halogen, C1-3 alkyl, halo C₁₋₃ alkyl, C₁₋₃ alkoxy, or C₃₋₈ heterocyclyl;
when Cy is 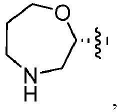 X, Y and Z are CH, R² is hydrogen, and A/B is H:
R¹ is selected from the group consisting of pyrimidine, pyrazine, pyridazine, pyrazole, furan, imidazole, thiazole, oxazole, isoxazole, triazole, quinazoline, quinoline, cycloalkyl, cycloalkenyl, heterocycloalkyl, or heterocycloalkenyl, wherein pyrimidine, pyrazine, pyridazine, triazinyl, pyrazole, furan, imidazole, thiazole, triazole, quinazoline, quinoline, cycloalkyl, cycloalkenyl, heterocycloalkyl and heterocycloalkenyl may be substituted with one or more R^{1a} (R^{1a} is defined as described above), or wherein S or 1-2 carbon atoms may be optionally substituted with oxo;
and/or R¹ is selected from: 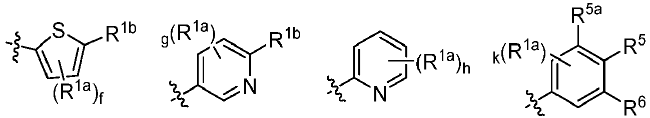
f is 0-2;
g is 0-3;
h is 0-5;
k is 0-2;
R^{1a} is as defined above, R^{1a} on the same ring may be simultaneously selected from the same substituent or from different substituents; two R^{1a} may, optionally with carbon or nitrogen atoms in the original ring, form saturated or unsaturated cyclic groups, which include but are not limited to cycloalkyl, cycloalkenyl, heterocycloalkyl, heterocycloalkenyl, bridged ring, spiro ring, fused ring, aryl and heteroaryl and may be further optionally substituted with one or more R³, and the C and S in the ring may also be optionally substituted with -CO-, -SO- and -SO2-;
R³ and R⁴ are as defined above unless otherwise specified;
when f and g are not 0 and R^{1a} is not hydrogen, R^{1b}=H, R^{1a}; R^{1b} and R^{1a}, or both R^{1a}, may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
when f and g are 0, or f and g are not 0 and R^{1a} is hydrogen, R^{1b} may be selected from the group consisting of hydrogen, deuterium, halogen, hydroxy, amino, mercapto, carboxyl, sulfone, sulfoxide, oxo, thio, nitro, alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, -SOR³, -S(O)2R³, -S(O)(NH)R³, - S(O)(NR⁴)R³, -S(O)2NR³R⁴, -OS(O)2R3, -NR³R⁴, -NR³(CO)R⁴, -NR³(SO₂)R⁴, -NR³R⁴ substituted alkyl, - CR³R⁴, -SR³, aryl, or 5-6 membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R₃; R^{1b} and R^{1a}, or both R^{1a}, may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl, heteroaryl, and may be further optionally substituted with one or more R³, and the C and S in the ring may also be optionally substituted with -CO-, -SO-, and -SO2-;
when k is not 0 and R^{1a} is not hydrogen, R^{5a}, R⁵ and R⁶=R^{1a}, or two of R^{5a}, R⁵, R⁶ and 1-2 R^{1a} may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
when k is 0, or when k is not 0 and R^{1a} is hydrogen:
if R^{5a} is deuterium, bromine, cyano, hydroxy, amino, mercapto, carboxyl, sulfone, sulfoxide, oxo, thio, nitro, cyano, C₂₋₈ alkyl, haloalkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocycloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴, alkylcarbonyloxy, cycloalkylcarbonyloxy, heterocyclylcarbonyloxy, , -SOR³, -S(O)2R³, -S(O)(NH)R³, - S(O)(NR⁴)R³, -S(O)2NR³R⁴, -OS(O)2R3, -NR³R⁴, -NR³(CO)R⁴, -NR³(SO₂)R⁴, -NR³R⁴substituted alkyl, - CR³R⁴, -SR³, aryl, or 5-6 membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R³; R⁵ and R⁶=R^{1a}, or two of R⁵, R⁶, R^{5a} and 1-2 R^{1a} may, optionally with carbon or nitrogen atoms in the original ring, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-;
if R^{5a} is chlorine, fluorine, or CH₃, R5 and R6 = R^{1a};
if R^{5a} is H, R⁶ = R^{1a}, and R⁵ may be selected from the group consisting of deuterium, hydroxyl, amino, mercapto, carboxyl, sulfone, sulfoxide, oxo, thio, nitro, cyano, alkyl, 1-2 fluoro substituted alkyl, 1-3 bromo substituted alkyl, 1-3 chloro substituted alkyl, saturated cycloalkyl, unsaturated cycloalkyl, saturated heterocyclyl, unsaturated heterocyclyl, aralkyl, heteroaralkyl, alkenyl, cycloalkenyl, heterocycloalkenyl, alkynyl, alkoxy, haloalkoxy, cycloalkoxy, heterocyclyloxy, aryloxy, heteroaryloxy, benzyloxy, alkylcarbonyl, alkoxycarbonyl, cycloalkylcarbonyl, cycloalkoxycarbonyl, aryloxycarbonyl, heteroaryloxycarbonyl, -CONR³R⁴ (wherein R³ and R⁴ are not simultaneously H), alkylcarbonyloxy, cycloalkylcarbonyloxy, heterocyclylcarbonyloxy, -SOR³, -S(O)2C₄₋₈ alkyl, -OS(O)2C₄₋₈ cycloalkyl, - OS(O)2C₄₋₈ heterocycloalkyl, -S(O)(NH)R³, -S(O)(NR⁴)R³, S(O)2NR³R⁴ (wherein R³ and R⁴ with the attached N atom may form heteroalkenyl, piperazinyl, 5 to 7-membered azaalkyl containing at least 1 O atom, morpholino, bridged morpholino, or R³ and R⁴ substituted alone do not constitute a ring), -S(O)2NH₂, -OS(O)2R³, -NR³R⁴, -NR³(CO)R⁴, -NR³(SO₂)R⁴, -NR³R⁴ substituted alkyl, -CR ³R⁴ , -SR³, aryl, 5 to 6-membered heteroaryl; wherein said alkyl, alkoxy, alkenyl, cycloalkenyl, heterocycloalkenyl, alkynyl, cycloalkyl, cycloalkoxy, heterocycloalkyl, heterocycloalkoxy, aryl, aralkyl, aralkoxy, heteroaryl, heteroaralkyl, and heteroaralkoxy may be optionally substituted with one or more R³;
or when R^{5a} is H, Cl, F, or CH₃, R¹ is selected from 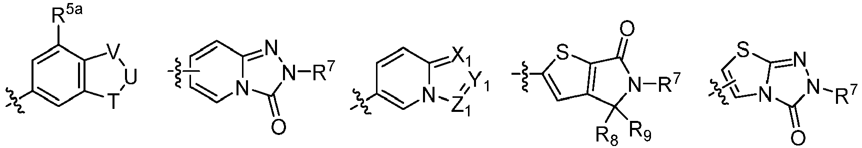 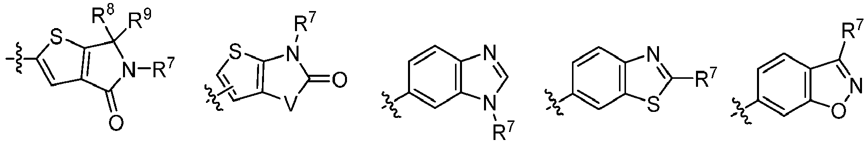
wherein
V is selected from the group consisting of -O-, -S-, -Se-, -CH₂-, -CF₂-, -CO-, -SO-, -SO2-, -N(R⁷)-, - C(R⁸R⁹)-, -O-CH(alkyl)-, -O-CH(cycloalkyl)-, -S-CH(cycloalkyl)-, -CH=C(alkyl)-, -N=C(alkyl)-, - CH=C(cycloalkyl)-, -N=C (cycloalkyl)-;
U is selected from the group consisting of -O-, -S-, -Se-, -CO-, -SO-, -S(O)2,-NR⁷-, or -CR⁸R⁹-;
when V is -O-, -S- or -CF2- and U is -CO-, T is-O-, -S-, -Se-, -CO, -SO-, -S(O)2, -CR⁸R⁹-, -NH-, - N(CHF)-, -N(CF₂)-, -N(CH₂-CH(OH)-CH₃)-, -N(CH₂-CH(OCH₃)-CH₃)-, -N(CH₂-CH₂-OCH₃)-, -N(C₄₋₈ alkyl)-, -N(cycloalkyl)-, -N(cycloalkoxy)-, -N(oxetane)-, -N(tetrahydrofuran)-, or -N(tetrahydropyran)-, wherein said -N(C₄₋₈ alkyl)-, -N(cycloalkyl)-, -N(cycloalkoxy)-, -N(oxetane)-, -N(tetrahydrofuran)-, and - N(tetrahydropyran) may be optionally substituted with C₁₋₃ alkoxy, C₁₋₃ alkyl, C₃₋₆ cycloalkyl, NH(C₁₋₃ alkyl), N(C₁₋₃ alkyl)₂, or 1-3 halogen; otherwise T is -O-, -S-, -Se-, -CO-, -SO-, -SO2-, -C(R⁸R⁹)-, -N(R⁷)-;
X1, Y1 and Z1 may be independently selected from CH and N;
R⁷=R^{1a};
R⁸, R⁹=R^{1a}, or R⁸ and R⁹ may, optionally with the atoms to which the both are originally attached, form a saturated or unsaturated cyclic group, which includes but is not limited to cycloalkyl, cycloalkenyl, heterocycloalkyl, heterocycloalkenyl, bridged ring, spiro ring, fused ring, aryl and heteroaryl, and may be further optionally substituted with one or more R³, and the C and S in the ring may also be optionally substituted with -CO-, -SO- and -SO2-.

2. The compound of formula (I) or a pharmaceutically acceptable salt thereof according to claim 1, **characterized in that**: said Cy is selected from 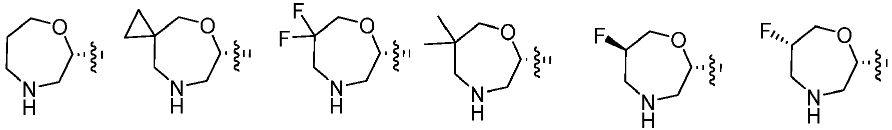 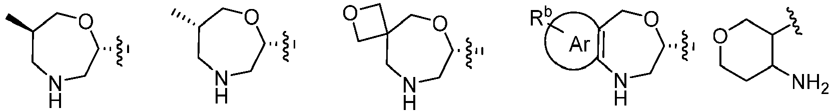
Ar is pyridine and benzene ring;
R^{b} is chlorine, fluorine, methyl, ethyl, propyl, isopropyl, cyclopropyl, trifluoromethyl, or trifluoromethoxy.

3. The compound of formula (I) or a pharmaceutically acceptable salt thereof according to claim 1, **characterized in that**: said A and B are both selected from H, or from F.

4. The compound of formula (I) or a pharmaceutically acceptable salt thereof according to claim 1, **characterized in that**: said X and Y are each independently selected from CH and N, and said Z is selected from CH.

5. The compound of formula (I) or a pharmaceutically acceptable salt thereof according to claim 1, **characterized in that**: said R² is selected from hydrogen, fluorine and methyl, and said q is selected from 1.

6. The compound of formula (I) or a pharmaceutically acceptable salt thereof according to claim 1, **characterized in that**: said R¹ is selected from 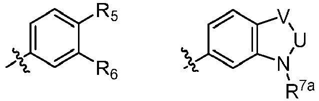 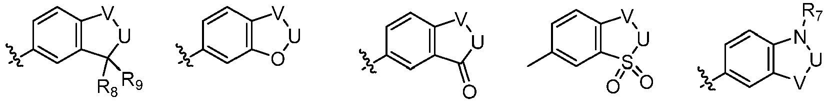 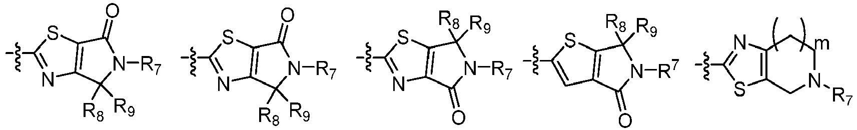 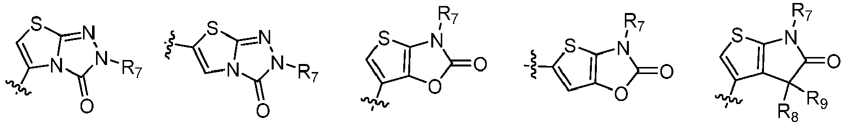 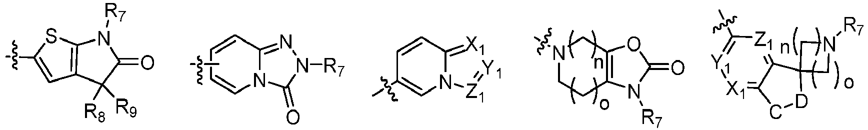 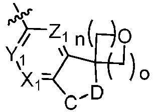 wherein
X₁, Y₁ and Z₁ are independently selected from CH and N;
m, n and o are 0-3;
U may be selected from -C(=O)-, -S(O)2-, -O-, -NR⁷-, -CR⁸R⁹-;
V may be selected from -C(=O)-, -S(O)2-, -O-, -S-, -Se-, -NR⁷-, -CR⁸R⁹-;
R7 is selected from the group consisting of hydrogen, CH₃OCH₂CH₂-, oxetanyl, azetidine, tetrahydrofuranyl, tetrahydropyran, pyrrolidine, piperazine, morpholine, piperidine, -C₁₋₃ alkyl, and C₃₋₆ cycloalkyl, wherein said C₁₋₃ alkyl, C3-6 cycloalkyl, azetidine, tetrahydrofuranyl, tetrahydropyran, pyrrolidine, piperazine, morpholine or piperidine is optionally substituted with 1, 2 or 3 fluorines, and/or is optionally substituted with one substituent selected from the group consisting of: C₁₋₃ alkyl, hydroxy, -OC₁₋₃ alkyl,-N (C₁₋₃ alkyl)₂, or cyclopropyl; the same substituent or different substituent may be selected for R⁷ at different positions in the same structure;
R⁸ and R⁹ are hydrogen, fluorine, -C₁₋₆ alkyl, or -C₁₋₆ haloalkyl, or R⁸ and R⁹ together with the nitrogen atom or carbon atom to which they are attached form C₃₋₆ cycloalkyl, oxetane, azetidine, pyrrolidine, piperidine ring, piperazine ring, morpholine ring, tetrahydrofuranyl, or tetrahydropyranyl, and said cyclopropane, oxetane, azetidine, pyrrolidine, piperidine ring, piperazine ring, morpholine ring, tetrahydrofuranyl or tetrahydropyranyl may be optionally substituted with C₁₋₃ alkyl, cyclopropane, oxetane, azetidine, cyclopropyloxy;
C and D are independently selected from -NR⁷C(O)-, -C(O)NR⁷-, -CH₂-CH₂-, -C(O)-O-, -O-C(O)-, -CH₂-O-, -O-CH₂-, -CH₂-NR⁷-, -NR⁷-CH₂-, -CH₂-; or one of C and D is a bond (single bond or double bond in the ring, with the atoms at both ends directly connected);
when Cy is 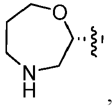 and R² is H, and X, Y and Z are all selected from CH, and -O-, -S-, or -CF₂- if V is present in the structure:
R5 is selected from the group consisting of -S(O)2C₁₋₃ alkyl, -CONH₂, -SO₂NHC₁₋₃ alkyl, or -SO₂NR³R⁴ (if R³ and R⁴, with the N atom to which they are attached, form a heterocyclic group, the said heterocyclic group is selected from the group consisting of piperazinyl or morpholinyl, which may be optionally substituted with C₁₋₃ alkyl or cyclopropyl);
R⁶ is selected from the group consisting of hydrogen, fluorine, chlorine, bromine, or methyl;
R^{1a} is selected from H, -CHF, -CF₂, -CH₂-CH (OH)-CH₃, -CH₂-CH(OCH₃)-CH₃, -C₄₋₈ alkyl, -cycloalkyl, - cycloalkoxy, -CH₂-CH₂-OCH₃, -oxetane, -tetrahydrofuran, -tetrahydropyran, pyrrolidine, piperazine, morpholine, or piperidine; wherein said C₄₋₈ alkyl, tetrahydrofuran, pyrrolidine, piperazine, morpholine, or piperidine is optionally substituted with 1, 2 or 3 fluorine and/or is optionally substituted with one substituent selected from the group consisting of: hydroxy,-OC₁₋₃ alkyl,-N (C₁₋₃ alkyl) 2, or cyclopropyl; otherwise: R⁵ is selected from cyano, -SO₂C₁₋₃ alkyl, -CONH₂, or -SO₂NR³R⁴, wherein R³ and R⁴ are hydrogen and -C₁₋₆ alkyl, or R³ and R⁴ together with the nitrogen atom to which they are attached form azetidine, oxetane, pyrrolidine, piperidine ring, piperazine ring, or morpholine ring, which may be optionally substituted with C1-3 alkyl or cyclopropyl;
R⁶ is selected from the group consisting of hydrogen, fluorine, chlorine, bromine, or methyl;
R^{7a}=R⁷.

7. The compound of formula (I) or a pharmaceutically acceptable salt thereof according to claim 1, **characterized in that**: said R¹ is selected from 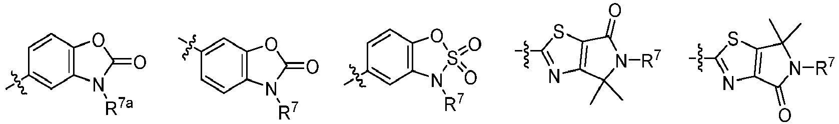 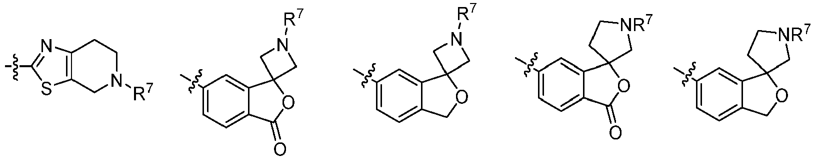 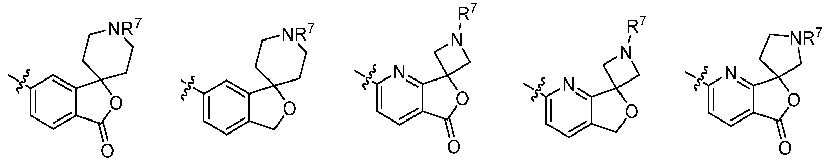 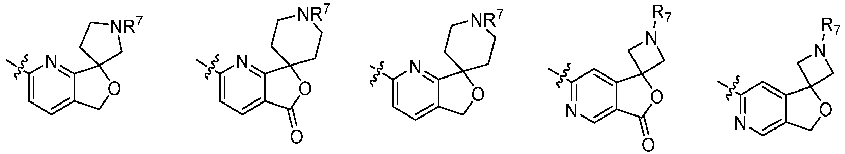 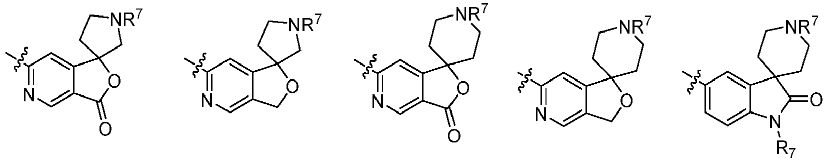 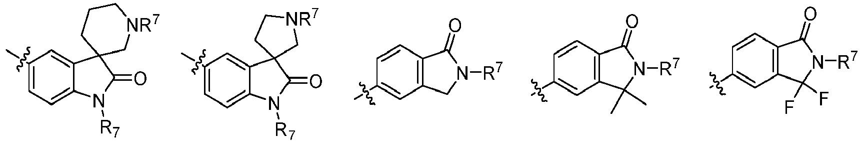 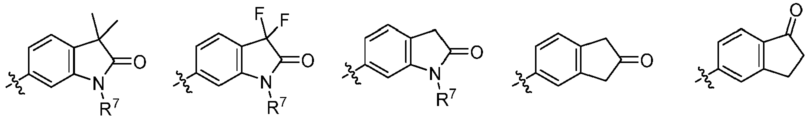 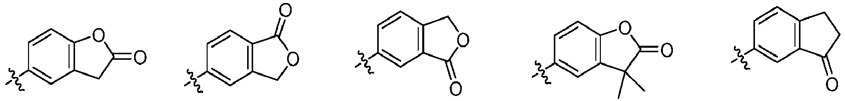 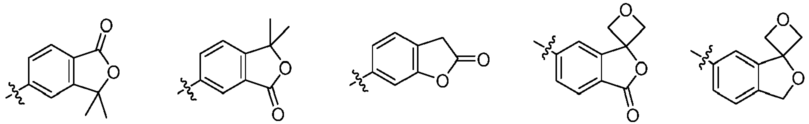
R⁷ is selected from the group consisting of hydrogen, -CF₂, -CHF, CH₃OCH₂CH₂-, oxetanyl, tetrahydrofuranyl, tetrahydropyran, -C₁₋₃ alkyl, or -C₃₋₆ cycloalkoxy; wherein said C₁₋₃ alkyl, oxetanyl, tetrahydrofuranyl, and tetrahydropyran may optionally be substituted with 1, 2 or 3 fluorines, and or optionally substituted with one substituent selected from the group consisting of: hydroxy, -OC₁₋₃ alkyl, - N(C₁₋₃ alkyl)₂, or cyclopropyl; the same substituent or different substituent may be selected for R⁷ at different positions in the same structure;
when Cy is 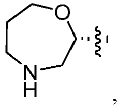 and R² is H, and X, Y and Z are all selected from CH, and A and B are both H:
R^{7a} is selected from H, -CHF, -CF₂, -CH₂-CH(OH)-CH₃, -CH₂-CH(OCH₃)-CH₃, -C₄₋₈ alkyl, -C₃₋₆ cycloalkyl, -C₃₋₆ cycloalkoxy, -CH₂-CH₂-OCH₃, -oxetane, -tetrahydrofuran, or -tetrahydropyran, and said oxetane, tetrahydrofuran, and tetrahydropyran may be optionally substituted with C₁₋₃alkyl, or cyclopropyl; otherwise R^{7a}=R⁷.

8. A compound of the following formula (I) or a pharmaceutically acceptable salt thereof:

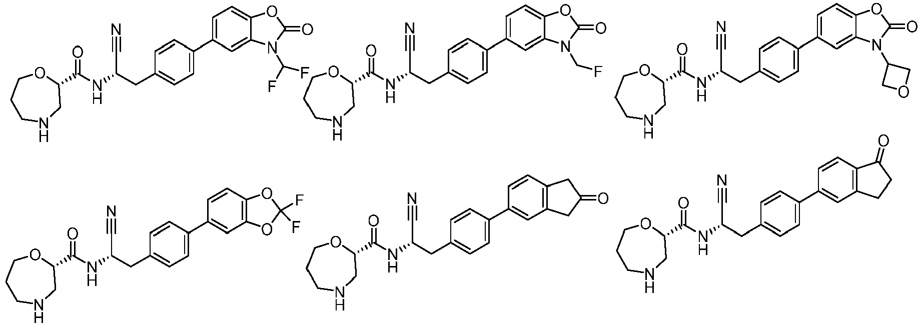

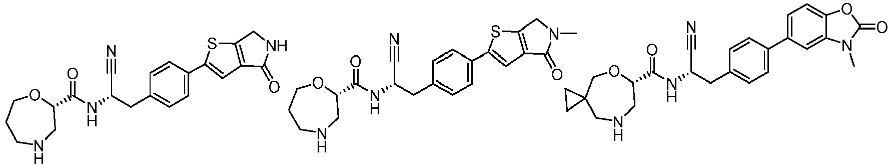

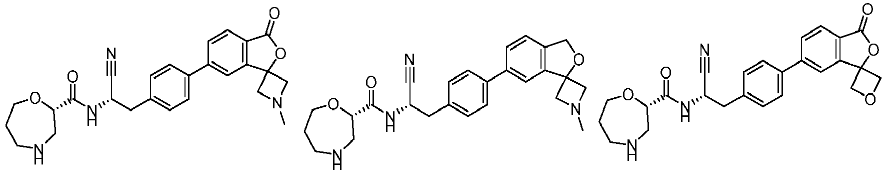

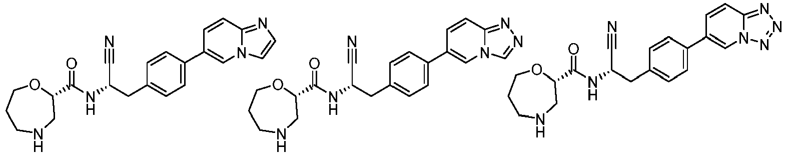

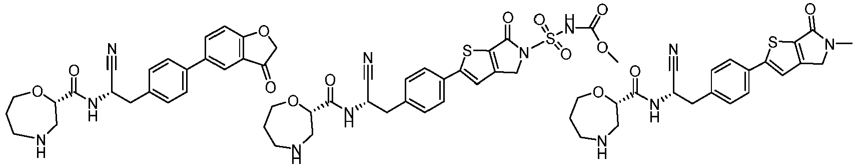

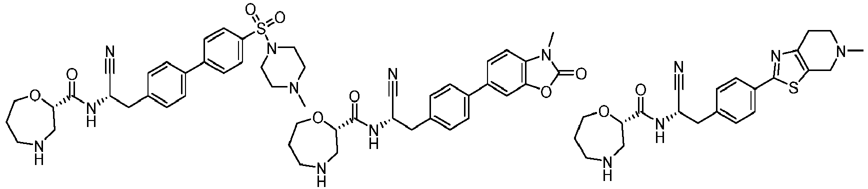

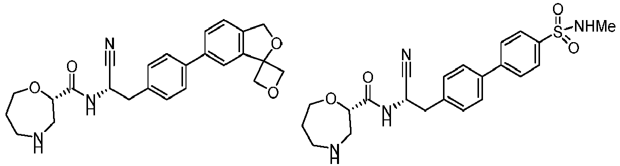

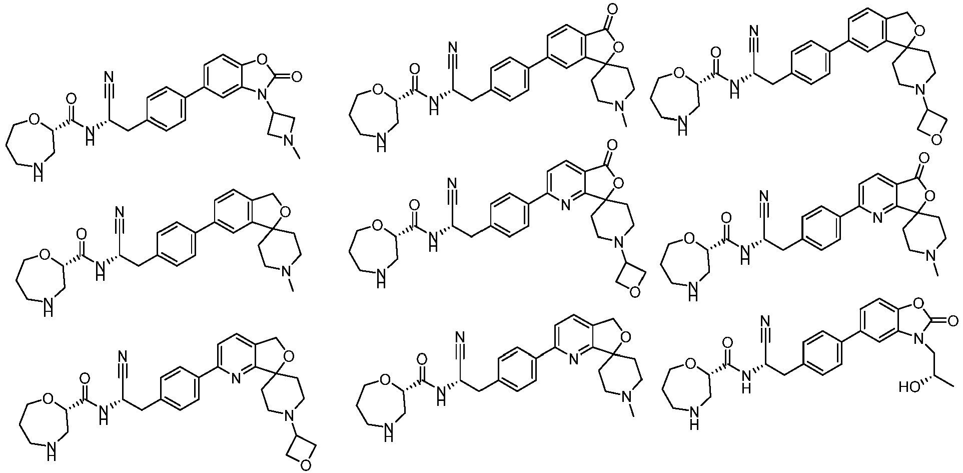

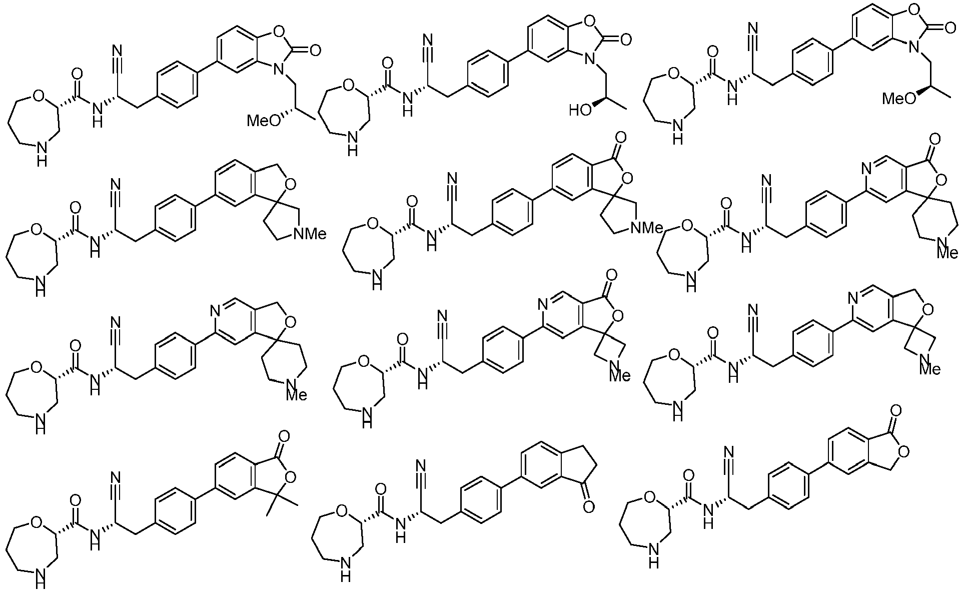

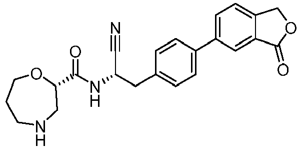

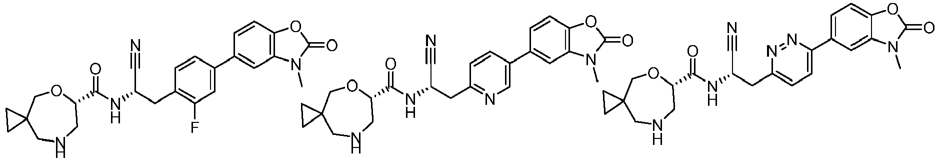

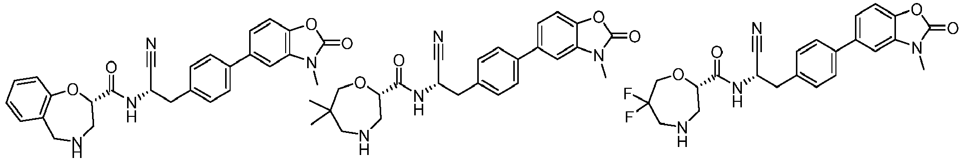

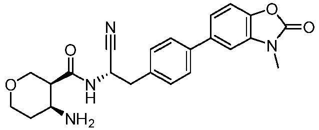

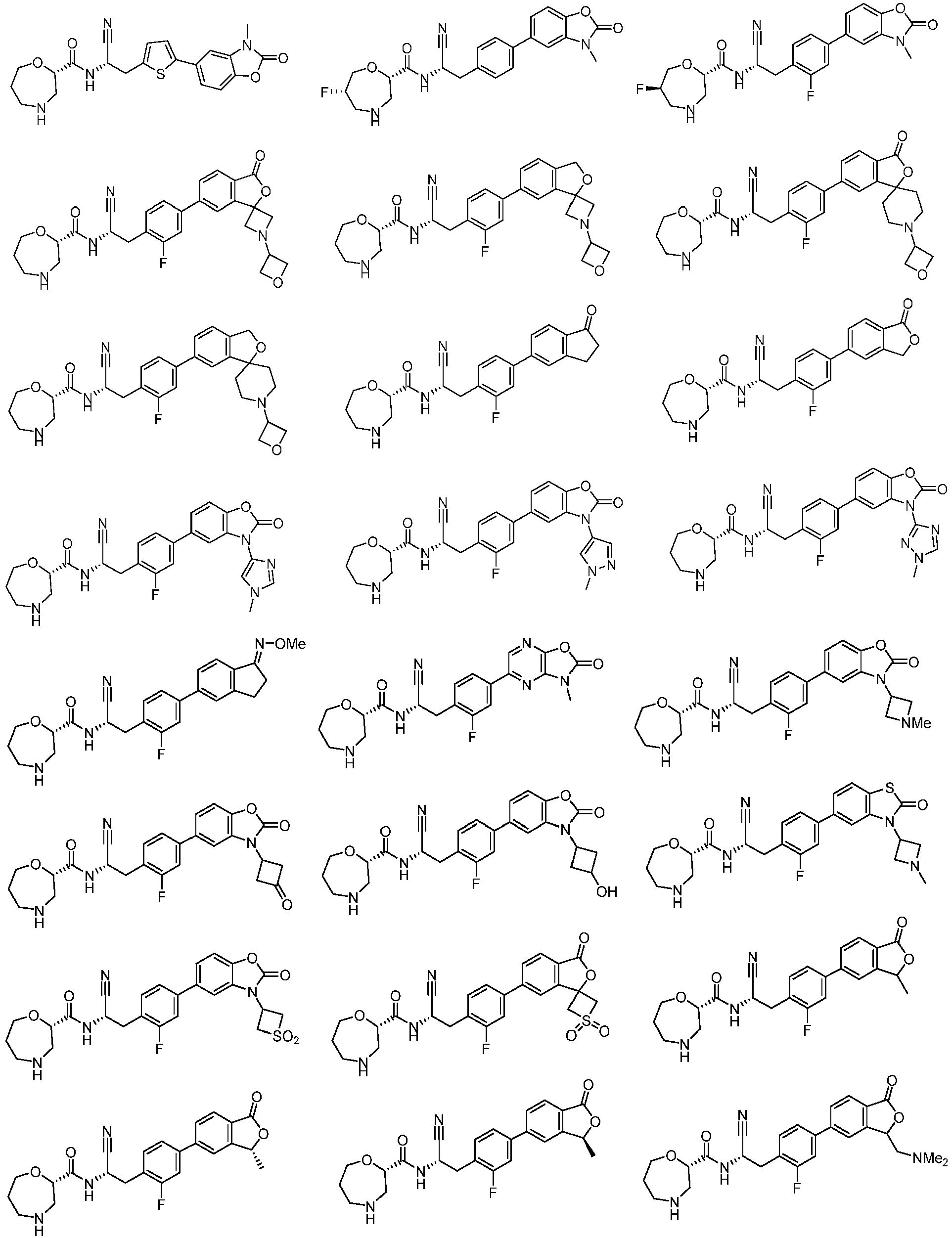

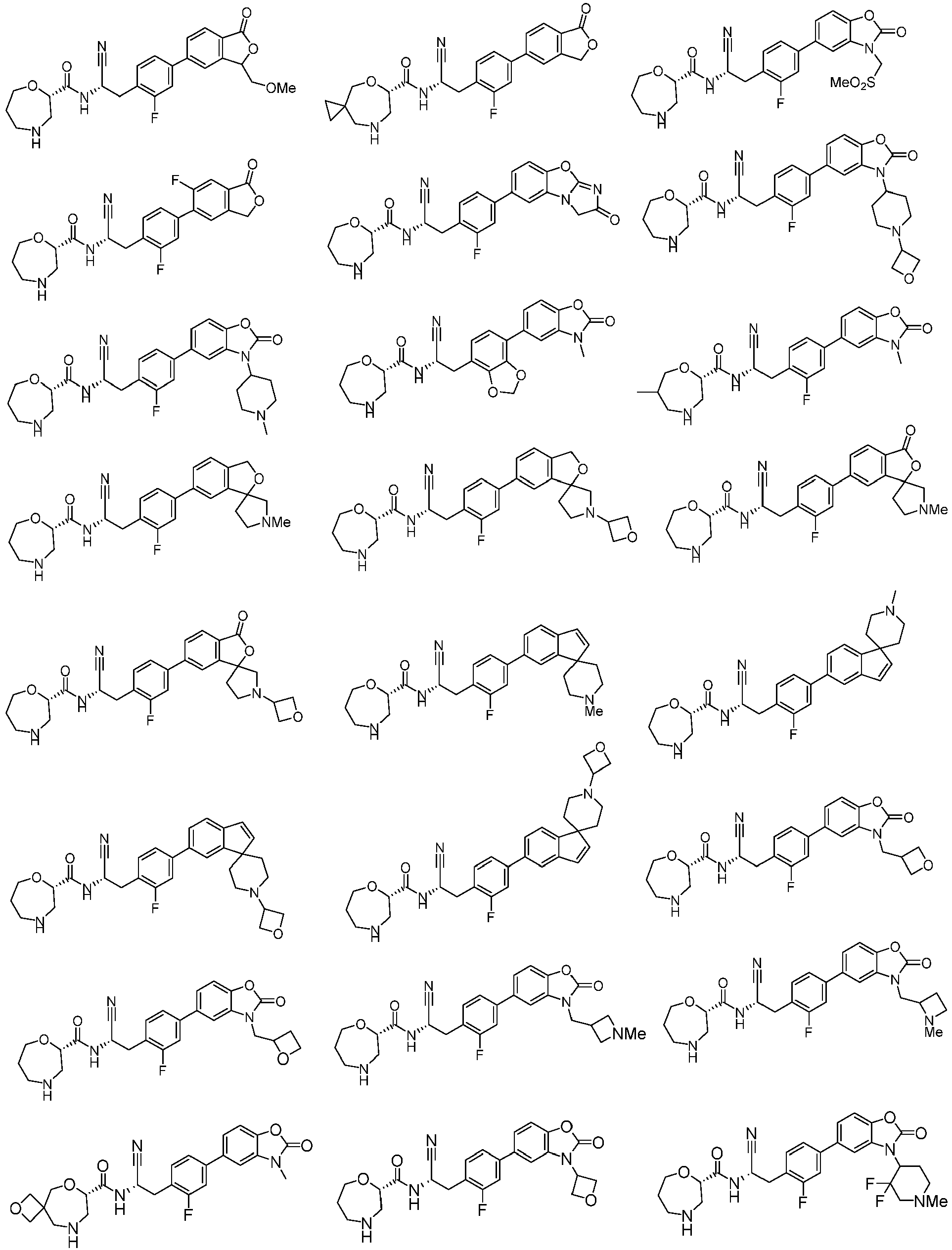

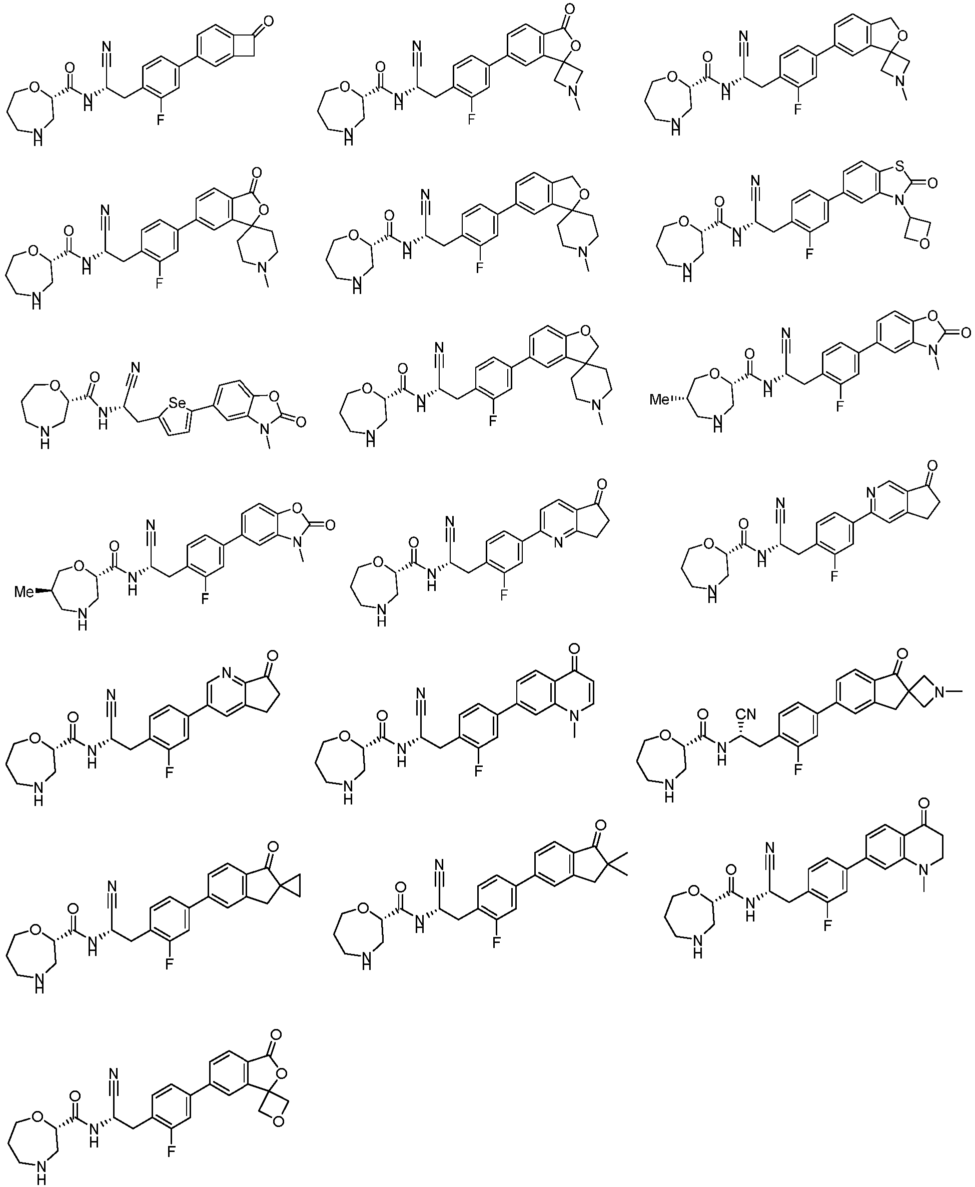

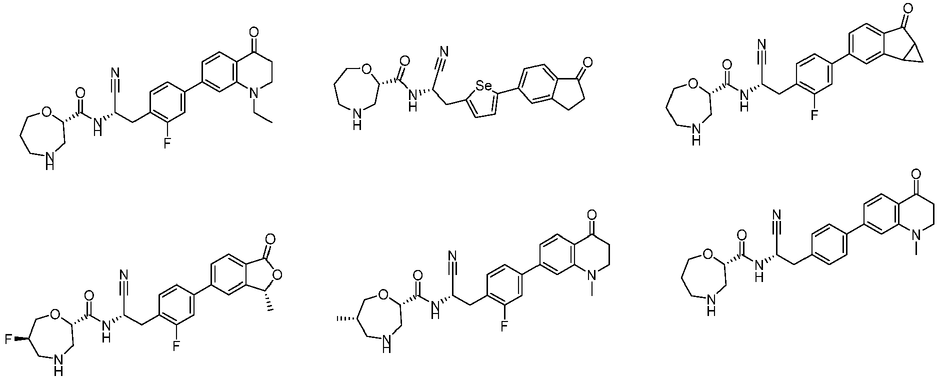

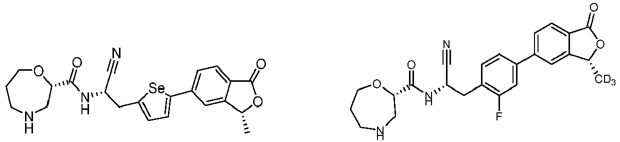

9. Use of the compound of formula (I) or a pharmaceutically acceptable salt thereof according to any one of claims 1-8 in the preparation of drugs for the treatment and prevention of disorders of cathepsin C and its downstream serine proteases NE, PR3, CaTG, and NSP4.

10. Use of the compound of formula (I) or a pharmaceutically acceptable salt thereof according to any one of claims 1-8 in the preparation of drugs for treating patients suffering from respiratory disease, metabolic disease, cardio-cerebrovascular disease, autoimmune disease, cancer, infectious disease and other inflammatory related diseases including asthma, chronic obstructive pulmonary disease, pulmonary fibrosis, pulmonary hypertension, pulmonary arterial hypertension, non-cystic fibrosis, cystic fibrosis, bronchiectasis, bronchitis, pneumonia, emphysema, acute lung injury (ALI), and acute respiratory distress syndrome (ARDS), sepsis, allergic disorders, immune inflammatory bowel disease, rheumatoid arthritis, glomerulonephritis, eosinophilic disorders, neutrophil disorders, ANCA-associated inflammation, ANCA-associated necrotizing crescentic glomerulonephritis, acute brain trauma, acute myocarditis, acute kidney injury, a-1-antitrypsin deficiency (AATD) and associated inflammation, liver fibrosis, fatty liver and hepatic steatosis, obesity, insulin resistance, diabetes, pathogenic microbial infections, infectious gastrointestinal inflammatory diseases, lung cancer and/or radiation injury syndrome or at risk for said diseases.

11. A pharmaceutical composition, comprising the compound of formula (I) or a pharmaceutically acceptable salt thereof according to any one of claims 1-8, and at least one pharmaceutical carrier or excipient.

12. The pharmaceutical composition according to claim 11, **characterized in that** it comprises one or more compounds of formula (I) according to claims 1-8, and pharmaceutically active compounds selected from the group consisting of other compounds including, but not limited to: b mimetics, anticholinergic drugs, corticosteroids, PDE4 inhibitors, LTD4 antagonists, EGFR inhibitors, CRTH2 inhibitors, 5-LO inhibitors, histamine receptor antagonists, CCR9 antagonists and SYK inhibitors, NE inhibitors, MMP9 inhibitors, MMP12 inhibitors and combinations of two or three active substances.

13. The pharmaceutical composition according to any one of claims 11-12, **characterized in that**: the pharmaceutical composition further comprises use in combination with a small molecule compound and/or a macromolecule antibody to treat cancer, inflammation, bone marrow-related diseases and autoimmune diseases; the small molecule compounds and/or large molecule antibodies include, but are not limited to, glucocorticoids, adrenergic agonists, cholinergic receptor antagonists, theophylline drugs, antioxidants, elastase inhibitors, metalloproteinase inhibitors, PDE4 inhibitors, LTD4 antagonists, EGFR inhibitors, CRTH2 inhibitors, 5-LO inhibitors, histamine receptor antagonists, CCR9 antagonists and SYK inhibitors, chemokine receptor inhibitors, interleukin antibodies such as IL-6 antibodies, IL-23 antibodies, targeted anti-thymic stromal lymphopoietin (TSLP) antibodies such as tezepelumab, complement inhibitors.

14. The pharmaceutical composition according to claim 13, **characterized in that**: the said composition is used in a medicament for treating and preventing diseases caused by cathepsin C and its downstream serine proteinases NE, PR3, CaTG, NSP4, said diseases being selected from respiratory diseases, metabolic diseases, cardiovascular and cerebrovascular diseases, autoimmune diseases, cancer, infectious diseases or inflammatory infectious diseases.

15. Solvates, racemic mixtures, enantiomers, diastereoisomers, tautomers or mixtures in any ratio including racemic mixtures of the compound of formula (I) according to any one of claims 1-8, **characterized in that** each of the substituents R¹, R², Cy, A, B, X, Y, Z and q:
